# EUROPÄISCHE PATENTSCHRIFT

(11) **EP 2 683 703 B1**
(45) Veröffentlichungstag und Bekanntmachung des Hinweises auf die Patenterteilung: **27.05.2015**
(21) Anmeldenummer: 12708011.7
(22) Anmeldetag: 07.03.2012
(51) Int. Cl.: C07D 291/06, A61K 31/54, C07D 419/04, C07D 419/10, A61P 3/10

(54) **NEUE SUBSTITUIERTE PHENYL-OXATHIAZINDERIVATE, VERFAHREN ZU DEREN HERSTELLUNG, DIESE VERBINDUNGEN ENTHALTENDE ARZNEIMITTEL UND DEREN VERWENDUNG**
NOVEL SUBSTITUTED PHENYL-OXATHIAZINE DERIVATIVES, METHOD FOR PRODUCING THEM, DRUGS CONTAINING SAID COMPOUNDS AND THE USE THEREOF
NOUVEAUX DÉRIVÉS PHÉNYL-OXATHIAZINE SUBSTITUÉS, PROCÉDÉ POUR LEUR PRÉPARATION, AGENT PHARMACEUTIQUE CONTENANT CES COMPOSÉS ET LEUR UTILISATION

(30) Priorität: 08.03.2011 EP 11305245
(43) Veröffentlichungstag der Anmeldung: 15.01.2014
(73) Patentinhaber: SANOFI, 75008 Paris (FR)
(72) Erfinder: BOEHME, Thomas, 65926 Frankfurt am Main (DE); ENGEL, Christian, 65926 Frankfurt am Main (DE); GUESSREGEN, Stefan, 65926 Frankfurt am Main (DE); HAACK, Torsten, 65926 Frankfurt am Main (DE); RITTER, Kurt, 65926 Frankfurt am Main (DE); TSCHANK, Georg, 65926 Frankfurt am Main (DE)
(74) Vertreter: Essler, Frank
(86) Internationale Anmeldenummer: PCT/EP2012/053940
(87) Internationale Veröffentlichungsnummer: WO 2012/120057

(56) Entgegenhaltungen:
- WO-A1-02/11722
- WO-A2-2008/073956
- SUZUE SEIGO ET AL: "Studies on Hypoglycemic Agents. IV. Synthesis of 1,4,3-Benzoxathiazine-4,4-dioxides", CHEMICAL AND PHARMACEUTICAL BULLETIN, PHARMACEUTICAL SOCIETY OF JAPAN, TOKYO, JP, Bd. 16, Nr. 5, 25. Mai 1968 (1968-05-25), Seiten 806-813, XP009109825, ISSN: 0009-2363
- IWAKAWA TSUNEO ET AL: "Cycloaddition in Synthesis of Sulfonamide Derivatives. IV. One-Pot Synthesis of 3-Dimethylamino-4,1,2-benzoxathiazine 1,1-Dioxides, 3-Methoxy-4-methyl-1,2,4-benzothiadiazine 1,1-Dioxide and 3-Dimethylamino-1,4,2-benzodithiazine 1,1-Dioxides", CHEMICAL AND PHARMACEUTICAL BULLETIN, PHARMACEUTICAL SOCIETY OF JAPAN, TOKYO, JP, Bd. 39, Nr. 8, 25. August 1991 (1991-08-25), Seiten 1939-1943, XP009109826, ISSN: 0009-2363
- EDWARD M. BURGESS, W. MICHAEL WILLIAMS: "The fragmentation of substituted 1,4,3,5-oxathiazine dioxides to N-sulfonylamides", J. ORG. CHEM., Bd. 38, Nr. 6, 1973, Seiten 1249-1250, XP002651414,

## Beschreibung

Die Erfindung betrifft substituierte Phenyl-Oxathiazinderivate und deren physiologisch verträgliche Salze.

In Suzue Seigo et al., Chem. Phar. Bull. 16(5), 806-813 (1986) sind Oxathiazinverbindungen zur Behandlung von Hypoglykämie beschrieben.

Der Erfindung lag die Aufgabe zugrunde, Verbindungen zur Verfügung zu stellen, die eine therapeutisch verwertbare Wirkung enthalten. Insbesondere bestand die Aufgabe darin, neue Verbindungen zu finden, die zur Behandlung von Diabetes, Hyperglykämie, Insulin Resistenz, Adipositas oder Lipidstoffwechselstörungen geeignet sind.

Die Erfindung betrifft daher Verbindungen der Formel I 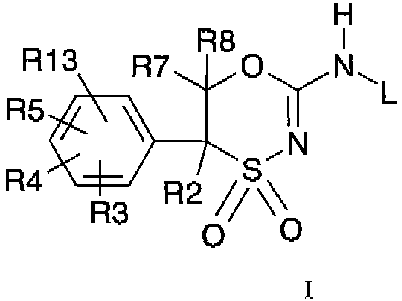 worin bedeuten
- L: R1, -CH(R10)(R11);
- R10, R11: unabhängig voneinander H, F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅, (C₁-C₆)-Alkylen-(R6), (C₃-C₈)-Cycloalkylen-(R6), (C₁-C₆)-Alkylen-(C₃-C₈)-Cycloalkylen-(R₆), (C₆-C₁₀)-Aryl, (C₁-C₆)-Alkylen-(C₆-C₁₀)-Aryl; wobei der Arylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)- Alkyl)₂, SF₅;
- R1: 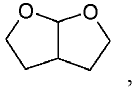 (C₆-C₁₀)-Aryl, (C₃-C₈)-Cycloalkyl, wobei der 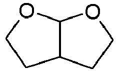 -rest, Arylrest oder Cycloalkylrest ein bis 3-fach substituiert sein kann mit F, Cl, Br, I, OH, CF₃, CHF₂, CH₂F, NO₂, CN, OCF₃, CHF₂, O-(C₁-C₆-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅,
- R2: H, F, (C₁-C₃)-Alkyl wobei der Alkylrest ein bis 3-fach mit Fluor substituiert sein kann;
- R3, R4, R5, R13: unabhängig voneinander H, F, Cl, Br, I, OH, CF₃, CHF₂, CH₂F, NO₂, CN, OCF₃, CHF₂, -(C₁-C₆)-Alkylen-(R9), O-(C₁-C₆)-Alkylen-(R9), tertiär-Butyl, iso-Propylen-(R9), (C=O)-(C₁-C₆)-Alkylen-(R9), (C₁-C₆)-Alkylen-(R9), NH₂, NH(C₁-C₆)-Alkylen-(R9), N((C₁-C₆)-Alkylen-R9)₂, (C₁-C₆)-Alkylen-NH₂, (C₁-C₆)-Alkylen-NH(C₁-C₆)-Alkylen-(R9), (C₁-C₆)-Alkylen-N((C₁-C₆)-Alkylen-R9)₂-O-(C₁-C₆)-Alkylen-NH₂, -O-(C₁-C₆)-Alkylen-NH(C₁-C₆)-Alkylen-(R9), -O-(C₁-C₆)-Alkylen-N((C₁-C₆)-Alkylen-R9)₂,-NH-(C₁-C₆)-Alkylen-NH₂, -NH-(C₁-C₆)-Alkylen-NH(C₁-C₆)-Alkylen-(R9), -NH-(C₁-C₆)-Alkylen-N((C₁-C₆)-Alkylen-R9)₂, SO₂-CH₃, SO₂-NH₂, SO₂-NH(C₁-C₆)-Alkyl, SO₂-N((C₁-C₆)-Alkyl)₂, COOH, COO-(C₁-C₆-Alkyl, CONH₂, CONH(C₁-C₆)-Alkyl, CON((C₁-C₆)-Alkyl)₂, SF₅; (C₆-C₁₀)-Aryl, -(C₁-C₆)-Alkylen-(C₆-C₁₀)-Aryl,, wobei der Arylrest ein bis 3-fach substituiert sein kann mit F, Cl, Br, I, OH, CF₃, CHF₂, CH₂F, NO₂, CN, OCF₃, CHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅, (C₆-C₁₀)-Aryl, (C₃-C₈)-Cycloalkyl, 4 bis12-gliediiger Heterocyclus, wobei der (C₆-C₁₀)-Arylrest, (C₃-C₈)-Cycloalkylrest, 4 bis 12-gliedriger Heterocyclusrest ein bis 3-fach substituiert sein kann mit F, Cl, Br, I, OH, CF₃, CHF, CH₂F, NO₂, CN, OCF₃, CHF₂, O-(C₁-C₆-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅; 4 bis 12-gliedriger Heterocyclus, -(C₁-C₆)-Alkylen-4 bis12-gliedriger Heterocyclus, wobei der Heterocyclylrest ein bis 3-fach substituiert sein kann mit F, Cl, Br, I, OH, CF₃, CHF₂, CH₂F, NO₂, CN, OCF₃, CHF₂, O-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-(C₁-C₆)-Alkylen-(R9), SO₂-C₂H₂F₃, SO₂-CH₃, SO₂-NH₂, SO₂-NH(C₁-C₆)-Alkylen-(R9), SO₂-N((C₁-C₆)-Alkyl)₂, COOH, COO-(C₁-C₆)-Alkylen-(R9), CONH₂, CONH(C₁-C₆)-Alkylen-(R9), CON((C₁-C₆)-Alkyl)₂, SF₅,(C₆-C₁₀)-Aryl, (C₃-C₈)-Cycloalkyl, 4 bis 12-gliedriger Heterocyclus; wobei der (C₆-C₁₀)-Arylrest, (C₃-C₈)-Cycloalkylrest, 4 bis 12-gliedriger Heterocyclusrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
- R6: H, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, O-(CO)-NH₂, SF₅;
- R7, R8: unabhängig voneinander H, (C₁-C₃)-Alkyl, wobei der Alkylrest ein bis 3-fach mit Fluor substituiert sein kann, oder R7 und R8 bilden gemeinsam mit dem Kohlenstoffatom an das sie gebunden sind einen 3-8 gliedrigen Carbocyclus oder Heterocyclus;
- R9: H, OH, OCH₃, OCF₃, CHF₂, CF₃;
sowie deren pharmazeutisch verträgliche Salze.

Bevorzugt sind Verbindungen der Formel I, worin bedeuten
- L: R1, -CH(R10)(R11);
- R10: F, Cl, Br, I, OH, CF₃, CHF₂, CH₂F, NO₂, CN, OCF₃, CHF₂, O-(C₁-C₆)-Alkyl, C₁-C₆)-Alkyl, NH₂, NH(C₁-C₆)-Alkyl, N((C₁-C₆)-Alkyl)₂, COOH, COO-(C₁-C₆-Alkyl, CONH₂, CONH(C₁-C₆)-Alkyl, CON((C₁-C₆)-Alkyl)₂, SF₅, (C₁-C₆)-Alkylen-(R6), (C₃-C₈)-Cycloalkylen-(R6), (C₁-C₆)-Alkylen-(C₁-C₈)-Cycloalkylen-(R6), (C₆-C₁₀)-Aryl, (C₁-C₆)-Alkylen-((C₆-C₁₀)-Aryl, wobei der Arylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)- Alkyl)₂, SF₅;
- R11: H, F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅, (C₁-C₆)-Alkylen-(R6), (C₃-C₈)-Cycloalkylen-(R6), (C₁-C₆)-Alkylen-(C₃-C₈)-Cycloalkylen-(R6), (C₆-C₁₀)-Aryl, (C₁-C₆)-Alkylen-((C₆-C₁₀)-Aryl; wobei der Arylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)- Alkyl)₂, SF₅;
- R1: (C₃-C₈)-Cycloalkyl, wobei der Cycloalkylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
- R2: H, F, (C₁-C₃)-Alkyl wobei der Alkylrest ein bis 3-fach mit Fluor substituiert sein kann;
- R3, R4, R5, R13: unabhängig voneinander H, F, Cl, Br, I, OH, CF₃, CHF₂, CH₂F, NO₂, CN, OCF₃, OCHF₂, O-(C₁-C₆)-Alkylen-(R9), -(C₁-C₆)-Alkylen-(R9), (C=O)-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅; (C₆-C₁₀)-Aryl, -(C₁-C₆)-Alkylen-(C₆-C₁₀)-Aryl, wobei der Arylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅; 4 bis 12-gliedriger Heterocyclus, -(C₁-C₆)-Alkylen-4 bis 12-gliedriger Heterocyclus, wobei der Heterocyclylrest ein bis 3-fach substituiert sein kann mit F, Cl, Br, I, OH, CF₃, CHF₂, CH₂F, NO₂, CN, OCF₃, OCHF₂, O-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-(C₁-C₆)-Alkylen-(R9), SO₂-C₂H₂F₃, SO₂-CH₃, SO₂-NH₂, SO₂-NH(C₁-C₆)-Alkylen-(R9), SO₂-N((C₁-C₆)-Alkyl)₂, COOH, COO-(C₁-C₆)-Alkylen-(R9), CONH₂, CONH(C₁-C₆)-Alkylen-(R9), CON((C₁-C₆)-Alkyl)₂, SF₅, (C₆-C₁₀)-Aryl, (C₃-C₈)-Cycloalkyl, 4-12-gliedriger Heterocyclus;
- R6: H, OH, O-(CO)-NH₂, SO₂NH₂;
- R7, R8: unabhängig voneinander H, (C₁-C₃)-Alkyl, wobei der Alkylrest ein bis 3-fach mit Fluor substituiert sein kann;
- R9: H, OH, OCH₃, OCF₃, CHF₂, CF₃;
sowie deren pharmazeutisch verträgliche Salze.

Weiter bevorzugt sind Verbindungen der Formel I, worin bedeuten
- L: R1, -CH(R10)(R11);
- R10: (C₆-C₁₀)-Aryl, (C₁-C₆)-Alkylen-((C₆-C₁₀)-Aryl, wobei der Arylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)- Alkyl)₂, SF₅;
- R11: F, Cl, Br, I, OH, CF₃, CHF₂, CH₂F, NO₂, CN, OCF₃, OCHF₂, O-(C₁-C₆)-Alkyl, C₁-C₆)-Alkyl, NH₂, NH(C₁-C₆)-Alkyl, N((C₁-C₆)-Alkyl)₂, COOH, COO-(C₁-C₆-Alkyl, CONH₂, CONH(C₁-C₆)-Alkyl, CON((C₁-C₆)-Alkyl)₂, SF₅" (C₁-C₆)-Alkylen-(R6);
- R1: (C₃-C₈)-Cycloalkyl, wobei der Cycloalkylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
- R2: H, (C₁-C₃)-Alkyl;
- R3, R4, R5, R13: unabhängig voneinander H, F, Cl, Br, I, OH, CF₃, CHF₂, CH₂F, NO₂, CN, OCF₃, OCHF₂, O-(C₁-C₆)-Alkylen-(R9), -(C₁-C₆)-Alkylen-(R9), (C=O)-(C1-C6)-Alkylen-(R9), (C₁-C₆)-Alkylen-(R9), NH₂, NH(C₁-C₆)-Alkylen-(R9), N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅; (C₆-C₁₀)-Aryl, -C₁-C₆)-Alkylen-(C₆-C₁₀)-Aryl,, wobei der Arylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅; 4 bis 12-gliedriger Heterocyclus, -(C₁-C₆)-Alkylen-4 bis12-gliedriger Heterocyclus, wobei der Heterocyclylrest ein bis 3-fach substituiert sein kann mit F, Cl, Br, I, OH, CF₃, CHF₂, CH₂F, NO₂, CN, OCF₃, OCHF₂, O-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-(C₁-C₆)-Alkylen-(R9), SO₂-C₂H₂F₃, SO₂-CH₃, SO₂-NH₂, SO₂-NH(C₁-C₆)-Alkylen-(R9), SO₂-N((C₁-C₆)-Alkyl)₂, COOH, COO-(C₁-C₆)-Alkylen-(R9), CONH₂, CONH(C₁-C₆)-Alkylen-(R9), CON((C₁-C₆)-Alkyl)₂, SF₅,(C₆-C₁₀)-Aryl, (C₃-C₈)-Cycloalkyl, 4-12-gliedriger Heterocyclus;
- R6: H, OH, O-(CO)-NH₂, SO₂NH₂;
- R7, R8: unabhängig voneinander (C₁-C₃)-Alkyl;
- R9: H, OH, OCH₃, OCF₃, CHF₂, CF₃;
sowie deren pharmazeutisch verträgliche Salze.

Weiter bevorzugt sind Verbindungen der Formel I, worin bedeuten
- L: R1, -CH(R10)(R11);
- R10: Phenyl, wobei der Phenylrest ein bis 3-fach substituiert sein kann mit F, Cl, Br,-(C₁-C₆)-Alkyl;
- R11: (C₁-C₆)-Alkyl, (C₁-C₆)-Alkylen-(R6);
- R1: (C₃-C₈)-Cycloalkyl, wobei der Cycloalkylrest ein bis 3-fach substituiert sein kann mit F, Cl, Br, OH;
- R2: H, (C₁-C₃)-Alkyl;
- R3, R4, R5, R13: unabhängig voneinander H , F, Cl, Br, I, OH, CF₃, CHF₂, CH₂F, NO₂, CN, OCF₃, OCHF₂, O-(C₁-C₆)-Alkylen-(R9), -(C₁-C₆)-Alkylen-(R9), (C=O)-(C1-C6)-Alkylen-(R9), (C₁-C₆)-Alkylen-(R9), NH₂, NH(C₁-C₆)-Alkylen-(R9), N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅; (C₆-C₁₀)-Aryl, -(C₁-C₆)-Alkylen-(C₆-C₁₀)-Aryl,, wobei der Arylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅; 4 bis 12-gliedriger Heterocyclus, -(C₁-C₆)-Alkylen-4 bis12-gliedriger Heterocyclus, wobei der Heterocyclylrest ein bis 3-fach substituiert sein kann mit F, Cl, Br, I, OH, CF₃, CHF₂, CH₂F, NO₂, CN, OCF₃, OCHF₂, O-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-(C₁-C₆)-Alkylen-(R9), SO₂-C₂H₂F₃, SO₂-CH₃, SO₂-NH₂, SO₂-NH(C₁-C₆)-Alkylen-(R9), SO₂-N((C₁-C₆)-Alkyl)₂, COOH, COO-(C₁-C₆)-Alkylen-(R9), CONH₂, CONH(C₁-C₆)-Alkylen-(R9), CON((C₁-C₆)-Alkyl)₂, SF₅, (C₆-C₁₀)-Aryl, (C₃-C₈)-Cycloalkyl, 4-12-gliedriger Heterocyclus;
- R6: OH;
- R7, R8: unabhängig voneinander (C₁-C₃)-Alkyl;
- R9: H, OH, OCH₃, OCF₃, CHF₂, CF₃;
sowie deren pharmazeutisch verträgliche Salze.

Können Reste oder Substituenten mehrfach in den Verbindungen der Formeln I auftreten, so können sie alle unabhängig voneinander die angegebene Bedeutung haben und gleich oder verschieden sein.

Die Erfindung bezieht sich auf Verbindungen der Formel I, in Form ihrer Tautomere, Racemate, racemischen Mischungen, Stereoisomerengemische, reinen Stereoisomere, Diastereoisomerengemische, reinen Diastereoisomere. Die Trennung der Gemische erfolgt zum Beispiel auf chromatographischem Weg.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze und Solvate wie hierin beschrieben.

Unter einem Alkylrest wird eine geradkettige oder verzweigte Kohlenwasserstoffkette mit einem bis acht Kohlenstoffen verstanden, wie z.B. Methyl, Ethyl, iso-Propyl, tert.-Butyl, Hexyl, Heptyl, Octyl. Die Alkylreste können einfach oder mehrfach wie oben beschrieben substituiert sein.

Unter einem Alkylenrest wird eine geradkettige oder verzweigte Kohlenwasserstoffkette mit zwei freien Valenzen verstanden, wie z.B. Methylen, Ethylen, iso-Propylen, tert.-Butylen.

Unter einem Carbozyklus bzw. Carbocyclylrest wird ein Ring, welcher gesättigt oder partiell ungesättigt (mit einer oder zwei Doppelbindungen) vorliegt, verstanden, der ausschließlich aus Kohlenstoffatomen aufgebaut ist.

Unter einem Arylrest wird ein Phenyl, Naphthyl-, Biphenyl-, Tetrahydronaphthyl-, alpha- oder beta-Tetralon-, Indanyl- oder Indan-1-on-ylrest verstanden.

Die Arylreste können ein oder mehrfach mit geeigneten Gruppen wie oben beschrieben substituiert sein.

Unter Heterocyclus bzw. heterocyclischer Rest werden Ringe und Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heterocylus bzw. der heterocyclische Rest mit einem weiteren Ringsystem anelliert ist. Der Heterocylus bzw. der heterocyclische Rest kann gesättigt, teilweise gesättigt oder aromatisch sein.

Geeignete "Heterocyclen" bzw. "heterocyclische Reste" sind Acridinyl, Azepanyl, Azocinyl, Benzimidazolyl, Benzofuryl, Benzothienyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, 5,6-Dihydro-4H-cyclopentathiazol-2-yl, 4,5-Dihydro-thiazol-2-yl, Furyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pyridooxazole, Pyridoimidazole, Pyridothiazole, Pyridinyl, Pyridyl, Pyrimidinyl, Pyrrolidinyl, Pyrrolinyl, 2H-Pyrrolyl, Pyrrolyl, 4,5,6,7-Tetrahydrobenzooxazol-2-yl, 4,5,6,7-Tetrahydro-benzothiazol-2-yl, 4,5,6,7-Tetrahydro-benzoimidazol-2-yl, 4,5,6,7-Tetrahydro-pyrazolo[1,5-a]pyridin-2-yl, Tetrahydrofuranyl, Tetrahydropyranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazinyl, Triazolyl, Tetrazolyl, Thiazolo[4,5-b]pyridinyl, Thieno[2,3-d]thiazol-2-yl, Tropanyl und Xanthenyl.

Die Heterocyclen bzw. heterocyclischen Reste können ein oder mehrfach mit geeigneten Gruppen wie oben beschrieben substituiert sein.,

Die Verbindung(en) der Formel I können auch in Kombination mit weiteren Wirkstoffen verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im Allgemeinen liegt die Tagesdosis im Bereich von 0.3 mg bis 100 mg (typischerweise von 3 mg und 50 mg) pro Tag pro Kilogramm Körpergewicht, z.B. 3-10 mg / kg / Tag. Eine intravenöse Dosis kann z.B. im Bereich von 0.3 mg bis 1.0 mg / kg liegen, die geeigneterweise als Infusion von 10 ng bis 100 ng pro Kilogramm pro Minute verabreicht werden kann. Geeignete Infusionslösungen für diese Zwecke können z.B. von 0.1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1.0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0.05 % bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0.1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im Allgemeinen in einer Konzentration von 0.1 bis 15 Gew.-% der Zusammensetzung vorhanden, beispielsweise von 0.5 bis 2 %.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1 % bis 35 %, vorzugsweise ca. 3 % bis 15 %. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Als weitere Wirkstoffe für die Kombinationspräparate sind geeignet:

Alle Antidiabetika, die in der Roten Liste 2006, Kapitel 12 genannt sind; alle Abmagerungsmittel/Appetitzügler, die in der Roten Liste 2006, Kapitel 1 genannt sind; alle Lipidsenker, die in der Roten Liste 2006, Kapitel 58 genannt sind. Sie können mit der erfindungsgemäßen Verbindung der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary ofUSAN and International Drug Names, US Pharmacopeia, Rockville 2001, offenbart.

Antidiabetika umfassen Insulin und Insulinderivate, wie z.B. Lantus^{®} (siehe www.lantus.com) oder HMR 1964 oder Levemir^{®} (insulin detemir) oder solche, wie sie in WO2005005477 (Novo Nordisk) beschrieben sind, schnell wirkende Insuline (siehe US 6,221,633), inhalierbare Insuline, wie z. B. Exubera ^{®} oder orale Insuline, wie z. B. IN-105 (Nobex) oder Oral-lyn ™ (Generex Biotechnology), GLP-1-Derivate wie z.B. Exenatide, Liraglutide oder diejenigen die in WO 98/08871, WO2005027978, WO2006037811, WO2006037810 von Novo Nordisk A/S, in WO 01/04156 von Zealand oder in WO 00/34331 von Beaufour-Ipsen offenbart wurden, Pramlintide Acetat (Symlin; Amylin Pharmaceuticals), sowie oral wirksame hypoglykämische Wirkstoffe.

Die oral wirksamen hypoglykämischen Wirkstoffe umfassen vorzugsweise Sulfonylharnstoffe, Biguanidine,
Meglitinide,
Oxadiazolidindione,
Thiazolidindione,
Glukosidase-Inhibitoren,
Hemmstoffe der Glykogenphosphorylase,
Glukagon-Antagoni sten,
Glukokinaseaktivatoren,
Inhibitoren der Fructose-1,6-bisphosphatase
Modulatoren des Glukosetransporters-4 (GLUT4),
Inhibitoren der Glutamin-Fructose-6-Phosphat-Amidotransferase (GFAT),

GLP-1-Agonisten, Kaliumkanalöffner, wie z.B. diejenigen, die in WO 97/26265 und WO 99/03861 von Novo Nordisk A/S offenbart wurden oder die, die in WO2006045799 beschrieben sind (Solvay),
Inhibitoren der Dipeptidylpeptidase-IV (DPP-IV),
Insulin-Sensitizer,
Inhibitoren von Leberenzymen, die an der Stimulation der Glukoneogenese und/oder Glykogenolyse beteiligt sind,
Modulatoren der Glukoseaufnahme, des Glukosetransports und der Glukoserückresorption, Hemmstoffe der 11ß-HSD1,
Inhibitoren der Protein-Tyrosin-Phosphatase-1B (PTP1B),
Modulatoren des natrium-abhängigen Glukosetransporters 1 oder 2 (SGLT1, SGLT2),
den Fettstoffwechsel verändernde Verbindungen wie antihyperlipidämische Wirkstoffe und antilipidämische Wirkstoffe,
Verbindungen, die die Nahrungsmitteleinnahme verringern,
Verbindungen, die die Thermogenese erhöhen,
PPAR- und RXR-Modulatoren und
Wirkstoffe, die auf den ATP-abhängigen Kaliumkanal der Betazellen wirken.

Bei einer Ausführungsform der Erfindung wird die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin, L-659699 verabreicht.

Bei einer Ausführungsform der Erfindung wird die Verbindung der Formel I in Kombination mit einem Cholesterinresorptionsinhibitor, wie z.B. Ezetimibe, Tiqueside, Pamaqueside, FM-VP4 (sitostanol/campesterol ascorbyl phosphat; Forbes Medi-Tech, WO2005042692, WO2005005453), MD-0727 (Microbia Inc., WO2005021497, WO2005021495) oder mit Verbindungen, wie in WO2002066464 (Kotobuki Pharmaceutical Co. Ltd.) oder WO2005044256 oder WO2005062824 (Merck & Co.) oder WO2005061451 und WO2005061452 (AstraZeneca AB) und WO2006017257 (Phenomix) oder WO2005033100 (Lipideon Biotechnology AG) beschrieben, verabreicht.

Bei einer Ausführungsform der Erfindung wird die Verbindung der Formel I in Kombination mit Vytorin^{™}, einer festen Kombination von Ezetimibe mit Simvastatin, verabreicht.

Bei einer Ausführungsform der Erfindung wird die Verbindung der Formel I in Kombination mit einer festen Kombination von Ezetimibe mit Fenofibrat verabreicht.

Bei einer weiteren Ausführungsform der Erfindung wird die Verbindung der Formel I in Kombination mit einer festen Kombination von Fenofibrat mit Rosuvastatin verabreicht.

Bei einer Ausführungsform der Erfindung wird die Verbindung der Formel I in Kombination mit ISIS-301012, einem Antisense-Oligonukleotid, welches in der Lage ist, das Apolipoprotein B Gen zu regulieren, verabreicht.

Bei einer Ausführungsform der Erfindung wird die Verbindung der Formel I in Kombination mit einem PPAR gamma Agonisten, wie z.B. Rosiglitazon, Pioglitazon, JTT-501, Gl 262570, R-483, CS-011 (Rivoglitazon) verabreicht.

Bei einer Ausführungsform der Erfindung wird die Verbindung der Formel I in Kombination mit Competact^{™}, einer festen Kombination von Pioglitazon Hydrochlorid mit Metformin Hydrochlorid, verabreicht.

Bei einer Ausführungsform der Erfindung wird die Verbindung der Formel I in Kombination mit duetact^{™}, einer festen Kombination von Pioglitazon Hydrochlorid mit Glimepirid, verabreicht.

Bei einer Ausführungsform der Erfindung wird die Verbindung der Formel I in Kombination mit Avandamet^{®}, einer festen Kombination von Rosiglitazon Maleat mit Metformin Hydrochlorid, verabreicht.

Bei einer Ausführungsform der Erfindung wird die Verbindung der Formel I in Kombination mit PPAR alpha Agonisten, wie z.B. GW9578, GW-590735, K-111, LY-674, KRP-101, DRF-10945 verabreicht.

Bei einer Ausführungsform der Erfindung wird die Verbindung der Formel I in Kombination mit einem gemischten PPAR alpha/gamma Agonisten, wie z.B. Naveglitazar, LY-510929, ONO-5129, E-3030, AVE 8042, AVE 8134, AVE 0847, oder wie in PCT/US 00/11833, PCT/US 00/11490, DE10142734.4 oder in J.P.Berger et al., TRENDS in Pharmacological Sciences 28(5), 244-251, 2005 beschrieben, verabreicht.

Bei einer Ausführungsform der Erfindung wird die Verbindung der Formel I in Kombination mit einem PPAR delta Agonisten, wie z.B. GW-501516 verabreicht.

Bei einer Ausführungsform wird die Verbindung der Formel I in Kombination mit Metaglidasen oder mit MBX-2044 oder anderen partiellen PPAR gamma Agonisten/Antagonisten verabreicht

Bei einer weiteren Ausführungsform der Erfindung wird die Verbindung der Formel I in Kombination mit einem Aktivator der AMP-aktivierten Proteinkinase (AMPK), wie z. B. A-769662 oder solchen Verbindungen wie sie in US20050038068 beschrieben sind, verabreicht.

Bei einer Ausführungsform der Erfindung wird die Verbindung der Formel I in Kombination mit einem Fibrat, wie z.B. Fenofibrat, Clofibrat, Bezafibrat, verabreicht.

Bei einer Ausführungsform der Erfindung wird die Verbindung der Formel I in Kombination mit einem MTP-Inhibitor, wie z.B. Implitapide , BMS-201038, R-103757 oder solchen wie in WO2005085226, WO2005121091, WO2006010423 beschrieben, verabreicht.

Bei einer Ausführungsform der Erfindung wird die Verbindung der Formel I in Kombination mit einem CETP-Inhibitor, wie z.B. Torcetrapib oder JTT-705 oder solchen wie sie in WO2006002342, WO2006010422, WO2006012093 beschrieben sind, verabreicht.

Bei einer Ausführungsform der Erfindung wird die Verbindung der Formel I in Kombination mit Gallensäureresorptionsinhibitor (siehe z.B. US 6,245,744, US 6,221,897 oder WO00/61568) wie z.B. HMR 1741 oder solchen wie in DE 10 2005 033099.1 und DE 10 2005 033100.9 beschrieben, verabreicht.

Bei einer Ausführungsform der Erfindung wird die Verbindung der Formel I in Kombination mit einem polymeren Gallensäureadsorber, wie z.B. Cholestyramin, Colesevelam, verabreicht.

Bei einer Ausführungsform der Erfindung wird die Verbindung der Formel I in Kombination mit einem LDL-Rezeptorinducer (siehe US 6,342,512), wie z.B. HMR1171, HMR1586, oder solchen wie in WO2005097738 beschrieben, verabreicht.

Bei einer Ausführungsform wird die Verbindung der Formel I in Kombination mit Omacor® (Omega-3-Fettsäuren; hochkonzentrierte Ethylester der Eicosapentaensäure und der Docosahexaensäure) verabreicht.

Bei einer Ausführungsform der Erfindung wird die Verbindung der Formel I in Kombination mit einem ACAT-Inhibitor, wie z.B. Avasimibe oder SMP-797, verabreicht.

Bei einer Ausführungsform der Erfindung wird die Verbindung der Formel I in Kombination mit einem Antioxidans, wie z.B. OPC-14117, Probucol, Tocopherol, Ascorbinsäure, ß-Caroten oder Selen verabreicht.

Bei einer Ausführungsform der Erfindung wird die Verbindung der Formel I in Kombination mit einem Vitamin, wie z. B. Vitamin B6 oder Vitamin B 12 verabreicht.

Bei einer Ausführungsform der Erfindung wird die Verbindung der Formel I in Kombination mit einem Lipoprotein-Lipase Modulator, wie z.B. Ibrolipim (NO-1886), verabreicht.

Bei einer Ausführungsform der Erfindung wird die Verbindung der Formel I in Kombination mit einem ATP-Citrat-Lyase Inhibitor, wie z.B. SB-204990, verabreicht.

Bei einer Ausführungsform der Erfindung wird die Verbindung der Formel I in Kombination mit einem Squalen Synthetase Inhibitor, wie z.B. BMS-188494 oder wie in WO2005077907 beschrieben, verabreicht.

Bei einer Ausführungsform der Erfindung wird die Verbindung der Formel I in Kombination mit einem Lipoprotein(a) antagonist, wie z.B. Gemcabene (CI-1027) verabreicht.

Bei einer Ausführungsform der Erfindung wird die Verbindung der Formel I in Kombination mit einem Aqonisten des GPR109A (HM74A Rezeptor Agonisten), wie z.B. Nicotinsäure oder "extended release niacin" in Verbindung mit MK-0524A oder solchen Verbindungen, wie sie in WO2006045565, WO2006045564, WO2006069242 beschrieben sind, verabreicht.

Bei einer anderen Ausführungsform der Erfindung wird die Verbindung der Formel I in Kombination mit einem Aqonisten des GPR116, wie sie z.B. in WO2006067531, WO2006067532 beschrieben sind, verabreicht.

Bei einer Ausführungsform der Erfindung wird die Verbindung der Formel I in Kombination mit einem Lipase Inhibitor, wie z.B. Orlistat oder Cetilistat (ATL-962), verabreicht.

Bei einer Ausführungsform der Erfindung wird die Verbindung der Formel I in Kombination mit Insulin verabreicht.

Bei einer Ausführungsform wird die Verbindung der Formel I in Kombination mit einem Sulfonylharnstoff, wie z.B. Tolbutamid, Glibenclamid, Glipizid oder Glimepirid verabreicht.

Bei einer Ausführungsform wird die Verbindung der Formel I in Kombination mit einer die Insulinsekretion verstärkende Substanz, wie z. B. KCP-265 (WO2003097064), verabreicht.

Bei einer Ausführungsform wird die Verbindung der Formel I in Kombination mit Agonisten des glucose-abhängigen insulinotropischen Rezeptors (GDIR) wie z. B. APD-668 verabreicht

Bei einer Ausführungsform wird die Verbindung der Formel I in Kombination mit einem Biguanid, wie z.B. Metformin, verabreicht.

Bei wieder einer anderen Ausführungsform wird die Verbindung der Formel I in Kombination mit einem Meglitinid, wie z.B. Repaglinide oder Nateglinid, verabreicht.

Bei einer Ausführungsform wird die Verbindung der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform wird die Verbindung der Formel I in Kombination mit einem α-Glukosidase-Inhibitor, wie z.B. Miglitol oder Acarbose, verabreicht.

Bei einer Ausführungsform wird die Verbindung der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform wird die Verbindung der Formel I in Kombination mit mehr als einer der vorstehend genannten Verbindungen, z.B. in Kombination mit einem Sulfonylharnstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc. verabreicht.

Bei einer Ausführungsform wird die Verbindung der Formel I in Kombination mit einem Hemmstoff der Glykogenphosphorylase, wie z.B. PSN-357 oder FR-258900 oder solchen wie in WO2003084922, WO2004007455, WO2005073229-31, WO2005067932 beschrieben, verabreicht.

Bei einer Ausführungsform wird die Verbindung der Formel I in Kombination mit Glukagon-Rezeptor-Antagonisten, wie z.B. A-770077 oder NNC-25-2504 oder wie in WO2004100875, WO2005065680, beschrieben, verabreicht.

Bei einer Ausführungsform wird die Verbindung der Formel I in Kombination mit Aktivatoren der Glukokinase, wie z. B. LY-2121260 (WO2004063179), PSN-105, PSN-110, GKA-50 oder solchen wie sie z. B. in WO2004072031, WO2004072066, WO2005080360, WO2005044801, WO2006016194, WO2006058923 beschrieben sind, verabreicht.

Bei einer Ausführungsform wird die Verbindung der Formel I in Kombination mit einem Inhibitor der Glukoneogenese, wie z. B. FR-225654, verabreicht.

Bei einer Ausführungsform wird die Verbindung der Formel I in Kombination mit Inhibitoren der Fructose-1,6-bisphosphatase (FBPase) wie z.B. CS-917 (MB-06322) oder MB-07803 oder solchen wie sie in WO2006023515 beschrieben sind, verabreicht.

Bei einer Ausführungsform wird die Verbindung der Formel I in Kombination mit Modulatoren des Glukosetransporters-4 (GLUT4), wie z. B. KST-48 (D.-O. Lee et al.: Arzneim.-Forsch. Drug Res. 54 (12), 835 (2004)), verabreicht.

Bei einer Ausführungsform wird die Verbindung der Formel I in Kombination mit Inhibitoren der Glutamin-Fructose-6-Phosphat-Amidotransferase (GFAT), wie sie z. B. in WO2004101528 beschrieben sind, verabreicht.

Bei einer Ausführungsform wird die Verbindung der Formel I in Kombination mit Inhibitoren der Dipeptidylpeptidase-IV (DPP-IV), wie z. B. Vildagliptin (LAF-237), Sitagliptin (MK-0431), Saxagliptin ((BMS-477118), GSK-823093, PSN-9301, SYR-322, SYR-619, TA-6666, TS-021, GRC-8200, GW-825964X, KRP-104, DP-893 oder wie sie in WO2003074500, WO2003106456, WO200450658, WO2005058901, WO2005012312, WO2005/012308, WO2006039325, WO2006058064, PCT/EP2005/007821, PCT/EP2005/008005, PCT/EP2005/008002, PCT/EP2005/008004, PCT/EP2005/008283, DE 10 2005 012874.2 oder DE 10 2005 012873.4 beschrieben sind, verabreicht.

Bei einer Ausführungsform wird die Verbindung der Formel I in Kombination mit Januvia™, einer festen Kombination von Sitagliptin Phosphat mit Metformin hydrochlorid, verabreicht.

Bei einer Ausführungsform wird die Verbindung der Formel I in Kombination mit Hemmstoffen der 11-beta-Hydroxysteroid-Dehydrogenase-1(11ß-HSD1), wie z. B. BVT-2733, JNJ-25918646, INCB-13739 oder solche, wie sie z. B. in WO200190090-94, WO200343999, WO2004112782, WO200344000, WO200344009, WO2004112779, WO2004113310, WO2004103980, WO2004112784, WO2003065983, WO2003104207, WO2003104208, WO2004106294, WO2004011410, WO2004033427, WO2004041264, WO2004037251, WO2004056744, WO2004058730, WO2004065351, WO2004089367, WO2004089380, WO2004089470-71, WO2004089896, WO2005016877, WO2005097759, WO2006010546, WO2006012227, WO2006012173, WO2006017542, WO2006034804, WO2006040329, WO2006051662, WO2006048750, WO2006049952, WO2006048331, WO2006050908, WO2006024627, WO2006040329, WO2006066109 beschrieben sind, verabreicht.

Bei einer Ausführungsform wird die Verbindung der Formel I in Kombination mit Inhibitoren der Protein-Tyrosin-Phosphatase-1B (PTP1B), wie sie z. B. in WO200119830-31, WO200117516, WO2004506446, WO2005012295, WO2005116003, PCT/EP2005/005311, PCT/EP2005/005321, PCT/EP2005/007151, PCT/EP2005/01294 oder DE 10 2004 060542.4 beschrieben sind, verabreicht.

Bei einer Ausführungsform wird die Verbindung der Formel I in Kombination mit Modulatoren des natrium-abhängigen Glukosetransporters 1 oder 2 (SGLT1, SGLT2), wie z.B. KGA-2727, T-1095, SGL-0010, AVE 2268 und SAR 7226 oder wie sie z. B. in WO2004007517, WO200452903, WO200452902, PCT/EP2005/005959, WO2005085237, JP2004359630, WO2005121161, WO2006018150, WO2006035796, WO2006062224, WO2006058597 oder von A. L. Handlon in Expert Opin. Ther. Patents (2005) 15(11), 1531-1540 beschrieben sind, verabreicht.

Bei einer Ausführungsform wird die Verbindung der Formel I in Kombination mit Modulatoren des GPR40 verabreicht.

Bei einer Ausführungsform wird die Verbindung der Formel I in Kombination mit Modulatoren des GPR119b, wie sie z. B. in WO2004041274 beschrieben sind, verabreicht.

Bei einer Ausführungsform wird die Verbindung der Formel I in Kombination mit Modulatoren des GPR119, wie sie z. B. in WO2005061489 (PSN-632408) beschrieben sind, verabreicht. Bei einer Ausführungsform wird die Verbindung der Formel I in Kombination mit Inhibitoren der hormon-sensitiven Lipase (HSL), wie z. B. in WO2005073199 beschrieben, verabreicht.

Bei einer Ausführungsform wird die Verbindung der Formel I in Kombination mit Hemmstoffen der Acetyl-CoA Carboxylase (ACC) wie z. B. solchen wie in WO199946262, WO200372197, WO2003072197, WO2005044814, WO2005108370, JP2006131559 beschrieben, verabreicht.

Bei einer Ausführungsform wird die Verbindung der Formel I in Kombination mit einem Inhibitor der Phosphoenolpyruvatcarboxykinase (PEPCK), wie z.B. solchen, wie in WO2004074288 beschrieben, verabreicht.

Bei einer Ausführungsform wird die Verbindung der Formel I in Kombination mit einem Inhibitor der Glykogen Synthase Kinase-3 beta (GSK-3 beta), wie z. B. in US2005222220, WO2005085230, PCT/EP2005/005346, WO2003078403, WO2004022544, WO2003106410, WO2005058908, US2005038023, WO2005009997, US2005026984, WO2005000836, WO2004106343, EP1460075, WO2004014910, WO2003076442, WO2005087727, WO2004046117 beschrieben.

Bei einer Ausführungsform wird die Verbindung der Formel I in Kombination mit einem Inhibitor der Protein Kinase C beta (PKC beta), wie z. B. Ruboxistaurin, verabreicht.

Bei einer Ausführungsform wird die Verbindung der Formel I in Kombination mit einem Endothelin-A-Rezeptor Antagonisten, wie z. B. Avosentan (SPP-301), verabreicht.

Bei einer Ausführungsform wird die Verbindung der Formel I in Kombination mit Inhibitoren der "I-kappaB kinase" (IKK Inhibitoren), wie sie z. B. in WO2001000610, WO2001030774, WO2004022553, WO2005097129 beschrieben sind, verabreicht.

Bei einer Ausführungsform wird die Verbindung der Formel I in Kombination mit Modulatoren des Glucocorticoidrezeptors, wie sie z. B. in WO2005090336 beschrieben sind, verabreicht.

Bei einer weiteren Ausführungsform wird die Verbindung der Formel I in Kombination mit CART-Modulatoren (siehe "Cocaine-amphetamine-regulated transcript influences energy metabolism, anxiety and gastric emptying in mice" Asakawa, A. et al.: Hormone and Metabolic Research (2001), 33(9), 554-558);
NPY-Antagonisten wie z.B. Naphthalin-1-sulfonsäure-{4-[(4-amino-quinazolin-2-ylamino)-methyl]-cyclohexylmethyl}-amid Hydrochlorid (CGP 71683A);
NPY-5 Rezeptorantagonisten wie L-152804, S-2367 oder wie sie z. B. in WO2006001318 beschrieben sind;
Peptid YY 3-36 (PYY3-36) oder analoge Verbindungen wie z. B. CJC-1682 (PYY3-36 konjugiert mit humanem Serum Albumin über Cys34) oder CJC-1643 (Derivat des PYY3-36, welches sich in vivo an Serum Albumin konjugiert) oder solche, wie sie in WO2005080424 beschrieben sind;
CB1R (Cannabinoid Rezeptor 1) Antagonisten (wie z.B. Rimonabant, SR147778, SLV-319, AVE-1625, MK-0364 oder Salze davon oder solche wie sie in z. B. EP 0656354, WO 00/15609, WO2001/64632, WO2001/64633, WO2001/64634, WO 02/076949, WO2005080345, WO2005080328, WO2005080343, WO2005075450, WO2005080357, WO200170700, WO2003026647-48, WO200302776, WO2003040107, WO2003007887, WO2003027069, US6,509,367, WO200132663, WO2003086288, WO2003087037, WO2004048317, WO2004058145, WO2003084930, WO2003084943, WO2004058744, WO2004013120, WO2004029204, WO2004035566, WO2004058249, WO2004058255, WO2004058727, WO2004069838, US20040214837, US20040214855, US20040214856, WO2004096209, WO2004096763, WO2004096794, WO2005000809, WO2004099157, US20040266845, WO2004110453, WO2004108728, WO2004000817, WO2005000820, US20050009870, WO200500974, WO2004111033-34, WO200411038-39, WO2005016286, WO2005007111, WO2005007628, US20050054679, WO2005027837, WO2005028456, WO2005063761-62, WO2005061509, WO2005077897, WO2006047516, WO2006060461, WO2006067428, WO2006067443 beschrieben sind);
MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chlor-phenyl)-2-oxo-ethyl]-amid; (WO 01/91752)) oder LB53280, LB53279, LB53278 oder THIQ, MB243, RY764, CHIR-785, PT-141 oder solche wie sie in WO2005060985, WO2005009950, WO2004087159, WO2004078717, WO2004078716, WO2004024720, US20050124652, WO2005051391, WO2004112793, WOUS20050222014, US20050176728, US20050164914, US20050124636, US20050130988, US20040167201, WO2004005324, WO2004037797, WO2005042516, WO2005040109, WO2005030797, US20040224901, WO200501921, WO200509184, WO2005000339, EP1460069, WO2005047253, WO2005047251, EP1538159, WO2004072076, WO2004072077, WO2006021655-57 beschrieben sind;
Orexin-Rezeptor Antagonisten (z.B. 1-(2-Methyl-benzoxazol-6-yl)-3-[1,5]naphthyridin-4-yl-harnstoff Hydrochlorid (SB-334867-A) oder solche, wie sie z. B. in WO200196302, WO200185693, WO2004085403, WO2005075458, WO2006067224 beschrieben sind); Histamin H3 Rezeptor Agonisten (z. B. 3-Cyclohexyl-1-(4,4-dimethyl-1,4,6,7-tetrahydroimidazo[4,5-c]pyridin-5-yl)-propan-1-on Oxalsäuresalz (WO 00/63208) oder solche, wie sie in WO200064884, WO2005082893 beschrieben sind);
CRF-Antagonisten (z.B. [2-Methyl-9-(2,4,6-trimethyl-phenyl)-9H-1,3,9-triaza-fluoren-4-yl]-dipropyl-amin (WO 00/66585));
CRF BP-Antagonisten (z.B. Urocortin);
Urocortin-Agonisten;
Agonisten des beta-3 Adrenoceptors wie z.B. 1-(4-Chlor-3-methansulfonylmethyl-phenyl)-2-[2-(2,3-dimethyl-1H-indol-6-yloxy)-ethylamino]-ethanol Hydrochlorid (WO 01/83451) oder Solabegron (GW-427353) oder N-5984 (KRP-204) oder solche, wie sie in JP2006111553 beschrieben sind;
MSH (Melanocyt-stimulierendes Hormon)-Agonisten;
MCH (melanin-konzentrierendes Hormon) Rezeptor Antagonisten (wie z. B. NBI-845, A-761, A-665798, A-798, ATC-0175, T-226296, T-71, GW-803430 oder solche Verbindungen, wie sie in WO2003/15769, WO2005085200, WO2005019240, WO2004011438, WO2004012648, WO2003015769, WO2004072025, WO2005070898, WO2005070925, WO2004039780, WO2003033476, WO2002006245, WO2002089729, WO2002002744, WO2003004027, FR2868780, WO2006010446, WO2006038680, WO2006044293, WO2006044174 beschrieben sind);
CCK-A Agonisten (wie z.B. {2-[4-(4-Chlor-2,5-dimethoxy-phenyl)-5-(2-cyclohexyl-ethyl)-thiazol-2-ylcarbamoyl]-5,7-dimethyl-indol-1-yl}-essigsäure Trifluoressigsäuresalz (WO 99/15525) oder SR-146131 (WO 0244150) oder SSR-125180) oder solchen, wie sie in WO2005116034 beschrieben sind;
Serotonin-Wiederaufnahme-Inhibitoren (z.B. Dexfenfluramine);
gemischte Sertonin- und noradrenerge Verbindungen (z.B. WO 00/71549);
5-HT-Rezeptor Agonisten z.B. 1-(3-Ethyl-benzofuran-7-yl)-piperazin Oxalsäuresalz (WO 01/09111);
5-HT2C Rezeptor Agonisten (wie z.B. Lorcaserin Hydrochlorid (APD-356) oder BVT-933 oder solche, wie sie in WO200077010, WO20077001-02, WO2005019180, WO2003064423, WO200242304, WO2005082859 beschrieben sind);
5-HT6 Rezeptor Antagonisten, wie sie z.B. in WO2005058858 beschrieben sind; Bombesin-Rezeptor Agonisten (BRS-3 Agonisten;
Galanin-Rezeptor Antagonisten;
Wachstumshormon (z.B. humanes Wachstumshormon oder AOD-9604);
Wachstumshormon freisetzende Verbindungen (6-Benzyloxy-1-(2-diisopropylaminoethylcarbamoyl)-3,4-dihydro-1H-isochinolin-2-carbonsäuretertiärbutylester (WO 01/85695)); Growth Hormone Secretagogue Receptor Antagonisten (Ghrelin Antagonisten) wie z. B. A-778193 oder solchen, wie sie in WO2005030734 beschrieben sind;
TRH-Agonisten (siehe z.B. EP 0 462 884);
entkoppelnde Protein 2- oder 3-Modulatoren;
Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);
Dopaminagonisten (DA-Agonisten, z.B. Bromocriptin, Doprexin);
Lipase/Amylase-Inhibitoren (z.B. WO 00/40569);
Inhibitoren der Diacylglycerol O-Acyltransferasen (DGATs) wie z. B. BAY-74-4113 oder wie z. B. in US2004/0224997, WO2004094618, WO200058491, WO2005044250, WO2005072740, JP2005206492, WO2005013907, WO2006004200, WO2006019020, WO2006064189 beschrieben;
Inhibitoren der Fettsäuresynthase (FAS) wie z.B. C75 oder solchen, wie in WO2004005277 beschrieben;
Oxyntomodulin;
Oleoyl-Estron oder Agonisten des Schilddrüsenhormonrezeptors (thyroid hormone receptor agonists) wie z. B: KB-2115 oder solche, wie in WO20058279, WO200172692, WO200194293, WO2003084915, WO2004018421, WO2005092316 beschrieben, verabreicht.

Bei einer Ausführungsform ist der weitere Wirkstoff Varenicline Tartrate, ein partieller Agonist des alpha 4-beta 2 nikotinischen Acetylcholinrezeptors.

Bei einer Ausführungsform ist der weitere Wirkstoff Trodusquemine.

Bei einer Ausführungsform ist der weitere Wirkstoff ein Modulator des Enzyms SIRT1, eines Mitglieds der humanen Sirtuinenzymfamilie.

Bei einer Ausführungsform der Erfindung ist der weitere Wirkstoff Leptin;
siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphetamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Mazindol oder Phentermin.

Bei einer Ausführungsform ist der weitere Wirkstoff ein Diphenylazetidinonderivat, wie z.B. in US 6,992,067 oder US 7,205,290 beschrieben.

Bei einer Ausführungsform wird die Verbindung der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

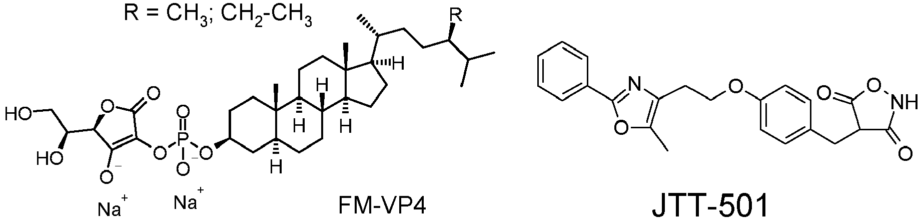

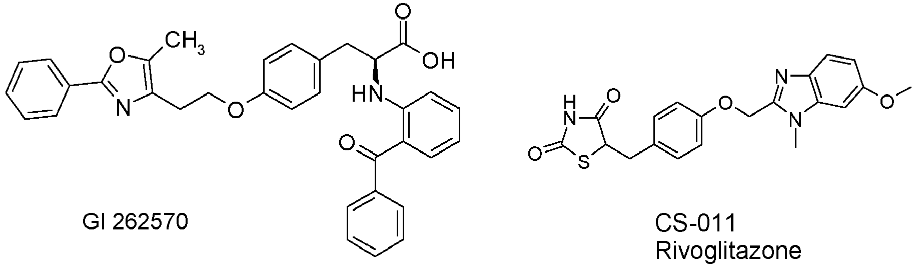

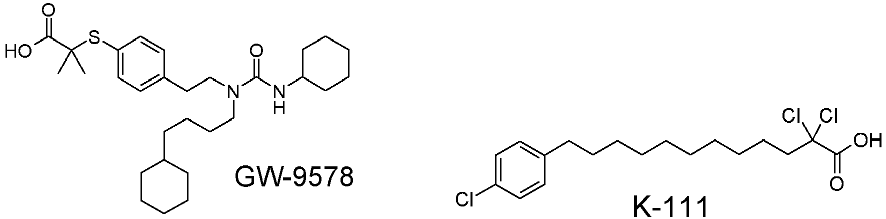

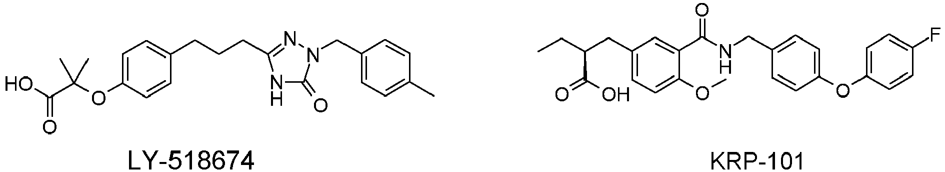

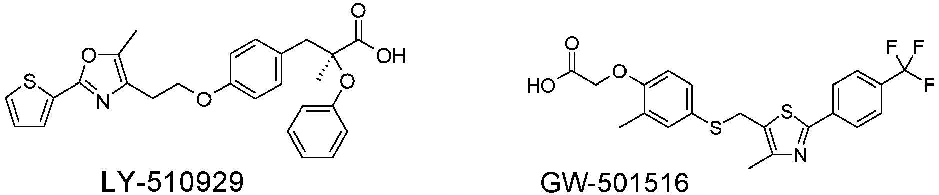

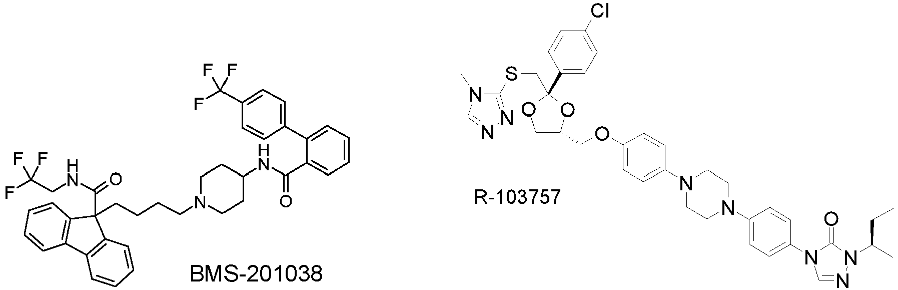

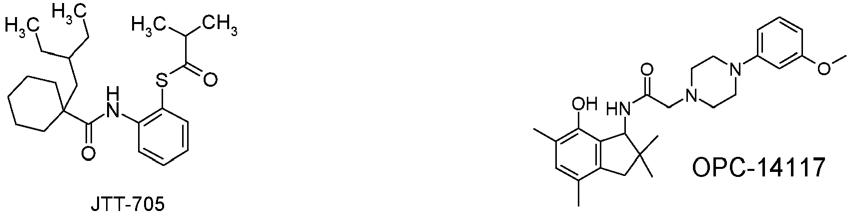

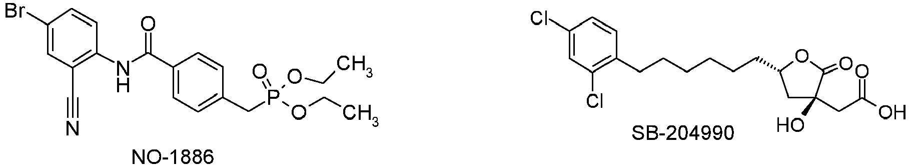

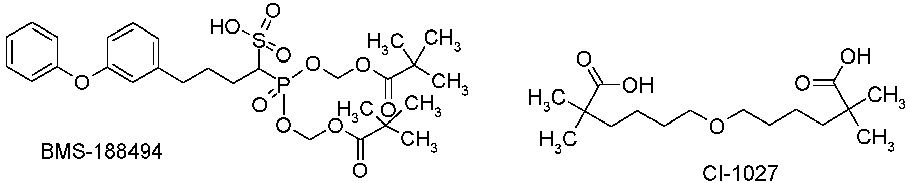

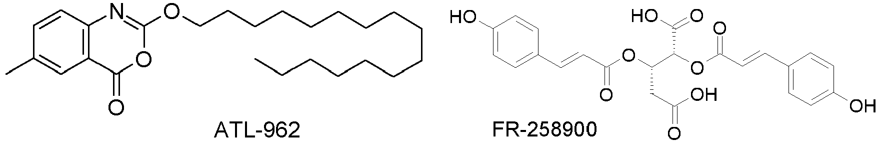

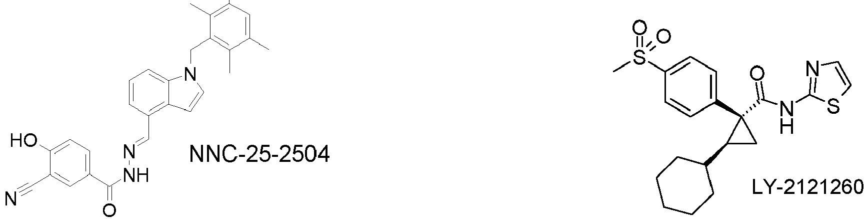

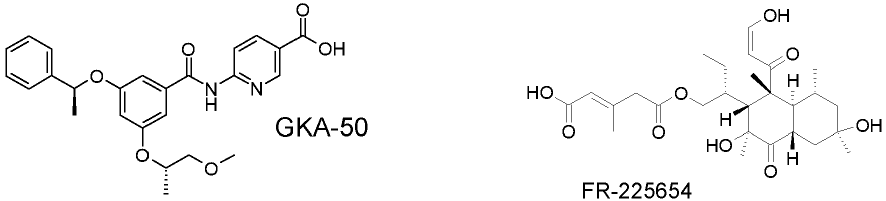

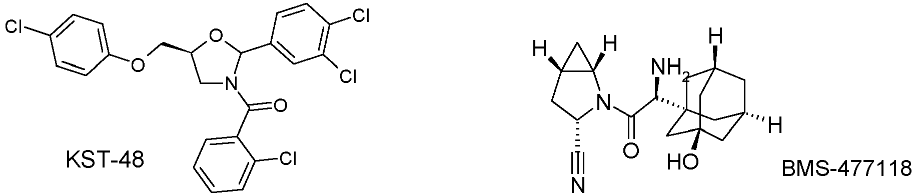

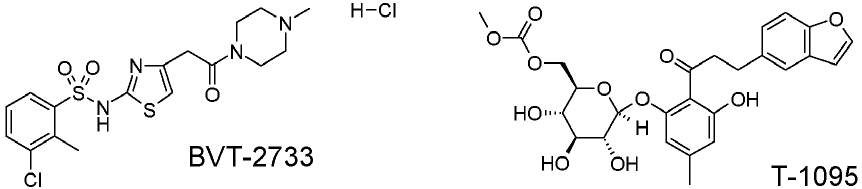

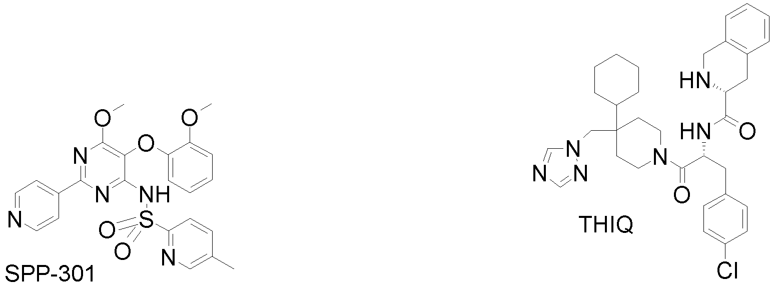

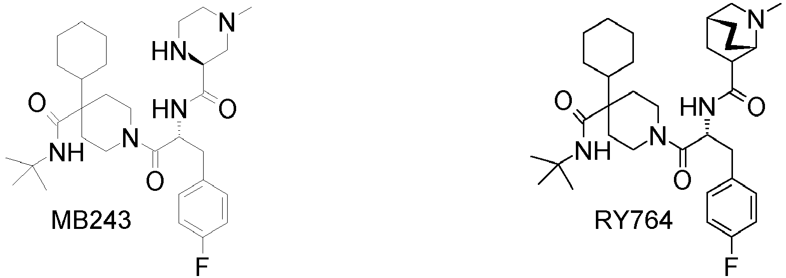

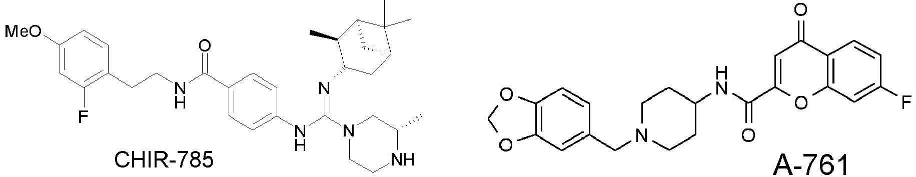

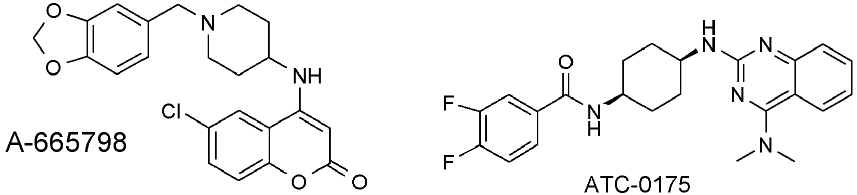

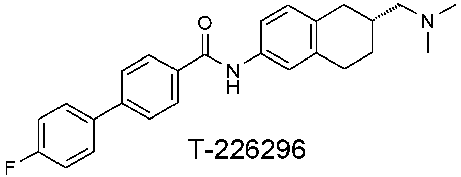

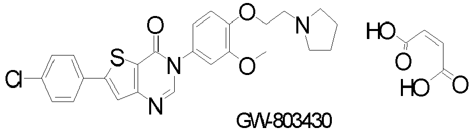

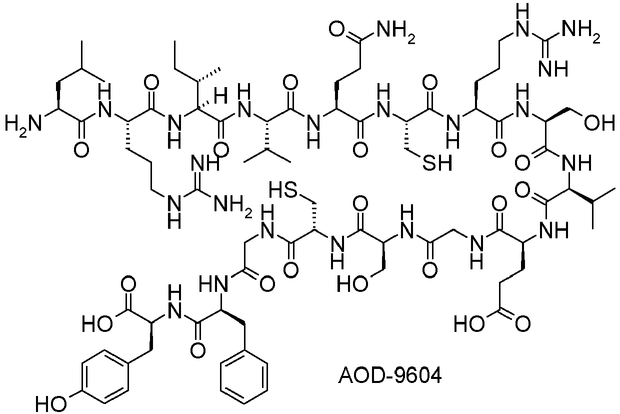

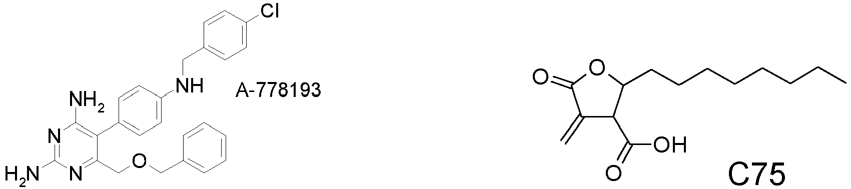

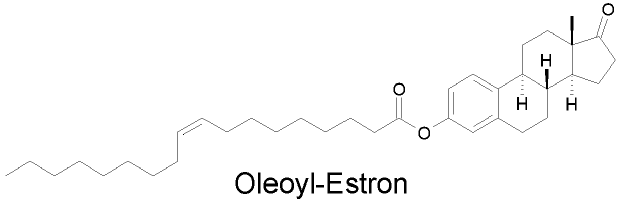

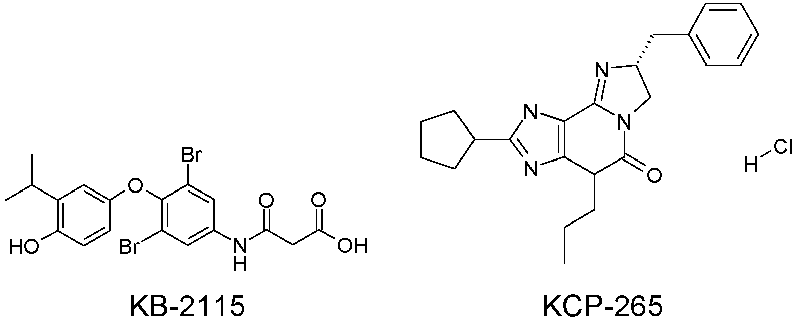

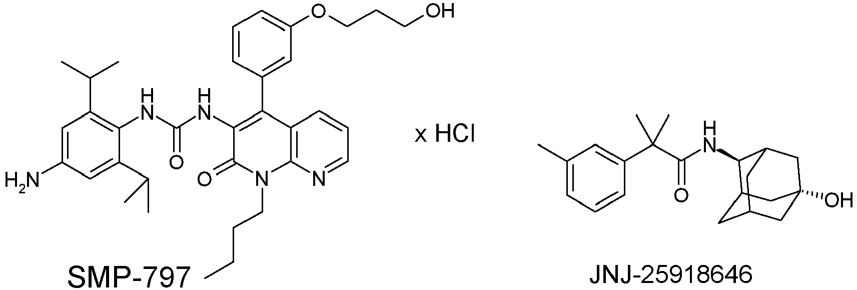

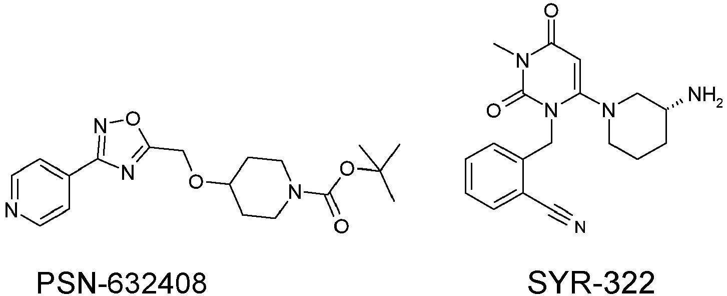

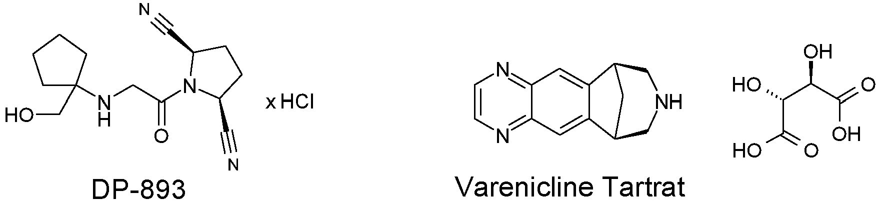

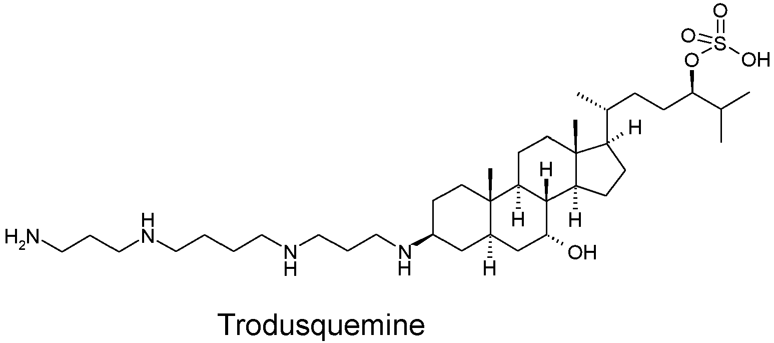

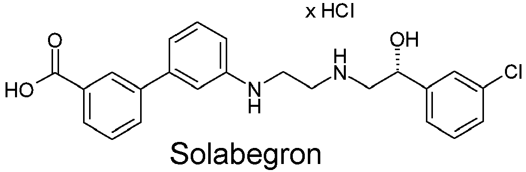

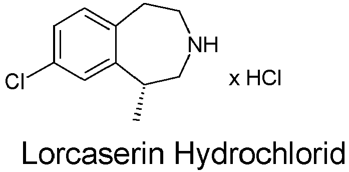

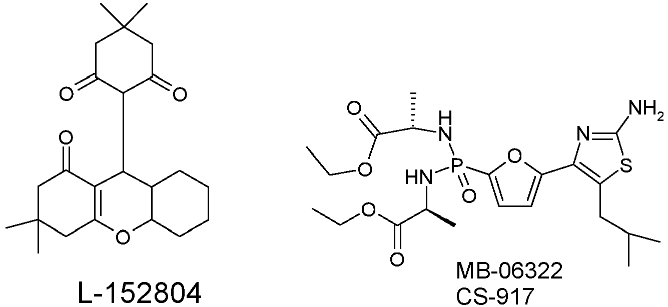

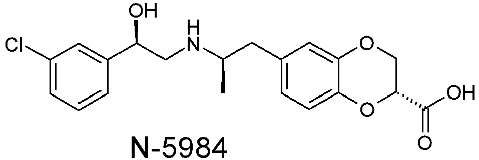

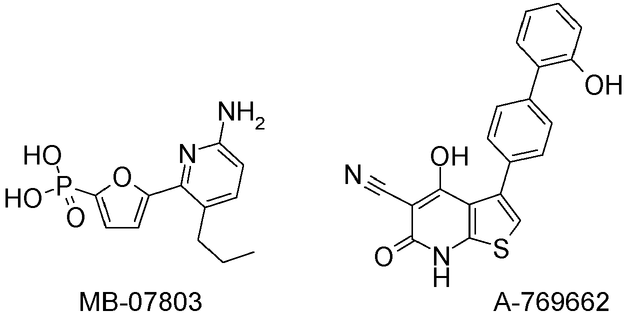

### Beispiele

Die nachfolgend aufgeführten Beispiele und Herstellungsmethoden dienen zur Erläuterung der Erfindung, ohne diese jedoch einzuschränken.

Die erfindungsgemäßen Verbindungen der Formel I können mit Hilfe von im Prinzip bekannten Reaktionen hergestellt werden. Beispielsweise wurden die Verbindungen nach folgenden allgemeinen Reaktionsschemata erhalten.

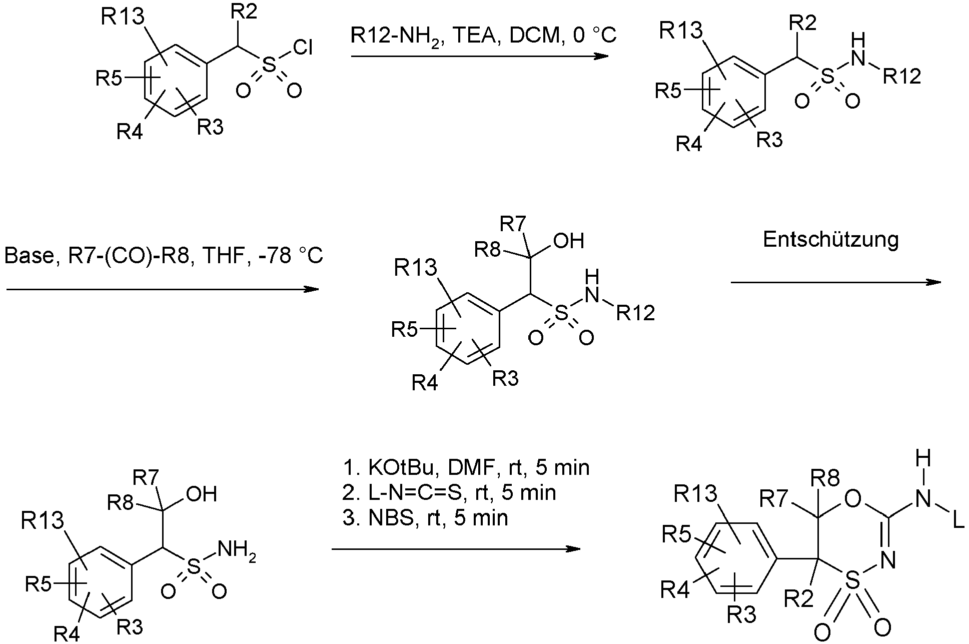

Aus einem mit einem Phenylrest und R2 substituierten Methansulfonsäurechlorid wird durch die Reaktion mit einem geeigneten Amin und Triethylamin (TEA) in einem geeigneten Lösungsmittel z.B. Dichlormethan (DCM) ein entsprechendes mit Phenyl und R2 substituiertes und mit R12 (z.B. Boc, Benzyl, 2,4-Dimethoxybenzyl) geschütztes Methansulfonsäureamid hergestellt. Durch Umsetzung mit einer geeigneten Base (z.B. Methyllithium) kann bei tiefer Temperatur dann ein Dianion erzeugt werden, das mit einem Keton (z.B. R7-(CO)-R8) oder einem Aldehyd (z.B. R7-CHO) in einem geeigneten Lösungsmittel z.B. Tetrahydrofuran (THF) zum mit R12 geschützten Hydroxysulfonamid reagiert. Durch Entschützung von R12 (z.B. bei einer Boc-Gruppe oder einer 2,4-Dimethoxybenzylgruppe durch Säurebehandlung oder bei einer Benzylgruppe durch Hydrierung) entsteht das freie Hydroxysulfonamid. Die Umsetzung des so erhaltenen Hydroxysulfonamids mit Base und einem Isothiocyanat (L-N=C=S) sowie nachfolgendem oxidativen Ringschluss mit N-Bromsuccinimid (NBS) liefert die gewünschten 4,4-dioxo-oxathiazine.

In den Fällen, in denen funktionelle Gruppen (z.B. eine Hydroxylgruppe) in geschützter Form (z.B. benzylgeschützt oder als Ester) eingebracht werden, werden diese am Schluss der Synthese durch ein geeignetes Verfahren (z.B. Hydrierung oder Reduktion) freigesetzt.

Die verwendeten Isothiocyanate werden durch die Reaktion eines primären Amins mit Thiocarbonyldiimidazol erhalten, wobei eventuell vorhandene störende funktionelle Gruppen, z.B. Hydroxylgruppen mit geeigneten Schutzgruppen, z.B. Silylether blockiert werden. Die Schutzgruppen werden am Ende der Sequenz durch geeignete Verfahren entfernt, z.B. Silylgruppen durch Behandlung mit methanolischer Salzsäure.

In den Fällen, in denen während der Synthese Diastereomere oder Racemate entstehen, können diese durch präparative HPLC voneinander getrennt werden.

Einige der eingesetzten primären Amine sind kommerziell erhältlich.
4-Fluoro-bicyclo[2.2.2]octan-1-amin kann hergestellt werden wie in der Literatur beschrieben (JOC 1982, 47, 1952-7).

Andere erfindungsgemäße Verbindungen können auf weiteren Wegen erhalten werden, die im folgenden Schema beispielhaft skizziert sind.

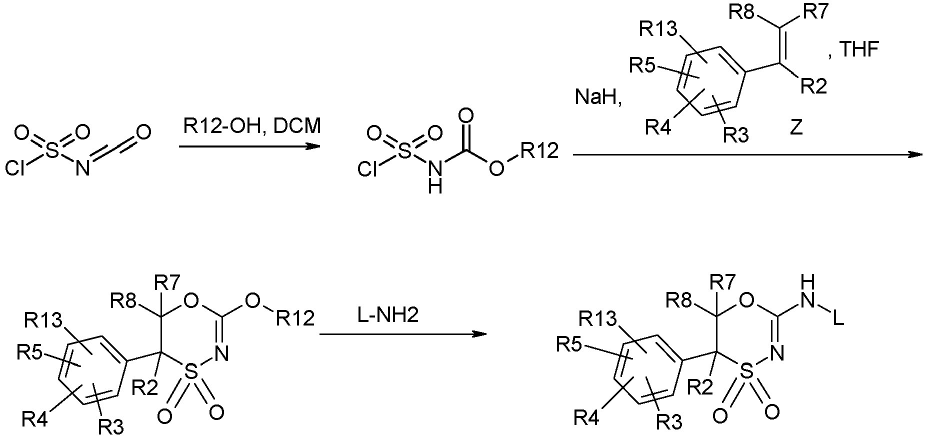

In Analogie zu einem literaturbekannten Verfahren (JACS 1972, 94, 4386-7) wird Chrosulfonylisocyanat mit einem Alkohol (R12-OH, z.B. Methanol) behandelt, wobei ein entsprechendes Carboalkoxysulfamoylchlorid entsteht (z.B. Carbomethoxysulfamoylchlorid). Dieses wird mit Natriumhydrid deprotoniert werden und die nach Chloridabspaltung entstehende Zwischenstufe (z.B. Methyl-N-Sulfonylurethan) reagiert in einer 2+4-Cycloaddition mit Alkenen der Formel Z zum alkoxysubstitierten (z.B. methoxysubstitierten) 4,4-Dioxooxathiazins umgesetzt. Die Alkoxygruppe lässt sich hier in einem geeigneten Lösemittel (z.B. Dichlormethan) durch ein Amin ersetzen, wobei die gewünschten 4,4-Dioxooxathiazine entstehen.

In den Fällen, in denen während der Synthese Diastereomere oder Racemate entstehen, können diese durch präparative HPLC voneinander getrennt werden.

Andere erfindungsgemäße Verbindungen können auf weiteren Wegen erhalten werden, die im folgenden Schema beispielhaft skizziert sind.

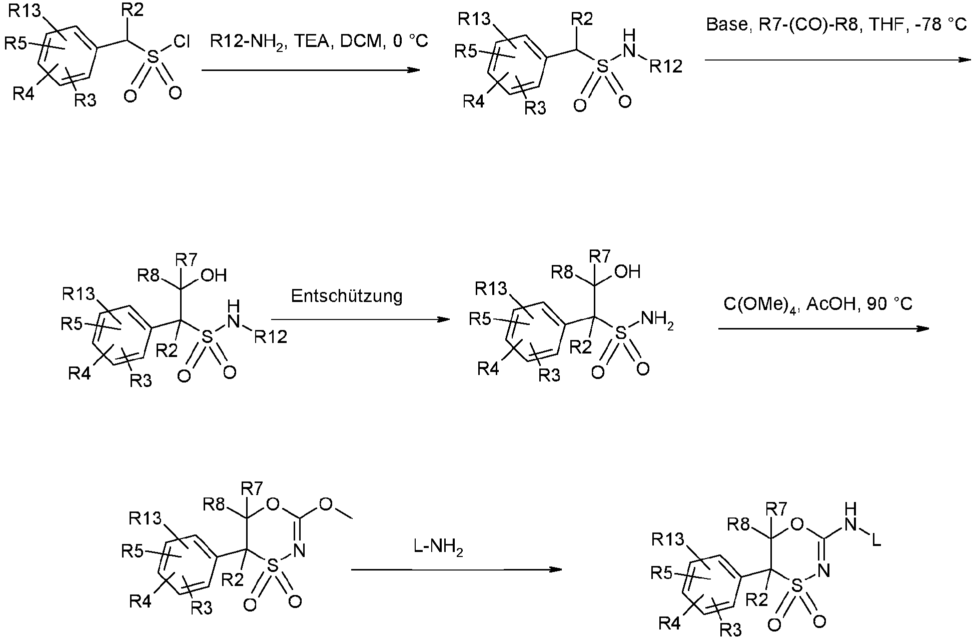

Aus einem mit Phenyl und R2 substituierten Methansulfonsäurechlorid wird durch die Reaktion mit einem geeigneten Amin und Triethylamin (TEA) in einem geeigneten Lösungsmittel z.B. Dichlormethan (DCM) ein entsprechendes mit Phenyl und R2 substituiertes und mit R12 (z.B. Boc, Benzyl, 2,4-Dimethoxybenzyl) geschütztes Methansulfonsäureamid hergestellt. Durch Behandlung mit einer geeigneten Base (z.B. Methyllithium) kann bei tiefer Temperatur dann ein Dianion erzeugt werden, das mit einem Keton (z.B. R7-(CO)-R8) oder einem Aldehyd (z.B. R7-CHO) in einem geeigneten Lösungsmittel z.B. Tetrahydrofuran (THF) zum mit R12 geschützten Hydroxysulfonamid umgesetzt wird. Durch Entschützung von R12 (z.B. bei einer Boc-Gruppe oder einer 2,4-Dimethoxybenzylgruppe durch Säurebehandlung oder bei einer Benzylgruppe durch Hydrierung) entsteht das freie Hydroxysulfonamid. Die Umsetzung des so erhaltenen Hydroxysulfonamids mit Orthokohlensäuretetramethylester liefert methoxysubstitierte 4,4-Dioxooxathiazine. Die Methoxygruppe (-O-CH₃) wird nun in einem geeigneten Lösemittel (z.B. Dichlormethan) durch ein Amin (L-NH₂) ersetzt, wobei die gewünschten 4,4-Dioxooxathiazine entstehen.

In den Fällen, in denen funktionelle Gruppen (z.B. eine Hydroxylgruppe) in geschützter Form (z.B. benzylgeschützt oder als Ester) eingebracht werden, werden diese am Schluss der Synthese durch ein geeignetes Verfahren (z.B. Hydrierung oder Reduktion) freigesetzt.

Falls das entstehende Molekül eine Halogenaryleinheit beinhaltet, kann das Halogen durch gängige metallkatalyiserte Kupplungsverfahren ersetzt werden. So kann z.B. ein Bromid mit Hilfe einer Suzuki-Reaktion oder eine Sonogashira-Reaktion oder einer palladiumkatalysierten Aminierung weiter umgesetzt werden. Weiterhin lassen sich in einigen Fällen einfache Syntheseschritte anschließen, so kann z.B. eine Dreifachbindung zur Einfachbindung hydriert werden oder ein Boc-geschütztes Amin kann entschützt und anschließend alkyliert werden.

Einige der verwendeten Amine sind kommerziell erhältlich oder können durch literaturbekannte Verfahren hergestellt werden.

Andere der verwendeten primären Amine wurden hergestellt wie im folgenden Schema beispielhaft skizziert ist.

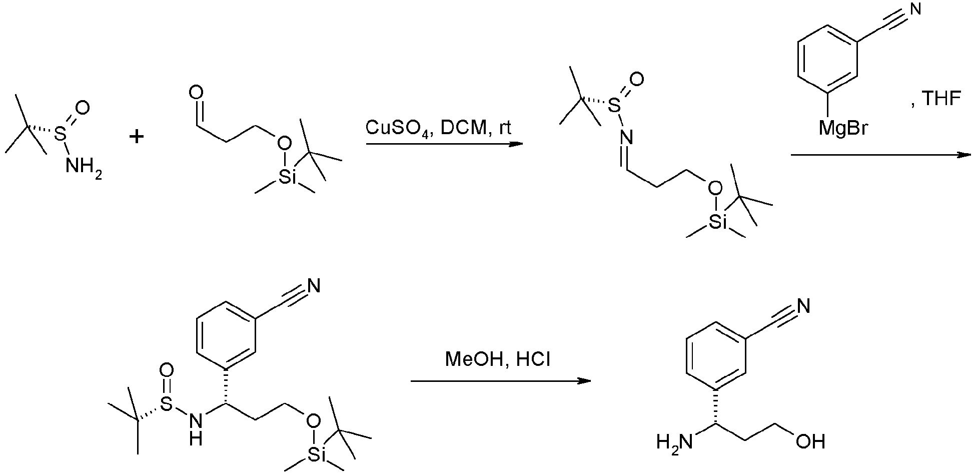

Die nachfolgend aufgeführten Beispiele dienen zur Erläuterung der Erfindung ohne diese jedoch einzuschränken.

**Tabelle 1:**

| Beispiel | CHEMISTRY | Methode | Retentions zeit | Molgewicht (g/mol) | Name |
|---|---|---|---|---|---|
| 1 | 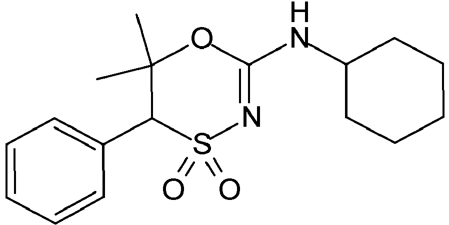 | B | 0,942 | 336,4 | Cyclohexyl-(6,6-dimethyl-4,4-dioxo-5-phenyl-5,6-dihydro-4H-4lambda6-[1,4,3]oxathiazin-2-yl)-amin |
| 2 | 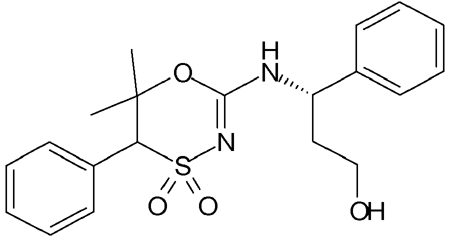 | B | 0,821 | 388,5 | (S)-3-(6,6-Dimethyl-4,4-dioxo-5-phenyl-5,6-dihydro-4H-4lambda6-[1,4,3]oxathiazin-2-ylamino)-3-phenyl-propan-1-ol |
| 3 | 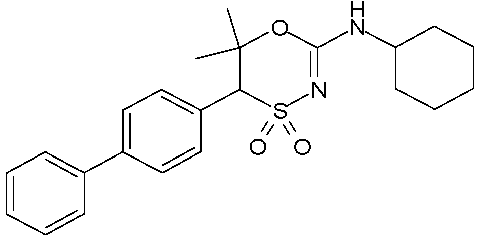 | A | 1,151 | 412,5 | (5-Biphenyl-4-yl-6,6-dimethyl-4,4-dioxo-5,6-dihydro-4H-4lambda6-[1,4,3]oxathiazin-2-yl)-cyclohexyl-amin |
| 4 | 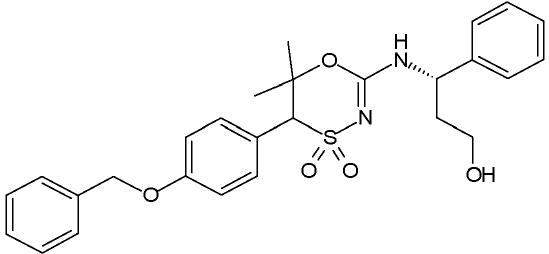 | B | 0,976 | 494,6 | (S)-3-[5-(4-Benzyloxy-phenyl)-6,6-dimethyl-4,4-dioxo-5,6-dihydro-4H-4lambda6-[1,4,3]oxathiazin-2-ylamino]-3-phenyl-propan-1-ol |
| 5 | 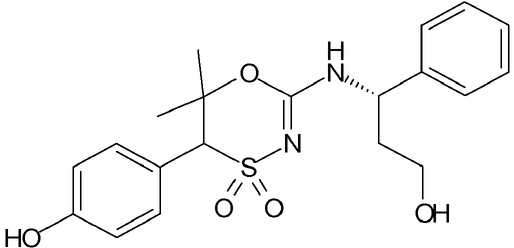 | B | 0,719 | 404,5 | 4-[2-((S)-3-Hydroxy-1-phenyl-propylamino)-6,6-dimethyl-4,4-dioxo-5,6-dihydro-4H-4lambda6-[1,4,3]oxathiazin-5-yl]-phenol |
| 6 | 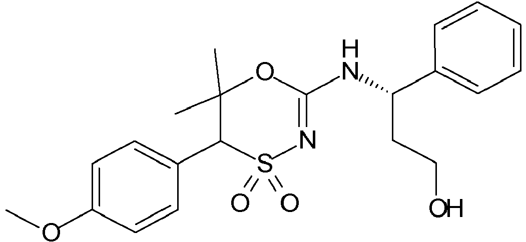 | B | 0,829 | 418,5 | (S)-3-[5-(4-Methoxy-phenyl)-6,6-dimethyl-4,4-dioxo-5,6-dihydro-4H-4lambda6-[1,4,3]oxathiazin-2-ylamino]-3-phenyl-propan-1-ol |
| 7 | 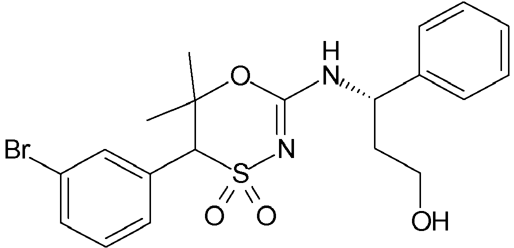 | B | 0,898 | 467,4 | (S)-3-[5-(3-Brom-phenyl)-6,6-d imethyl-4,4-dioxo-5,6-dihydro-4H-4lambda6-[1,4,3]oxathiazin-2-ylamino]-3-phenyl-propan-1-ol |
| 8 | 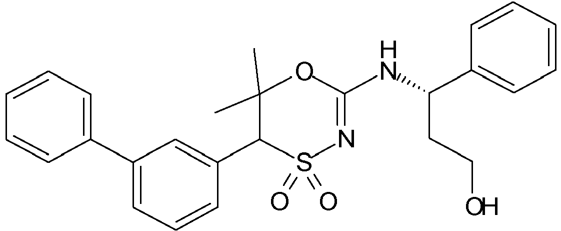 | A | 1,038 | 464,6 | (S)-3-(5-Biphenyl-3-yl-6,6-dimethyl-4,4-dioxo-5,6-dihydro-4H-4lambda6-[1,4,3]oxathiazin-2-ylamino)-3-phenyl-propan-1-ol |
| 9 | 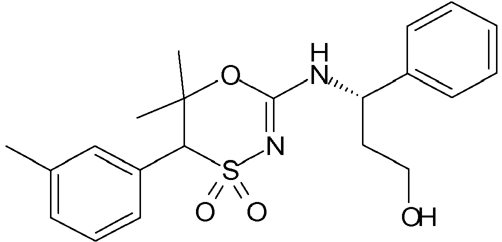 | A | 0,942 | 402,5 | (S)-3-(6,6-Dimethyl-4,4-dioxo-5-m-tolyl-5,6-dihydro-4H-4lambda6-[1,4,3]oxathiazin-2-ylamino)-3-phenyl-propan-1-ol |
| 10 | 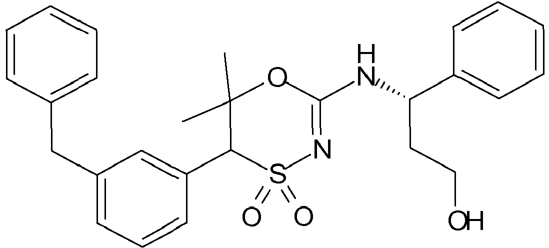 | A | 1,063 | 478,6 | (S)-3-[5-(3-Benzyl-phenyl)-6,6-dimethyl-4,4-dioxo-5,6-dihydro-4H-4lambda6-[1,4,3]oxathiazin-2-ylamino]-3-phenyl-propan-1-ol |
| 11 | 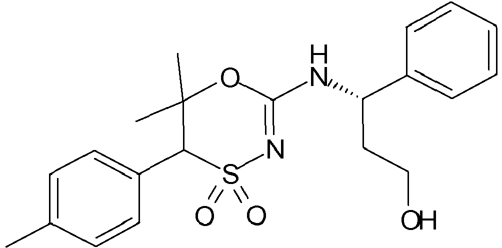 | A | 0,948 | 402,5 | (S)-3-(6,6-Dimethyl-4,4-dioxo-5-p-tolyl-5,6-dihydro-4H-4lambda6-[1,4,3]oxathiazin-2-ylamino)-3-phenyl-propan-1-ol |
| 12 | 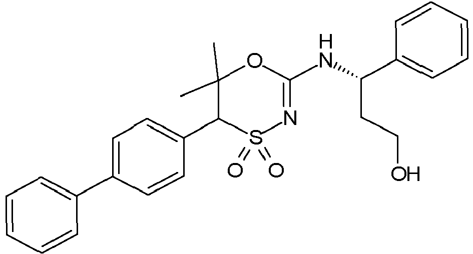 | B | 0,973 | 464,6 | (S)-3-(5-Biphenyl-4-yl-6,6-dimethyl-4,4-dioxo-5,6-dihydro-4H-4lambda6-[1,4,3]oxathiazin-2-ylamino)-3-phenyl-propan-1-ol |
| 13 | 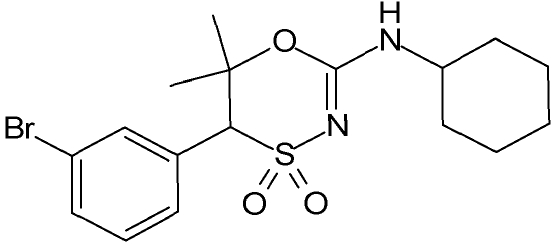 | B | 0,986 | 415,3 | [5-(3-Brom-phenyl)-6,6-dimethyl-4,4-dioxo-5,6-dihydro-4H-4lambda6-[1,4,3]oxathiazin-2-yl]-cyclohexyl-amin |
| 14 | 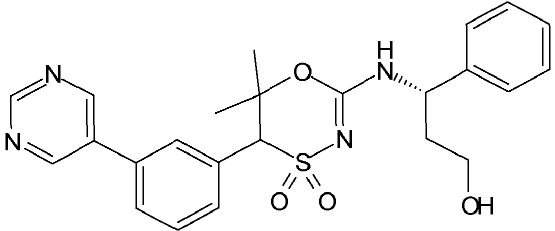 | B | 0,732 | 466,6 | (S)-3-[6,6-Dimethyl-4,4-dioxo-5-(3-pyrimidin-5-yl-phenyl)-5,6-dihydro-4H-4lambda6-[1,4,3]oxathiazin-2-ylamino]-3-phenyl-propan-1-ol |
| 15 | 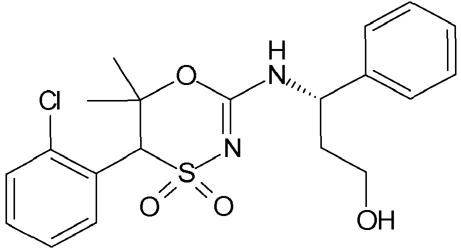 | B | 0,852 | 422,9 | (S)-3-[5-(2-Chlor-phenyl)-6,6-dimethyl-4,4-dioxo-5,6-dihydro-4H-4lambda6-[1,4,3]oxathiazin-2-ylamino]-3-phenyl-propan-1-ol |
| 16 | 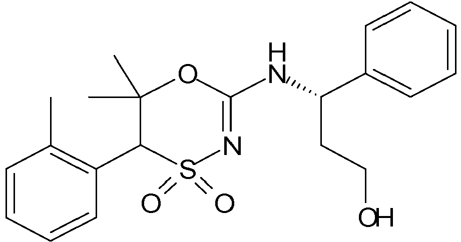 | B | 0,843 | 402,5 | (S)-3-(6,6-Dimethyl-4,4-dioxo-5-o-tolyl-5,6-dihydro-4H-4lambda6-[1,4,3]oxathiazin-2-ylamino)-3-phenyl-propan-1-ol |
| 17 | 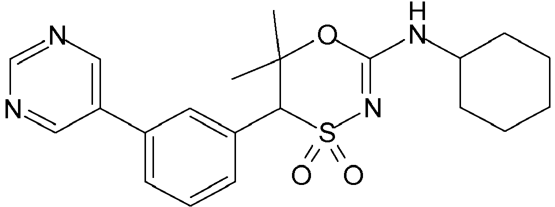 | B | 0,84 | 414,5 | Cyclohexyl-[6,6-dimethyl-4,4-dioxo-5-(3-pyrimidin-5-yl-phenyl)-5,6-dihydro-4H-4lambda6-[1,4,3]oxathiazin-2-yl]-amin |
| 18 | 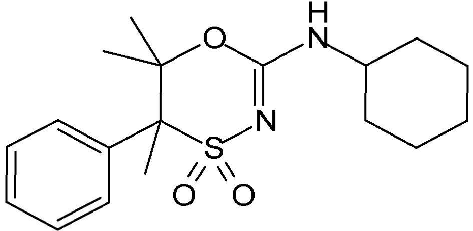 | B | 0,938 | 350,5 | Cyclohexyl-(5,6,6-trimethyl-4,4-dioxo-5-phenyl-5,6-dihydro-4H-4lambda6-[1,4,3]oxathiazin-2-yl)-amin |
| 19 | 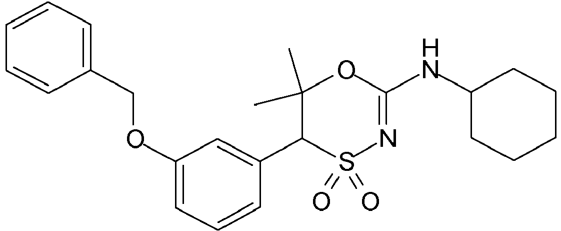 | B | 1,095 | 442,6 | [5-(3-Benzyloxy-phenyl)-6,6-dimethyl-4,4-dioxo-5,6-dihydro-4H-4lambda6-[1,4,3]oxathiazin-2-yl]-cyclohexyl-amin |
| 20 | 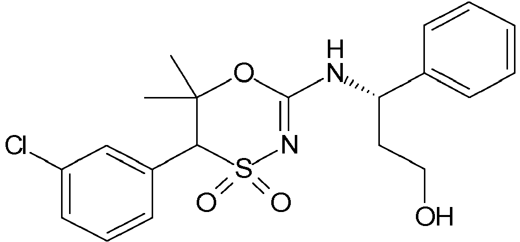 | B | 0,877 | 422,9 | (S)-3-[5-(3-Chlor-phenyl)-6,6-dimethyl-4,4-dioxo-5,6-dihydro-4H-4lambda6-[1,4,3]oxathiazin-2-ylamino]-3-phenyl-propan-1-ol |
| 21 | 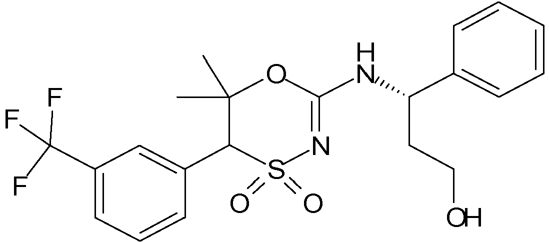 | B | 0,908 | 456,5 | (S)-3-[6,6-Dimethyl-4,4-dioxo-5-(3-trifluormethyl-phenyl)-5,6-dihydro-4H-4lambda6-[1,4,3]oxathiazin-2-ylamino]-3-phenyl-propan-1-ol |
| 22 | 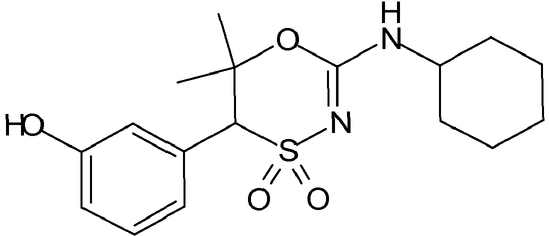 | B | 0,846 | 352,4 | 3-(2-Cyclohexylamino-6,6-dimethyl-4,4-dioxo-5,6-dihydro-4H-4lambda6-[1,4,3]oxathiazin-5-yl)-phenol |
| 23 | 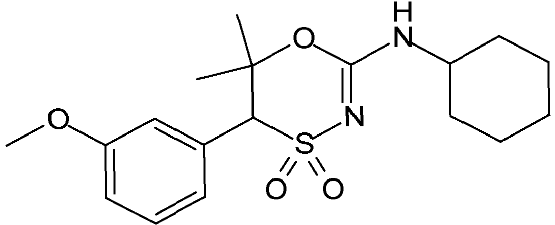 | B | 0,935 | 366,5 | Cyclohexyl-[5-(3-methoxy-phenyl)-6,6-dimethyl-4,4-dioxo-5,6-dihydro-4H-4lambda6-[1,4,3]oxathiazin-2-yl]-amin |
| 24 | 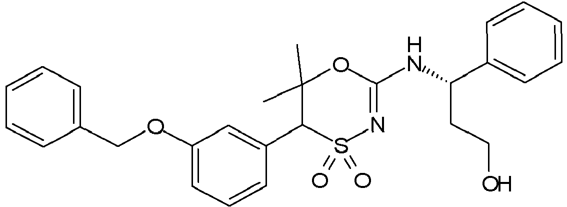 | B | 0,958 | 494,6 | (S)-3-[5-(3-Benzyloxy-phenyl)-6,6-dimethyl-4,4-dioxo-5,6-dihydro-4H-4lambda6-[1,4,3]oxathiazin-2-ylamino]-3-phenyl-propan-1-ol |
| 25 | 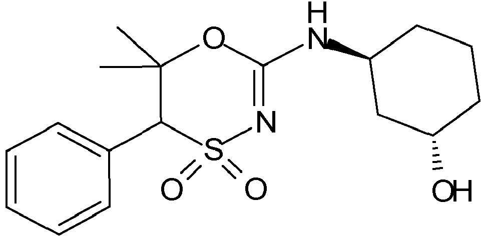 | A | 0,883 | 352,4 | (1S,3S)-3-(6,6-Dimethyl-4,4-dioxo-5-phenyl-5,6-dihydro-4H-4lambda6-[1,4,3]oxathiazin-2-ylamino)-AyAlohexanol |
| 26 | 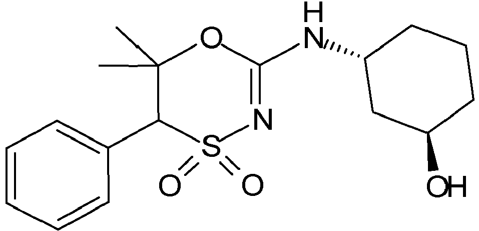 | A | 0,877 | 352,4 | (1R,3R)-3-(6,6-Dimethyl-4,4-dioxo-5-phenyl-5,6-dihydro-4H-4lambda6-[1,4,3]oxathiazin-2-ylamino)-cyclohexanol |
| 27 | 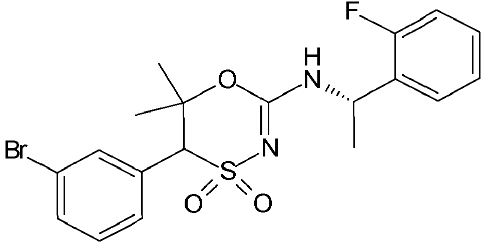 | B | 0,988 | 455,3 | [5-(3-Brom-phenyl)-6,6-dimethyl-4,4-dioxo-5,6-dihydro-4H-4lambda6-[1,4,3]oxathiazin-2-yl]-[(S)-1-(2-fluor-phenyl)-ethyl]-amin |
| 28 | 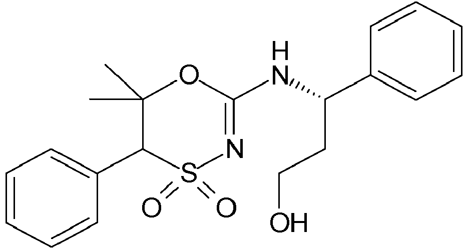 | A | 0,965 | 388,5 | (S)-3-(6,6-Dimethyl-4,4-dioxo-5-phenyl-5,6-dihydro-4H-4lambda6-[1,4,3]oxathiazin-2-ylamino)-3-phenyl-propan-1-ol* |
| 29 | 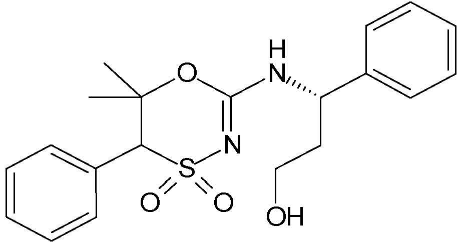 | A | 0,963 | 388,5 | (S)-3-(6,6-Dimethyl-4,4-dioxo-5-phenyl-5,6-dihydro-4H-4lambda6-[1,4,3]oxathiazin-2-ylamino)-3-phenyl-propan-1-ol* |
| 30 | 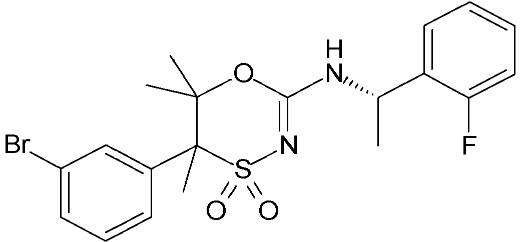 | B | 1,002 | 469,4 | [5-(3-Brom-phenyl)-5,6,6-trimethyl-4,4-dioxo-5,6-dihydro-4H-4lambda6-[1,4,3]oxathiazin-2-yl]-[(S)-1-(2-fluor-phenyl)-ethyl]-amin |
| 31 | 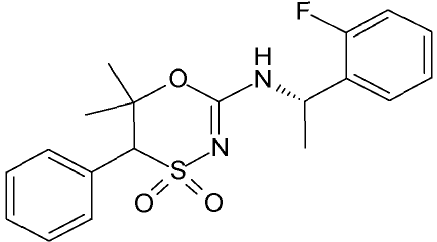 | B | 0,939 | 376,4 | (6,6-Dimethyl-4,4-dioxo-5-phenyl-5,6-dihydro-4H-4lambda6-[1,4,3]oxathiazin-2-yl)-[(S)-1-(2-fluor-phenyl)-ethyl]-amin |
| 32 | 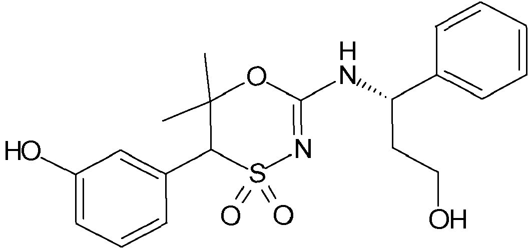 | B | 0,729 | 404,5 | 3-[2-((S)-3-Hydroxy-1-phenyl-propylamino)-6,6-dimethyl-4,4-dioxo-5,6-dihydro-4H-4lambda6-[1,4,3]oxathiazin-5-yl]-phenol |
| 33 | 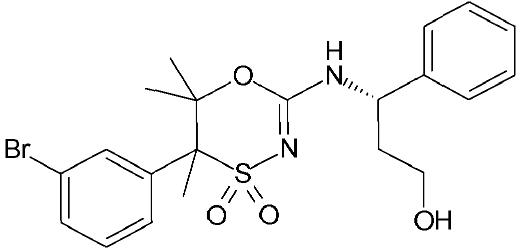 | B | 0,884 | 481,4 | (S)-3-[5-(3-Brom-phenyl)-5,6,6-trimethyl-4,4-dioxo-5,6-dihydro-4H-4lambda6-[1,4,3]oxathiazin-2-ylamino]-3-phenyl-propan-1-ol |
| 34 | 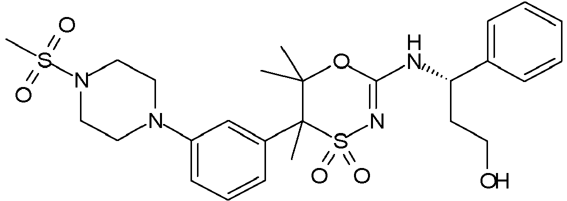 | B | 0,793 | 564,7 | (S)-3-{5-[3-(4-Methansulfonyl-piperazin-1-yl)-phenyl]-5,6,6-trimethyl-4,4-dioxo-5,6-dihydro-4H-4lambda6-[1,4,3]oxathiazin-2-ylamino}-3-phenyl-propan-1-ol |
| 35 | 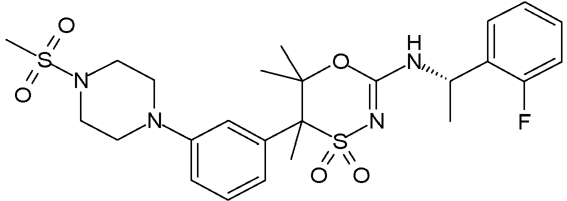 | B | 0,907 | 552,7 | [(S)-1-(2-Fluor-phenyl)-ethyl]-{5-[3-(4-methansulfonyl-piperazin-1-yl)-phenyl]-5,6,6-trimethyl-4,4-dioxo-5,6-dihydro-4H-4lambda6-[1,4,3]oxathiazin-2-yl}-amin |
| 36 | 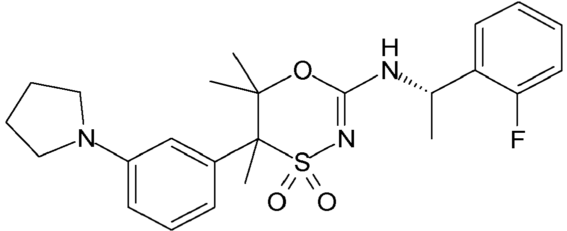 | B | 0,939 | 459,6 | [(S)-1-(2-Fluor-phenyl)-ethyl]-[5,6,6-trimethyl-4,4-dioxo-5-(3-pyrrolidin-1-yl-phenyl)-5,6-dihydro-4H-4lambda6-[1,4,3]oxathiazin-2-yl]-amin |
| 37 | 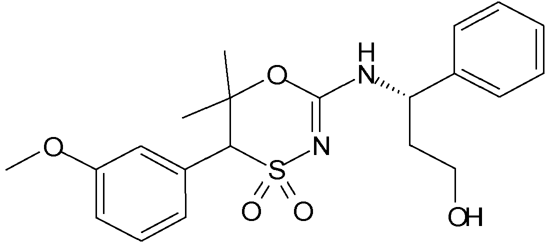 | B | 0,832 | 418,5 | (S)-3-[5-(3-Methoxy-phenyl)-6,6-dimethyl-4,4-dioxo-5,6-dihydro-4H-4lambda6-[1,4,3]oxathiazin-2-ylamino]-3-phenyl-propan-1-ol |
| 38 | 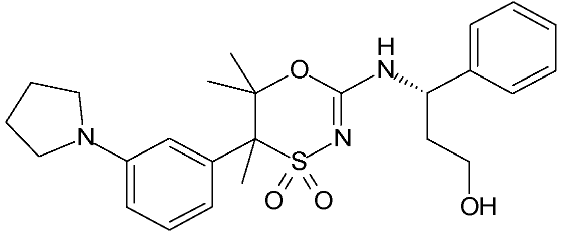 | B | 0,756 | 471,6 | (S)-3-Phenyl-3-[5,6,6-trimethyl-4,4-dioxo-5-(3-pyrrolidin-1-yl-phenyl)-5,6-dihydro-4H-4lambda6-[1,4,3]oxathiazin-2-ylamino]-propan-1-ol * |
| 39 | 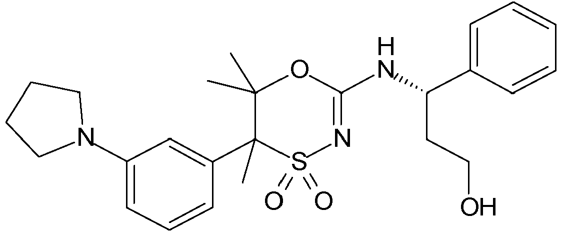 | B | 0,782 | 471,6 | (S)-3-Phenyl-3-[5,6,6-trimethyl-4,4-dioxo-5-(3-pyrrolidin-1-yl-phenyl)-5,6-dihydro-4H-4lambda6-[1,4,3]oxathiazin-2-ylamino]-propan-1-ol * |
| 40 | 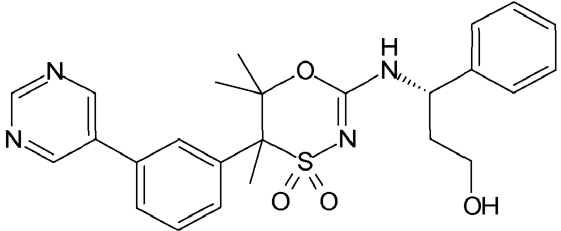 | B | 0,729 | 480,6 | (S)-3-Phenyl-3-[5,6,6-trimethyl-4,4-dioxo-5-(3-pyrimidin-5-yl-phenyl)-5,6-dihydro-4H-4lambda6-[1,4,3]oxathiazin-2-ylamino]-propan-1 -ol |
| 41 | 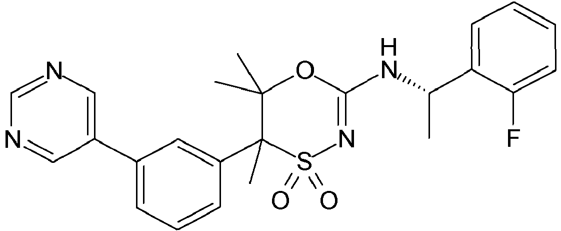 | B | 0,845 | 468,5 | [(S)-1-(2-Fluor-phenyl)-ethyl]-[5,6,6-trimethyl-4,4-dioxo-5-(3-pyrimidin-5-yl-phenyl)-5,6-dihydro-4H-4lambda6-[1,4,3]oxathiazin-2-yl]-amin |
| 42 | 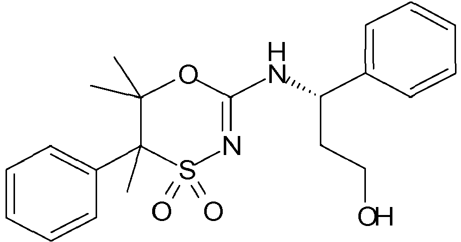 | B | 0,816 | 402,5 | (S)-3-Phenyl-3-(5,6,6-trimethyl-4,4-dioxo-5-phenyl-5,6-dihydro-4H-4lambda6-[1,4,3]oxathiazin-2-ylamino)-propan-1-ol |
| 43 | 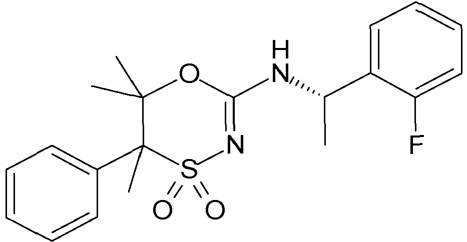 | B | 0,94 | 390,5 | [(S)-1-(2-Fluor-phenyl)-ethyl]-(5,6,6-trimethyl-4,4-dioxo-5-phenyl-5,6-dihydro-4H-4lambda6-[1,4,3]oxathiazin-2-yl)-amin |
| 44 | 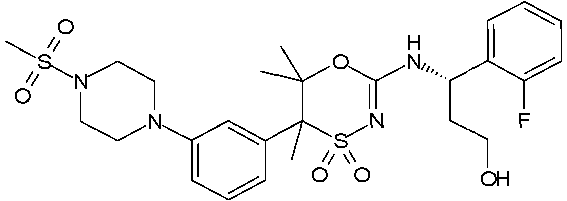 | B | 0,806 | 582,7 | (S)-3-(2-Fluor-phenyl)-3-{5-[3-(4-methansulfonyl-piperazin-1-yl)-phenyl]-5,6,6-trimethyl-4,4-dioxo-5,6-dihydro-4H-4lambda6-[1,4,3]oxathiazin-2-ylamino}-propan-1-ol |
| 45 | 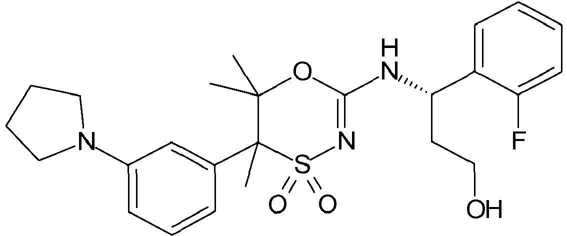 | B | 0,798 | 489,6 | (S)-3-(2-Fluor-phenyl)-3-[5,6,6-trimethyl-4,4-dioxo-5-(3-pyrrolidin-1-yl-phenyl)-5,6-dihydro-4H-4lambda6-[1,4,3]oxathiazin-2-ylamino]-propan-1-ol |
| 46 | 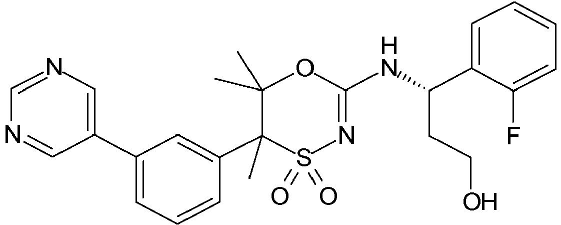 | B | 0,732 | 498,6 | (S)-3-(2-Fluor-phenyl)-3-[5,6,6-trimethyl-4,4-dioxo-5-(3-pyrimidin-5-yl-phenyl)-5,6-dihydro-4H-4lambda6-[1,4,3]oxathiazin-2-ylamino]-propan-1-ol |
| 47 | 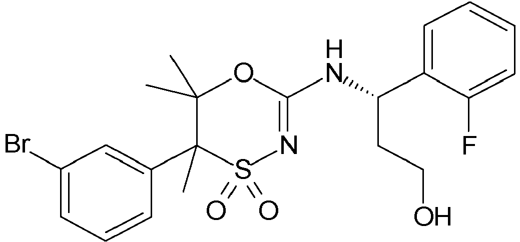 | B | 0,888 | 499,4 | (S)-3-[5-(3-Brom-phenyl)-5,6,6-trimethyl-4,4-dioxo-5,6-dihydro-4H-4lambda6-[1,4,3]oxathiazin-2-ylamino]-3-(2-fluor-phenyl)-propan-1-ol |
| 48 | 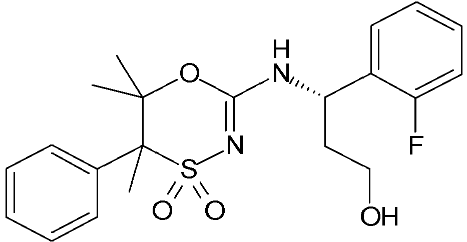 | B | 0,82 | 420,5 | (S)-3-(2-Fluor-phenyl)-3-(5,6,6-trimethyl-4,4-dioxo-5-phenyl-5,6-dihydro-4H-4lambda6-[1,4,3]oxathiazin-2-ylamino)-propan-1 -ol |
| 49 | 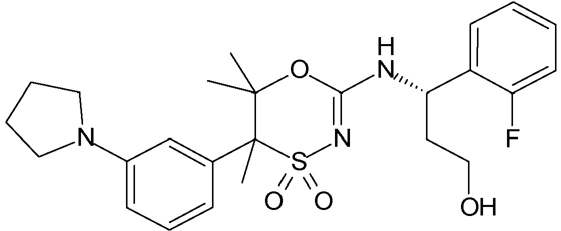 | B | 0,812 | 489,6 | (S)-3-(2-Fluor-phenyl)-3-[5,6,6-trimethyl-4,4-dioxo-5-(3-pyrrolidin-1-yl-phenyl)-5,6-dihydro-4H-4lambda6-[1,4,3]oxathiazin-2-ylamino]-propan-1-ol |
| 50 | 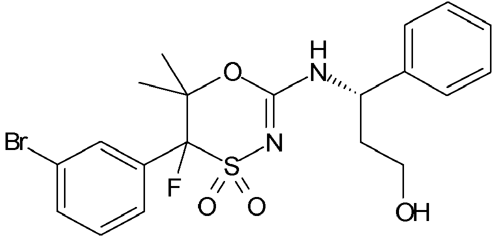 | B | 1.021 | 485,4 | (S)-3-[5-(3-Brom-phenyl)-5-fluor-6,6-dimethyl-4,4-dioxo-5,6-dihydro-4H-4lambda6-[1,4,3]oxathiazin-2-ylamino]-3-phenyl-propan-1-ol |
| 51 | 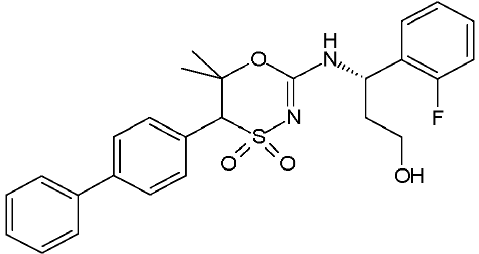 | B | 0.973 | 482,6 | (S)-3-(5-Biphenyl-4-yl-6,6-dimethyl-4,4-dioxo-5,6-dihydro-4H-4lambda6-[1,4,3]oxathiazin-2-ylamino)-3-(2-fluor-phenyl)-propan-1-ol |
| 52 | 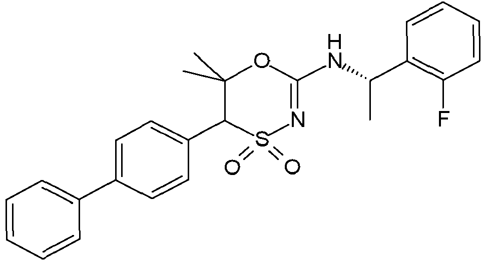 | B | 1.105 | 452,5 | (5-Biphenyl-4-yl-6,6-dimethyl-4,4-dioxo-5,6-dihydro-4H-4lambda6-[1,4,3]oxathiazin-2-yl)-[(S)-1-(2-fluor-phenyl)-ethyl]-amin |
| 53 | 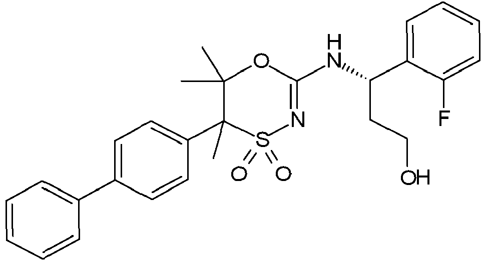 | B | 0.960 | 496,6 | (S)-3-(5-Biphenyl-4-yl-5,6,6-trimethyl-4,4-dioxo-5,6-dihydro-4H-4lambda6-[1,4,3]oxathiazin-2-ylamino)-3-(2-fluor-phenyl)-propan-1-ol |
| 54 | 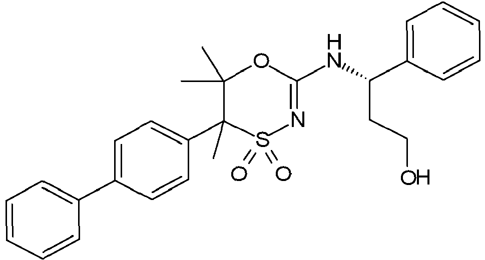 | B | 1.003 | 478,6 | (S)-3-(5-Biphenyl-4-yl-5,6,6-trimethyl-4,4-dioxo-5,6-dihydro-4H-4lambda6-[1,4,3]oxathiazin-2-ylamino)-3-phenyl-propan-1-ol |
| 55 | 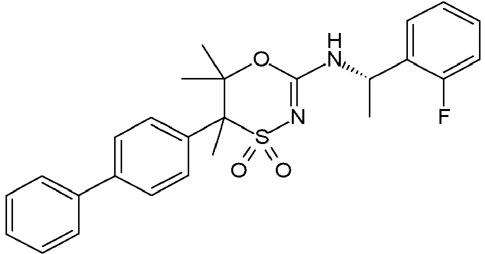 | B | 1.131 | 466,6 | (5-Biphenyl-4-yl-5,6,6-trimethyl-4,4-dioxo-5,6-dihydro-4H-4lambda6-[1,4,3]oxathiazin-2-yl)-[(S)-1-(2-fluor-phenyl)-ethyl]-amin |
| 56 | 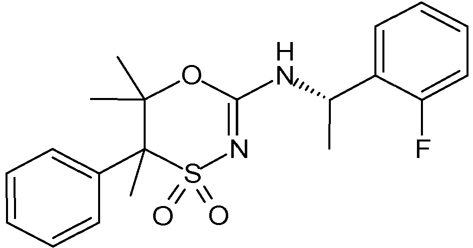 | B | 0.936 | 390,5 | [(S)-1-(2-Fluor-phenyl)-ethyl]-(5,6,6-trimethyl-4,4-dioxo-5-phenyl-5,6-dihydro-4H-4lambda6-[1,4,3]oxathiazin-2-yl)-amin * |
| 57 | 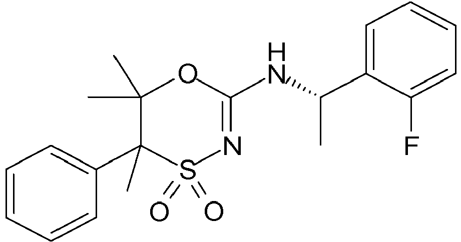 | B | 0.930 | 390,5 | [(S)-1-(2-Fluor-phenyl)-ethyl]-(5,6,6-trimethyl-4,4-dioxo-5-phenyl-5,6-dihydro-4H-4lambda6-[1,4,3]oxathiazin-2-yl)-amin * |
| 58 | 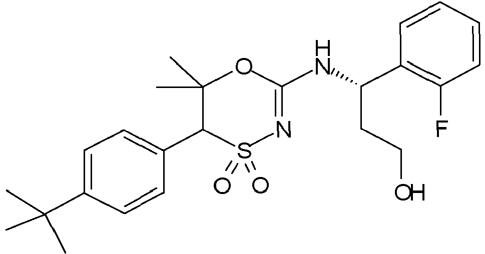 | B | 0.996 | 462,6 | (S)-3-[5-(4-tert-Butyl-phenyl)-6,6-dimethyl-4,4-dioxo-5,6-dihydro-4H-4lambda6-[1,4,3]oxathiazin-2-ylamino]-3-(2-fluor-phenyl)-propan-1-ol |
| 59 | 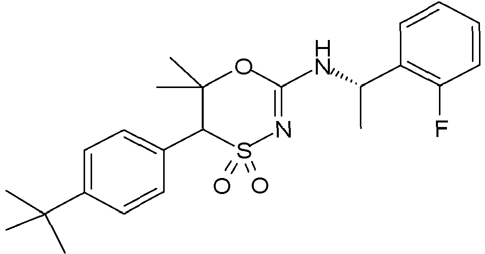 | B | 1.111 | 432,6 | [5-(4-tert-Butyl-phenyl)-6,6-dimethyl-4,4-dioxo-5,6-dihydro-4H-4lambda6-[1,4,3]oxathiazin-2-yl]-[(S)-1-(2-fluor-phenyl)-ethyl]-amin |
| 60 | 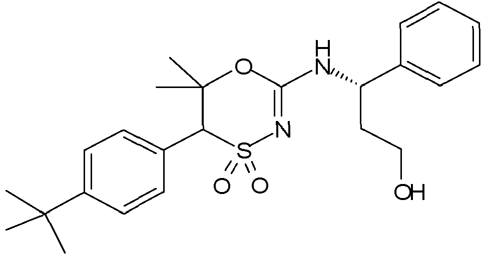 | B | 0.989 | 444,6 | (S)-3-[5-(4-tert-Butyl-phenyl)-6,6-dimethyl-4,4-dioxo-5,6-dihydro-4H-4lambda6-[1,4,3]oxathiazin-2-ylamino]-3-phenyl-propan-1-ol |
| 61 | 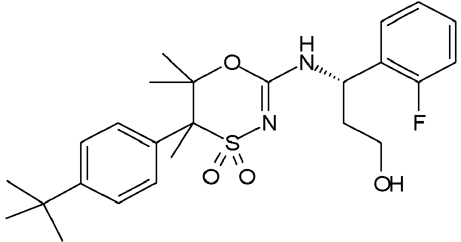 | B | 0.998 | 476,6 | (S)-3-[5-(4-tert-Butyl-phenyl)-5,6,6-trimethyl-4,4-dioxo-5,6-dihydro-4H-4lambda6-[1,4,3]oxathiazin-2-ylamino]-3-(2-fluor-phenyl)-propan-1-ol |
| 62 | 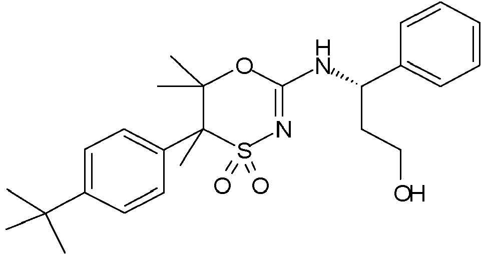 | B | 0.989 | 458,6 | (S)-3-[5-(4-tert-Butyl-phenyl)-5,6,6-trimethyl-4,4-dioxo-5,6-dihydro-4H-4lambda6-[1,4,3]oxathiazin-2-ylamino]-3-phenyl-propan-1-ol |
| 63 | 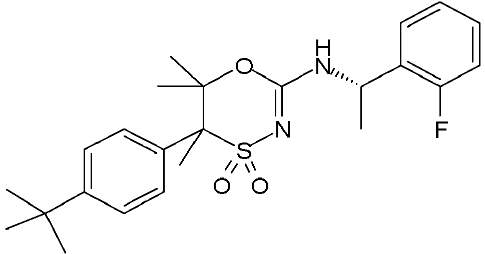 | B | 1.114 | 446,6 | [5-(4-tert-Butyl-phenyl)-5,6,6-trimethyl-4,4-dioxo-5,6-dihydro-4H-4lambda6-[1,4,3]oxathiazin-2-yl]-[(S)-1-(2-fluor-phenyl)-ethyl]-amin |
| 64 | 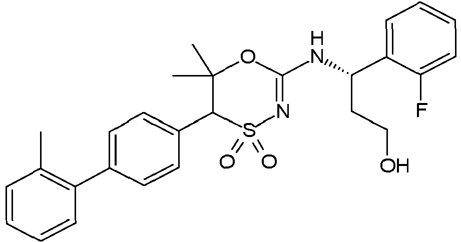 | C | 1.802 | 496,6 | (S)-3-[6,6-Dimethyl-5-(2'-methyl-biphenyl-4-yl)-4,4-dioxo-5,6-dihydro-4H-4lambda6-[1,4,3]oxathiazin-2-ylamino]-3-(2-fluor-phenyl)-propan-1-ol |
| 65 | 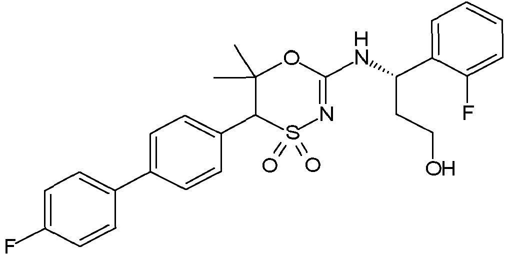 | C | 1.753 | 500,6 | (S)-3-[5-(4'-Fluor-biphenyl-4-yl)-6,6-dimethyl-4,4-dioxo-5,6-dihydro-4H-4lambda6-[1,4,3]oxathiazin-2-ylamino]-3-(2-fluor-phenyl)-propan-1-ol |
| 66 | 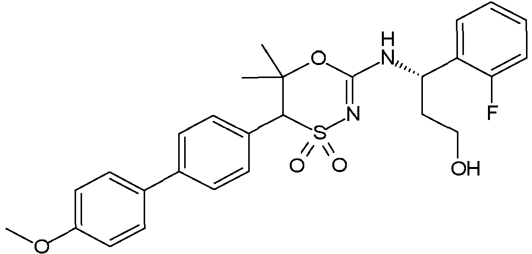 | C | 1.715 | 512,6 | (S)-3-(2-Fluor-phenyl)-3-[5-(4'-methoxy-biphenyl-4-yl)-6,6-dimethyl-4,4-dioxo-5,6-dihydro-4H-4lambda6-[1,4,3]oxathiazin-2-ylamino]-propan-1-ol |
| 67 | 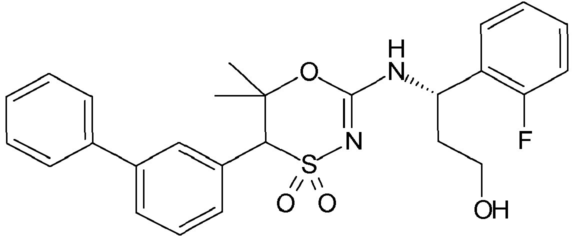 | B | 0.964 | 482,6 | (S)-3-(5-Biphenyl-3-yl-6,6-dimethyl-4,4-dioxo-5,6-dihydro-4H-4lambda6-[1,4,3]oxathiazin-2-ylamino)-3-(2-fluor-phenyl)-propan-1-ol |
| 68 | 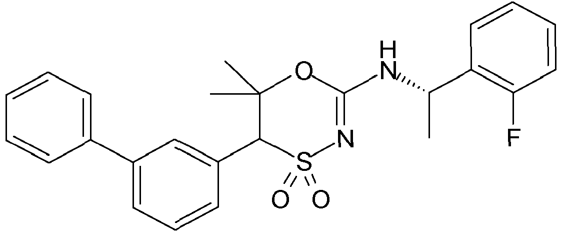 | B | 1.072 | 452,5 | (5-Biphenyl-3-yl-6,6-dimethyl-4,4-dioxo-5,6-dihydro-4H-4lambda6-[1,4,3]oxathiazin-2-yl)-[(S)-1-(2-fluor-phenyl)-ethyl]-amin |
| 69 | 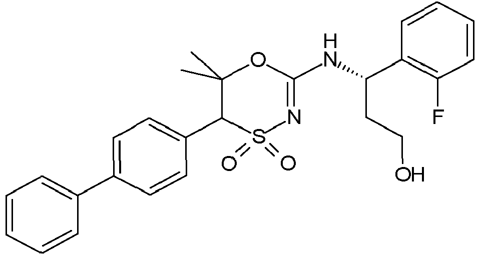 | B | 0.973 | 482,6 | (S)-3-(5-Biphenyl-4-yl-6,6-dimethyl-4,4-dioxo-5,6-dihydro-4H-4lambda6-[1,4,3]oxathiazin-2-ylamino)-3-(2-fluor-phenyl)-propan-1 -ol * |
| 70 | 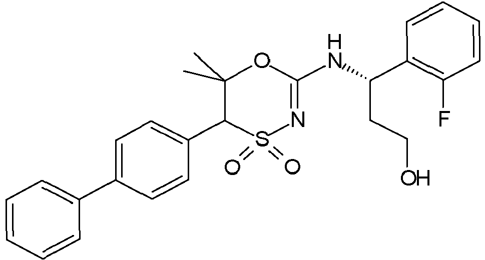 | B | 0.973 | 482,6 | (S)-3-(5-Biphenyl-4-yl-6,6-dimethyl-4,4-dioxo-5,6-dihydro-4H-4lambda6-[1,4,3]oxathiazin-2-ylamino)-3-(2-fluor-phenyl)-propan-1 -ol * |
| 71 | 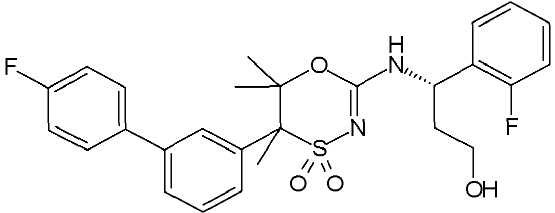 | B | 0.956 | 514,6 | (S)-3-[5-(4'-Fluor-biphenyl-3-yl)-5,6,6-trimethyl-4,4-dioxo-5,6-dihydro-4H-4lambda6-[1,4,3]oxathiazin-2-ylamino]-3-(2-fluor-phenyl)-propan-1-ol |
| 72 | 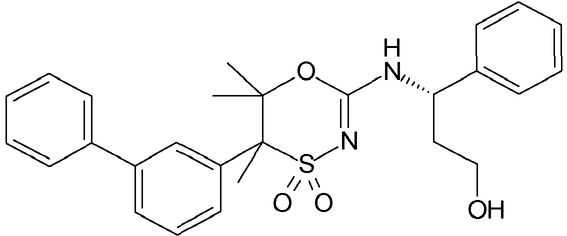 | B | 0.972 | 478,6 | (S)-3-(5-Biphenyl-3-yl-5,6,6-trimethyl-4,4-dioxo-5,6-dihydro-4H-4lambda6-[1,4,3]oxathiazin-2-ylamino)-3-phenyl-propan-1-ol |
| 73 | 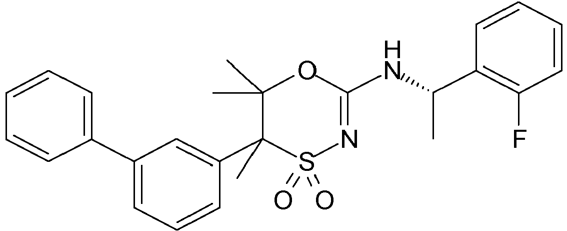 | B | 1.069 | 466,6 | (5-Biphenyl-3-yl-5,6,6-trimethyl-4,4-dioxo-5,6-dihydro-4H-4lambda6-[1,4,3]oxathiazin-2-yl)-[(S)-1-(2-fluor-phenyl)-ethyl]-amin |
| 74 | 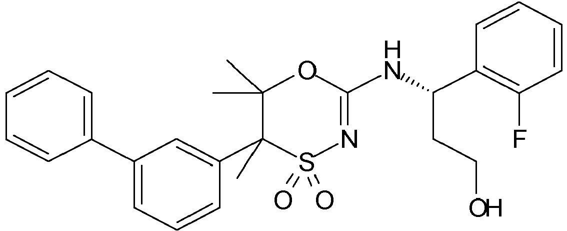 | B | 0.967 | 496,6 | (S)-3-(5-Biphenyl-3-yl-5,6,6-trimethyl-4,4-dioxo-5,6-dihydro-4H-4lambda6-[1,4,3]oxathiazin-2-ylamino)-3-(2-fluor-phenyl)-propan-1-ol |
| 75 | 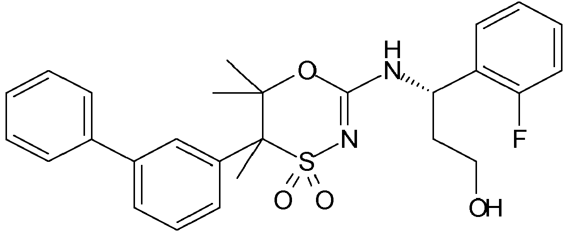 | B | 0.962 | 496,6 | (S)-3-(5-Biphenyl-3-yl-5,6,6-trimethyl-4,4-dioxo-5,6-dihydro-4H-4lambda6-[1,4,3]oxathiazin-2-ylamino)-3-(2-fluor-phenyl)-propan-1-ol |
| 76 | 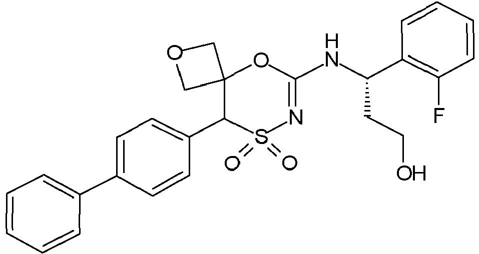 | B | 0.935 | 496,6 | (S)-3-(9-Biphenyl-4-yl-8,8-dioxo-2,5-dioxa-8lambda6-thia-7-aza-spiro[3.5]non-6-en-6-ylamino)-3-(2-fluor-phenyl)-propan-1-ol |
| 77 | 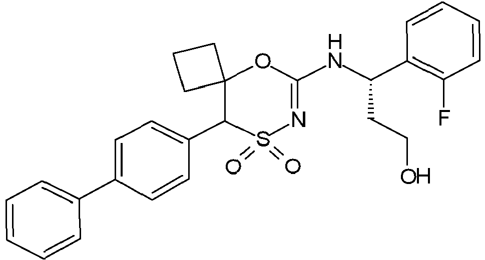 | B | 1.009 | 494,6 | (S) -3-(9-Biphenyl-4-yl-8,8-dioxo-5-oxa-8lambda6-thia-7-aza-spiro[3.5]non-6-en-6-ylamino)-3-(2-fluor-phenyl)-propan-1-ol |
| 78 | 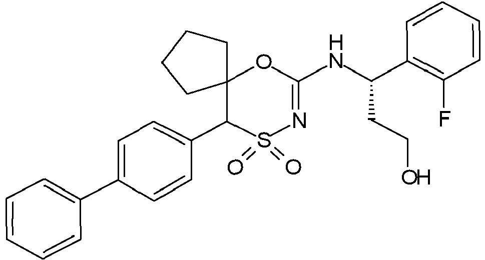 | C | 1.796 | 508,6 | (S)-3-(10-Biphenyl-4-yl-9,9-dioxo-6-oxa-9lambda6-thia-8-aza-spiro[4.5]dec-7-en-7-ylamino)-3-(2-fluor-phenyl)-propan-1-ol |
| 79 | 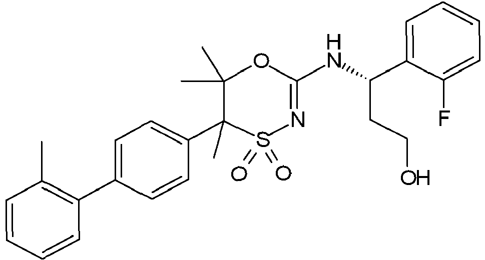 | B | 1.028 | 510,6 | (S)-3-(2-Fluor-phenyl)-3-[5,6,6-trimethyl-5-(2'-methyl-biphenyl-4-yl)-4,4-dioxo-5,6-dihydro-4H-4lambda6-[1,4,3]oxathiazin-2-ylamino]-propan-1-ol |
| 80 | 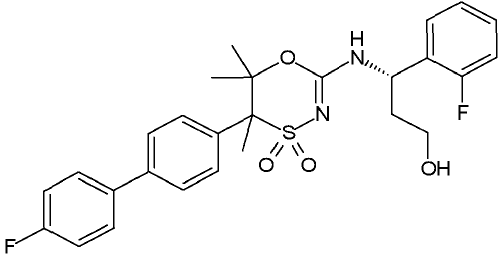 | B | 1.007 | 514,6 | (S)-3-[5-(4'-Fluor-biphenyl-4-yl)-5,6,6-trimethyl-4,4-dioxo-5,6-dihydro-4H-4lambda6-[1,4,3]oxathiazin-2-ylamino]-3-(2-fluor-phenyl)-propan-1-ol |
| 81 | 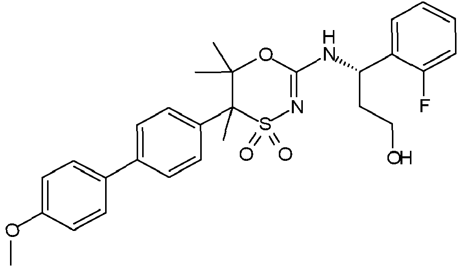 | B | 0.988 | 526,6 | (S)-3-(2-Fluor-phenyl)-3-[5-(4'-methoxy-biphenyl-4-yl)-5,6,6-trimethyl-4,4-dioxo-5,6-dihydro-4H-4lambda6-[1,4,3]oxathiazin-2-ylamino]-propan-1-ol |
| 82 | 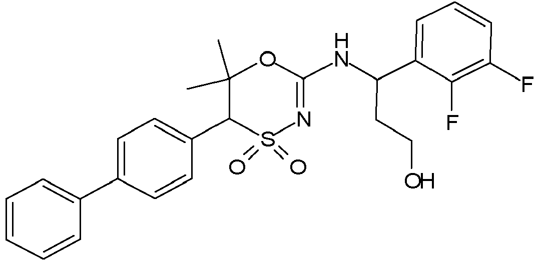 | B | 1.035 | 500,6 | 3-(5-Biphenyl-4-yl-6,6-dimethyl-4,4-dioxo-5,6-dihydro-4H-4lambda6-[1,4,3]oxathiazin-2-ylamino)-3-(2,3-difluor-phenyl)-propan-1-ol |
| 83 | 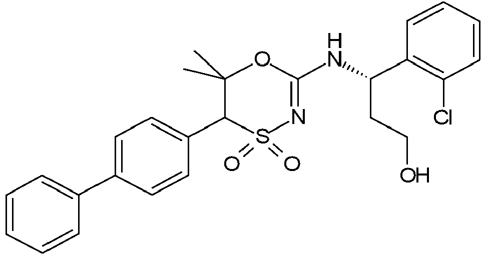 | B | 1.058 | 499,0 | (S)-3-(5-Biphenyl-4-yl-6,6-dimethyl-4,4-dioxo-5,6-dihydro-4H-4lambda6-[1,4,3]oxathiazin-2-ylamino)-3-(2-chloro-phenyl)-propan-1-ol |
| 84 | 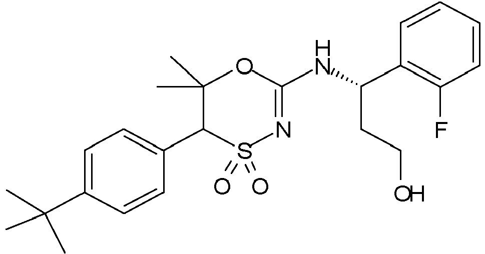 | B | 0.996 | 462,6 | (S)-3-[5-(4-tert-Butyl-phenyl)-6,6-dimethyl-4,4-dioxo-5,6-dihydro-4H-4lambda6-[1,4,3]oxathiazin-2-ylamino]-3-(2-fluor-phenyl)-propan-1-ol * |
| 85 | 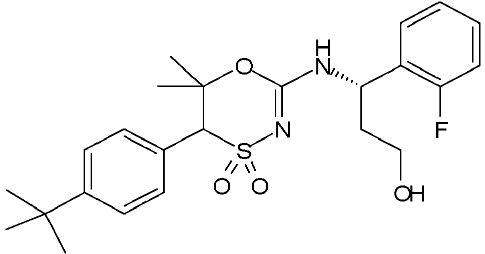 | B | 0.996 | 462,6 | (S)-3-[5-(4-tert-Butyl-phenyl)-6,6-dimethyl-4,4-dioxo-5,6-dihydro-4H-4lambda6-[1,4,3]oxathiazin-2-ylamino]-3-(2-fluor-phenyl)-propan-1-ol * |
| 86 | 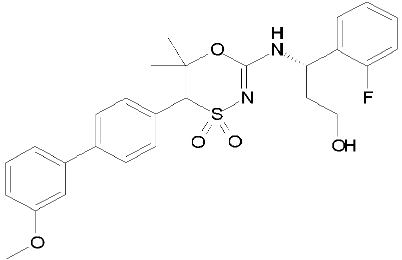 | B | 0.970 | 512,6 | (S)-3-(2-Fluor-phenyl)-3-[5-(3'-methoxy-biphenyl-4-yl)-6,6-dimethyl-4,4-dioxo-5,6-dihydro-4H-4lambda6-[1,4,3]oxathiazin-2-ylamino]-propan-1-ol |
| 87 | 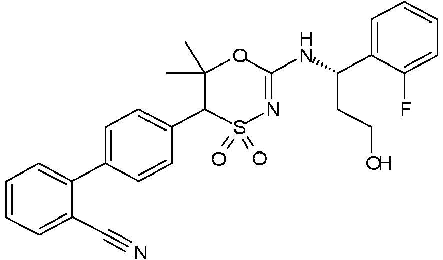 | B | 0.905 | 507,6 | 4'-{2-[(S)-1-(2-Fluor-phenyl)-3-hydroxy-propylamino]-6,6-dimethyl-4,4-dioxo-5,6-dihydro-4H-4lambda6-[1,4,3]oxathiazin-5-yl}-biphenyl-2-carbonitril |
| 88 | 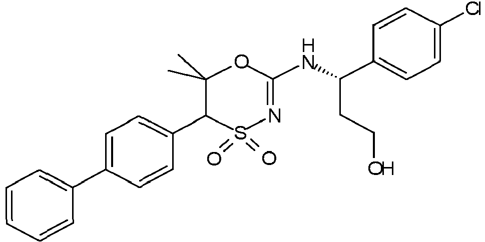 | A | 1,042 | 499,0 | (S)-3-(5-Biphenyl-4-yl-6,6-dimethyl-4,4-dioxo-5,6-dihydro-4H-4lambda6-[1,4,3]oxathiazin-2-ylamino)-3-(4-chloro-phenyl)-propan-1-ol |
| 89 | 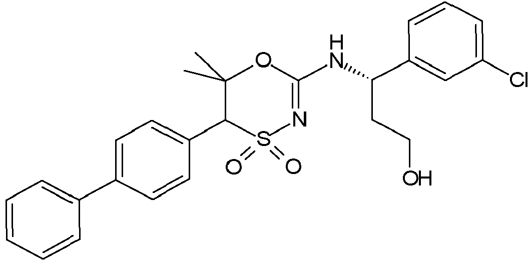 | A | 1,034 | 499,0 | (S)-3-(5-Biphenyl-4-yl-6,6-dimethyl-4,4-dioxo-5,6-dihydro-4H-4lambda6-[1,4,3]oxathiazin-2-ylamino)-3-(3-chloro-phenyl)-propan-1-ol |
| 90 | 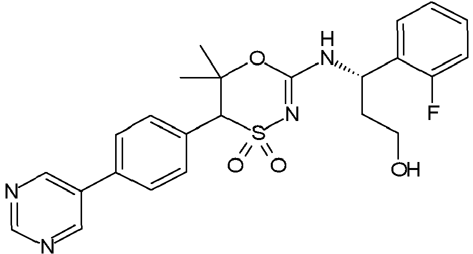 | B | 0.764 | 484,5 | (S)-3-[6,6-Dimethyl-4,4-dioxo-5-(4-pyrimidin-5-yl-phenyl)-5,6-dihydro-4H-4lambda6-[1,4,3]oxathiazin-2-ylamino]-3-(2-fluor-phenyl)-propan-1-ol |
| 91 | 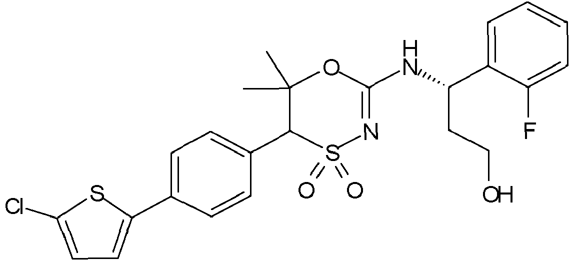 | B | 1.068 | 523,0 | (S)-3-{5-[4-(5-Chlor-thiophen-2-yl)-phenyl]-6,6-dimethyl-4,4-dioxo-5,6-dihydro-4H-4lambda6-[1,4,3]oxathiazin-2-ylamino}-3-(2-fluor-phenyl)-propan-1-ol |
| 92 | 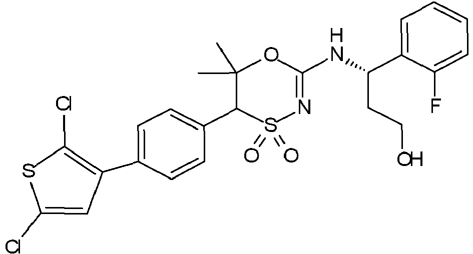 | B | 1.124 | 557,5 | (S)-3-{5-[4-(2,5-Dichlor-thiophen-3-yl)-phenyl]-6,6-dimethyl-4,4-dioxo-5,6-dihydro-4H-4lambda6-[1,4,3]oxathiazin-2-ylamino}-3-(2-fluor-phenyl)-propan-1-ol |
| 93 | 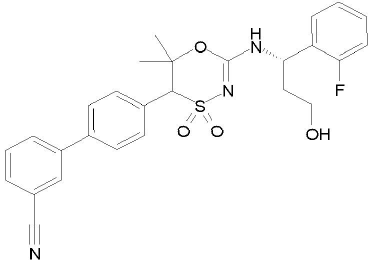 | C | 1.659 | 507,6 | 4'-{2-[(S)-1-(2-Fluor-phenyl)-3-hydroxy-propylamino]-6,6-dimethyl-4,4-dioxo-5,6-dihydro-4H-4lambda6-[1,4,3]oxathiazin-5-yl}-biphenyl-3-carbonitril |
| 94 | 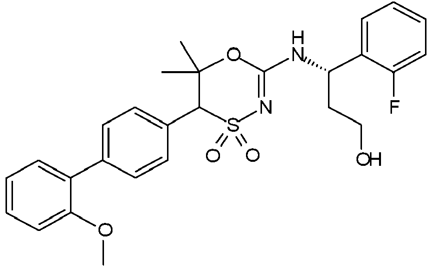 | B | 0.969 | 512,6 | (S)-3-(2-Fluor-phenyl)-3-[5-(2'-methoxy-biphenyl-4-yl)-6,6-dimethyl-4,4-dioxo-5,6-dihydro-4H-4lambda6-[1,4,3]oxathiazin-2-ylamino]-propan-1-ol |
| 95 | 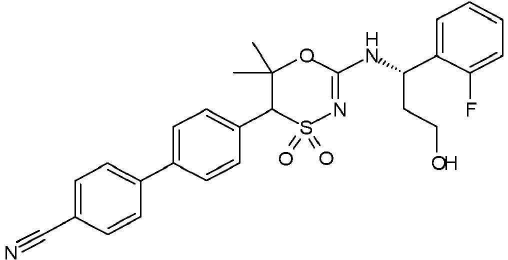 | B | 0.941 | 507,6 | 4'-{2-[(S)-1-(2-Fluor-phenyl)-3-hydroxy-propylamino]-6,6-dimethyl-4,4-dioxo-5,6-dihydro-4H-4lambda6-[1,4,3]oxathiazin-5-yl}-biphenyl-4-carbonitril |
| 96 | 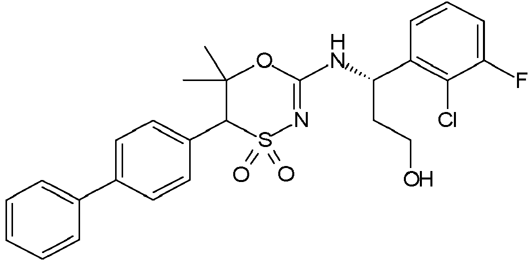 | A | 1,111 | 517,0 | (S)-3-(5-Biphenyl-4-yl-6,6-dimethyl-4,4-dioxo-5,6-dihydro-4H-4lambda6-[1,4,3]oxathiazin-2-ylamino)-3-(2-chlor-3-fluor-phenyl)-propan-1-ol |
| 97 | 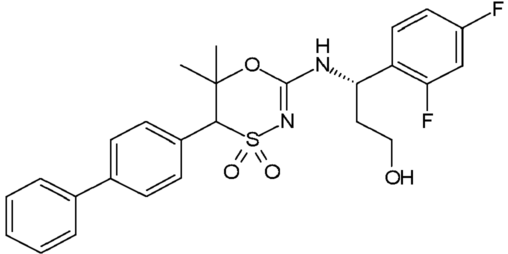 | A | 1,103 | 500,6 | (S)-3-(5-Biphenyl-4-yl-6,6-dimethyl-4,4-dioxo-5,6-dihydro-4H-4lambda6-[1,4,3]oxathiazin-2-ylamino)-3-(2,4-difluor-phenyl)-propan-1-ol |
| 98 | 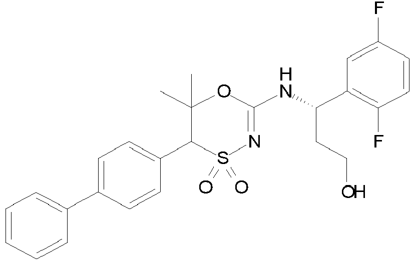 | A | 1,101 | 500,6 | (S)-3-(5-Biphenyl-4-yl-6,6-dimethyl-4,4-dioxo-5,6-dihydro-4H-4lambda6-[1,4,3]oxathiazin-2-ylamino)-3-(2,5-difluor-phenyl)-propan-1-ol |
| 99 | 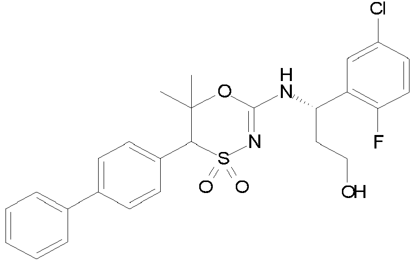 | A | 1,104 | 517,0 | (S)-3-(5-Biphenyl-4-yl-6,6-dimethyl-4,4-dioxo-5,6-dihydro-4H-4lambda6-[1,4,3]oxathiazin-2-ylamino)-3-(5-chlor-2-fluor-phenyl)-propan-1-ol |
| 99 | 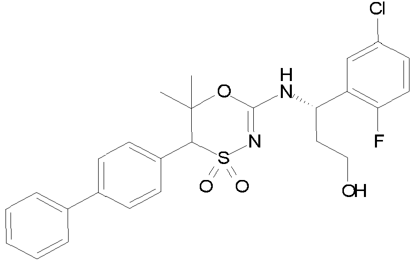 | A | 1.104 | 517.0 | (S)-3-(5-Biphenyl-4-yl-6,6-dimethyl-4,4-dioxo-5,6-dihydro-4H-4lambda6-[1,4,3]oxathiazin-2-ylamino)-3-(5-chlor-2-fluor-phenyl)-propan-1-ol |
| 100 | 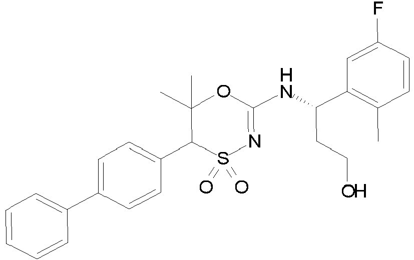 | A | 1.107 | 496.6 | (S)-3-(5-Biphenyl-4-yl-6,6-dimethyl-4,4-dioxo-5,6-dihydro-4H-4lambda6-[1,4,3]oxathiazin-2-ylamino)-3-(5-fluor-2-methyl-phenyl)-propan-1-ol |
| 101 | 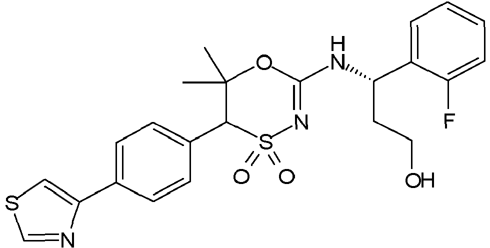 | B | 0.828 | 489.6 | (S)-3-[6,6-Dimethyl-4,4-dioxo-5-(4-thiazol-4-yl-phenyl)-5,6-dihydro-4H-4lambda6-[1,4,3]oxathiazin-2-ylamino]-3-(2-fluor-phenyl)-propan-1-ol |
| 102 | 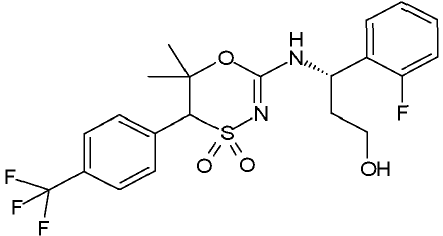 | B | 0.944 | 474.5 | (S)-3-[6,6-Dimethyl-4,4-dioxo-5-(4-trifluormethyl-phenyl)-5,6-dihydro-4H-4lambda6-[1,4,3]oxathiazin-2-ylamino]-3-(2-fluor-phenyl)-propan-1-ol |
| 103 | 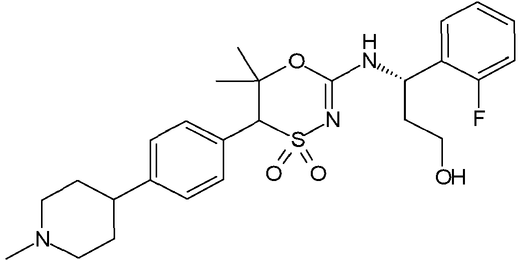 | B | 0.659 | 503.6 | (S)-3-{6,6-Dimethyl-5-[4-(1-methyl-piperidin-4-yl)-phenyl]-4,4-dioxo-5,6-dihydro-4H-4lambda6-[1,4,3]oxathiazin-2-ylamino}-3-(2-fluor-phenyl)-propan-1-ol |
| 104 | 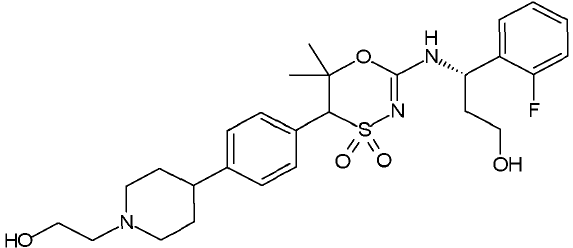 | B | 0.653 | 533.7 | (S)-3-(2-Fluor-phenyl)-3-(5-{4-[1-(2-hydroxy-ethyl)-piperidin-4-yl]-phenyl}-6,6-dimethyl-4,4-dioxo-5,6-dihydro-4H-4lambda6-[1,4,3]oxathiazin-2-ylamino)-propan-1-ol |
| 105 | 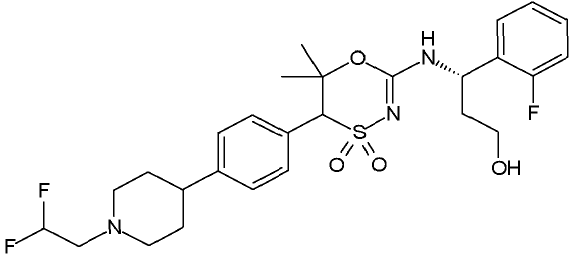 | B | 0.681 | 553.6 | (S)-3-(5-{4-[1-(2,2-Difluor-ethyl)-piperidin-4-yl]-phenyl}-6,6-dimethyl-4,4-dioxo-5,6-dihydro-4H-4lambda6-[1,4,3]oxathiazin-2-ylamino)-3-(2-fluor-phenyl)-propan-1-ol |
| 106 | 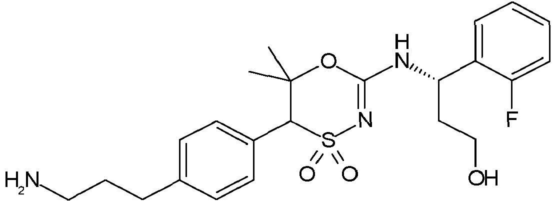 | B | 0.658 | 463.6 | (S)-3-{5-[4-(3-Amino-propyl)-phenyl]-6,6-dimethyl-4,4-dioxo-5,6-dihydro-4H-4Hlambda6-[1,4,3]oxathiazin-2-ylamino}-3-(2-fluor-phenyl)-propan-1-ol |
| 107 | 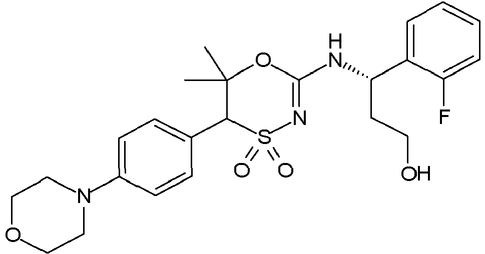 | B | 0.809 | 491.6 | (S)-3-[6,6-Dimethyl-5-(4-morpholin-4-yl-phenyl)-4,4-dioxo-5,6-dihydro-4H-4lambda6-[1,4,3]oxathiazin-2-ylamino]-3-(2-fluor-phenyl)-propan-1-ol |
| 108 | 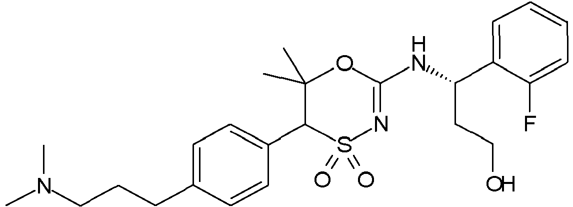 | B | 0.671 | 491.6 | (S)-3-{5-[4-(3-Dimethylamino-propyl)-phenyl]-6,6-dimethyl-4,4-dioxo-5,6-dihydro-4H-4lambda6-[1,4,3]oxathiazin-2-ylamino}-3-(2-fluor-phenyl)-propan-1-ol |
| 109 | 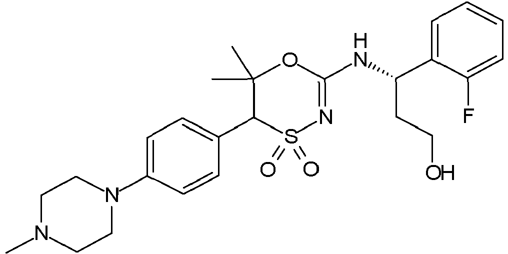 | C | 1.088 | 504.6 | (S)-3-{6,6-Dimethyl-5-[4-(4-methyl-piperazin-1-yl)-phenyl]-4,4-dioxo-5,6-dihydro-4H-41ambda6-[1,4,3]oxathiazin-2-ylamino}-3-(2-fluor-phenyl)-propan-1-ol |
| 110 | 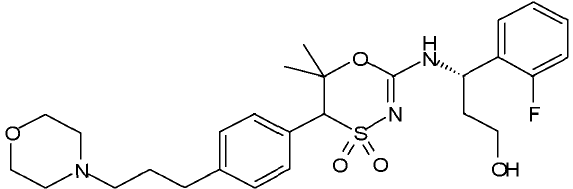 | B | 0.648 | 533.7 | (S)-3-{6,6-Dimethyl-5-[4-(3-morpholin-4-yl-propyl)-phenyl]-4,4-dioxo-5,6-dihydro-4H-4lambda6-[1,4,3]oxathiazin-2-ylamino}-3-(2-fluor-phenyl)-propan-1-ol |
| 111 | 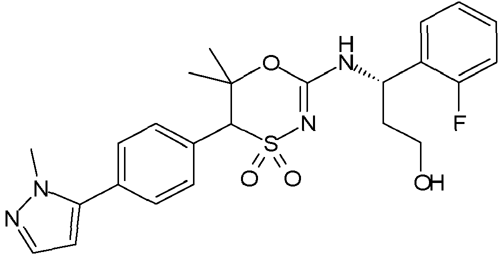 | B | 0.833 | 486.6 | (S)-3-{6,6-Dimethyl-5-[4-(2-methyl-2H-pyrazol-3-yl)-phenyl]-4,4-dioxo-5,6-dihydro-4 H-4lambda6-[1,4,3]oxathiazin-2-ylamino}-3-(2-fluor-phenyl)-propan-1-ol |
| 112 | 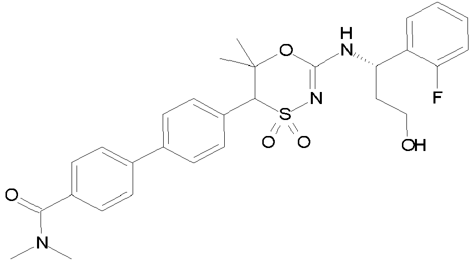 | B | 0.862 | 553.6 | 4'-{2-[(S)-1-(2-fluor-phenyl)-3-hydroxy-propylamino]-6,6-dimethyl-4,4-dioxo-5,6-dihydro-4H-4lambda6-[1,4,3]oxathiazin-5-yl}-biphenyl-4-carboxylic acid dimethylamide |
| 113 | 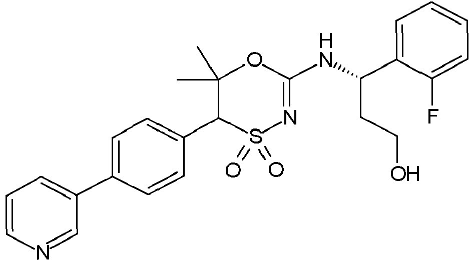 | B | 0.663 | 483.6 | (S)-3-[6,6-Dimethyl-4,4-dioxo-5-(4-pyridin-3-yl-phenyl)-5,6-dihydro-4H-4lambda6-[1,4,3]oxathiazin-2-ylamino]-3-(2-fluor-phenyl)-propan-1-ol |
| 114 | 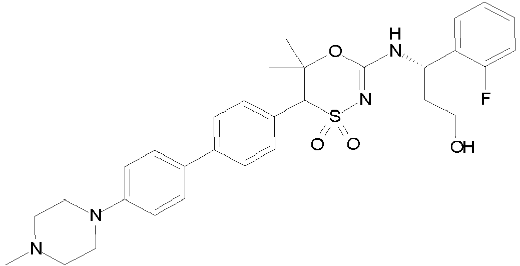 | B | 0.739 | 580.7 | (S)-3-{6,6-Dimethyl-5-[4'-(4-methyl-piperazin-1-yl)-biphenyl-4-yl]-4,4-dioxo-5,6-dihydro-4H-4lambda6-[1,4,3]oxathiazin-2-ylamino}-3-(2-fluor-phenyl)-propan-1-ol |
| 115 | 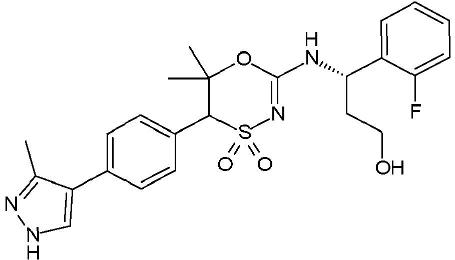 | B | 0.763 | 486.6 | (S)-3-{6,6-Dimethyl-5-[4-(3-methyl-1H-pyrazol-4-yl)-phenyl]-4,4-dioxo-5,6-dihydro-4H-41ambda6-[1,4,3]oxathiazin-2-ylamino}-3-(2-fluor-phenyl)-propan-1-ol |
| 116 | 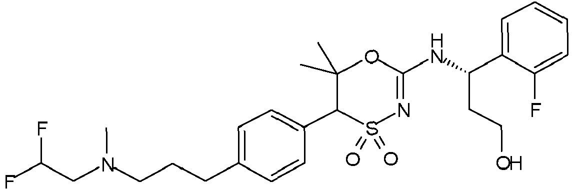 | B | 0.697 | 541.6 | (S)-3-[5-(4-{3-[(2,2-Difluor-ethyl)-methyl-amino]-propyl}-phenyl)-6,6-dimethyl-4,4-dioxo-5,6-dihydro-4H-4lambda6-[1,4,3]oxathiazin-2-ylamino]-3-(2-fluor-phenyl)-propan-1-ol |
| 117 | 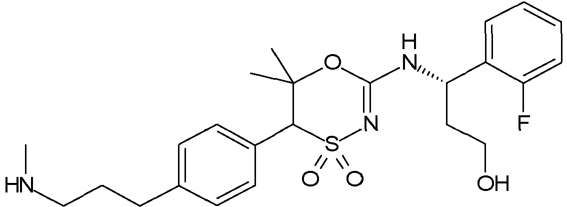 | B | 0.671 | 477.6 | (S)-3-{6,6-Dimethyl-5-[4-(3-methylamino-propyl)-phenyl]-4,4-dioxo-5,6-dihydro-4H-4lambda6-[1,4,3]oxathiazin-2-ylamino}-3-(2-fluor-phenyl)-propan-1-ol |
| 118 | 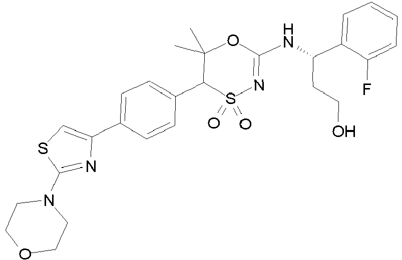 | B | 0.898 | 574.7 | (S)-3-{6,6-Dimethyl-5-[4-(2-morpholin-4-yl-thiazol-4-yl)-phenyl]-4,4-dioxo-5,6-dihydro-4H-4lambda6-[1,4,3]oxathiazin-2-ylamino}-3-(2-fluor-phenyl)-propan-1-ol |
| 119 | 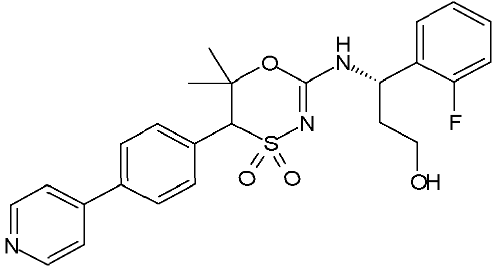 | B | 0.655 | 483.6 | (S)-3-[6,6-Dimethyl-4,4-dioxo-5-(4-pyridin-4-yl-phenyl)-5,6-dihydro-4H-4lambda6-[1,4,3]oxathiazin-2-ylamino]-3-(2-fluor-phenyl)-propan-1-ol |
| 120 | 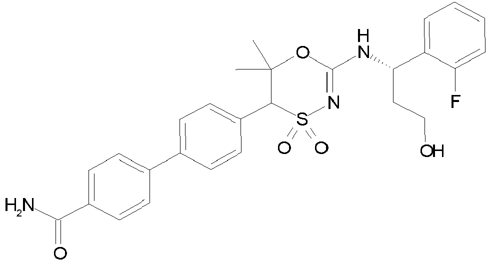 | B | 0.814 | 525.6 | 4'-{2-[(S)-1-(2-fluor-phenyl)-3-hydroxy-propylamino]-6,6-dimethyl-4,4-dioxo-5,6-dihydro-4H-4lambda6-[1,4,3]oxathiazin-5-yl}-biphenyl-4-carboxylic acid amide |
| 121 | 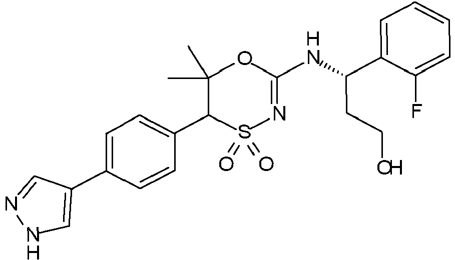 | B | 0.782 | 472.5 | (S)-3-{6,6-Dimethyl-4,4-dioxo-5-[4-(1H-pyrazol-4-yl)-phenyl]-5,6-dihydro-4H-41ambda6-[1,4,3]oxathiazin-2-ylamino}-3-(2-fluor-phenyl)-propan-1-ol |
| 122 | 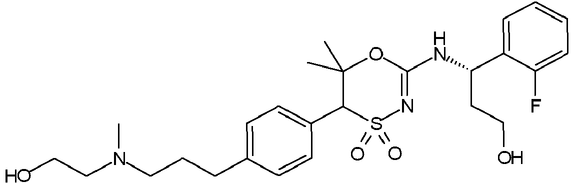 | B | 0.678 | 521.6 | (S)-3-(2-fluor-phenyl)-3-[5-(4-{3-[(2-hyd roxy-ethyl)-methyl-amino]-propyl}-phenyl)-6,6-dimethyl-4,4-dioxo-5,6-dihydro-4H-4lambda6-[1,4,3]oxathiazin-2-ylamino]-propan-1-ol |
| 123 | 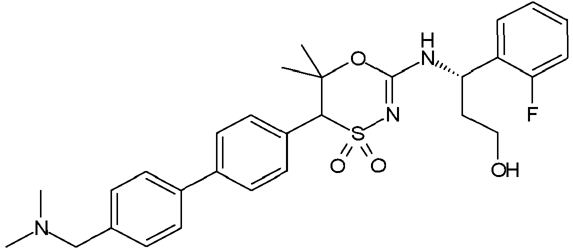 | B | 0.724 | 539.7 | (S)-3-[5-(4'-Dimethylaminomethyl-biphenyl-4-yl)-6,6-dimethyl-4,4-dioxo-5,6-dihydro-4H-4lambda6-[1,4,3]oxathiazin-2-ylamino]-3-(2-fluor-phenyl)-propan-1-ol |
| 124 | 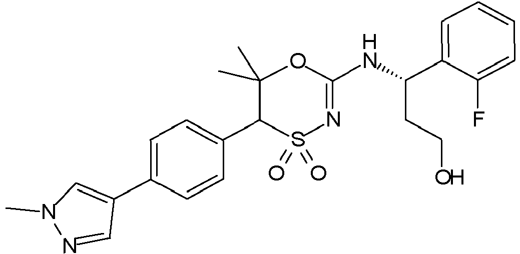 | B | 0.817 | 486.6 | (S)-3-{6,6-Dimethyl-5-[4-(1-methyl-1H-pyrazol-4-yl)-phenyl]-4,4-dioxo-5,6-dihydro-4H-4lambda6-[1,4,3]oxathiazin-2-ylamino}-3-(2-fluor-phenyl)-propan-1-ol |
| 125 | 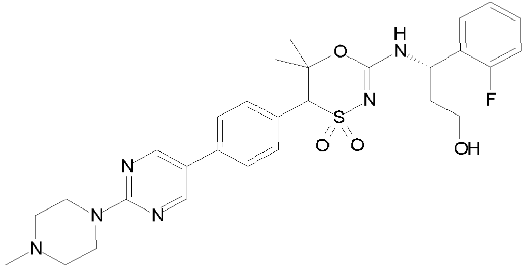 | B | 0.71 | 582.7 | (S)-3-(6,6-Dimethyl-5-{4-[2-(4-methyl-piperazin-1-yl)-pyrimidin-5-yl]-phenyl}-4,4-dioxo-5,6-dihydro-4H-4lambda6-[1,4,3]oxathiazin-2-ylamino)-3-(2-fluor-phenyl)-propan-1-ol |
| 126 | 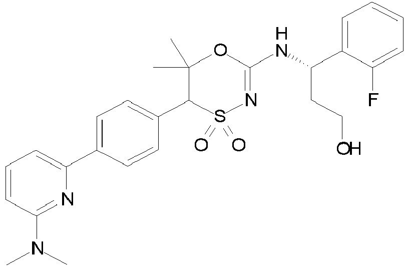 | B | 0.734 | 526.6 | (S)-3-{5-[4-(6-Dimethylamino-pyridin-2-yl)-phenyl]-6,6-dimethyl-4,4-dioxo-5,6-dihydro-4H-4lambda6-[1,4,3]oxathiazin-2-ylamino}-3-(2-fluor-phenyl)-propan-1-ol |
| 127 | 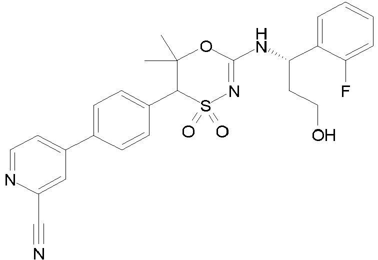 | C | 1.348 | 508.6 | 4-(4-{2-[(S)-1-(2-fluor-phenyl)-3-hydroxy-propylamino]-6,6-dimethyl-4,4-dioxo-5,6-dihydro-4H-4lambda6-[1,4,3]oxathiazin-5-yl}-phenyl)-pyridine-2-carbonitrile |
| 128 | 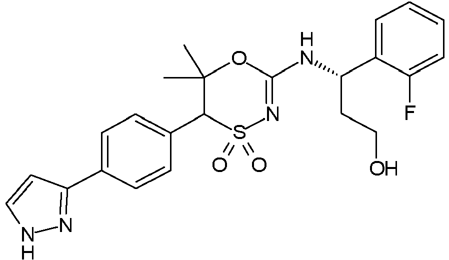 | B | 0.75 | 472.5 | (S)-3-{6,6-Dimethyl-4,4-dioxo-5-[4-(1H-pyrazol-3-yl)-phenyl]-5,6-dihydro-4H-4lambda6-[1,4,3]oxathiazin-2-ylamino}-3-(2-fluor-phenyl)-propan-1-ol |
| 129 | 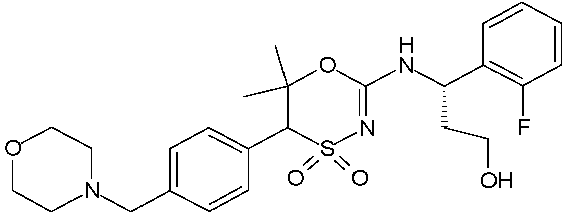 | B | 0.600 | 505.6 | (S)-3-[6,6-Dimethyl-5-(4-morpholin-4-ylmethyl-phenyl)-4,4-dioxo-5,6-dihydro-4H-4lambda6-[1,4,3]oxathiazin-2-ylamino]-3-(2-fluor-phenyl)-propan-1-ol |
| 130 | 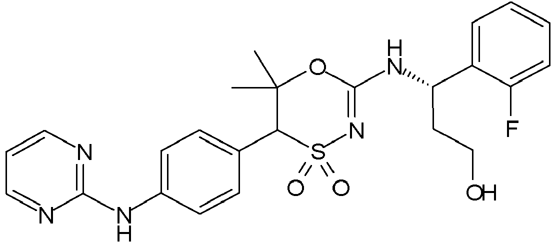 | B | 0.763 | 499.6 | (S)-3-{6,6-Dimethyl-4,4-dioxo-5-[4-(pyrimidin-2-ylamino)-phenyl]-5,6-dihydro-4H-4lambda6-[1,4,3]oxathiazin-2-ylamino}-3-(2-fluor-phenyl)-propan-1-ol |
| 131 | 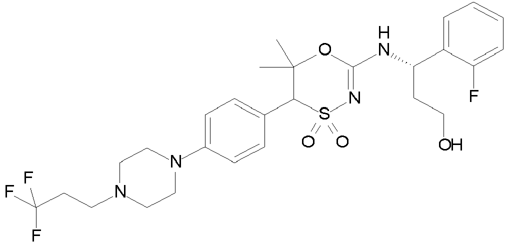 | B | 0.699 | 586.6 | (S)-3-(6,6-Dimethyl-4,4-dioxo-5-{4-[4-(3, 3, 3-trifluor-propyl)-piperazin-1-yl]-phenyl}-5,6-dihydro-4H-4lambda6-[1,4,3]oxathiazin-2-ylamino)-3-(2-fluor-phenyl)-propan-1-ol |
| 132 | 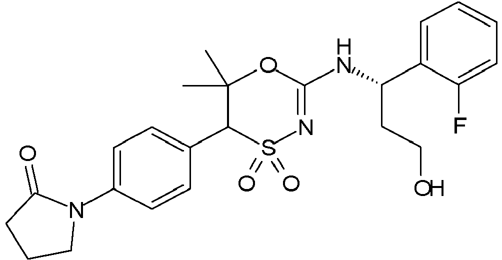 | B | 0.776 | 489.6 | 1-(4-{2-[(S)-1-(2-fluor-phenyl)-3-hydroxy-propylamino]-6,6-dimethyl-4,4-dioxo-5,6-dihydro-4H-4lambda6-[1,4,3]oxathiazin-5-yl}-phenyl)-pyrrolidin-2-on |
| 133 | 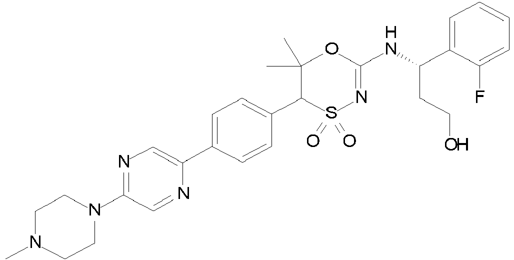 | B | 0.682 | 582.7 | (S)-3-{6,6-Dimethyl-5-[4-(4-methyl-3,4,5,6-tetrahydro-2H-[1,2']bipyrazinyl-5'-yl)-phenyl]-4,4-dioxo-5,6-dihydro-4H-4lambda6-[1,4,3]oxathiazin-2-ylamino}-3-(2-fluor-phenyl)-propan-1-ol |
| 134 | 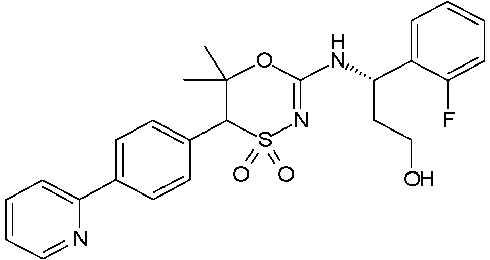 | B | 0.668 | 483.6 | (S)-3-[6,6-Dimethyl-4,4-dioxo-5-(4-pyrid in-2-yl-phenyl)-5,6-dihydro-4H-4lambda6-[1,4,3]oxathiazin-2-ylamino]-3-(2-fluor-phenyl)-propan-1-ol |
| 135 | 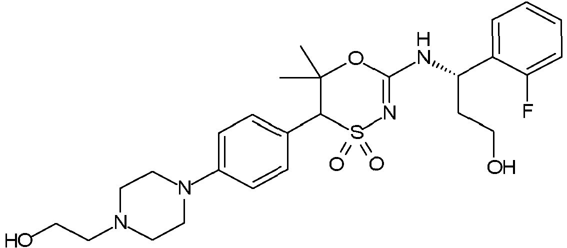 | B | 0.638 | 534.6 | (S)-3-(2-fluor-phenyl)-3-(5-{4-[4-(2-hydroxy-ethyl)-piperazin-1-yl]-phenyl}-6,6-dimethyl-4,4-dioxo-5,6-dihydro-4H-4lambda6-[1,4,3]oxathiazin-2-ylamino)-propan-1-ol |
| 136 | 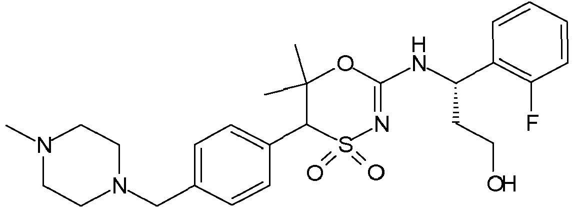 | B | 0.588 | 518.6 | (S)-3-{6,6-Dimethyl-5-[4-(4-methyl-piperazin-1-ylmethyl)-phenyl]-4,4-dioxo-5,6-dihydro-4H-4lambda6-[1,4,3]oxathiazin-2-ylamino}-3-(2-fluor-phenyl)-propan-1-ol |
| 137 | 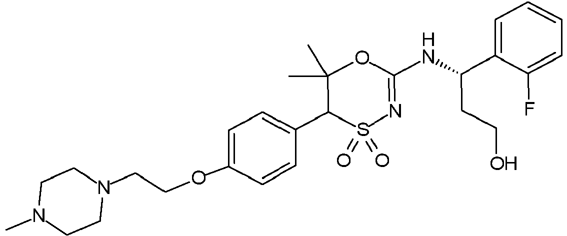 | B | 0.618 | 548.7 | (S)-3-(6,6-Dimethyl-5-{4-[2-(4-methyl-piperazin-1-yl)-ethoxy]-phenyl}-4,4-dioxo-5,6-dihydro-4H-4lambda6-[1,4,3]oxathiazin-2-ylamino)-3-(2-fluor-phenyl)-propan-1-ol |
| 138 | 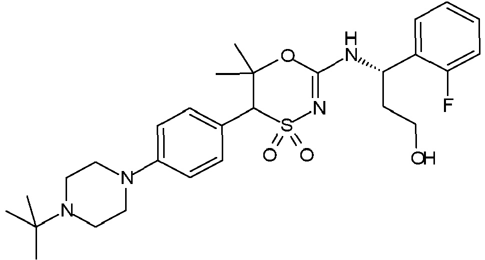 | B | 0.684 | 546.7 | (S)-3-{5-[4-(4-tert-Butyl-piperazin-1-yl)-phenyl]-6,6 dimethyl-4,4-dioxo-5,6-dihydro-4H-4lambda6-[1,4,3]oxathiazin-2-ylamino}-3-(2-fluor-phenyl)-propan-1-ol |
| 139 | 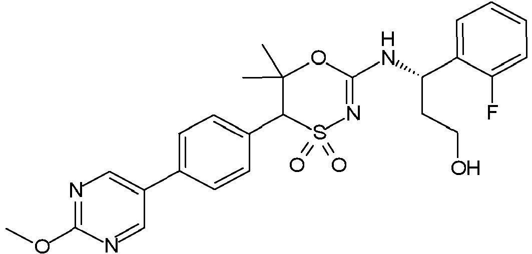 | B | 0.811 | 514.6 | (S)-3-(2-fluor-phenyl)-3-{5-[4-(2-methoxy-pyrimidin-5-yl)-phenyl]-6,6-dimethyl-4,4-dioxo-5,6-dihydro-4H-4lambda6-[1,4,3]oxathiazin-2-ylamino}-propan-1-ol |
| 140 | 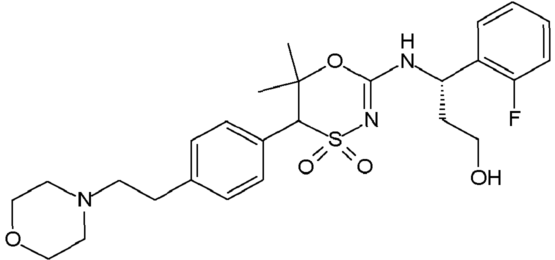 | B | 0.642 | 519.6 | (S)-3-{6,6-Dimethyl-5-[4-(2-morpholin-4-yl-ethyl)-phenyl]-4,4-dioxo-5,6-dihydro-4H-4lambda6-[1,4,3]oxathiazin-2-ylamino}-3-(2-fluor-phenyl)-propan-1-ol |
| 141 | 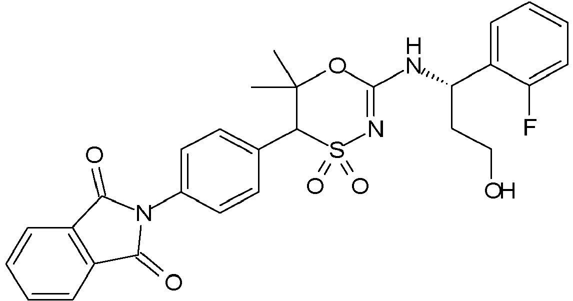 | B | 0.910 | 551.6 | 2-(4-{2-[(S)-1-(2-fluor-phenyl)-3-hydroxy-propylamino]-6,6-dimethyl-4,4-dioxo-5,6-dihydro-4H-4lambda6-[1,4,3]oxathiazin-5-yl}-phenyl)-isoindole-1,3-dion |
| 142 | 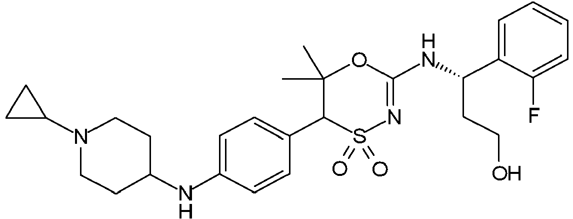 | B | 0.683 | 544.7 | (S)-3-{5-[4-(1-Cyclopropyl-piperidin-4-ylamino)-phenyl]-6,6-dimethyl-4,4-dioxo-5,6-dihydro-4H-4lambda6-[1,4,3]oxathiazin-2-ylamino}-3-(2-fluor-phenyl)-propan-1-ol |
| 143 | 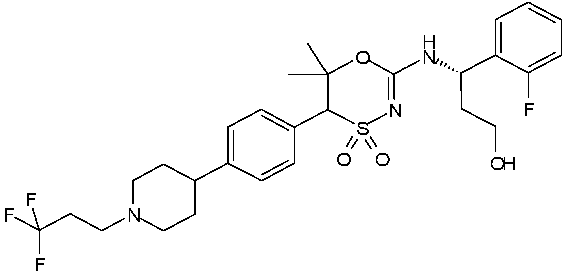 | B | 0.722 | 585.7 | (S)-3-(6,6-Dimethyl-4,4-dioxo-5-{4-[1-(3,3,3-trifluor-propyl)-piperidin-4-yl]-phenyl}-5,6-dihydro-4H-4lambda6-[1,4,3]oxathiazin-2-ylamino)-3-(2-fluor-phenyl)-propan-1-ol |
| 144 | 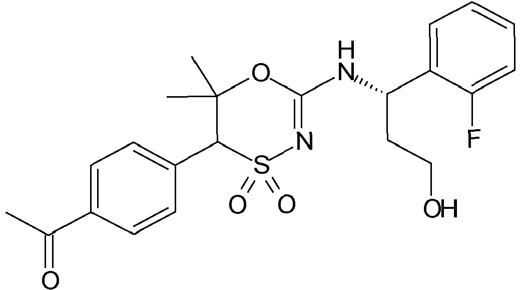 | B | 0.806 | 448.5 | 1-(4-{2-[(S)-1-(2-fluor-phenyl)-3-hydroxy-propylamino]-6,6-dimethyl-4,4-dioxo-5,6-dihydro-4H-4lambda6-[1,4,3]oxathiazin-5-yl}-phenyl)-ethanon |
| 145 | 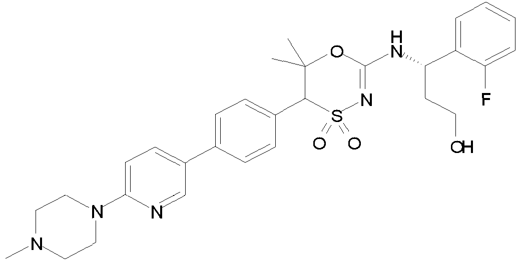 | B | 0.642 | 581.7 | (S)-3-(6,6-Dimethyl-5-{4-[6-(4-methyl-piperazin-1-yl)-pyridin-3-yl]-phenyl}-4,4-dioxo-5,6-dihydro-4H-4lambda6-[1,4,3]oxathiazin-2-ylamino)-3-(2-fluor-phenyl)-propan-1-ol |
| 146 | 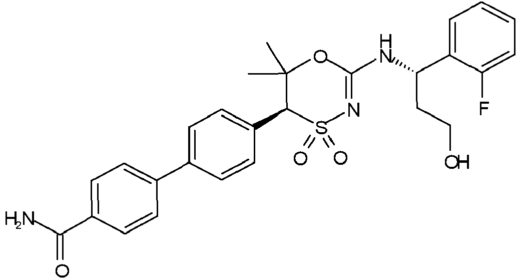 | B | 0.797 | 525.6 | 4'-{(S)-2-[(S)-1-(2-fluor-phenyl)-3-hydroxy-propylamino]-6,6-dimethyl-4,4-dioxo-5,6-dihydro-4H-4lambda6-[1,4,3]oxathiazin-5-yl}-biphenyl-4-carbonsäureamid |
| 147 | 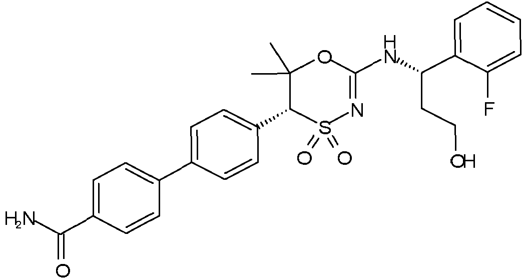 | B | 0.799 | 525.6 | 4'-{(R)-2-[(S)-1-(2-fluor-phenyl)-3-hydroxy-propylamino]-6,6-dimethyl-4,4-dioxo-5,6-dihydro-4H-4lambda6-[1,4,3]oxathiazin-5-yl}-biphenyl-4-carbonsäureamid |
| 148 | 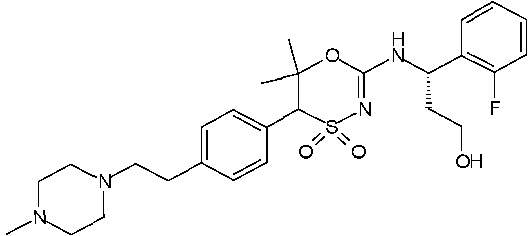 | B | 0.597 | 532.7 | (S)-3-(6,6-Dimethyl-5-{4-[2-(4-methyl-piperazin-1-yl)-ethyl]-phenyl}-4,4-dioxo-5,6-dihydro-4H-4lambda6-[1,4,3]oxathiazin-2-ylamino)-3-(2-fluor-phenyl)-propan-1-ol |
| 149 | 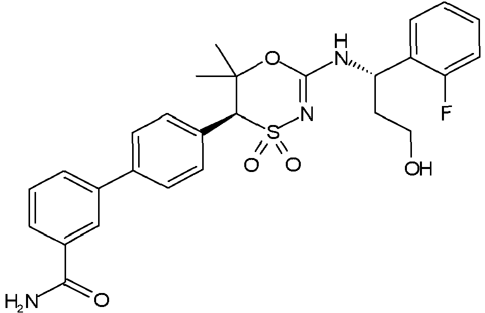 | B | 0.823 | 525.6 | 4'-{(S)-2-[(S)-1-(2-fluor-phenyl)-3-hydroxy-propylamino]-6,6-dimethyl-4,4-dioxo-5,6-dihydro-4H-4lambda6-[1,4,3]oxathiazin-5-yl}-biphenyl-3-carbonsäureamid |
| 150 | 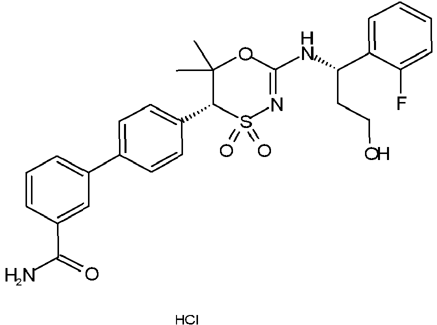 | B | 0.824 | 525.6 | 4'-{(R)-2-[(S)-1-(2-fluor-phenyl)-3-hydroxy-propylamino]-6,6-dimethyl-4,4-dioxo-5,6-dihydro-4H-4lambda6-[1,4,3]oxathiazin-5-yl}-biphenyl-3-carbonsäureamid |
| 151 | 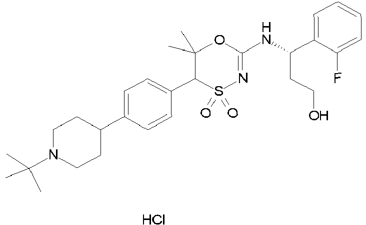 | B | 0.714 | 545.7 | (S)-3-{5-[4-(1-tert-Butyl-piperidin-4-yl)-phenyl]-6,6-dimethyl-4,4-dioxo-5,6-dihydro-4H-4lambda6-[1,4,3]oxathiazin-2-ylamino}-3-(2-fluor-phenyl)-propan-1-ol |
| 152 | 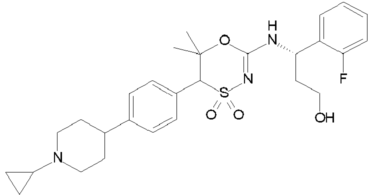 | B | 0.697 | 529.7 | (S)-3-{5-[4-(1-Cyclopropyl-piperidin-4-yl)-phenyl]-6,6-dimethyl-4,4-dioxo-5,6-dihydro-4H-4lambda6-[1,4,3]oxathiazin-2-ylamino}-3-(2-fluor-phenyl)-propan-1-ol |
| 153 | 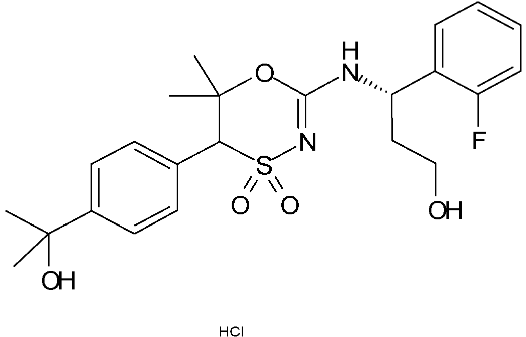 | B | 0.821 | 464.6 | (S)-3-(2-fluor-phenyl)-3-{5-[4-(1-hydroxy-1-methyl-ethyl)-phenyl]-6,6-dimethyl-4,4-dioxo-5,6-dihydro-4H-4lambda6-[1,4,3]oxathiazin-2-ylamino}-propan-1-ol |
| 154 | 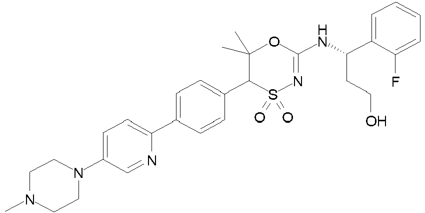 | B | 0.637 | 581.7 | (S)-3-(6,6-Dimethyl-5-{4-[5-(4-methyl-piperazin-1-yl)-pyridin-2-yl]-phenyl}-4,4-dioxo-5,6-dihydro-4H-4lambda6-[1,4,3]oxathiazin-2-ylamino)-3-(2-fluor-phenyl)-propan-1-ol |
| 155 | 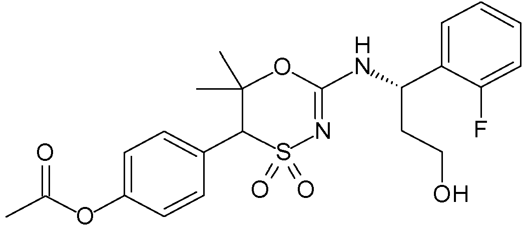 | B | 0.863 | 464.5 | Acetic acid 4-{2-[(S)-1-(2-fluor-phenyl)-3-hydroxy-propylamino]-6,6-dimethyl-4,4-dioxo-5,6-dihydro-4H-4lambda6-[1,4,3]oxathiazin-5-yl}-phenyl ester |
| 156 | 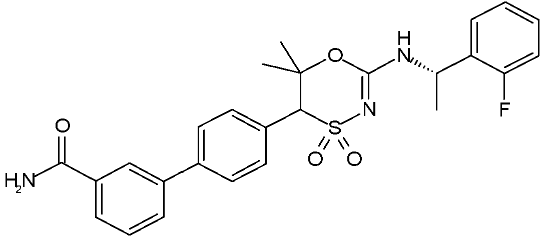 | B | 0.940 | 495.6 | 4'-{2-[(S)-1-(2-fluor-phenyl)-ethylamino]-6,6-dimethyl-4,4-dioxo-5,6-dihydro-4H-4lambda6-[1,4,3]oxathiazin-5-yl}-biphenyl-3-carbonsäureamid |
| 157 | 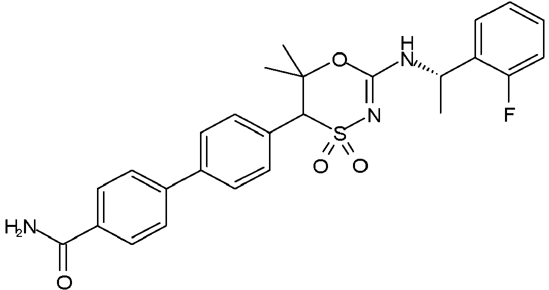 | B | 0.927 | 495.6 | 4'-{2-[(S)-1-(2-fluor-phenyl)-ethylamino]-6,6-dimethyl-4,4-dioxo-5,6-dihydro-4H-41ambda6-[1,4,3]oxathiazin-5-yl}-biphenyl-4-carbonsäureamid |
| 158 | 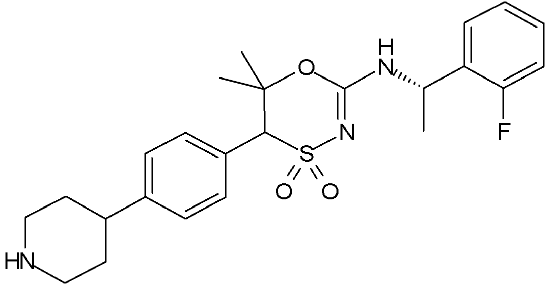 | B | 0.783 | 459.6 | [6,6-Di methyl-4, 4-d ioxo-5-(4-pi pe rid i n-4-yl-ph e nyl) 5,6-dihydro-4H-4lambda6-[1,4,3]oxathiazin-2-yl]-[(S)-1-(2-fluor-phenyl)-ethyl]-amin |
| 159 | 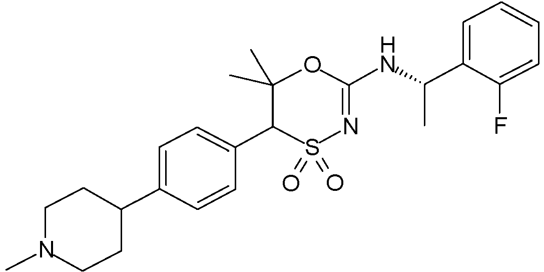 | B | 0.777 | 473.6 | {6,6-Dimethyl-5-[4-(1-methyl-piperidin-4-yl)-phenyl]-4,4-dioxo-5,6-dihydro-4H-4lambda6-[1,4,3]oxathiazin-2-yl}-[(S)-1-(2-fluor-phenyl)-ethyl]-amin |
| 160 | 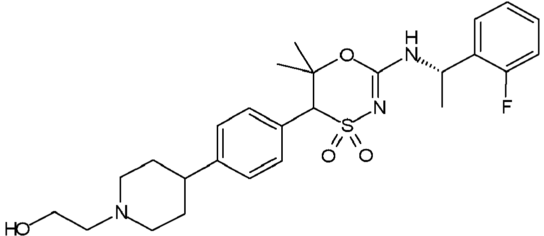 | B | 0.769 | 503.6 | 2-[4-(4-{2-[(S)-1-(2-fluor-phenyl)-ethylamino]-6, 6-dimethyl-4,4-dioxo-5,6-dihydro-4H-41ambda6-[1,4,3]oxathiazin-5-yl}-phenyl)-piperidin-1-yl]-ethanol |
| 161 | 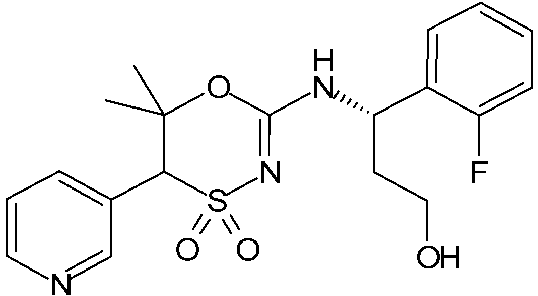 | B | 0.619 | 407.5 | (S)-3-(6,6-Dimethyl-4,4-dioxo-5-pyridin-3-yl-5,6-dihydro-4H-4lambda6-[1,4,3]oxathiazin-2-ylamino)-3-(2-fluor-phenyl)-propan-1-ol |
| 162 | 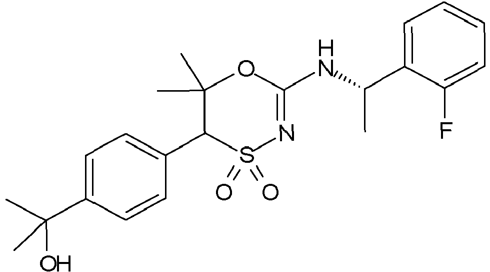 | C | 1.568 | 434.5 | 2-(4-{2-[(S)-1-(2-flu or-phenyl)-ethylamino]-6,6-dimethyl-4,4-dioxo-5,6-dihydro-4H-4lambda6-[1,4,3]oxathiazin-5-yl}-phenyl)-propan-2-ol |
| 163 | 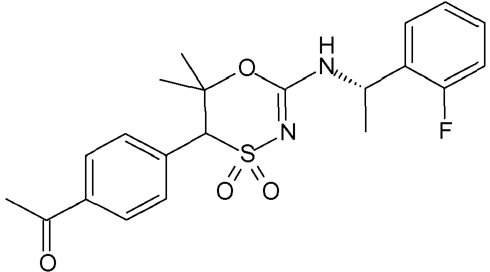 | B | 0.939 | 418.5 | 1-(4-{2-[(S)-1-(2-fluor-phenyl)-ethylamin o]-6,6-dimethyl-4,4-dioxo-5,6-dihydro-4H-4lambda6-[1,4,3]oxathiazin-5-yl}-phenyl)-ethanon |
| 164 | 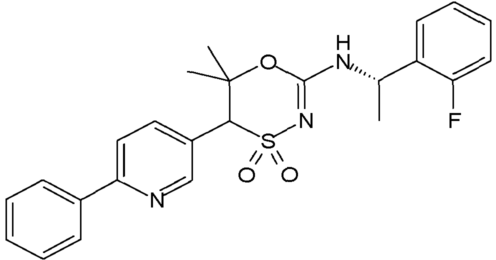 | B | 0.963 | 453.5 | [6,6-Dimethyl-4,4-dioxo-5-(6-phenyl-pyridin-3-yl)-5,6-dihydro-4H-4lambda6-[1,4,3]oxathiazin-2-yl]-[(S)-1-(2-fluor-phenyl)-ethyl]-amin |
| 165 | 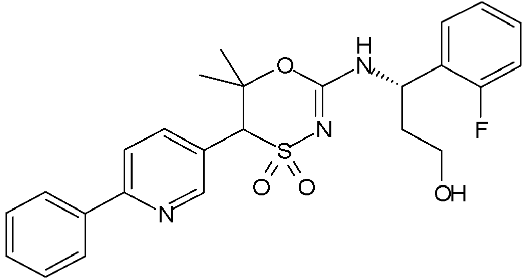 | B | 0.863 | 483.6 | (S)-3-[6,6-Dimethyl-4,4-dioxo-5-(6-phenyl-pyridin-3-yl)-5,6-dihydro-4H-4lambda6-[1,4,3]oxathiazin-2 ylamino]-3-(2-fluor-phenyl)-propan-1-ol |
| 166 | 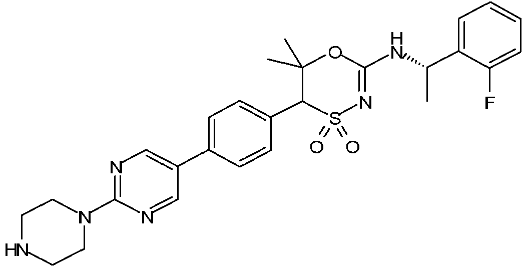 | B | 0.797 | 538.6 | {6,6-Dimethyl-4,4-dioxo-5-[4-(2-piperazin-1-yl-pyrimidin-5-yl)-phenyl]-5,6-dihydro-4H-4lambda6-[1,4,3]oxathiazin-2-yl}-[(S)-1-(2-fluor-phenyl)-ethyl]-amin |
| 167 | 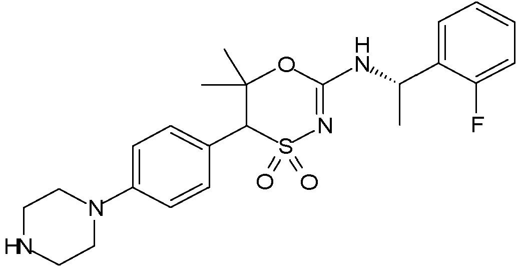 | B | 0.765 | 460.6 | [6,6-Dimethyl-4,4-dioxo-5-(4-piperazin-1-yl-phenyl)-5,6-dihydro-4H-4lambda6-[1,4,3]oxathiazin-2-yl]-[(S)-1-(2-fluor-phenyl)-ethyl] amin |
| 168 | 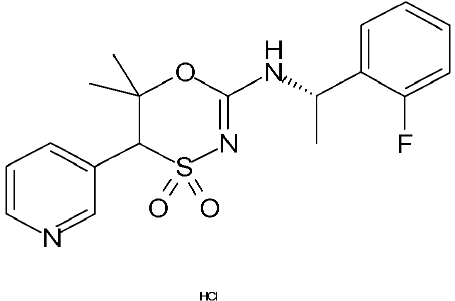 | B | 0.712 | 377.4 | (6,6-Dimethyl-4,4-dioxo-5-pyridin-3-yl-5,6-dihydro-4H-4lambda6-[1,4,3]oxathiazin-2-yl)-[(S)-1-(2-fluor-phenyl)-ethyl]-amin |
| 169 | 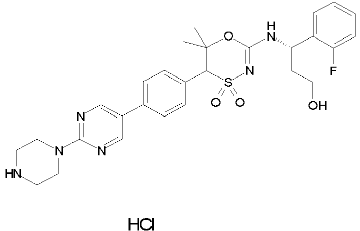 | B | 0.718 | 568.7 | (S)-3-{6,6-Dimethyl-4,4-dioxo-5-[4-(2-piperazin-1-yl-pyrimidin-5-yl)-phenyl]-5,6-dihydro-4H-4lambda6-[1,4,3]oxathiazin-2-ylamino}-3-(2-fluor-phenyl)-propan-1-ol |
| 170 | 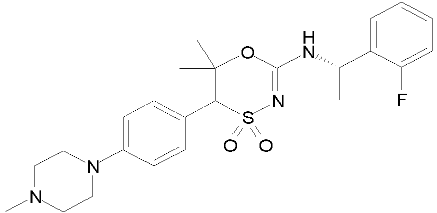 | B | 0.684 | 474.6 | {6,6-Dimethyl-5-[4-(4-methyl-piperazin-1-yl)-phenyl]-4,4-dioxo-5,6-dihydro-4H-4lambda6-[1,4,3]oxathiazin-2-yl}-[(S)-1-(2-fluor-phenyl)-ethyl]-amin |
| | 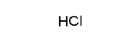 | | | | |
| 171 | 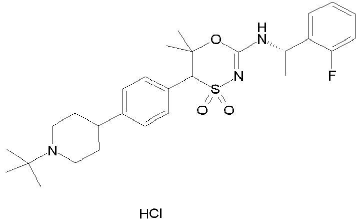 | B | 0.745 | 515.7 | {5-[4-(1-tert-Butyl-piperidin-4-yl)-phenyl]-6,6-dimethyl-4,4-dioxo-5,6-dihydro-4H-4lambda6-[1,4,3]oxathiazin-2-yl}-[(S)-1-(2-fluor-phenyl)-ethyl]-amin |
| 172 | 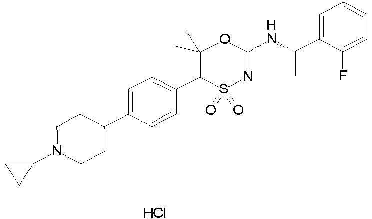 | B | 0.730 | 499.6 | {5-[4-(1-Cyclopropyl-piperidin-4-yl)-phenyl]-6,6-dimethyl-4,4-dioxo-5,6-dihydro-4H-4lambda6-[1,4,3]oxathiazin-2-yl}-[(S)-1-(2-fluor-phenyl)-ethyl]-amin |
| 173 | 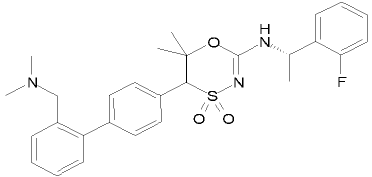 | B | 0.753 | 509.6 | [5-(2'-Dimethylaminomethyl-biphenyl-4-yl)-6,6-dimethyl-4,4-dioxo-5,6-dihydro-4H-4lambda6-[1,4,3]oxathiazin-2-yl]-[(S)-1-(2-fluor-phenyl)-ethyl] amin |
| 174 | 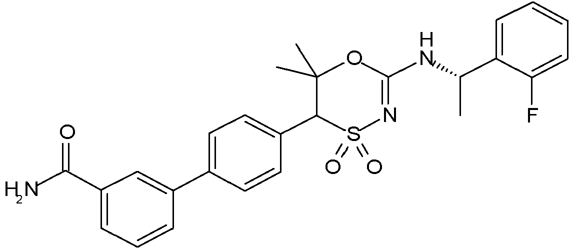 | B | 0.853 | 495.6 | 4'-{(S)-2-[(S)-1 -(2-fluor-phenyl)-ethylaminol-6,6-dimethyl-4,4-dioxo-5,6-dihydro-4H-41ambda6-[1,4,3]oxathiazin-5-yl}-biphenyl-3-carbonsäureamid |
| 175 | 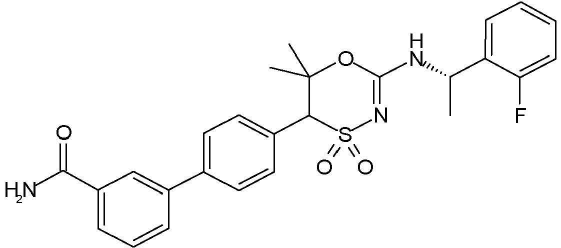 | B | 0.859 | 495.6 | 4'-{(R)-2-[(S)-1-(2-fluor-phenyl)-ethylamino]-6,6-dimethyl-4,4-dioxo-5,6-dihydro-4H-4lambda6-[1,4,3]oxathiazin-5-yl}-biphenyl-3-carbonsäureamid |
| 176 | 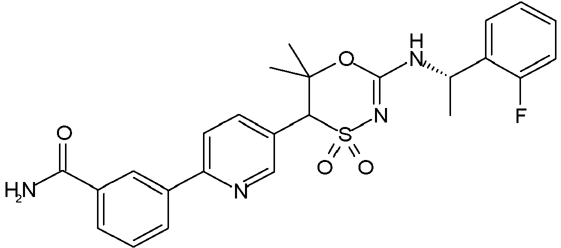 | C | 1.444 | 496.6 | 3-(5-{2-[(S)-1-(2-fluor-phenyl)-ethylaminol-6,6-dimethyl-4,4-dioxo-5,6-dihydro-4H-41ambda6-[1,4,3]oxathiazin-5-yl}-pyridin-2-yl)-benzamid |
| 177 | 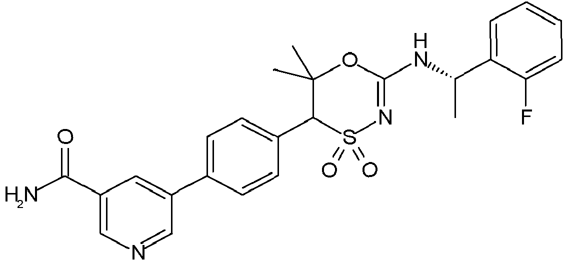 | B | 0.750 | 496.6 | 5-(4-{(S)-2-[(S)-1-(2-fluor-phenyl)-ethylamino]-6,6-dimethyl-4,4-dioxo-5,6-dihydro-4H-4lambda6-[1,4,3]oxathiazin-5-yl}-phenyl)-nicotinamid |
| 178 | 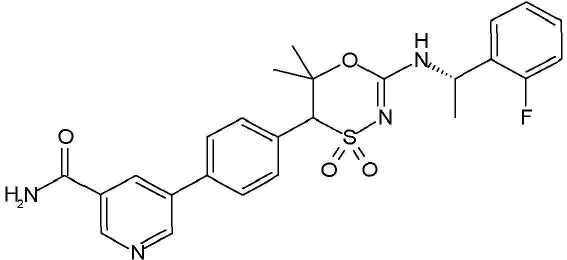 | B | 0.755 | 496.6 | 5-(4-{(R)-2-[(S)-1-(2-fluor-phenyl)-ethylamino]-6,6-dimethyl-4,4-dioxo-5,6-dihydro-4H-4lambda6-[1,4,3]oxathiazin-5-yl}-phenyl)-nicotinamid |
| 179 | 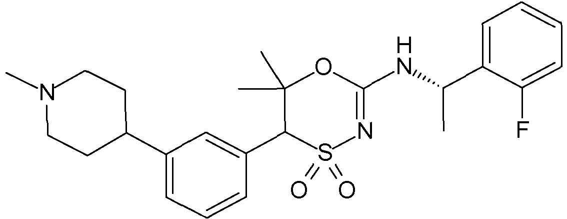 | B | 0.714 | 473.6 | {6,6-Dimethyl-5-[3-(1-methyl-piperidin-4-yl)-phenyl]-4,4-dioxo-5,6-dihydro-4H-4lambda6-[1,4,3]oxathiazin-2-yl}-[(S)-1-(2-fluor-phenyl)-ethyl]-amin |
| 180 | 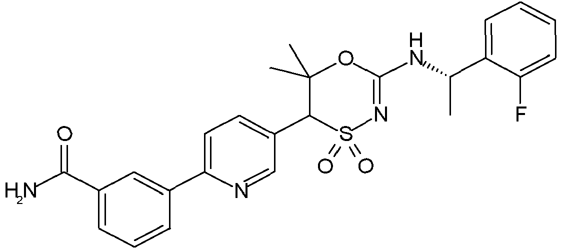 | B | 0.788 | 496.6 | 3-(5-{2-[(S)-1-(2-fluor-phenyl)-ethylamino]-6,6-dimethyl-4,4-dioxo-5,6-dihydro-4H-41ambda6-[1,4,3]oxathiazin-5-yl}-pyridin-2-yl)-benzamid |
| 181 | 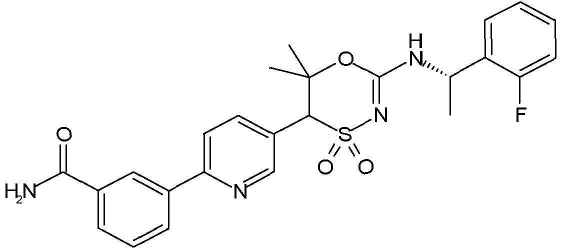 | B | 0.795 | 496.6 | 3-(5-{2-[(S)-1-(2-fluor-phenyl)-ethylamino]-6,6-dimethyl-4,4-dioxo-5,6-dihydro-4H-41ambda6-[1,4,3]oxathiazin-5-yl}-pyridin-2-yl)-benzamid |
| 182 | 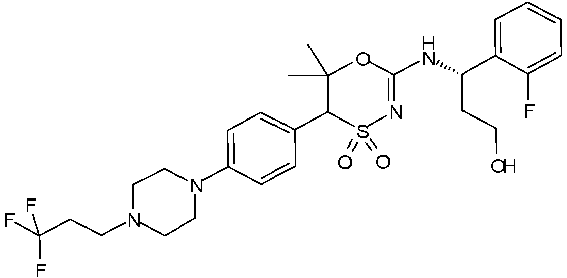 | B | 0.678 | 586.6 | (S)-3-(6,6-Dimethyl-4,4-dioxo-5-{4-[4-(3,3,3-rrifluor-propyl)-piperazin-1-yl]-phenyl}-5,6-dihydro-4H-4lambda6-[1,4,3]oxathiazin-2-ylamino)-3-(2-fluor-phenyl)-propan-1-ol |
| 183 | 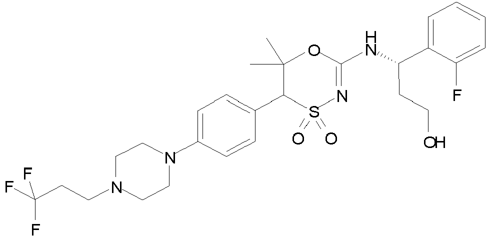 | B | 0.677 | 586.6 | (S)-3-(6,6-Dimethyl-4,4-dioxo-5-{4-[4-(3,3,3-trifluor-propyl)-piperazin-1-yl]-phenyl}-5,6-dihydro-4H-4lambda6-[1,4,3]oxathiazin-2-ylamino)-3-(2-fluor-phenyl)-propan-1-ol |
| 184 | 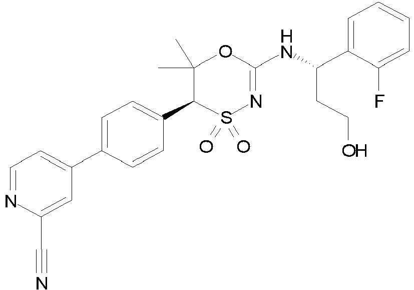 | B | 0.856 | 508.6 | 4-(4-{(S)-2-[(S)-1-(2-fluor-phenyl)-3-hydroxy-propylamino]-6,6-dimethyl-4,4-dioxo-5,6-dihydro-4H-4lambda6-[1,4,3]oxathiazin-5-yl}-phenyl)-pyridin-2-carbonitril |
| 185 | 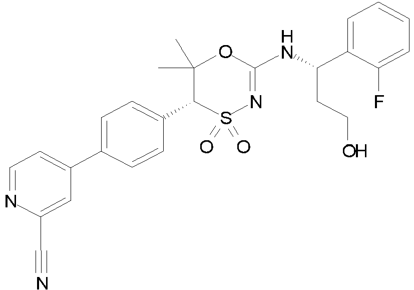 | B | 0.857 | 508.6 | 4-(4-{(R)-2-[(S)-1-(2-fluor-phenyl)-3-hydroxy-propylamino]-6,6-dimethyl-4,4-dioxo-5,6-dihydro-4H-4lambda6-[1,4,3]oxathiazin-5-yl}-phenyl)-pyridin-2-carbonitril |
| 186 | 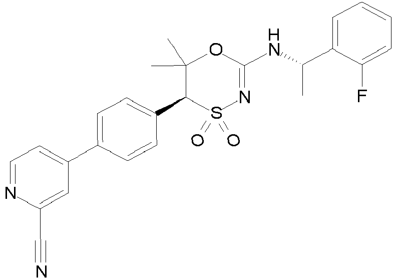 | B | 0.964 | 478.5 | 4-(4-{(S)-2-[(S)-1-(2-fluor-phenyl)-ethylamino]-6,6-dimethyl-4,4-dioxo-5,6-dihydro-4H-4lambda6-[1,4,3]oxathiazin-5-yl}-phenyl)-pyridin-2-carbonitril |
| 187 | 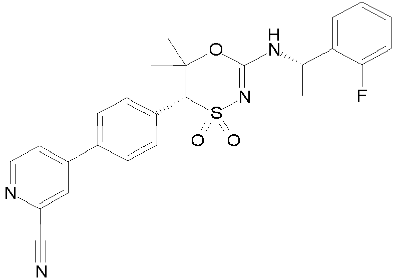 | B | 0.973 | 478.5 | 4-(4-{(R)-2-[(S)-1-(2-fluor-phenyl)-ethyla mino]-6,6-dimethyl-4,4-dioxo-5,6-dihydro-4H-4lambda6-[1,4,3]oxathiazin-5-yl}-phenyl)-pyridin-2-carbonitril |
| 188 | 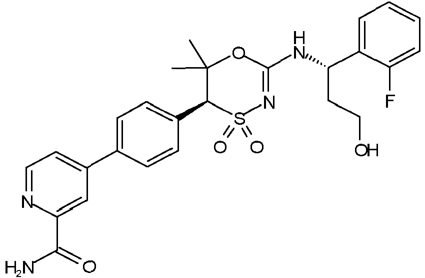 | B | 0.744 | 526.6 | 4-(4-{(S)-2-[(S)-1-(2-fluor-phenyl)-3-hydroxy-propylamino]-6,6-dimethyl-4,4-dioxo-5,6-dihydro-4H-4lambda6-[1,4,3]oxathiazin-5-yl}-phenyl)-pyridin-2-carbonsäureamid |
| 189 | 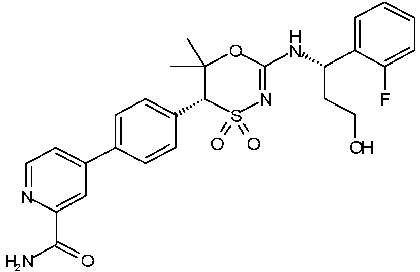 | B | 0.745 | 526.6 | 4-(4-{(R)-2-[(S)-1-(2-fluor-phenyl)-3-hydroxy-propylamino]-6,6-dimethyl-4,4-dioxo-5,6-dihydro-4H-4lambda6-[1,4,3]oxathiazin-5-yl}-phenyl)-pyridin-2-carbonsäureamid |
| 190 | 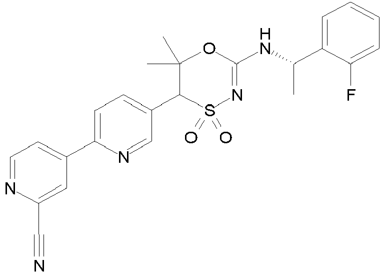 | B | 0.908 | 479.5 | 5-{2-[(S)-1-(2-fluor-phenyl)-ethylamino]-6,6-dimethyl-4,4-dioxo-5,6-dihydro-4H-4lambda6-[1,4,3]oxathiazin-5-yl}-[2,4']bipyridinyl-2'-carbonitril |
| 191 | 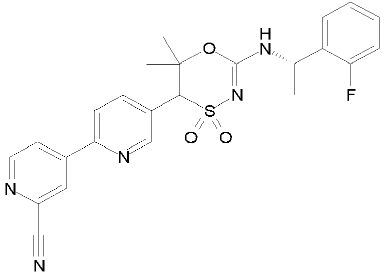 | B | 0.919 | 479.5 | 5-{2-[(S)-1-(2-fluor-phenyl)-ethylamino]-6,6-dimethyl-4,4-dioxo-5,6-dihydro-4H-4lambda6-[1,4,3]oxathiazin-5-yl}-[2,4']bipyridinyl-2'-carbonitril |
| 192 | 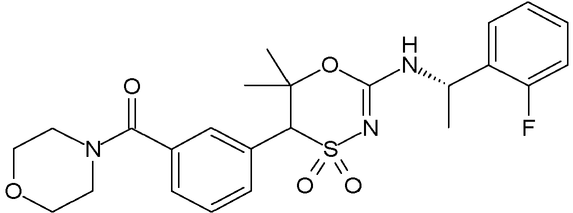 | C | 1.467 | 489.6 | (3-{2-[(S)-1-(2-fluor-phenyl)-ethylamino]-6,6-dimethyl-4,4-dioxo-5,6-dihydro-4H-4lambda6-[1,4,3]oxathiazin-5-yl}-phenyl)-morpholin-4-yl-methanon |
| 193 | 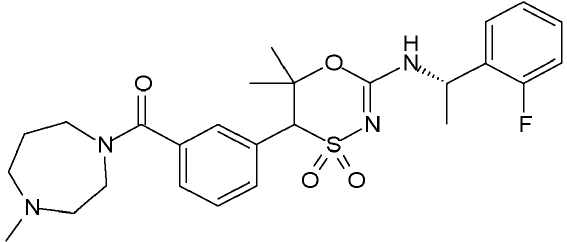 | C | 1.211 | 516.6 | (3-{2-[(S)-1-(2-fluor-phenyl)-ethylaminol-6,6-dimethyl-4,4-dioxo-5,6-dihydro-4H-41ambda6-[1,4,3]oxathiazin-5-yl}-phenyl)-(4-methyl-[1,4]diazepan-1-yl)-methanon |
| 194 | 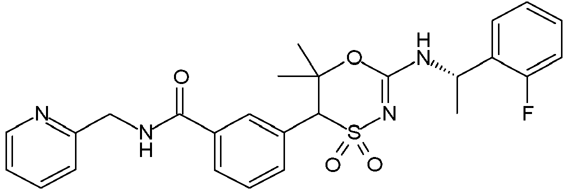 | C | 1.269 | 510.6 | 3-{2-[(S)-1-(2-fluor-phenyl)-ethylaminol-6,6-dimethyl-4,4-dioxo-5,6-dihydro-4H-41ambda6-[1,4,3]oxathiazin-5-yl}-N-pyridin-2-ylmethyl-benzamid |
| 195 | 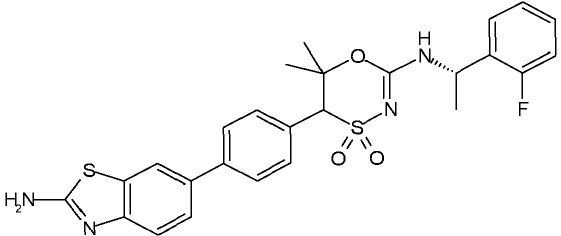 | C | 1.434 | 524.6 | 6-(4-{2-[(S)-1-(2-fluor-phenyl)-ethylamino]-6,6-dimethyl-4,4-dioxo-5,6-dihydro-4H-4lambda6-[1,4,3]oxathiazin-5-yl}-phenyl)-benzothiazol-2-ylamin |
| 196 | 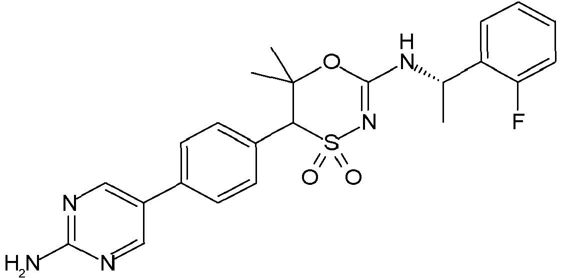 | C | 1.396 | 469.5 | {5-[4-(2-Amino-pyrimidin-5-yl)-phenyl]-6,6-dimethyl-4,4-dioxo-5,6-dihydro-4H-4lambda6-[1,4,3]oxathiazin-2-yl}-[(S)-1-(2-fluor-phenyl)-ethyl]-amin |

| | | | | | |
|---|---|---|---|---|---|
| * Isomerenreine Verbindung ** trans Verbindung | | | | | |

### Chromatographie-Methoden:

### Methode A

LC UV/MS: Agilent 1200 Series

Säule: Mercury MS, Luna C18(2), S-3 µm, 10 x 2.0 mm

Laufmittel: 0 min 93 % H₂O (0.05 % TFA) - 1.0 min 95 % Acetonitril - 1.45 min 95 % Acetonitril - 1.5 min 7 % Acetonitril (30 °C, Fluss 1.1 ml / min)

### Methode B

LC UV/MS: Agilent 1100 Series

Säule: Mercury MS, Luna C18(2), S-3 µm, 10 x 2,0 mm

Laufmittel: 0 min 93 % H₂O (0.05 % TFA) - 1.2 min 95 % Acetonitril - 1.4 min 95 % Acetonitril - 1.45 min 7 % Acetonitril (30 °C, Fluss 1.1 ml / min)

### Methode C

LC UV/MS: Agilent 1100 Series

Säule: YMC J'spere ODS H80, 80 Ǻ, S-4 µm, 20 x 2.1 mm

Laufmittel: 0 min 96 % H₂O (0.05 % TFA) - 2.0 min 95 % Acetonitril - 2.4 min 95 % Acetonitril - 2.45 min 4 % Acetonitril (30 °C, Fluss 1 ml / min)

Die Wirksamkeit der Verbindungen wurde wie folgt getestet:

### Enzymatischer 11beta-HSD Test:

Zum Messen der Wirkung der Verbindungen wurde ein auf SPA basierendes Nachweisverfahren (Solly *et al.* 2005) angewendet. Zunächst wurden 20 µl der humanen 11β-HSD1-Mikrosomenfraktion (0.2 µg Protein), zubereitet in 50 mM HEPES, 0.1% BSA (w/v), in eine Platte mit 384 Vertiefungen gegeben. Die Testverbindungen (0.09 µl) wurden in 100 % DMSO auf die Assayplatte übertragen. Die Reaktion wurde durch Zugabe von 20 µl [1,2-³H] Cortison (0.1 µCi / 100 mM) in Assaypuffer mit 25 mM HEPES, 100 mM KCl, 5 mM NaCl, 2 mM MgCl₂ und 0.25 mM NADPH gestartet. Die Platte wurde 1 Stunde lang bei 37 °C geschüttelt. Gleichzeitig wurde eine Stopplösung mit 20 mg / ml SPA-PVT-Perlen, 1,6 mg / ml monoklonalem Cortisol-Antikörper und 0.01 mM SSR110887 (Inhibitor aus dem Biovitrium-Patent) in 50 mM HEPES, 1 M NaCl und 1 M KCl bei Raumtemperatur gerührt. Zum Abbruch der Reaktion wurden in alle Vertiefungen jeweils 25 µl der Stopplösung gegeben. Die Platte wurde 1 weitere Stunde lang sachte bei Raumtemperatur geschüttelt und dann 1 Minute bei 500 gₐᵥ zentrifugiert, damit sich die SPA-Perlen absetzen konnten. Die Platte wurde dann in einem Wallac-1450-Microbeta-Gerät mit einem Standard-SPA-Programm gelesen (1 min Zählzeit / Vertiefung). Die Vergleichsverbindung war Glycyrrhetinsäure.

Protein und radioaktives Substrat wurden mit einem Biomek FX-Gerät (Beckman Coulter) zur Handhabung von Flüssigkeiten dispensiert. Die Testverbindungen wurden mit einem mit einem 90-nl-Pin-Tool ausgestatteten Cybi-Well (CyBio) zugesetzt.

Lit.: Solly S, Mundt SS, Zokian HJ, Juy-Fang Ding G, Hermanowski-Vosatka A, Strulovici B and Zheng W. High-throughput screening of 11β-Hydroxysteroid dehydrogenase type 1 in scintillation proximity format. Assay Drug Dev Technol 2005;3:377-384.

**Tabelle 2: Biologische Aktivität**

| Beispiel | IC₅₀ (nM) |
|---|---|
| 1 | 113 |
| 2 | 17 |
| 3 | 1270 |
| 4 | 14 |
| 5 | 1670 |
| 6 | 29 |
| 7 | 20 |
| 8 | 22 |
| 9 | 22 |
| 10 | 15 |
| 11 | 9 |
| 12 | 45 |
| 13 | 47 |
| 14 | 32 |
| 15 | 11 |
| 16 | 11 |
| 18 | 36 |
| 19 | 352 |
| 20 | 21 |
| 21 | 22 |
| 22 | 163 |
| 23 | 114 |
| 24 | 21 |
| 27 | 13 |
| 28 | 16 |
| 29 | 23 |
| 30 | 7 |
| 31 | 10 |
| 32 | 24 |
| 33 | 8 |
| 34 | 17 |
| 35 | 15 |
| 36 | 15 |
| 37 | 13 |
| 38 | 19 |
| 39 | 24 |
| 40 | 15 |
| 41 | 10 |
| 42 | 17 |
| 43 | 7 |
| 44 | 13 |
| 45 | 11 |
| 46 | 18 |
| 47 | 5 |
| 48 | 5 |
| 49 | 24 |
| 50 | 40 |
| 51 | 15 |
| 52 | 64 |
| 53 | 8 |
| 54 | 9 |
| 55 | 28 |
| 56 | 8 |
| 57 | 7 |
| 58 | 21 |
| 59 | 55 |
| 60 | 43 |
| 61 | 14 |
| 62 | 47 |
| 63 | 32 |
| 64 | 12 |
| 65 | 21 |
| 66 | 23 |
| 67 | 15 |
| 68 | 34 |
| 69 | 13 |
| 70 | 19 |
| 71 | 22 |
| 72 | 46 |
| 73 | 28 |
| 74 | 12 |
| 75 | 44 |
| 76 | 332 |
| 77 | 26 |
| 78 | 35 |
| 79 | 8 |
| 80 | 7 |
| 81 | 10 |
| 82 | 23 |
| 83 | 17 |
| 84 | 15 |
| 85 | 9 |
| 86 | 10 |
| 87 | 7 |
| 88 | 198 |
| 89 | 21 |
| 90 | 7 |
| 91 | 11 |
| 92 | 23 |
| 93 | 6 |
| 94 | 14 |
| 95 | 6 |
| 96 | 10 |
| 97 | 16 |
| 98 | 12 |
| 99 | 25 |
| 100 | 19 |
| 101 | 5 |
| 102 | 6 |
| 103 | 32 |
| 104 | 28 |
| 105 | 8 |
| 106 | 26 |
| 107 | 33 |
| 108 | 42 |
| 109 | 79 |
| 110 | 27 |
| 111 | 7 |
| 112 | 7 |
| 113 | 4 |
| 114 | 8 |
| 115 | 5 |
| 116 | 5 |
| 117 | 23 |
| 118 | 8 |
| 119 | 6 |
| 120 | 6 |
| 121 | 8 |
| 122 | 35 |
| 123 | 13 |
| 124 | 8 |
| 125 | 4 |
| 126 | 19 |
| 127 | 6 |
| 128 | 10 |
| 129 | 18 |
| 130 | 20 |
| 131 | 17 |
| 132 | 14 |
| 133 | 7 |
| 134 | 11 |
| 135 | 59 |
| 136 | 63 |
| 137 | 70 |
| 138 | 61 |
| 139 | 5 |
| 140 | 69 |
| 141 | 14 |
| 142 | 64 |
| 143 | 13 |
| 144 | 10 |
| 145 | 5 |
| 146 | 8 |
| 147 | 9 |
| 148 | 144 |
| 149 | 4 |
| 150 | 3 |
| 151 | 34 |
| 152 | 26 |
| 153 | 48 |
| 154 | 13 |
| 155 | 17 |
| 156 | 12 |
| 157 | 20 |
| 158 | 38 |
| 159 | 108 |
| 160 | 64 |
| 161 | 11 |
| 162 | 52 |
| 163 | 15 |
| 164 | 27 |
| 165 | 7 |
| 166 | 4 |
| 167 | 122 |
| 168 | 21 |
| 169 | 2 |
| 170 | 246 |
| 171 | 108 |
| 172 | 55 |
| 173 | 137 |
| 174 | 18 |
| 175 | 20 |
| 176 | 6 |
| 177 | 17 |
| 178 | 3 |
| 179 | 34 |
| 180 | 10 |
| 181 | 7 |
| 182 | 6 |
| 183 | 20 |
| 184 | 3 |
| 185 | 4 |
| 186 | 11 |
| 187 | 5 |
| 188 | 7 |
| 189 | 5 |
| 190 | 331 |
| 191 | 460 |
| 192 | 252 |
| 193 | 476 |
| 194 | 14 |
| 195 | 40 |
| 196 | 7 |

Aus den Messdaten ist abzulesen, dass die Verbindungen der Formel I 11beta-HSD1 (11beta-Hydroxysteroid Dehydrogenase Type 1) inhibieren und dadurch gut zur Behandlung von Hyperglykämie, Insulin Resistenz, Diabetes, Adipositas, Lipidstoffwechselstörungen und anderen Erkrankungen geeignet sind.

Nachfolgend wird die Herstellung einiger Beispiele detailliert beschrieben, die übrigen Verbindungen der Formel I wurden analog erhalten:

### Experimenteller Teil:

### 2-Methyl-propan-2-sulfinsäure 1-(2-chlor-3-fluor-phenyl)-meth-(E)-ylideneamid

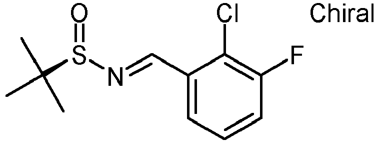

2-Chlor-3-fluor-benzaldehyd (2.5 g) und (R)-(+)-2-Methyl-2-propansulfinamid wurden in Dichlormethan (50 ml) gelöst und dann Titan(IV)-isopropoxid (23.6 ml) zugegeben. Es wurde drei Stunden unter Rückfluß erhitzt, der Ansatz dann auf Eis (150 g) gegossen und zehn Minuten kräftig gerührt. Es wurde über Kieselgur filtiert und das Filtrat mit Dichlormethan (3 x 50 ml) extrahiert. Die organische Phase wurden getrocknet (Na₂SO₄), filtriert und im Vakuum konzentriert. Das Produkt (4 g) wurde ohne weitere Reinigung weiter verwendet.

Auf gleiche Weise wurden folgende Verbindungen hergestellt:
2-Methyl-propan-2-sulfinsäure 1-(2,4-difluor-phenyl)-meth-(E)-ylideneamid 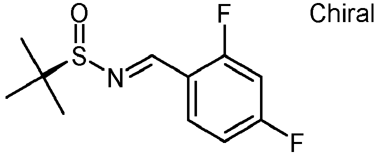 3-Amino-3-(2,5-difluor-phenyl)-propionsäure

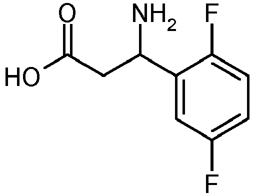

Eine Lösung aus 2,5-Difluor-benzaldehyd (5 g), Malonsäure (3.66 g) und Ammoniumacetat (5.42 g) in Ethanol (50 ml) wurde sechs Stunden unter Rückfluss erhitzt. Der Ansatz wurde abgekühlt und über Nacht stehen gelassen. Die entstandenen Kristalle wurden abfiltriert und mit Ethanol (5 ml) nachgewaschen. Das Produkt (2.94 g) wurde ohne weitere Reinigung verwendet.

Auf gleiche Weise wurden folgende Verbindungen hergestellt:
3-Amino-3-(5-fluor-2-methyl-phenyl)-propionsäure 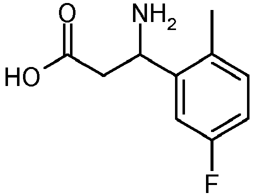
(S)-3-(2-Chlor-3-fluor-phenyl)-3-((R)-2-methyl-propan-2-sulfinylamino)-propionsäure methylester

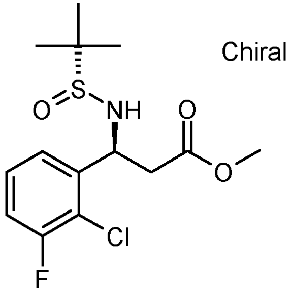

Zu Diisopropylamin (4.13 ml), gelöst in THF (200 ml), wurde langsam Butyllithium 1.6 N (17.55 ml) bei 0 °C zugetropft und dann dreißig Minuten gerührt. Anschließend wurde auf -75 °C abgekühlt und Methylacetat (2.13 ml) gelöst in THF (5 ml) zugegeben und weitere dreißig Minuten gerührt. Nun wurde Chlortitan-triisopropoxid (56.15 ml, 1 molar in Hexan) bei gleicher Temperatur zugetropft und wieder dreißig Minuten gerührt. 2-Methyl-propan-2-sulfinsäure 1-(2-chlor-3-fluor-phenyl)-meth-(E)-ylideneamid wurde in THF (10 ml) gelöst und bei -75 °C zugetropft und drei Stunden bei gleicher Temperatur gerührt. Die Mischung wurde auf kalte, gesättigte Ammoniumchloridlösung gegossen und mit Essigsäureethylester versetzt und fünfzehn Minuten gerührt. Das Phasengemisch wurde dann über Kieselgur geklärt, die organische Phase abgetrennt und die wässrige Phase mit Ethylacetat (2 x 50 ml) extrahiert. Die vereinigten organischen Phasen wurden getrocknet (Na₂SO₄), filtriert und konzentriert. Der Rückstand wurde chromatographisch (Kieselgel 40-63 µm, Ethylacetat) gereinigt. Man erhielt so das Produkt (4.1 g) mit einem de von 72 % laut NMR

Auf gleiche Weise wurden folgende Verbindungen hergestellt:
(S)-3-(2,4-difluor-phenyl)-3-((R)-2-methyl-propan-2-sulfinylamino)-propionsäure methylester 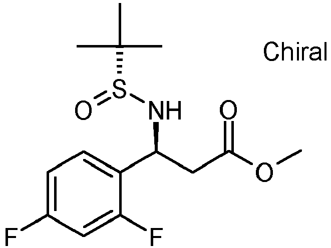
3-Amino-3-(2,5-difluor-phenyl)-propionsäure ethylester

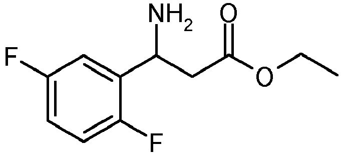

In einen Rundkolben mit Ethanol (50 ml) wurde Acetylchlorid (4 ml) bei Raumtemperatur getropft. Zu der so bereiteten ethanolischen Salzsäure Lösung wurde 3-Amino-3-(2,5-difluor-phenyl)-propionsäure (2.94 g) gegeben und drei Stunden bei 50 °C gerührt. Anschließend wurde im Vakuum konzentriert, der Rückstand mit Natronlauge 1N (100 ml) versetzt und sofort mit Dichlormethan (3 x 50 ml) extrahiert. Die vereinigten organischen Phasen wurden getrocknet (Na₂SO₄), filtriert und konzentriert. Das Produkt (3.2 g) wurde ohne weitere Reinigung verwendet.

Auf gleiche Weise wurden folgende Verbindungen hergestellt:
(S)-3-Amino-3-(5-fluor-2-methyl-phenyl)-propionsäure ethylester 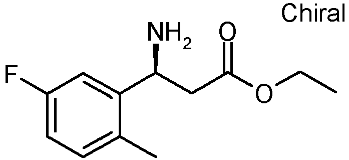
(S)-3-Amino-3-(2,5-difluor-phenyl)-propionsäure methylester 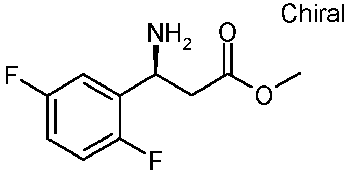 (S)-3-Amino-3-(5-fluor-2-methyl-phenyl)-propionsäure methylester 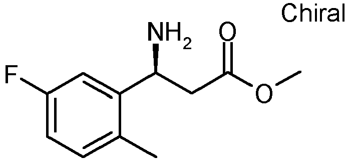 (S)-3-Amino-3-(2,5-difluor-phenyl)-propionsäure

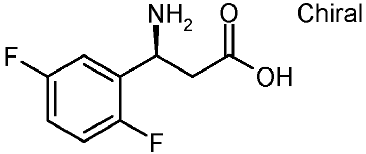

3-Amino-3-(2,5-difluor-phenyl)-propionsäure ethylester (3 g) wurden in Wasser emulgiert (pH = 9.2) und mit Kailumdihydrogenphosphat (5 mg) versetzt (pH = 8.5). Nach Zugabe von Amono Lipase PS (150 mg) wurde über Nacht gerührt. Der pH-Wert bleibt bei 7.5. Der Ansatz wurde nun mit Wasser (30 ml) versetzt und über Kieselgur filtriert und noch mehrmals mit Wasser nachgewaschen. Die wässrige Phase wurde nun mit Dichlormethan extrahiert (3 x 30 ml), die organische Phase getrocknet (Na2S04) und konzentriert. Man erhielt so das Produkt (1.3 g) mit einem ee = 90 % nach HPLC.

Auf gleiche Weise wurden folgende Verbindungen hergestellt:
(S)-3-Amino-3-(5-fluor-2-methyl-phenyl)-propionsäure 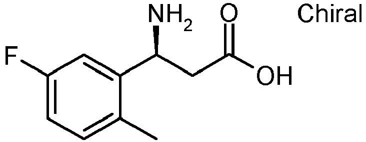
(S)-3-Amino-3-(3-chlor-2-fluor-phenyl)-propionsäure methylester

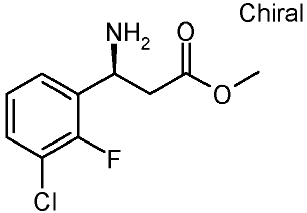

Zu einer Lösung aus Acetylchlorid (4 ml) in Methanol (50 ml) wurde (S)-3-(2-Chlor-3-fluor-phenyl)-3-((R)-2-methyl-propan-2-sulfinylamino)-propionsäure methylester (4.1 g) gelöst in Methanol (10 ml) bei Raumtemperatur zugegeben. Nach einer Stunde wurde die Mischung konzentriert, in Dichlormethan (100 ml) aufgenommen und mit Natronlauge 1N (100 ml) gewaschen. Die wässrige Phase wurde noch zweimal mit Dichlormethan gewaschen. Die vereinigten organischen Phasen wurden getrocknet (Na₂SO₄), filtriert und konzentriert. Der Rückstand (2.8 g) wurde ohne weitere Reinigung verwendet.

Auf gleiche Weise wurden folgende Verbindungen hergestellt:
(S)-3-Amino-3-(2,4-difluor-phenyl)-propionsäure methylester 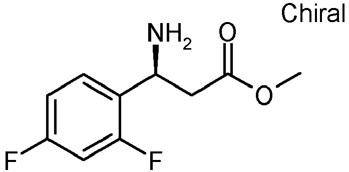
(S)-3-Amino-3-(2-chlor-3-fluor-phenyl)-propan-1-ol

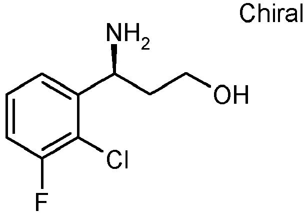

Zu einer Lösung aus Lithiumaluminiumhydrid 1N (24.2 ml in THF) in THF (20 ml) wurde bei 0 °C (S)-3-Amino-3-(3-chlor-2-fluor-phenyl)-propionsäure methylester (2.8 g) gelöst in THF (5 ml) zugetropft und eine Stunde gerührt. Dann wurde der Ansatz auf gesättigte Natriumchloridlösung (50 ml) getropft und fünf Minuten gerührt. Dann wurde Natronlauge 1N (50 ml) zugegeben und weitere fünf Minuten gerührt. Die Mischung wurde dann mit Ethylacatat (3 x 50 ml) extrahiert, getrocknet (Na₂SO₄) und konzentriert. Das Produkt (2.23 g) wurde ohne weitere Aufreinigung verwendet.

Auf gleiche Weise wurden folgende Verbindungen hergestellt:
(S)-3-Amino-3-(2,4-difluor-phenyl)-propan-1-ol 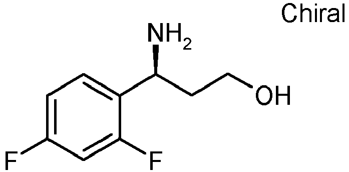
(S)-3-Amino-3-(2,5-difluor-phenyl)-propan-1-ol 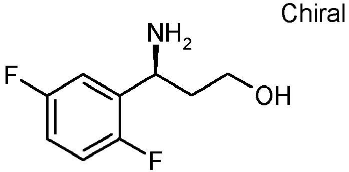 (S)-3-Amino-3-(5-fluor-2-methyl-phenyl)-propan-1-ol 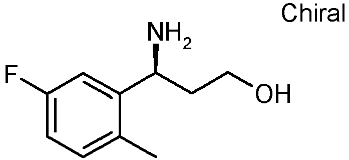
(S)-3-(tert-Butyl-dimethyl-silanyloxy)-1-(5-chlor-2-fluor-phenyl)-propylamin

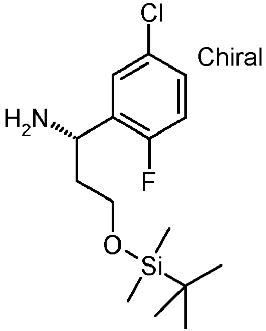

Das Lithiumaluminiumhydrid 1N (100 ml) in THF wurde vorgelegt und (S)-3-Amino-3-(5-chloro-2-fluoro-phenyl)-propionsäuremethylester (in THF unlöslich) portionsweise unter Eiskühlung zugegeben. Die Kühlung wurde dann entfernt und 1 Stunde nachgerührt. Anschließend wurde unter Eis 5ml H₂O und 5 ml 5M NaOH zugetropft und vorsichtig 15ml H₂O zugegeben, dann wurde 4 Tage gerührt. Der Niederschlag wurde über Celite filtriert, mit THF (3 x 30 ml) nachgewewaschen, dann das Filtrat eingeengt. Der Rückstand wurde mit Dichlormethan (50 ml) aufgenommen und über MgS04 getrocknet, filtriert und einrotiert. Der Rückstand (2.95 g) wurde in Dichlormethan (20 ml) gelöst, mit DIPEA und portionsweise mit t-Butyldimethyl-chlorsilan versetzt. Es wurde bei 25 °C über Nacht gerührt.

Der Ansatz wurde zweimal mit 50 ml 5% NaHCO₃-Lsg. gewaschen, die organische Phase über Na₂SO₄ getrocknet und eingeengt. Auswaage: 5.16 g. Dann wurde mit Hilfe des Flashmasters gereinigt. 70 g Säule (Normalphase); Fraktionsgröße: 20ml; Fluss: 19ml/min Fraktionen 17 - 40 vereinigt. Man erhielt so das Produkt (3.64 g)mit dem Molekulargewicht 317.9 (C₁₅H₂₆ClFNOSi); MS (ESI): 318 (M+H+).

### (S)-3-(tert-Butyl-dimethyl-silanyloxy)-1-phenyl-propylamin

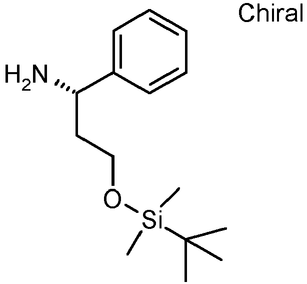

S-3-Amino-3-phenyl-1-propanol (4.72 g) wurde in Dichlormethan (60 mL) gelöst, mit Triethylamin (6.36 g) und tert.-Butyl-dimethyl-chlorosilan (4.1 g) versetzt und 3 Stunden bei Raumtemperatur gerührt. Dann wurde mit Wasser (3 x 50 mL) gewaschen und über eine Phasenseparatorkartusche getrocknet. Man erhielt so das Produkt mit dem Molekulargewicht 265.5 g / mol (C15H27NOSi), MS (ESI): (M+H+) 266 g / mol.

Auf gleiche Weise wurden folgende Verbindungen hergestellt:
(S)-3-(tert-Butyl-dimethyl-silanyloxy)-1-(2-fluor-phenyl)-propylamin 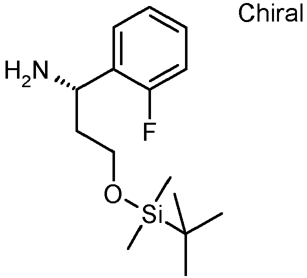
(S)-3-(tert-Butyl-dimethyl-silanyloxy)-1-(3-chlor-phenyl)-propylamin 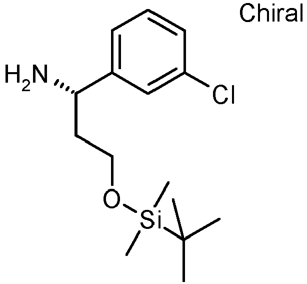
(S)-3-(tert-Butyl-dimethyl-silanyloxy)-1-(4-chlor-phenyl)-propylamin 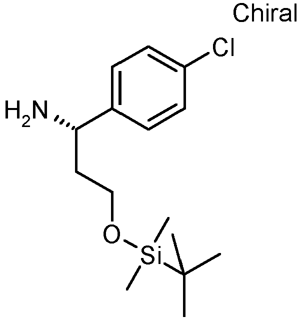 (S)-3-(tert-Butyl-dimethyl-silanyloxy)-1-(2-chlor-phenyl)-propylamin 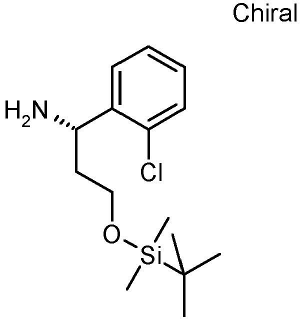
(S)-3-(tert-Butyl-dimethyl-silanyloxy)-1-(2-chlor-3-fluor--phenyl)-propylamin 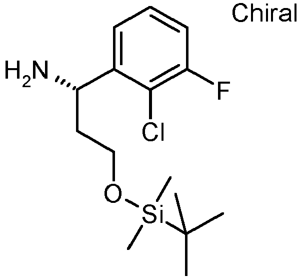 (S)-3-(tert-Butyl-dimethyl-silanyloxy)-1-(2,4-difluor-phenyl)-propylamin 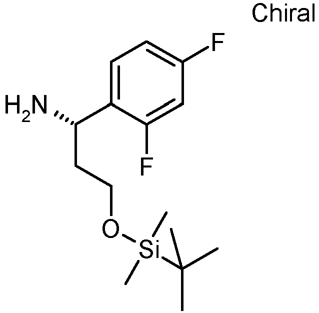 (S)-3-(tert-Butyl-dimethyl-silanyloxy)-1-(2,5-difluor-phenyl)-propylamin 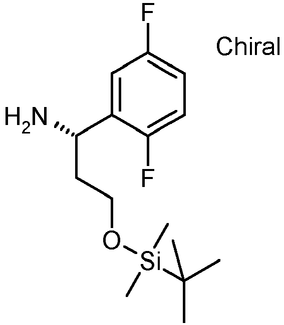 (S)-3-(tert-Butyl-dimethyl-silanyloxy)-1-(5-fluor-2-methyl-phenyl)-propylamin

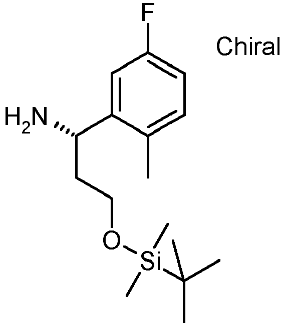

Nachfolgend wird die Synthese von 2-Hydroxy-2-methyl-1-phenyl-propan-1-sulfonsäureamid beschrieben. Dieser Baustein wurde anschließend in verschiedene Reaktionen eingesetzt.

### N-(2,4-Dimethoxy-benzyl)-C-phenyl-methansulfonamid

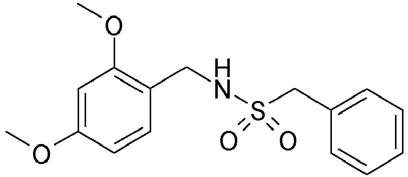

Unter Eiskühlung wurden 5.00 g Phenyl-methansulfonylchlorid in 40 ml Dichlormethan vorgelegt und langsam eine Lösung aus 8.04 ml 2,4-Dimethoxybenzylamin in 10 ml Dichlormethan zugetropft. Zur verbesserten Rührbarkeit wurden weitere 25 ml Dichlormethan zugegeben und die Reaktionsmischung auf Raumtemperatur kommen gelassen. Nach 2 Stunden Rühren wurde die Reaktionsmischung mit 100 ml Wasser, mit 100 ml 0.2 N wässriger Salzsäure, mit 100 ml gesättigter wässriger Natriumhydrogencarbonat-Lösung und abschließend noch einmal mit 100 ml Wasser gewaschen. Die organische Phase wurde über Na₂SO₄ getrocknet, einrotiert und im Hochvakuum getrocknet. Der Rückstand (8.42 g) wurde ohne weitere Reinigung in die nächste Reaktion eingesetzt.

### 2-Hydroxy-2-methyl-1-phenyl-propan-1-sulfonsäure-2,4-dimethoxy-benzylamid

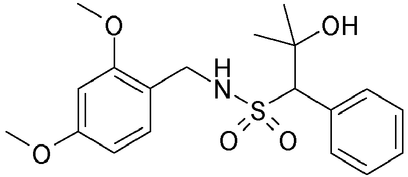

Unter Inertgas wurden 2.00 g N-(2,4-Dimethoxy-benzyl)-C-phenyl-methansulfonamid in 40 ml THF vorgelegt und anschließend bei einer Temperatur von -78 °C 9.54 ml einer 1.5 N Butyllithium-Lösung in Hexan tropfenweise zugegeben und 5 Minuten unter Eiskühlung gerührt. Anschließend wurde die Reaktionslösung erneut auf -78 °C abgekühlt und eine Lösung von 2.01 ml Aceton in 4 ml THF zugegeben. Die Mischung wurde auf Raumtemperatur kommen gelassen und 30 Minuten gerührt. Die Reaktionslösung wurde mit 0.82 ml Essigsäure und 50 ml Ethylacetat versetzt, mit 30 ml einer gesättigten wässrigen Natriumhydrogencarbonat-Lösung und 30 ml einer gesättigten wässrigen Natriumchlorid-Lösung gewaschen, die organische Phase über Na₂SO₄ getrocknet und einrotiert. Der Rückstand (2.67 g) wurde ohne weitere Reinigung in die nächste Reaktion eingesetzt.

### 2-Hydroxy-2-methyl-1-phenyl-propan-1-sulfonsäureamid

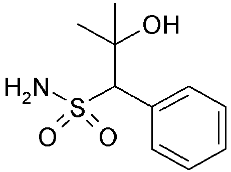

Eine Lösung aus 2.67 g 2-Hydroxy-2-methyl-1-phenyl-propan-1-sulfonsäure-2,4-dimethoxy-benzylamid in 20 ml Dichlormethan wurde mit 3.74 ml Trifluoressigsäure versetzt und 2 Stunden bei Raumtemperatur gerührt. Die Ansatzlösung wurde 2 x mit 100 ml Toluol koevaporiert und das Rohprodukt mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (0.90 g) mit dem Molekulargewicht 229.3 g / mol (C₁₀H₁₅NO₃S).

### Cyclohexyl-(6,6-dimethyl-4,4-dioxo-5-phenyl-5,6-dihydro-4H-4lambda6-[1,4,3]oxathiazin-2-yl)-amin

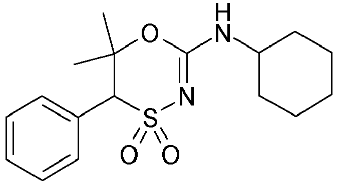

Eine Lösung aus 150 mg 2-Hydroxy-2-methyl-1-phenyl-propan-1-sulfonsäureamid und 97 mg Cylohexylisothiocyanat in 1.5 ml NMP wurde mit 0.20 ml einer 2 N Lösung von Natrium-bis(trimethylsilyl)-amidin THF versetzt. Nach 20 Minuten Rühren wurden 71 mg N-Bromsuccinimid zugegeben und weitere 15 Minuten bei Raumtemperatur gerührt. Die Reaktionslösung wurde mit 50 ml Wasser versetzt und 2 x mit 50 ml Ethylacetat extrahiert. Die vereinten organischen Phasen wurden über Na₂SO₄ getrocknet und einrotiert. Der Rückstand wurde im Reinigungslabor über präparative HPLC gereinigt und die produkthaltigen Fraktionen unter vermindertem Druck vom organischen Lösungsmittel befreit. Der wässrige Rückstand wurde 3 x mit Dichlormethan extrahiert, die vereinten organischen Phasen über eine Phasenseparator-Kartusche (Chromabond^{®} PTS) getrocknet und einrotiert. Der Rückstand wurde in einer Mischung aus Acetonitril und Wasser gelöst und lyophilisiert. Man erhielt so das Produkt (46 mg) mit dem Molekulargewicht 336.4 g / mol (C₁₇H₂₄N₂O₃S); MS (ESI): m / e = 337 (M+H⁺).

### (1S,3S)-3-(6,6-Dimethyl-4,4-dioxo-5-phenyl-5,6-dihydro-4H-4lambda6-[1,4,3]oxathiazin-2-ylamino)-cyclohexanol

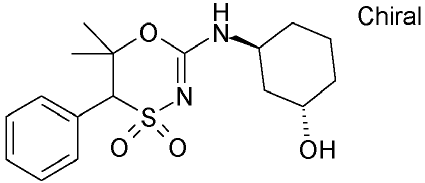

173 mg S,S-Aminocyclohexanol wurden in 5 ml Dichlormethan gelöst und mit 311 mg 1,1'-Thiocarbonyldiimidazol versetzt. Nach 5 Minuten Rühren bei Raumtemperatur wurden noch einmal 5 ml Dichlormethan nachgegeben, um die Rührbarkeit der Suspension zu verbessern und weitere 80 Minuten gerührt. Die Reaktionslösung wurde mit einem Gemisch aus 10 ml Diethylether und 10 ml n-Pentan versetzt und 3 x mit 15 ml Wasser gewaschen, über Na₂SO₄ getrocknet und einrotiert. Unter Inertgas wurden der Rückstand und 300 mg 2-Hydroxy-2-methyl-1-phenyl-propan-1-sulfonsäureamid in 3 ml NMP gelöst und mit 0.39 ml einer 2 N Lösung von Natrium-bis(trimethylsilyl)-amid in THF versetzt. Nach 30 Minuten Rühren wurden 140 mg N-Bromsuccinimid zugesetzt und die resultierende Reaktionslösung 45 Minuten bei gleichbleibender Temperatur gerührt. Die Umsetzung wurde mit LCMS überprüft. Da sie noch unvollständig war, wurden weitere 70 mg N-Bromsuccinimid zugegeben und der Ansatz für 48 Stunden gerührt. Das Reaktionsgemisch wurde mit 1 ml einer Mischung aus Acetonitril und Wasser (9:1) verdünnt und im Reinigungslabor über präparative HPLC gereinigt. Die produkthaltigen Fraktionen wurden vereinigt und der wässrige Rückstand lyophilisiert. So erhielt man das Produkt (148 mg) mit dem Molekulargewicht 352.4 g / mol (C₁₇H₂₄N₂O₄S); MS (ESI): m / e = 353 (M+H⁺).

(1R,3R)-3-(6,6-Dimethyl-4,4-dioxo-5-phenyl-5,6-dihydro-4H-4lambda6-[1,4,3]oxathiazin-2-ylamino)-cyclohexanol wurde ebenfalls mit dieser Herstellungsvorschrift synthetisiert.

Die verwendeten Aminocyclohexanole wurden wie folgt hergestellt:
P. Bernardelli, M. Bladon, E. Lorthiois, A.C. Manage, F. Vernige, R. Wriggleswort, Tetrahedron: Asymmetry 15 2004, 1451-1455
L.M. Levy, G. de Gonzalo, V. Gotor, Tetrahedron: Asymmetry 15 2004, 2051-2056

### (S)-3-(6,6-Dimethyl-4,4-dioxo-5-phenyl-5,6-dihydro-4H-4lambda6-[1,4,3]oxathiazin-2-ylamino)-3-phenyl-propan-1-ol

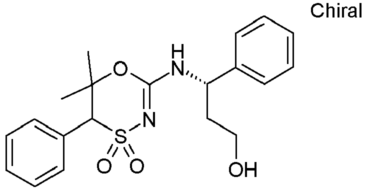

### (S)-3-(tert-Butyl-dimethyl-silanyloxy)-1-phenyl-propylamin

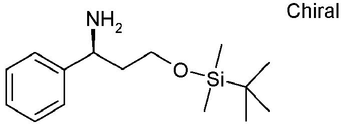

Es wurden 1.50 g (S)-3-Amino-3-phenylpropan-1-ol Hydrochlorid in 20 ml Dichlormethan vorgelegt und anschließend 2.77 ml Triethylamin und 1.30 g tert-Butyldimethylchlorsilan zugegeben. Nach 3 Stunden Rühren bei Raumtemperatur wurde die Reaktionslösung 3 x mit 30 ml Wasser extrahiert, über eine Phasenseparator-Kartusche (Chromabond^{®} PTS) getrocknet, einrotiert und im Hochvakuum getrocknet. Man erhielt so das Produkt (2.06 g) mit dem Molekulargewicht 265.5 g / mol (C₁₅H₂₇NOSi); MS (ESI): m / e = 266 (M+H⁺).

### (S)-3-(6,6-Dimethyl-4,4-dioxo-5-phenyl-5,6-dihydro-4H-4lambda6-[1,4,3]oxathiazin-2-ylamino)-3-phenyl-propan-1-ol

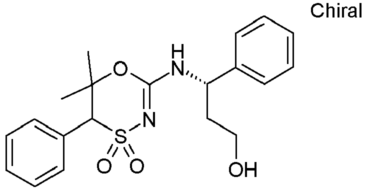

### 175 mg (S)-3-(tert-Butyl-dimethyl-silanyloxy)-1-phenyl-propylamin wurden in 2 ml

Dichlormethan gelöst und mit 121 mg 1,1'-Thiocarbonyldiimidazol versetzt. Nach 20 Minuten Rühren bei Raumtemperatur wurde die Reaktionslösung mit einem Gemisch aus 20 ml Diethylether und 20 ml n-Pentan versetzt und 3 x mit 30 ml Wasser gewaschen, über MgSO₄ getrocknet und einrotiert. Unter Inertgas wurden der Rückstand und 130 mg 2-Hydroxy-2-methyl-1-phenyl-propan-1-sulfonsäureamid in 1.5 ml NMP gelöst und mit 0.17 ml einer 2 N Lösung von Natrium-bis(trimethylsilyl)-amid in THF versetzt. Nach 15 Minuten Rühren wurden 61 mg N-Bromsuccinimid zugesetzt und die resultierende Reaktionslösung 5 Minuten bei gleichbleibender Temperatur gerührt. Die Reaktionslösung wurde mit 50 ml Wasser versetzt und 2 x mit 50 ml Ethylacetat extrahiert. Die vereinten organischen Phasen wurden über MgSO4 getrocknet und einrotiert. Zur Abspaltung der Schutzgruppe wurde der Rückstand in 4 ml Methanol aufgenommen und nach Zugabe von 0.20 ml konzentrierter Salzsäure über Nacht bei Raumtemperatur gerührt. Die Reaktionslösung wurde mit DCM und Wasser versetzt, die organische Phase über MgSO₄ getrocknet, einrotiert und der Rückstand im Reinigungslabor über präparative HPLC gereinigt. Die produkthaltigen Fraktionen wurden vereinigt, die enthaltene Trifluoressigsäure mit gesättigter wässriger Natriumhydrogencarbonat-Lösung neutralisiert und unter vermindertem Druck vom Lösungsmittel befreit. Der wässrige Rückstand wurde 3 x mit Dichlormethan extrahiert, die vereinten organischen Phasen über eine Phasenseparator-Kartusche (Chromabond^{®} PTS) getrocknet und einrotiert. Der Rückstand wurde in einer Mischung aus Acetonitril und Wasser gelöst und lyophilisiert. Man erhielt so das Produkt (42 mg) mit dem Molekulargewicht 388.5 g / mol (C₂₀H₂₄N₂O₄S); MS (ESI): m / e = 389 (M+H⁺).

Die Herstellung der beiden nachfolgenden Verbindungen erfolgte analog:
(S)-3-(6,6-Dimethyl-4,4-dioxo-5-phenyl-5,6-dihydro-4H-4lambda6-[1,4,3]oxathiazin-2-ylamino)-3-phenyl-propan-1-ol Stereomer 1 und Stereomer 2

Auf der Stufe des 2-Hydroxy-2-methyl-1-phenyl-propan-1-sulfonsäure-2,4-dimethoxy-benzylamids wurde eine chirale Trennung von 1.28 g des Racemats durchgeführt. Anschließend wurden beide Stereomere einzeln weiter umgesetzt.
HPLC-Anlage: Waters Pump 2695, PAD 2996
Säule: Chiralpak AS-H/53, 5 µm, 250 x 4.6 mm
Laufmittel: n-Heptan : Ethanol : Methanol 5 : 1 : 1 (30 °C, Fluss 1 ml / min) Retentionszeiten:
5.323 Minuten (Stereomer 1, 616 mg)
6.585 Minuten (Stereomer 2, 630 mg).

### (S)-3-[5-(4-Benzyloxy-phenyl)-6,6-dimethyl-4,4-dioxo-5,6-dihydro-4H-4lambda6-1,4,3-oxathiazin-2-ylamino]-3-phenyl-propan-1-ol

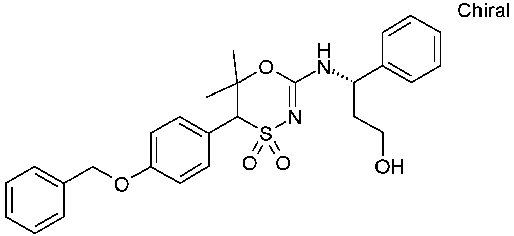

### (4-Benzyloxy-phenyl)-methansulfonsäure Natriumsalz

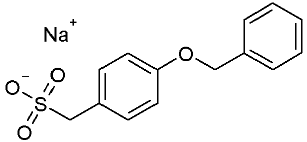

Eine Mischung aus 25.51 g 4-Benzyloxybenzylchlorid und 28.78 g Natriumsulfit in 96 ml Wasser wurde für 21 Stunden bei 75 °C gerührt. Nach dem Abkühlen auf Raumtemperatur wurde die Reaktionslösung mit 100 ml tert-Butyl-methylether verrührt, der Feststoff abgesaugt, 2 x mit 20 ml tert-Butyl-methylether, 1 x mit 20 ml Eiswasser, 2 x mit 20 ml Aceton und 2 x mit 20 ml Dichlormethan gewaschen. Der Niederschlag wurde über Nacht im Vakuumtrockenschrank bei 40 °C getrocknet. Man erhielt so das Produkt (6.20 g) mit dem Molekulargewicht 300.3 g / mol (C₁₄H₁₃O₄S.Na), MS (ESI): m / e = 301 (M+H⁺).

### (4-Benzyloxy-phenyl)-methansulfonylchlorid

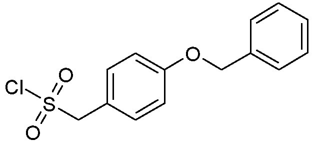

Unter Inertgas wurden 5.70 g (4-Benzyloxy-phenyl)-methansulfonsäure Natriumsalz und 1.48 ml N,N-Dimethyl-formamid in 110 ml THF vorgelegt, anschließend bei einer Temperatur von -20 °C 4.17 ml Oxalylchlorid zugetropft und die Reaktionslösung innerhalb von 15 Minuten auf 0 °C kommen gelassen. Die Reaktionslösung wurde mit 100 ml Diethylether verdünnt und mit Wasser, verdünnter wässriger Natriumhydrogencarbonat-Lösung und gesättigter Natriumchlorid-Lösung gewaschen. Die organische Phase wurde über MgSO₄ getrocknet und einrotiert. Der Rückstand (4.89 g) wurde ohne weitere Reinigung in die nächste Reaktion eingesetzt.

### C-(4-Benzyloxy-phenyl)-N-(2,4-dimethoxy-benzyl)-methansulfonamid

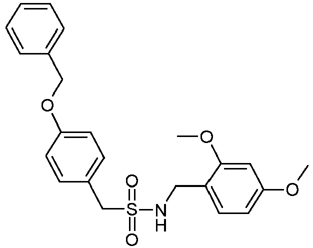

Unter Inertgas wurden 4.89 g (4-Benzyloxy-phenyl)-methansulfonylchlorid 120 ml THF vorgelegt, anschließend bei einer Temperatur von -20 °C 6.10 ml 2,4-Dimethoxybenzylamin zugetropft und die Reaktionslösung innerhalb von 30 Minuten auf 0 °C kommen gelassen. Die Reaktionslösung wurde mit 150 ml Ethylacetat verdünnt und mit Wasser, verdünnter wässriger Kaliumhydrogensulfat-Lösung, gesättigter wässriger Natriumhydrogencarbonat-Lösung und gesättigter Natriumchlorid-Lösung gewaschen. Die organische Phase wurde über MgSO₄ getrocknet und einrotiert. Das Rohprodukt wurde mittels Normalphasenchromatographie über den Flashmaster mit einem n-Heptan/Ethylacetat-Gradienten gereinigt. Die produkthaltigen Fraktionen wurden vereinigt, einrotiert und im Hochvakuum getrocknet. Man erhielt so das Produkt (3.23 g) mit dem Molekulargewicht 427.5 g / mol (C₂₃H₂₅NO₄S).

### 1-(4-Benzyloxy-phenyl)-2-hydroxy-2-methyl-propan-1-sulfonsäure-2,4-dimethoxy-benzylamid

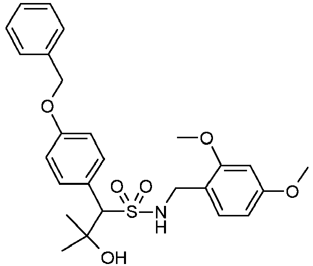

Unter Inertgas wurden 3.22 g C-(4-Benzyloxy-phenyl)-N-(2,4-dimethoxy-benzyl)-methansulfonamid in 70 ml THF vorgelegt und anschließend bei einer Temperatur von -78 °C 12.50 ml einer 1.8 N Phenyllithium-Lösung tropfenweise zugegeben. Die Reaktionsmischung wurde auf 0 °C kommen gelassen und kurz nachgerührt, dann erneut auf -78 °C abgekühlt und 4.00 ml Aceton tropfenweise zugegeben. Die Mischung wurde innerhalb von 15 Minuten auf -20 °C kommen gelassen, mit 1.29 ml Essigsäure und 100 ml Ethylacetat versetzt und mit gesättigter wässriger Natriumhydrogencarbonat-Lösung und gesättigter wässriger Natriumchlorid-Lösung gewaschen. Die organische Phase wurde über MgSO₄ getrocknet und einrotiert. Das Rohprodukt wurde mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (3.65 g) mit dem Molekulargewicht 485.6 g / mol (C₂₆H₃₁NO₆S).

### 1-(4-B enzyloxy-phenyl)-2-hydroxy-2-methyl-prop an-1-sulfonsäureamid

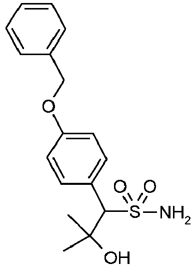

Eine Lösung aus 3.65 g 1-(4-Benzyloxy-phenyl)-2-hydroxy-2-methyl-propan-1-sulfonsäure-2,4-dimethoxy-benzylamid in 20 ml Dichlormethan wurde mit 4.00 ml Trifluoressigsäure versetzt und 20 Minuten bei Raumtemperatur gerührt. Die Umsetzung wurde mit LCMS überprüft. Da sie unvollständig war, wurden noch einmal 2.00 ml Trifluoressigsäure zugegeben und weitere 2 Stunden gerührt. Die Ansatzlösung wurde mit 20 ml Wasser versetzt und mit gesättigter wässriger Natriumhydrogencarbonat-Lösung neutralisiert. Der Niederschlag wurde über eine Glasfritte abgesaugt, mit Dichlormethan und Wasser gewaschen und im Exsikkator über P₂O₅ getrocknet. Man erhielt so das Produkt (1.95 g) mit dem Molekulargewicht 335.4 g / mol (C₁₇H₂₁NO₄S).

### [5-(4-Benzyloxy-phenyl)-6,6-dimethyl-4,4-dioxo-5,6-dihydro-4H-4lambda6-1,4,3-oxathiazin-2-yl]-[(S)-3-(tert-butyl-dimethyl-silanyloxy)-1-phenyl-propyl]-amin

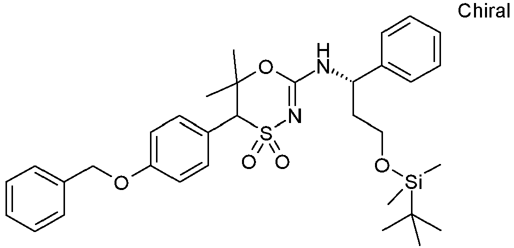

921 mg (S)-3-(tert-Butyl-dimethyl-silanyloxy)-1-phenyl-propylamin wurden in 10 ml Dichlormethan gelöst und mit 638 mg 1,1'-Thiocarbonyldiimidazol versetzt. Nach 30 Minuten Rühren bei Raumtemperatur wurde die Reaktionslösung mit einem Gemisch aus 50 ml Diethylether und 50 ml n-Pentan versetzt und 3 x mit 50 ml Wasser gewaschen, über MgSO₄ getrocknet und einrotiert. Unter Inertgas wurden der Rückstand und 1000 mg 1-(4-Benzyloxy-phenyl)-2-hydroxy-2-methyl-propan-l-sulfonsäureamid in 8 ml NMP gelöst und mit 1.49 ml einer 2 N Lösung von Natrium-bis(trimethylsilyl)-amid in THF tropfenweise versetzt. Nach 15 Minuten Rühren wurden 531 mg N-Bromsuccinimid zugesetzt und die resultierende Reaktionslösung 10 Minuten bei gleichbleibender Temperatur gerührt. Die Reaktionslösung wurde mit 100 ml Wasser versetzt und 2 x mit 100 ml Ethylacetat extrahiert. Die vereinten organischen Phasen wurden über MgSO₄ getrocknet, einrotiert und der Rückstand mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (805 mg) mit dem Molekulargewicht 608.9 g / mol (C₃₃H₄₄N₂O₅SSi); MS (ESI): m / e = 609 (M+H⁺).

### (S)-3-[5-(4-Benzyloxy-phenyl)-6,6-dimethyl-4,4-dioxo-5,6-dihydro-4H-4lambda6-1,4,3-oxathiazin-2-ylamino]-3-phenyl-propan-1-ol

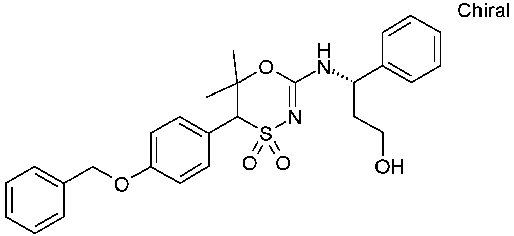

Zur Abspaltung der Schutzgruppe wurden 40 mg [5-(4-Benzyloxy-phenyl)-6,6-dimethyl-4,4-dioxo-5,6-dihydro-4H-4lambda6-1,4,3-oxathiazin-2-yl]-[(S)-3-(tert-butyl-dimethyl-silanyloxy)-1-phenyl-propyl]-amin in 5 ml Methanol gelöst und nach Zugabe von 1 ml 2 N Salzsäure für 1 Stunde bei Raumtemperatur gerührt. Die Reaktionslösung wurde mit 2 ml 1 N wässriger Natronlauge neutralisiert, einrotiert und der Rückstand im Reinigungslabor über präparative HPLC gereinigt. Die produkthaltigen Fraktionen wurden vereinigt und lyophilisiert. Man erhielt so das Produkt (19.7 mg) mit dem Molekulargewicht 494.6 g / mol (C₂₇H₃₀N₂O₅S), MS (ESI): m / e = 495 (M+H⁺).

### 4-[2-((S)-3-Hydroxy-1-phenyl-propylamino)-6,6-dimethyl-4,4-dioxo-5,6-dihydro-4H-4lambda6-1,4,3-oxathiazin-5-yl]-phenol

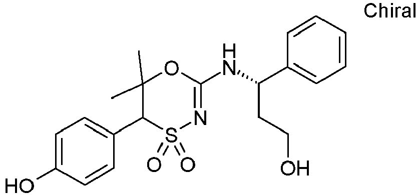

Unter Inertgas wurden 762 mg [5-(4-Benzyloxy-phenyl)-6,6-dimethyl-4,4-dioxo-5,6-dihydro-4H-4lambda6-1,4,3-oxathiazin-2-yl]-[(S)-3-(tert-butyl-dimethyl-silanyloxy)-l-phenyl-propyl]-amin in 20 ml Methanol vorgelegt und unter Zusatz von 133 mg Pd/C 10 % für 1 Stunde unter Wasserstoff-Atmosphäre gerührt. Die Vollständigkeit der Umsetzung wurde mit LCMS geprüft, die Reaktion nochmals mit 133 mg Pd/C 10 % und zusätzlich 0.52 ml Triethylamin versetzt und weitere 60 Minuten unter Wasserstoff-Atmosphäre gerührt. Laut LCMS blieb die Abspaltung der Silyl-Schutzgruppe unvollständig. Nach Filtration von den festen Rückständen wurde das Filtrat unter vermindertem Druck vom Lösungsmittel befreit. Das Rohprodukt wurde mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (104 mg) mit dem Molekulargewicht 404.5 g / mol (C₂₀H₂₄N₂O₅S); MS (ESI): m / e = 405 (M+H⁺).

Durch unvollständige Abspaltung der Silyl-Schutzgruppe wurde als Nebenprodukt 4-{2-[(S)-3-(tert-Butyl-dimethyl-silanyloxy)-1-phenyl-propylamino]-6,6-dimethyl-4,4-dioxo-5,6-dihydro-4H-4lambda6-1,4,3-oxathiazin-5-yl}-phenol (454 mg) mit dem Molekulargewicht 518.8 g / mol (C₂₆H₃₈N₂O₅SSi); MS (ESI): m / e = 519 (M+H⁺) isoliert.

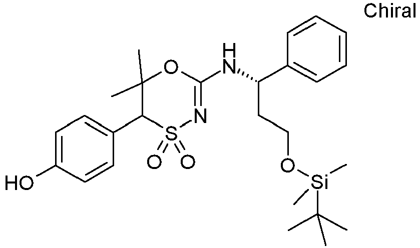

### (S)-3-[5-(4-Methoxy-phenyl)-6,6-dimethyl-4,4-dioxo-5,6-dihydro-4H-4lambda6-1,4,3-oxathiazin-2-ylamino]-3-phenyl-propan-1-ol

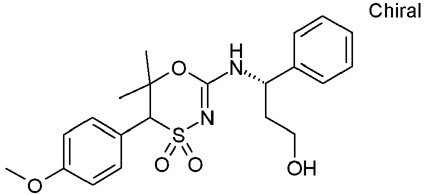

Zu einer Lösung von 40 mg 4-[2-((S)-3-Hydroxy-1-phenyl-propylamino)-6,6-dimethyl-4,4-dioxo-5,6-dihydro-4H-4lambda6-1,4,3-oxathiazin-5-yl]-phenol in 1 ml N,N-Dimethylformamid wurden bei Raumtemperatur 11 mg Kalium-tert-butylat und 28 mg Methyliodid zugegeben und die Reaktionsmischung für 3 Stunden bei gleichbleibender Temperatur gerührt. Die Reaktionslösung wurde im Reinigungslabor über präparative HPLC gereinigt. Die produkthaltigen Fraktionen wurden vereinigt und lyophilisiert. Man erhielt so das Produkt (10 mg) mit dem Molekulargewicht 418.5 g / mol (C₂₁H₂₆N₂O₅S); MS (ESI): m / e = 419 (M+H⁺).

### Trifluor-methansulfonsäure-4-{2-[(S)-3-(tert-butyl-dimethyl-silanyloxy)-1-phenyl-propylamino]-6,6-dimethyl-4,4-dioxo-5,6-dihydro-4H-4lambda6-1,4,3-oxathiazin-5-yl}-phenylester

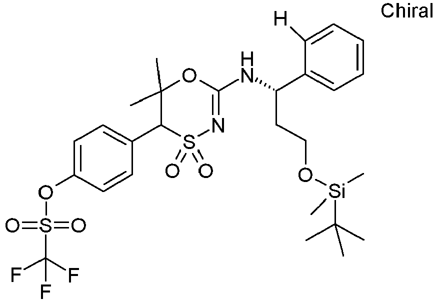

Unter Inertgas wurden 450 mg 4-{2-[(S)-3-(tert-Butyl-dimethyl-silanyloxy)-1-phenyl-propylamino]-6,6-dimethyl-4,4-dioxo-5,6-dihydro-4H-4lambda6-1,4,3-oxathiazin-5-yl}-phenol in 10 ml Methylenchlorid vorgelegt und anschließend unter Eiskühlung 0.18 ml Triethylamin und 0.18 ml Trifluormethansulfonsäureanhydrid zugegeben. Die Reaktionsmischung wurde auf Raumtemperatur kommen gelassen und 2 Stunden nachgerührt. Anschließend wurden 20 ml Wasser zugegeben, die Phasen getrennt und die wässrige Phase noch 2 x mit 20 ml Methylenchlorid extrahiert. Die vereinten organischen Phasen wurden mit 1 N wässriger Salzsäure gewaschen, über MgSO₄ getrocknet und einrotiert. Der Rückstand (355 mg) mit dem Molekulargewicht 650.8 g / mol (C₂₇H₃₇F₃N₂O₇S₂Si); MS (ESI): m / e = 651 (M+H⁺) wurde ohne weitere Reinigung in die nächste Reaktion eingesetzt.

### (S)-3-(5-Biphenyl-4-yl-6,6-dimethyl-4,4-dioxo-5,6-dihydro-4H-4lambda6-1,4,3-oxathiazin-2-ylamino)-3-phenyl-propan-1-ol

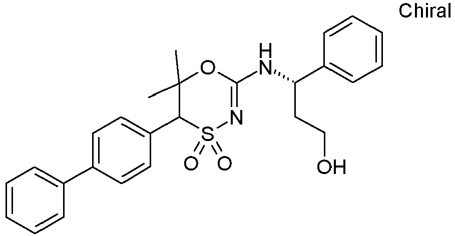

Unter Inertgas wurden 1.38 ml einer 0.5 N Zinkchlorid-Lösung in THF auf -78 °C gekühlt und tropfenweise mit 0.26 ml einer 1.8 N Phenyllithium-Lösung in Dibutylether versetzt. Die Reaktionslösung wurde auf Raumtemperatur kommen gelassen und 30 Minuten gerührt. Dann wurde diese Lösung zu einer Lösung aus 60 mg Trifluor-methansulfonsäure-4-{2-[(S)-3-(tert-butyl-dimethyl-silanyloxy)-1-phenyl-propylamino]-6,6-dimethyl-4,4-dioxo-5,6-dihydro-4H-4lambda6-1,4,3-oxathiazin-5-yl}-phenylester und 8 mg Dichlor[1,1'-bis(diphenylphosphino)ferrocen]palladium in 1.5 ml THF gegeben. Nach 45 Minuten Rühren bei 60 °C wurde die Umsetzung mit LCMS überprüft und erneut 8 mg des Katalysators zugegeben. Nach weiteren 2 Stunden bei gleichbleibender Temperatur wurde noch einmal die gleiche Menge der selbst hergestellten Lösung aus Phenyllithium und Zinkchlorid zugegeben und weitere 60 Minuten bei 60 °C gerührt. Nach Entfernen des Lösungsmittels unter vermindertem Druck wurde der Rückstand in 1.5 ml Methanol gelöst, mit 0.15 ml konzentrierter Salzsäure versetzt und 1 Stunde bei Raumtemperatur gerührt. Die Reaktionslösung wurde mit 20 ml Ethylacetat und 10 ml Wasser verdünnt und mit Natronlauge neutralisiert. Die organische Phase wurde nochmals mit gesättigter wässriger Natriumchlorid-Lösung gewaschen, über MgSO₄ getrocknet und einrotiert. Der Rückstand wurde mittels Normalphasenchromatographie über den Flashmaster mit einem n-Heptan/Ethylacetat-Gradienten gereinigt. Die produkthaltigen Fraktionen wurden vereinigt, einrotiert und das Rohprodukt im Reinigungslabor über präparative HPLC weiter gereinigt. Die produkthaltigen Fraktionen wurden vereinigt, das Acetonitril unter vermindertem Druck entfernt, der wässrige Rückstand mit gesättigter wässriger Natriumhydrogencarbonat-Lösung basisch gestellt und mit Dichlormethan extrahiert. Die organische Phase wurde über MgSO₄ getrocknet und einrotiert, der Rückstand erneut in einer Mischung aus Acetonitril und Wasser aufgenommen und lyophilisiert. Man erhielt so das Produkt (35.1 mg) mit dem Molekulargewicht 464.6 g / mol (C₂₆H₂₈N₂O₄S); MS (ESI): m / e = 465 (M+H⁺).

### (S)-3-(6,6-Dimethyl-4,4-dioxo-5-p-tolyl-5,6-dihydro-4H-4lambda6-[1,4,3]oxathiazin-2-ylamino)-3-phenyl-propan-1-ol

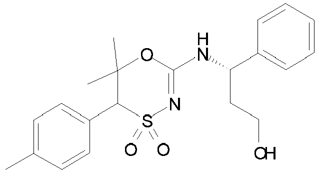

Unter Inertgas wurden 60 mg Trifluor-methansulfonsäure-4-{2-[(S)-3-(tert-butyl-dimethyl-silanyloxy)-1-phenyl-propylamino]-6,6-dimethyl-4,4-dioxo-5,6-dihydro-4H-4lambda6-1,4,3-oxathiazin-5-yl}-phenylester, 5.3 mg Bis(dibenzylidenaceton)palladium und 13.1 mg 1,2,3,4,5-Pentaphenyl-1-(di-tert-butylphosphino)ferrocen vorgelegt, in 1.2 ml Toluol gelöst und 5 Minuten bei Raumtemperatur gerührt. Nach der Zugabe von 0.09 ml einer 2 N Lösung von Trimethylaluminium wurde das Reaktionsgemisch für 2 Stunden bei 80 °C gerührt.Nach dem Abkühlen auf Raumtemperatur wurde 1 ml Methanol zugegeben und das Lösungsmittel unter vermindertem Druck entfernt. Der Rückstand wurde in 2 ml Methanol gelöst, mit 0.2 ml konzentrierter Salzsäure versetzt und 1 Stunde bei Raumtemperatur gerührt. Die Reaktionslösung wurde mit 20 ml Ethylacetat und 10 ml Wasser verdünnt und mit Natronlauge neutralisiert. Die organische Phase wurde nochmals mit gesättigter wässriger Natriumchlorid-Lösung gewaschen, über MgSO₄ getrocknet und einrotiert. Der Rückstand wurde im Reinigungslabor über präparative HPLC gereinigt. Die produkthaltigen Fraktionen wurden vereinigt, das Acetonitril unter vermindertem Druck entfernt, der wässrige Rückstand mit gesättigter wässriger Natriumhydrogencarbonat-Lösung basisch gestellt und mit Dichlormethan extrahiert. Die organische Phase wurde über MgSO₄ getrocknet und einrotiert, der Rückstand erneut in einer Mischung aus Acetonitril und Wasser aufgenommen und lyophilisiert. Man erhielt so das Produkt (22.8 mg) mit dem Molekulargewicht 402.5 g / mol (C₂₁H₂₆N₂O₄S); MS (ESI): m / e = 403 (M+H⁺).

### C-(3-Brom-phenyl)-N-(2,4-dimethoxy-benzyl)-methansulfonamid

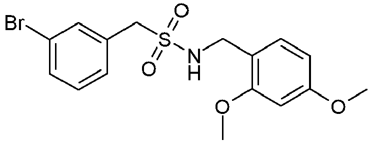

Unter Eiskühlung wurden 9.90 g 3-Brombenzylsulfonylchlorid in 120 ml Dichlormethan vorgelegt und innerhalb von 15 Minuten eine Lösung aus 11.55 ml 2,4-Dimethoxybenzylamin in 60 ml Dichlormethan zugetropft. Die Reaktionsmischung wurde auf Raumtemperatur kommen gelassen und 1 Stunde gerührt. Anschließend wurde der Ansatz mit 50 ml Wasser, mit 100 ml 1 N wässriger Salzsäure, mit 100 ml gesättigter wässriger Natriumhydrogencarbonat-Lösung und abschließend noch einmal mit 100 ml Wasser gewaschen. Die organische Phase wurde über MgSO₄ getrocknet und einrotiert. Der Rückstand (14.30 g) wurde ohne weitere Reinigung in die nächste Reaktion eingesetzt.

### 1-(3-Brom-phenyl)-2-hydroxy-2-methyl-propan-1-sulfonsäure-2,4-dimethoxy-benzylamid

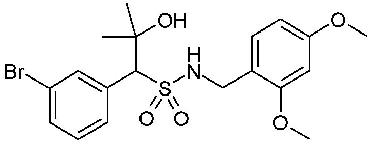

Unter Inertgas wurden 7.00 g C-(3-Brom-phenyl)-N-(2,4-dimethoxy-benzyl)-methansulfonamid in 120 ml THF vorgelegt und anschließend bei einer Temperatur von -78 °C 25.14 ml einer 1.6 N Methyllithium-Lösung in Diethylether tropfenweise zugegeben und 10 Minuten bei gleichbleibender Temperatur gerührt. Anschließend wurden 7 ml Aceton zugegeben. Nach 5 Minuten Rühren wurde die Reaktionslösung mit 5 ml Essigsäure versetzt, 5 Minuten nachgerührt und der Ansatz mit 100 ml Ethylacetat versetzt, mit 1 x 100 ml und 1 x 50 ml einer gesättigten wässrigen Natriumhydrogencarbonat-Lösung und 2 x 50 ml einer gesättigten wässrigen Natriumchlorid-Lösung gewaschen. Die organische Phase wurde über MgSO₄ getrocknet und einrotiert. Der Rückstand (8.63 g) wurde ohne weitere Reinigung in die nächste Reaktion eingesetzt.

### 1-(3-Brom-phenyl)-2-hydroxy-2-methyl-propan-1-sulfonsäureamid

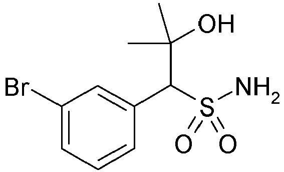

Eine Lösung aus 8.63 g 1-(3-Brom-phenyl)-2-hydroxy-2-methyl-propan-1-sulfonsäure-2,4-dimethoxy-benzylamid in 40 ml Dichlormethan wurde mit 10 ml Trifluoressigsäure versetzt und 30 Minuten bei Raumtemperatur gerührt. Die Ansatzlösung wurde mit 40 ml Wasser und langsam und vorsichtig mit 150 ml einer gesättigten wässrigen Natriumhydrogencarbonat-Lösung versetzt. Der Niederschlag wurde abgesaugt, mit Wasser gewaschen und über P₂O₅ unter reduziertem Druck getrocknet. Das Rohprodukt wurde mittels Normalphasenchromatographie über den Flashmaster mit einem Dichlormethan/Methanol-Gradienten gereinigt. Die produkthaltigen Fraktionen wurden vereinigt und einrotiert. Man erhielt so das Produkt (4.11 g) mit dem Molekulargewicht 308.2 g / mol (C₁₀H₁₄BrNO₃S); MS (ESI): m / e = 325 (M+H₂O+H⁺).

### [5-(3-Brom-phenyl)-6,6-dimethyl-4,4-dioxo-5,6-dihydro-4H-4lambda6-1,4,3-oxathiazin-2-yl]-cyclohexyl-amin

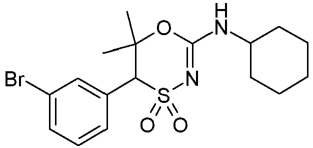

Eine Lösung aus 1.00 g 1-(3-Brom-phenyl)-2-hydroxy-2-methyl-propan-1-sulfonsäureamid und 0.483 g Cylohexylisothiocyanat in 7 ml NMP wurde mit 1.62 ml einer 2 N Lösung von Natrium-bis(trimethylsilyl)-amid versetzt. Nach 20 Minuten Rühren wurden 578 mg N-Bromsuccinimid zugegeben und weitere 60 Minuten bei Raumtemperatur gerührt. Die Reaktionslösung wurde mit 150 ml Wasser versetzt und 2 x mit 150 ml Ethylacetat extrahiert. Die vereinten organischen Phasen wurden mit gesättigter wässriger Natriumchlorid-Lösung gewaschen, über MgSO₄ getrocknet und einrotiert. Der Rückstand wurde im Reinigungslabor über präparative HPLC gereinigt und die produkthaltigen Fraktionen lyophilisiert. Man erhielt so das Produkt (411 mg) mit dem Molekulargewicht 415.3 g / mol (C₁₇H₂₃BrN₂O₃S), MS (ESI): m / e = 416 (M+H⁺).

### Cyclohexyl-[6,6-dimethyl-4,4-dioxo-5-(3-pyrimidin-5-yl-phenyl)-5,6-dihydro-4H-4lambda6-[1,4,3]oxathiazin-2-yl]-amin

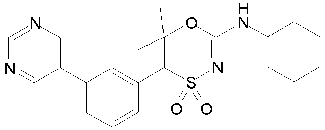

Unter Inertgas wurden 80 mg [5-(3-Brom-phenyl)-6,6-dimethyl-4,4-dioxo-5,6-dihydro-4H-4lambda6-1,4,3-oxathiazin-2-yl]-cyclohexyl-amin, 30 mg Pyrimidin-5-boronsäure und 251 mg Cäsiumcarbonat in einer Mischung aus 1.2 ml Dioxan und 0.4 ml Wasser gelöst. Nach 10 Minuten Spülen mit Argon wurden 15 mg Dichlor[1,1'-bis(diphenylphosphino)ferrocen]palladium zugegeben und die Reaktionsmischung für 2 Stunden bei 80 °C gerührt. Nach dem Abkühlen auf Raumtemperatur wurde mit 50 ml Ethylacetat versetzt, mit 15 ml Wasser und 15 ml gesättigter wässriger Natriumchlorid-Lösung gewaschen, die organische Phase über MgSO₄ getrocknet und einrotiert. Der Rückstand wurde im Reinigungslabor über präparative HPLC gereinigt. Die produkthaltigen Fraktionen wurden vereinigt und lyophilisiert. Man erhielt so das Produkt (6.4 mg) mit dem Molekulargewicht 414.5 g / mol (C₂₁H₂₆N₄O₃S); MS (ESI): m / e = 415 (M+H⁺).

### [5-(3-Brom-phenyl)-6,6-dimethyl-4,4-dioxo-5,6-dihydro-4H-4lambda6-[1,4,3]oxathiazin-2-yl]-[(S)-1-(2-fluor-phenyl)-ethyl]-amin

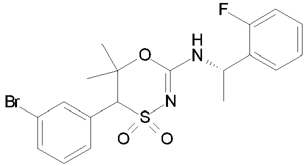

### 5-(3-Brom-phenyl)-2-methoxy-6,6-dimethyl-5,6-dihydro-1,4,3-oxathiazin-4,4-dioxid

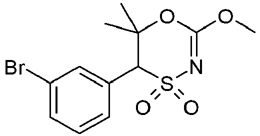

Eine Mischung aus 500 mg 1-(3-Brom-phenyl)-2-hydroxy-2-methyl-propan-1-sulfonsäureamid in 2 ml Tetramethoxymethan und 0.5 ml Essigsäure wurde für 3 Stunden bei 100 °C gerührt. Nach dem Entfernen der Lösungsmittel unter vermindertem Druck wurde der Rückstand (565 mg) ohne weitere Reinigung in die nächste Reaktion eingesetzt.

Analog wurden folgende Intermediate erhalten:
5-(4-Brom-phenyl)-2-methoxy-6,6-dimethyl-5,6-dihydro-[1,4,3]oxathiazin 4,4-dioxid 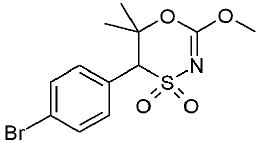
5-(6-Chlor-pyridin-3-yl)-2-methoxy-6,6-dimethyl-5,6-dihydro-[1,4,3]oxathiazin 4,4-dioxid 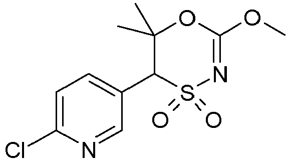 [5-(3-Brom-phenyl)-6,6-dimethyl-4,4-dioxo-5,6-dihydro-4H-4lambda6-[1,4,3]oxathiazin-2-yl]-[(S)-1-(2-fluor-phenyl)-ethyl]-amin

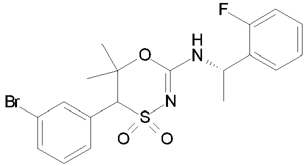

Eine Lösung aus 565 mg 5-(3-Brom-phenyl)-2-methoxy-6,6-dimethyl-5,6-dihydro-1,4,3-oxathiazin-4,4-dioxid und 270 mg (S)-1-(2-Fluor-phenyl)-ethylamin in 2 ml Methylenchlorid wurde einrotiert und der Rückstand über Nacht bei Raumtemperatur stehen gelassen. Das Rohprodukt wurde mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (162 mg) mit dem Molekulargewicht 455.3 g / mol (C₁₉H₂₀BrFN₂O₃S).

Auf gleiche Weise wurden folgende Produkte erhalten:
[5-(4-Brom-phenyl)-6,6-dimethyl-4,4-dioxo-5,6-dihydro-4H-4lambda6-[1,4,3]oxathiazin-2-yl]-[(S)-3-(tert-butyl-dimethyl-silanyloxy)-1-(2-fluor-phenyl)-propyl]-amin 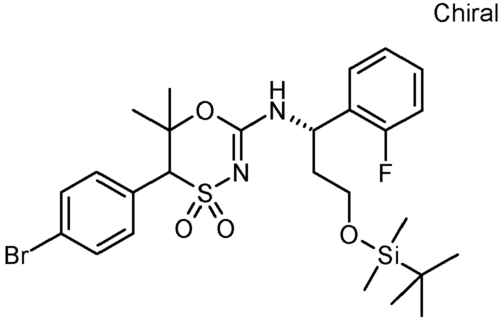 [5-(4-Brom-phenyl)-6,6-dimethyl-4,4-dioxo-5,6-dihydro-4H-4lambda6-[1,4,3]oxathiazin-2-yl]-[(S)-3-(tert-butyl-dimethyl-silanyloxy)-1-(2-chlor-phenyl)-propyl]-amin 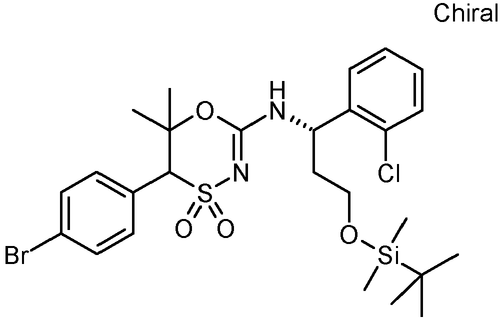
[5-(4-Brom-phenyl)-6,6-dimethyl-4,4-dioxo-5,6-dihydro-4H-4lambda6-[1,4,3]oxathiazin-2-yl]-[(S)-3-(tert-butyl-dimethyl-silanyloxy)-1-(3-chlor-phenyl)-propyl]-amin 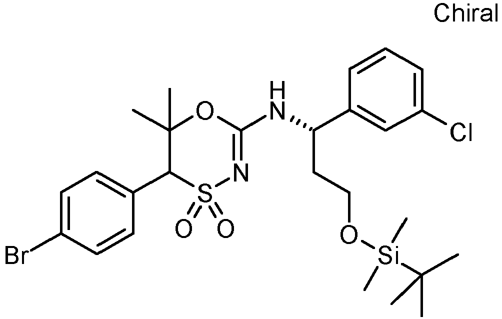
[5-(4-Brom-phenyl)-6,6-dimethyl-4,4-dioxo-5,6-dihydro-4H-4lambda6-[1,4,3]oxathiazin-2-yl]-[(S)-3-(tert-butyl-dimethyl-silanyloxy)-1-(4-chlor-phenyl)-propyl]-amin 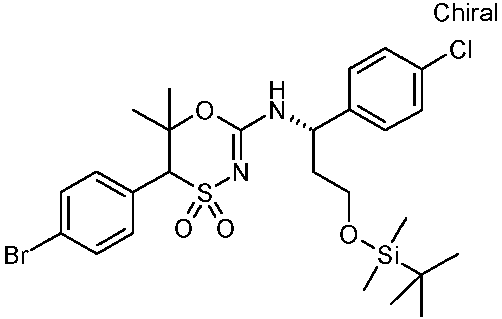
[5-(4-Brom-phenyl)-6,6-dimethyl-4,4-dioxo-5,6-dihydro-4H-4lambda6-[1,4,3]oxathiazin-2-yl]-[(S)-3-(tert-butyl-dimethyl-silanyloxy)-1-(2,3-difluor-phenyl)-propyl]-amin 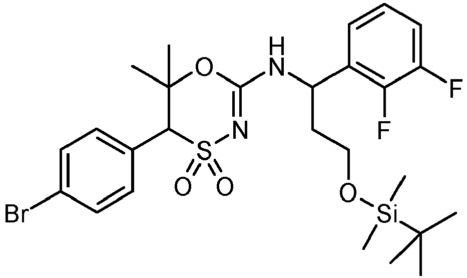
[5-(4-Brom-phenyl)-6,6-dimethyl-4,4-dioxo-5,6-dihydro-4H-4lambda6-[1,4,3]oxathiazin-2-yl]-[(S)-3-(tert-butyl-dimethyl-silanyloxy)-1-(2,4-difluor-phenyl)-propyl]-amin 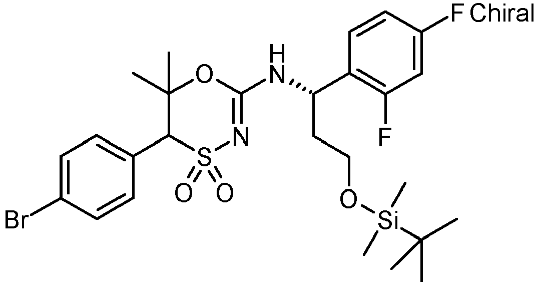
[5-(4-Brom-phenyl)-6,6-dimethyl-4,4-dioxo-5,6-dihydro-4H-4lambda6-[1,4,3]oxathiazin-2-yl]-[(S)-3-(tert-butyl-dimethyl-silanyloxy)-1-(2,5-difluor-phenyl)-propyl]-amin 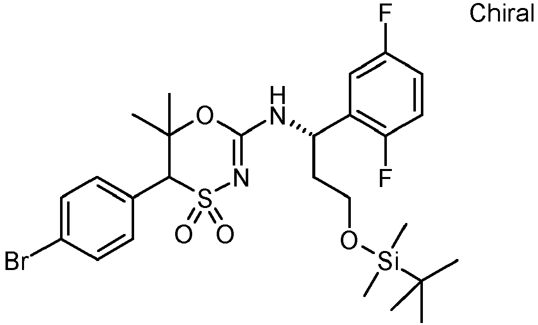
[5-(4-Brom-phenyl)-6,6-dimethyl-4,4-dioxo-5,6-dihydro-4H-4lambda6-[1,4,3]oxathiazin-2-yl]-[(S)-3-(tert-butyl-dimethyl-silanyloxy)-1-(5-chlor-2-fluor-phenyl)-propyl]-amin 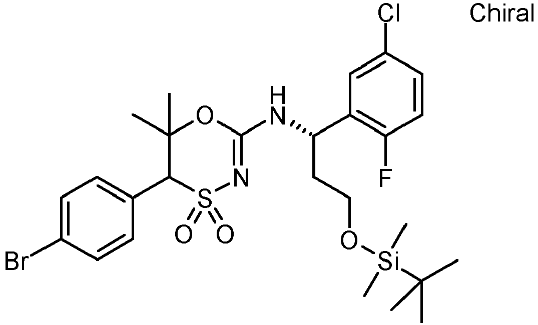
[5-(4-Brom-phenyl)-6,6-dimethyl-4,4-dioxo-5,6-dihydro-4H-4lambda6-[1,4,3]oxathiazin-2-yl]-[(S)-3-(tert-butyl-dimethyl-silanyloxy)-1-(2-chlor-3-fluor-phenyl)-propyl]-amin 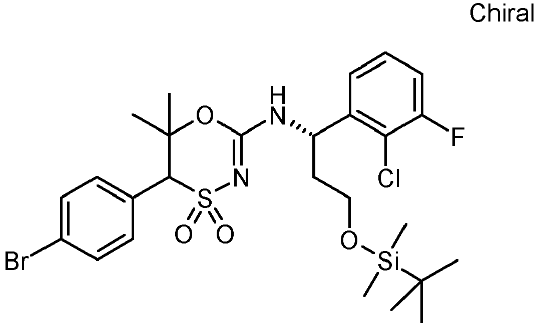
[5-(4-Brom-phenyl)-6,6-dimethyl-4,4-dioxo-5,6-dihydro-4H-4lambda6-[1,4,3]oxathiazin-2-yl]-[(S)-3-(tert-butyl-dimethyl-silanyloxy)-1-(5-fluor-2-methyl-phenyl)-propyl]-amin 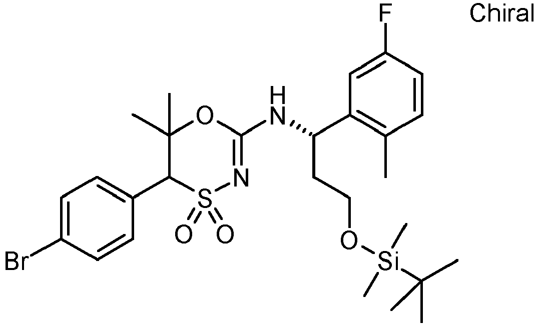
[(S)-3-(tert-Butyl-dimethyl-silanyloxy)-1-(2-fluoro-phenyl)-propyl]-[5-(6-chloro-pyridin-3-yl)-6,6-dimethyl-4,4-dioxo-5,6-dihydro-4H-4lambda*6*-[1,4,3]oxathiazin-2-yl]-amine 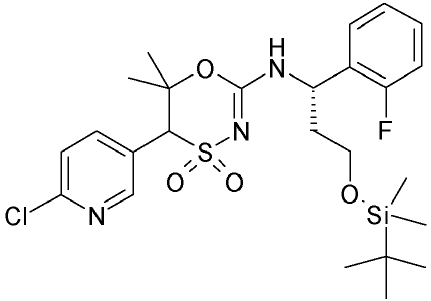
[5-(6-Chlor-pyridin-3-yl)-6,6-dimethyl-4,4-dioxo-5,6-dihydro-4H-4lambda*6*-[1,4,3]oxathiazin-2-yl]-[(S)-1-(2-fluoro-phenyl)-ethyl]-amin 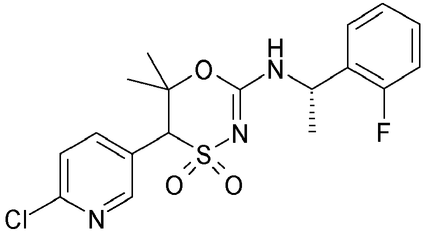
(6,6-Dimethyl-4,4-dioxo-5-phenyl-5,6-dihydro-4H-4lambda6-[1,4,3]oxathiazin-2-yl)-[(S)-1-(2-fluor-phenyl)-ethyl]-amin

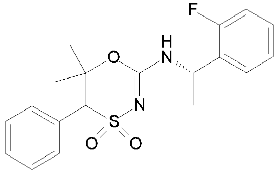

Unter Inertgas wurden 48 mg [5-(3-Brom-phenyl)-6,6-dimethyl-4,4-dioxo-5,6-dihydro-4H-4lambda6-[1,4,3]oxathiazin-2-yl]-[(S)-1-(2-fluor-phenyl)-ethyl]-amin in 5 ml Methanol vorgelegt und unter Zusatz von 22 mg Pd/C 10 % für 1.75 Stunden unter Wasserstoff-Atmosphäre gerührt. Nach Filtration von den festen Rückständen wurde das Filtrat unter vermindertem Druck vom Lösungsmittel befreit und das Rohprodukt im Reinigungslabor über präparative HPLC gereinigt. Die produkthaltigen Fraktionen wurden vereinigt, unter vermindertem Druck vom organischen Lösungsmittel befreit und der wässrige Rückstand lyophilisiert. Man erhielt so das Produkt (27.8 mg) mit dem Molekulargewicht 376.4 g / mol (C₁₉H₂₁FN₂O₃S); MS (ESI): m / e = 377 (M+H⁺).

### (S)-3-[6,6-Dimethyl-5-(2'-methyl-biphenyl-4-yl)-4,4-dioxo-5,6-dihydro-4H-4lambda6-[1,4,3]oxathiazin-2-ylamino]-3-(2-fluor-phenyl)-propan-1-ol

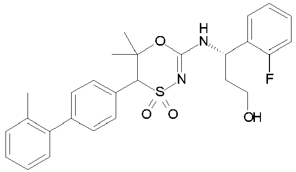

Die Synthese von [5-(4-Brom-phenyl)-6,6-dimethyl-4,4-dioxo-5,6-dihydro-4H-4lambda6-1,4,3-oxathiazin-2-yl]-[(S)-3-(tert-butyl-dimethyl-silanyloxy)-1-(2-fluor-phenyl)-propyl]-amin erfolgte analog zu [5-(3-Brom-phenyl)-6,6-dimethyl-4,4-dioxo-5,6-dihydro-4H-4lambda6-[1,4,3]oxathiazin-2-yl]-[(S)-1-(2-fluor-phenyl)-ethyl]-amin.

Unter Inertgas wurden zu einer Mischung aus 97 mg 2-Methylphenylboronsäure, 350 mg [5-(4-Brom-phenyl)-6,6-dimethyl-4,4-dioxo-5,6-dihydro-4H-4lambda6-1,4,3-oxathiazin-2-yl]-[(S)-3-(tert-butyl-dimethyl-silanyloxy)-1-(2-fluor-phenyl)-propyl]-amin und 571 mg Cäsiumcarbonat in 4.5 ml Dioxan und 1.5 ml Wasser 40 mg Dichlor[1,1'-bis(diphenylphosphino)ferrocen]palladium gegeben. Nach 30 Minuten Rühren bei 95 °C wurde die Reaktion auf Raumtemperatur abkühlen gelassen, mit 50 ml Ethylacetat versetzt und die organische Phase mit 20 ml Wasser und 20 ml gesättigter wässriger Natriumchlorid-Lösung gewaschen. Die organische Phase wurde über MgSO₄ getrocknet und einrotiert. Das Rohprodukt wurde mittels Normalphasenchromatographie über den Flashmaster mit einem n-Heptan/Ethylacetat-Gradienten gereinigt. Die produkthaltigen Fraktionen wurden vereinigt und einrotiert. Zur Abspaltung der Schutzgruppe wurde der Rückstand wurde in 4 ml Methanol gelöst, mit 0.4 ml konzentrierter Salzsäure versetzt und für 16 Stunden bei Raumtemperatur gerührt. Die Reaktionslösung wurde einrotiert, der Rückstand in 20 ml Dichlormethan aufgenommen und mit 10 ml Wasser gewaschen. Die wässrige Phase wurde nochmals mit 10 ml Dichlormethan extrahiert, die vereinigten organischen Phasen über eine Phasenseparator-Kartusche (Chromabond^{®} PTS) getrocknet und einrotiert. Das Rohprodukt wurde im Reinigungslabor über präparative HPLC gereinigt. Die produkthaltigen Fraktionen wurden vereinigt, das Acetonitril unter vermindertem Druck entfernt, der wässrige Rückstand mit gesättigter wässriger Natriumhydrogencarbonat-Lösung basisch gestellt und mit Dichlormethan extrahiert. Die organische Phase wurde über MgSO₄ getrocknet und einrotiert, der Rückstand erneut in einer Mischung aus Acetonitril und Wasser aufgenommen und lyophilisiert. Man erhielt so das Produkt (120 mg) mit dem Molekulargewicht 496.6 g / mol (C₂₇H₂₉FN₂O₄S); MS (ESI): m / e = 497 (M+H⁺).

Die Verbindungen 65 bis 68 und 71 bis75 wurden ebenfalls mit dieser Herstellungsvorschrift synthetisiert:
(S)-3-[5-(4'-Fluor-biphenyl-4-yl)-6,6-dimethyl-4,4-dioxo-5,6-dihydro-4H-4lambda6-[1,4,3] oxathiazin-2-ylamino]-3-(2-fluor-phenyl)-propan-1-ol
(S)-3-(2-Fluor-phenyl)-3-[5-(4'-methoxy-biphenyl-4-yl)-6,6-dimethyl-4,4-dioxo-5,6-dihydro-4H-4lambda6-[1,4,3]oxathiazin-2-ylamino]-propan-1-ol
(S)-3-(2-Fluor-phenyl)-3-[5-(4'-methoxy-biphenyl-4-yl)-6,6-dimethyl-4,4-dioxo-5,6-dihydro-4H-4lambda6-[1,4,3]oxathiazin-2-ylamino]-propan-1-ol
(S)-3-(5-Biphenyl-3-yl-6,6-dimethyl-4,4-dioxo-5,6-dihydro-4H-4lambda6-[1,4,3]oxathiazin-2-ylamino)-3-(2-fluor-phenyl)-propan-1-ol
(5-Biphenyl-3-yl-6,6-dimethyl-4,4-dioxo-5,6-dihydro-4H-4lambda6-[1,4,3]oxathiazin-2-yl)-[(S)-1-(2-fluor-phenyl)-ethyl]-amin
(S)-3-[5-(4'-Fluor-biphenyl-3-yl)-5,6,6-trimethyl-4,4-dioxo-5,6-dihydro-4H-4lambda6-[1,4,3]oxathiazin-2-ylamino]-3-(2-fluor-phenyl)-propan-1-ol
(S)-3-[5-(3-Brom-phenyl)-6,6-dimethyl-4,4-dioxo-5,6-dihydro-4H-4lambda6-[1,4,3]oxathiazin-2-ylamino]-3-phenyl-propan-1-ol
(S)-3-[5-(4'-Fluor-biphenyl-3-yl)-5,6,6-trimethyl-4,4-dioxo-5,6-dihydro-4H-4lambda6-[1,4,3]oxathiazin-2-ylamino]-3-(2-fluor-phenyl)-propan-1-ol
(S)-3-(5-Biphenyl-3-yl-5,6,6-trimethyl-4,4-dioxo-5,6-dihydro-4H-4lambda6-[1,4,3]oxathiazin-2-ylamino)-3-phenyl-propan-1-ol
(5-Biphenyl-3-yl-5,6,6-trimethyl-4,4-dioxo-5,6-dihydro-4H-4lambda6-[1,4,3]oxathiazin-2-yl)-[(S)-1-(2-fluor-phenyl)-ethyl]-amin
(5-Biphenyl-3-yl-5,6,6-trimethyl-4,4-dioxo-5,6-dihydro-4H-4lambda6-[1,4,3]oxathiazin-2-yl)-[(S)-1-(2-fluor-phenyl)-ethyl]-amin
(S)-3-(5-Biphenyl-3-yl-5,6,6-trimethyl-4,4-dioxo-5,6-dihydro-4H-4lambda6-[1,4,3]oxathiazin-2-ylamino)-3-(2-fluor-phenyl)-propan-1-ol
(S)-3-(5-Biphenyl-3-yl-5,6,6-trimethyl-4,4-dioxo-5,6-dihydro-4H-4lambda6-[1,4,3]oxathiazin-2-ylamino)-3-(2-fluor-phenyl)-propan-1-ol 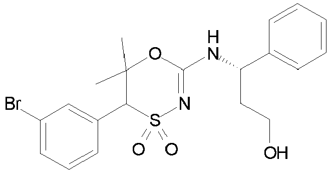
[5-(3-Brom-phenyl)-6,6-dimethyl-4,4-dioxo-5,6-dihydro-4H-4lambda6-1,4,3-oxathiazin-2-yl]-[(S)-3-(tert-butyl-dimethyl-silanyloxy)-1-phenyl-propyl]-amin

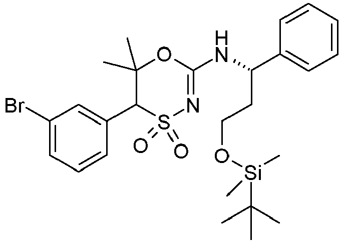

2.04 g (S)-3-(tert-Butyl-dimethyl-silanyloxy)-1-phenyl-propylamin wurden in 20 ml Dichlormethan vorgelegt und mit 1.41 g 1,1'-Thiocarbonyldiimidazol versetzt. Nach 45 Minuten Rühren bei Raumtemperatur wurde die Reaktionslösung mit einem Gemisch aus 80 ml Diethylether und 80 ml n-Pentan versetzt und 3 x mit 50 ml Wasser gewaschen, über MgSO₄ getrocknet und einrotiert. Unter Inertgas wurden der Rückstand und 2.03 g 1-(3-Brom-phenyl)-2-hydroxy-2-methyl-propan-1-sulfonsäureamid in 15 ml NMP gelöst und mit 3.29 ml einer 2 N Lösung von Natrium-bis(trimethylsilyl)-amid in THF tropfenweise versetzt. Nach 20 Minuten Rühren wurden 1.17 g N-Bromsuccinimid zugesetzt und die resultierende Reaktionslösung 20 Minuten bei gleichbleibender Temperatur gerührt. Die Reaktionslösung wurde mit 250 ml Wasser versetzt und 2 x mit 100 ml sowie 1 x mit 50 ml Ethylacetat extrahiert. Die vereinten organischen Phasen wurden über MgSO₄ getrocknet, einrotiert und der Rückstand mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (1.33 g) mit dem Molekulargewicht 581.7 g / mol (C₂₆H₃₇BrN₂O₄SSi); MS (ESI): m / e = 582 (M+H⁺).

### (S)-3-[5-(3-Brom-phenyl)-6,6-dimethyl-4,4-dioxo-5,6-dihydro-4H-4lambda6-[1,4,3]oxathiazin-2-ylamino]-3-phenyl-propan-1-ol

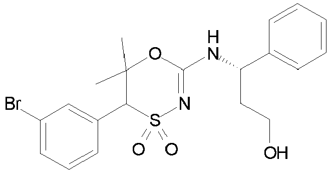

Zur Abspaltung der Schutzgruppe wurden 70 mg [5-(3-Brom-phenyl)-6,6-dimethyl-4,4-dioxo-5,6-dihydro-4H-4lambda6-1,4,3-oxathiazin-2-yl]-[(S)-3-(tert-butyl-dimethyl-silanyloxy)-1-phenyl-propyl]-amin in 1.5 ml Methanol aufgenommen und nach Zugabe von 0.15 ml konzentrierter Salzsäure 1 Stunde bei Raumtemperatur gerührt. Die Reaktionslösung wurde einrotiert und der Rückstand im Reinigungslabor über präparative HPLC gereinigt. Die produkthaltigen Fraktionen wurden vereinigt und unter vermindertem Druck vom Lösungsmittel befreit. Der Rückstand wurde in Acetonitril gelöst und lyophilisiert. Man erhielt so das Produkt (38.6 mg) mit dem Molekulargewicht 467.4 g / mol (C₂₀H₂₃BrN₂O₄S); MS (ESI): m / e = 468 (M+H⁺).

### (S)-3-(5-Biphenyl-3-yl-6,6-dimethyl-4,4-dioxo-5,6-dihydro-4H-4lambda6-[1,4,3]oxathiazin-2-ylamino)-3-phenyl-propan-1-ol

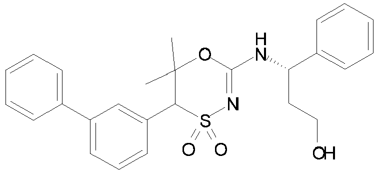

Unter Inertgas wurden 2.58 ml einer 0.5 N Zinkchlorid-Lösung in THF auf -78 °C gekühlt und tropfenweise mit 0.48 ml einer 1.8 N Phenyllithium-Lösung in Dibutylether versetzt. Die Reaktionslösung wurde auf Raumtemperatur kommen gelassen und 30 Minuten gerührt. Dann wurde diese Lösung zu einer Lösung aus 100 mg [5-(3-Brom-phenyl)-6,6-dimethyl-4,4-dioxo-5,6-dihydro-4H-4lambda6-1,4,3-oxathiazin-2-yl]-[(S)-3-(tert-butyl-dimethyl-silanyloxy)-1-phenyl-propyl]-amin und 14.1 mg Dichlor[1,1'-bis(diphenylphosphino)ferrocen]palladium in 2 ml THF gegeben. Nach 45 Minuten Rühren bei 60 °C wurde die Reaktion auf Raumtemperatur abkühlen gelassen, mit 10 ml Wasser und 10 ml Ethylacetat versetzt, die organische Phase über MgSO₄ getrocknet und einrotiert. Der Rückstand wurde in 1.5 ml Methanol gelöst, mit 0.15 ml konzentrierter Salzsäure versetzt und für 16 Stunden bei Raumtemperatur gerührt. Die Reaktionslösung wurde mit Ethylacetat verdünnt und mit gesättigter wässriger Natriumchlorid-Lösung gewaschen, über MgSO₄ getrocknet und einrotiert. Der Rückstand wurde im Reinigungslabor über präparative HPLC gereinigt. Die produkthaltigen Fraktionen wurden vereinigt, das Acetonitril unter vermindertem Druck entfernt, der wässrige Rückstand mit gesättigter wässriger Natriumhydrogencarbonat-Lösung basisch gestellt und mit Dichlormethan extrahiert. Die organische Phase wurde über MgSO₄ getrocknet und einrotiert, der Rückstand erneut in einer Mischung aus Acetonitril und Wasser aufgenommen und lyophilisiert. Man erhielt so das Produkt (57.8 mg) mit dem Molekulargewicht 464.6 g / mol (C₂₆H₂₈N₂O₄S); MS (ESI): m / e = 565 (M+H⁺).

### (S)-3-(6,6-Dimethyl-4,4-dioxo-5-m-tolyl-5,6-dihydro-4H-4lambda6-[1,4,3]oxathiazin-2-ylamino)-3-phenyl-propan-1-ol

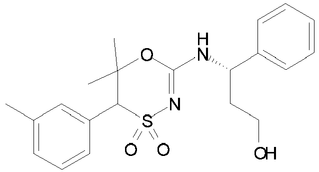

### [(S)-3-(tert-Butyl-dimethyl-silanyloxy)-1-phenyl-propyl]-(6,6-dimethyl-4,4-dioxo-5-m-tolyl-5,6-dihydro-4H-4lambda6-1,4,3-oxathiazin-2-yl)-amin

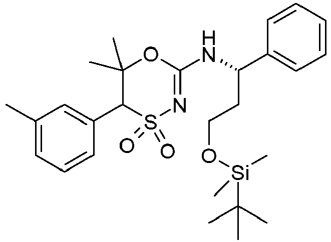

Unter Inertgas wurden 100 mg [5-(3-Brom-phenyl)-6,6-dimethyl-4,4-dioxo-5,6-dihydro-4H-4lambda6-1,4,3-oxathiazin-2-yl]-[(S)-3-(tert-butyl-dimethyl-silanyloxy)-1-phenyl-propyl]-amin, 8 mg Bis(dibenzylidenaceton)palladium und 19.6 mg 1,2,3,4,5-Pentaphenyl-1-(di-tert-butylphosphino)ferrocen vorgelegt, in 3 ml Toluol gelöst und 5 Minuten bei Raumtemperatur gerührt. Nach der Zugabe von 0.17 ml einer 2 N Lösung von Trimethylaluminium wurde das Reaktionsgemisch für 1.5 Stunden bei 70 °C gerührt.Nach dem Abkühlen auf Raumtemperatur wurde das Lösungsmittel unter vermindertem Druck entfernt und der Rückstand mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (51.7 mg) mit dem Molekulargewicht 516.8 g / mol (C₂₇H₄₀N₂O₄SSi); MS (ESI): m / e = 517 (M+H⁺).

### (S)-3-(6,6-Dimethyl-4,4-dioxo-5-m-tolyl-5,6-dihydro-4H-4lambda6-[1,4,3]oxathiazin-2-ylamino)-3-phenyl-propan-1-ol

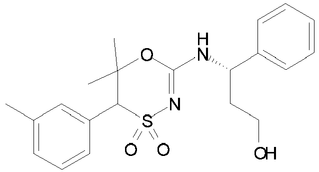

51.7 mg [(S)-3-(tert-Butyl-dimethyl-silanyloxy)-1-phenyl-propyl]-(6,6-dimethyl-4,4-dioxo-5-m-tolyl-5,6-dihydro-4H-4lambda6-1,4,3-oxathiazin-2-yl)-amin wurden in 2 ml Methanol gelöst, mit 0.1 ml konzentrierter Salzsäure versetzt und für 16 Stunden bei Raumtemperatur gerührt. Die Reaktionslösung wurde mit Ethylacetat verdünnt und mit gesättigter wässriger Natriumchlorid-Lösung gewaschen, über MgSO₄ getrocknet und einrotiert. Der Rückstand wurde im Reinigungslabor über präparative HPLC gereinigt. Die produkthaltigen Fraktionen wurden vereinigt, das Acetonitril unter vermindertem Druck entfernt, der wässrige Rückstand mit gesättigter wässriger Natriumhydrogencarbonat-Lösung basisch gestellt und mit Dichlormethan extrahiert. Die organische Phase wurde über MgSO₄ getrocknet und einrotiert, der Rückstand erneut in einer Mischung aus Acetonitril und Wasser aufgenommen und lyophilisiert. Man erhielt so das Produkt (22.9 mg) mit dem Molekulargewicht 402.5 g / mol (C₂₁H₂₆N₂O₄S); MS (ESI): m / e = 403 (M+H⁺).

### (S)-3-[5-(3-Benzyl-phenyl)-6,6-dimethyl-4,4-dioxo-5,6-dihydro-4H-4lambda6-[1,4,3]oxathiazin-2-ylamino]-3 -phenyl-propan- 1 -ol

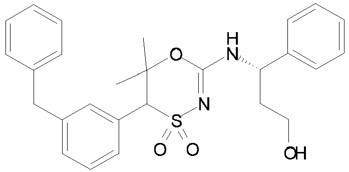

Unter Inertgas wurden 1.72 ml einer 0.5 N Lösung von Benzylzinkbromid in THF zu einer Lösung von 100 mg [5-(3-Brom-phenyl)-6,6-dimethyl-4,4-dioxo-5,6-dihydro-4H-4lambda6-1,4,3-oxathiazin-2-yl]-[(S)-3-(tert-butyl-dimethyl-silanyloxy)-1-phenyl-propyl]-amin und 14.1 mg Dichlor[1,1'-bis(diphenylphosphino)ferrocen]palladium in 2 ml THF gegeben. Nach 18 Stunden Rühren bei 60 °C wurden 2 ml Methanol und 0.5 ml konzentrierte Salzsäure zugegeben und die Reaktionsmischung für 1 Stunde bei Raumtemperatur gerührt. Die Reaktionslösung wurde mit 20 ml Ethylacetat und 10 ml Wasser verdünnt, mit wässriger Natriumhydroxid-Lösung neutralisiert, die organische Phase nochmals mit gesättigter wässriger Natriumchlorid-Lösung gewaschen, über MgSO₄ getrocknet und einrotiert. Der Rückstand wurde im Reinigungslabor über präparative HPLC gereinigt. Die produkthaltigen Fraktionen wurden vereinigt, mit Dichlormethan extrahiert und einrotiert. Der Rückstand wurde weiter mittels Normalphasenchromatographie über den Flashmaster mit einem n-Heptan/Ethylacetat-Gradienten gereinigt. Die produkthaltigen Fraktionen wurden vereinigt und einrotiert, der Rückstand erneut in einer Mischung aus Acetonitril und Wasser aufgenommen und lyophilisiert. Man erhielt so das Produkt (38.2 mg) mit dem Molekulargewicht 478.6 g / mol (C₂₇H₃₀N₂O₄S); MS (ESI): m / e = 479 (M+H⁺).

### (S)-3-[6,6-Dimethyl-4,4-dioxo-5-(3-pyrimidin-5-yl-phenyl)-5,6-dihydro-4H-4lambda6-[1,4,3]oxathiazin-2-ylamino]-3-phenyl-propan-1-ol

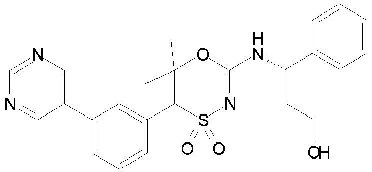

Unter Inertgas wurde eine Suspension von 100 mg [5-(3-Brom-phenyl)-6,6-dimethyl-4,4-dioxo-5,6-dihydro-4H-4lambda6-1,4,3-oxathiazin-2-yl]-[(S)-3-(tert-butyl-dimethyl-silanyloxy)-1-phenyl-propyl]-amin, 31 mg Pyrimidin-5-boronsäure und 110 mg Kaliumphosphat in 2 ml Toluol vorgelegt. Nach der Zugabe von 10 mg Bis(dibenzylidenaceton)palladium und 24 mg 1,2,3,4,5-Pentaphenyl-1-(di-tert-butylphosphino)ferrocen wurde die Reaktionsmischung für 1.5 h bei 80 °C gerührt.Der Fortgang der Reaktion wurde mit LCMS überprüft. Da noch keine Umsetzung stattgefunden hatte, wurden 0.8 ml Wasser, 73 mg Kaliumphosphat, 24 mg Pyrimidin-5-boronsäure, 10 mg Bis(dibenzylidenaceton)palladium und 24 mg 1,2,3,4,5-Pentaphenyl-1-(di-tert-butylphosphino)ferrocen zugegeben und weitere 20 Stunden bei gleichbleibender Temperatur gerührt. Nach dem Entfernen des Lösungsmittels unter vermindertem Druck wurde der Rückstand in 2 ml Methanol und 0.2 ml konzentrierter Salzsäure gelöst und für 1.5 Stunden bei Raumtemperatur gerührt. Die Reaktionslösung wurde mit 20 ml Ethylacetat und 10 ml Wasser verdünnt, die organische Phase nochmals mit gesättigter wässriger Natriumchlorid-Lösung gewaschen, über MgSO₄ getrocknet und einrotiert. Der Rückstand wurde mittels Normalphasenchromatographie über den Flashmaster mit einem Dichlormethan/Methanol-Gradienten gereinigt. Die produkthaltigen Fraktionen wurden vereinigt und einrotiert. Das Rohprodukt wurde im Reinigungslabor über präparative HPLC weiter gereinigt. Die produkthaltigen Fraktionen wurden vereinigt, das Acetonitril unter vermindertem Druck entfernt, der wässrige Rückstand mit gesättigter wässriger Natriumhydrogencarbonat-Lösung basisch gestellt und mit Dichlormethan extrahiert. Die organische Phase wurde über MgSO₄ getrocknet und einrotiert, der Rückstand erneut in einer Mischung aus Acetonitril und Wasser aufgenommen und lyophilisiert. Man erhielt so das Produkt (15.7 mg) mit dem Molekulargewicht 466.6 g / mol (C₂₄H₂₆N₄O₄S); MS (ESI): m / e = 467 (M+H⁺).

### (S)-3-[5-(2-Chlor-phenyl)-6,6-dimethyl-4,4-dioxo-5,6-dihydro-4H-4lambda6-[1,4,3]oxathiazin-2-ylamino]-3 -phenyl-propan- 1 -ol

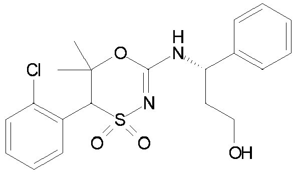

### C-(2-Chlor-phenyl)-N-(2,4-dimethoxy-benzyl)-methansulfonamid

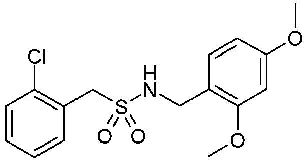

Bei Raumtemperatur wurden 1.24 g (2-Chlor-phenyl)-methansulfonylchlorid in 30 ml Dichlormethan vorgelegt und 1.66 ml 2,4-Dimethoxybenzylamin zugetropft. Die Reaktionsmischung wurde für 30 Minuten gerührt. Dann wurde der Ansatz mit 50 ml Wasser und 50 ml Wasser versetzt und anschließend die wässrige Phase nochmals mit 30 ml Ethylacetat gewaschen. Die vereinten organischen Phasen wurden mit 1 N wässriger Salzsäure gewaschen, über MgSO₄ getrocknet und einrotiert. Der Rückstand (1.93 g) wurde ohne weitere Reinigung in die nächste Reaktion eingesetzt.

### 1-(2-Chlor-phenyl)-2-hydroxy-2-methyl-propan-1-sulfonsäureamid

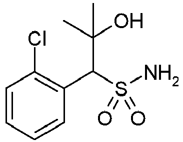

Unter Inertgas wurden 1.51 g C-(2-Chlor-phenyl)-N-(2,4-dimethoxy-benzyl)-methansulfonamid in 30 ml THF vorgelegt und anschließend bei einer Temperatur von -78 °C 6.09 ml einer 1.6 N Methyllithium-Lösung in Diethylether tropfenweise zugegeben und 10 Minuten bei gleichbleibender Temperatur gerührt. Anschließend wurden 1.83 ml Aceton zugegeben. Nach 10 Minuten Rühren wurde die Reaktionslösung mit 2 ml Trifluoressigsäure versetzt und das Lösungsmittel unter vermindertem Druck entfernt. Der Rückstand wurde in Ethylacetat gelöst, die Lösung mit gesättigter wässriger Natriumhydrogencarbonat-Lösung und Wasser gewaschen, über MgSO₄ getrocknet und einrotiert. Der Rückstand wurde in 20 ml Dichlormethan gelöst und mit 3 ml Trifluoressigsäure versetzt. Nach 2 Stunden Rühren bei Raumtemperatur wurde die Reaktionslösung einrotiert und der Rückstand in einer Mischung aus Wasser und Dichlormethan aufgenommen. Der dabei ausgefallene Niederschlag wurde mit LCMS und ¹H-NMR analysiert und anschließend verworfen, da kein Produkt enthalten war. Aus dem Filtrat fiel erneut ein Niederschlag aus, der mit LCMS als produkthaltig (512 mg) identifiziert wurde. Da noch weiteres Produkt in beiden Phasen des Filtrats enthalten war, wurde die wässrige Phase mit Dichlormethan extrahiert, die vereinten organischen Phasen einrotiert und der Rückstand mittels Normalphasenchromatographie über den Flashmaster mit einem n-Heptan/Ethylacetat-Gradienten gereinigt. Die produkthaltigen Fraktionen wurden vereinigt und einrotiert (707 mg). Man erhielt so das Produkt mit dem Molekulargewicht 263.7 g / mol (C₁₀H₁₄ClNO₃S); MS (ESI): m / e = 283 (M+H₂O+H⁺).

### (S)-3-[5-(2-Chlor-phenyl)-6,6-dimethyl-4,4-dioxo-5,6-dihydro-4H-4lambda6-[1,4,3]oxathiazin-2-ylamino]-3-phenyl-propan-1-ol

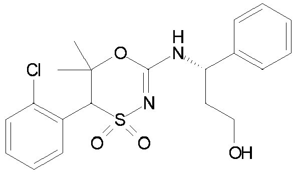

443 mg (S)-3-(tert-Butyl-dimethyl-silanyloxy)-1-phenyl-propylamin wurden in 10 ml Dichlormethan vorgelegt und mit 325 mg 1,1'-Thiocarbonyldiimidazol versetzt. Nach 40 Minuten Rühren bei Raumtemperatur wurde die Reaktionslösung mit einem Gemisch aus 50 ml Diethylether und 50 ml n-Pentan versetzt und 3 x mit 50 ml Wasser gewaschen, über MgSO₄ getrocknet und einrotiert. Unter Inertgas wurden der Rückstand und 400 mg 1-(2-Chlorphenyl)-2-hydroxy-2-methyl-propan-1-sulfonsäureamid in 5 ml NMP gelöst und mit 0.68 ml einer 2 N Lösung von Natrium-bis(trimethylsilyl)-amid in THF tropfenweise versetzt. Nach 15 Minuten Rühren wurden 270 mg N-Bromsuccinimid zugesetzt und die resultierende Reaktionsmischung 5 Minuten bei gleichbleibender Temperatur gerührt. Die Reaktionslösung wurde mit 70 ml Ethylacetat verdünnt und 1 x mit 180 ml und 2 x mit 75 ml Wasser gewaschen. Die organische Phase wurde über MgSO₄ getrocknet, einrotiert, der Rückstand in 8 ml Methanol aufgenommen und nach Zugabe von 1 ml konzentrierter Salzsäure 1 Stunde bei Raumtemperatur gerührt. Die Reaktionslösung wurde mit gesättigter wässriger Natriumhydrogencarbonat-Lösung auf pH 5 eingestellt, mit Dichlormethan verdünnt und mit Wasser gewaschen. Die organische Phase wurde nach dem Trocknen über MgSO₄ einrotiert und der Rückstand im Reinigungslabor über präparative HPLC gereinigt. Die produkthaltigen Fraktionen wurden vereinigt, das Acetonitril unter vermindertem Druck entfernt und der wässrige Rückstand mit gesättigter wässriger Natriumhydrogencarbonat-Lösung neutralisiert. Anschließend wurde 3 x mit 20 ml Ethylacetat extrahiert, die vereinten organischen Phasen über MgSO₄ getrocknet und einrotiert. Der Rückstand wurde erneut in einer Mischung aus Acetonitril und Wasser aufgenommen und lyophilisiert. Man erhielt so das Produkt (94.6 mg) mit dem Molekulargewicht 422.9 g / mol (C₂₀H₂₃ClN₂O₄S); MS (ESI): m / e = 423 (M+H⁺).

### (S)-3-(6,6-Dimethyl-4,4-dioxo-5-o-tolyl-5,6-dihydro-4H-4lambda6-[1,4,3]oxathiazin-2-ylamino)-3 -phenyl-propan- 1 -ol

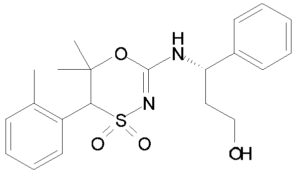

### N-(2,4-Dimethoxy-benzyl)-C-o-tolyl-methansulfonamid

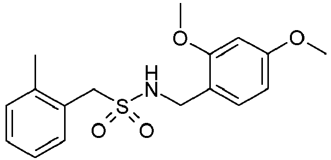

Bei Raumtemperatur wurde 1.00 g o-Tolyl-methansulfonylchlorid in 30 ml Dichlormethan vorgelegt und 1.46 ml 2,4-Dimethoxybenzylamin zugetropft. Die Reaktionsmischung wurde für 30 Minuten gerührt. Dann wurde der Ansatz mit 50 ml Wasser und 50 ml Ethylacetat versetzt und anschließend die wässrige Phase nochmals mit 30 ml Ethylacetat gewaschen. Die vereinten organischen Phasen wurden mit 1 N wässriger Salzsäure gewaschen, über MgSO₄ getrocknet und einrotiert. Der Rückstand (1.42 g) wurde ohne weitere Reinigung in die nächste Reaktion eingesetzt.

### 2-Hydroxy-2-methyl-1-o-tolyl-propan-1-sulfonsäureamid

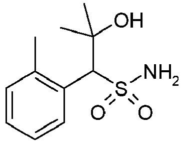

Unter Inertgas wurden 0.95 g N-(2,4-Dimethoxy-benzyl)-C-o-tolyl-methansulfonamid in 20 ml THF vorgelegt und anschließend bei einer Temperatur von -78 °C 4.06 ml einer 1.6 N Methyllithium-Lösung in Diethylether tropfenweise zugegeben und 10 Minuten bei gleichbleibender Temperatur gerührt. Anschließend wurden 1.22 ml Aceton zugegeben. Nach 10 Minuten Rühren wurde die Reaktionslösung mit 2 ml Trifluoressigsäure versetzt, auf Raumtemperatur erwärmen gelassen und das Lösungsmittel unter vermindertem Druck entfernt. Der Rückstand wurde in 10 ml Dichlormethan gelöst und mit 1.5 ml Trifluoressigsäure versetzt. Nach 1.5 Stunden Rühren bei Raumtemperatur wurde die Reaktionslösung einrotiert und der Rückstand in einer Mischung aus Wasser und Dichlormethan aufgenommen. Der dabei ausgefallene Niederschlag wurde abgesaugt und verworfen. Die organische Phase wurde über MgSO₄ getrocknet und einrotiert. Da das Produkt auch in der wässrigen Phase des Filtrats enthalten war, wurde diese 2 x mit 50 ml Ethylacetat extrahiert, die vereinten organischen Phasen über MgSO₄ getrocknet und einrotiert. Beide Rückstände wurden mittels Normalphasenchromatographie über den Flashmaster mit einem n-Heptan/Ethylacetat-Gradienten gereinigt. Die produkthaltigen Fraktionen wurden vereinigt und einrotiert (516 mg). Man erhielt so das Produkt mit dem Molekulargewicht 243.3 g / mol (C₁₁H₁₇NO₃S).

### (S)-3-(6,6-Dimethyl-4,4-dioxo-5-o-tolyl-5,6-dihydro-4H-4lambda6-[1,4,3]oxathiazin-2-ylamino)-3 -phenyl-propan- 1 -ol

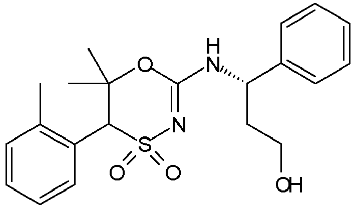

487 mg (S)-3-(tert-Butyl-dimethyl-silanyloxy)-1-phenyl-propylamin wurden in 7 ml Dichlormethan vorgelegt und mit 357 mg 1,1'-Thiocarbonyldiimidazol versetzt. Nach 40 Minuten Rühren bei Raumtemperatur wurde die Reaktionslösung mit einem Gemisch aus 30 ml Diethylether und 30 ml n-Pentan versetzt und 3 x mit 30 ml Wasser gewaschen, über MgSO₄ getrocknet und einrotiert. Unter Inertgas wurden der Rückstand und 255 mg 2-Hydroxy-2-methyl-1-o-tolyl-propan-1-sulfonsäureamid in 5 ml NMP gelöst und mit 0.75 ml einer 2 N Lösung von Natrium-bis(trimethylsilyl)-amid in THF tropfenweise versetzt. Nach 5 Minuten Rühren wurden 297 mg N-Bromsuccinimid zugesetzt und die resultierende Reaktionsmischung 5 Minuten bei gleichbleibender Temperatur gerührt. Die Reaktionslösung wurde mit 30 ml Ethylacetat verdünnt und 1 x mit 75 ml und 2 x mit 40 ml Wasser gewaschen. Die organische Phase wurde über MgSO₄ getrocknet, einrotiert, der Rückstand in 5 ml Methanol aufgenommen und nach Zugabe von 1.59 ml konzentrierter Salzsäure 1.75 Stunden bei Raumtemperatur gerührt. Die Reaktionslösung wurde nach Verdünnung mit 15 ml Wasser mit gesättigter wässriger Natriumhydrogencarbonat-Lösung auf pH 5 eingestellt und mit Ethylacetat extrahiert. Die organische Phase wurde nach dem Trocknen über MgSO₄ einrotiert und der Rückstand im Reinigungslabor über präparative HPLC gereinigt. Die produkthaltigen Fraktionen wurden vereinigt, das Acetonitril unter vermindertem Druck entfernt und der wässrige Rückstand mit gesättigter wässriger Natriumhydrogencarbonat-Lösung neutralisiert. Anschließend wurde 3 x mit 20 ml Ethylacetat extrahiert, die vereinten organischen Phasen über MgSO₄ getrocknet und einrotiert. Der Rückstand wurde erneut in einer Mischung aus Acetonitril und Wasser aufgenommen und lyophilisiert. Man erhielt so das Produkt (63.4 mg) mit dem Molekulargewicht 402.5 g / mol (C₂₁H₂₆N₂O₄S); MS (ESI): m / e = 403 (M+H⁺).

### [5-(3-Benzyloxy-phenyl)-6,6-dimethyl-4,4-dioxo-5,6-dihydro-4H-4lambda6-[1,4,3]oxathiazin-2-yl]-cyclohexyl-amin

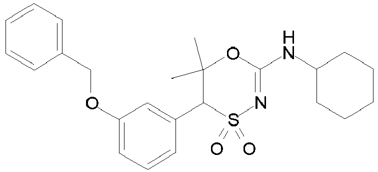

### Methansulfonsäure-3-benzyloxy-benzylester

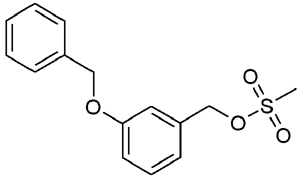

Bei Raumtemperatur wurden 5.00 g o-(3-Benzyloxy-phenyl)-methanol in 80 ml Dichlormethan vorgelegt, mit 3.90 ml Triethylamin versetzt und eine Lösung aus 1.92 ml Methansulfonylchlorid in 5 ml DCM tropfenweise zugegeben. Nach 45 Minuten Rühren wurde die Reaktionsmischung mit je 100 ml Wasser, 1 N wässriger Salzsäure, 1 N wässriger Natronlauge und verdünnter wässriger Natriumchlorid-Lösung gewaschen. Die organische Phase wurde über MgSO₄ getrocknet, einrotiert und im Hochvakuum getrocknet. Der Rückstand (5.86 g) wurde ohne weitere Reinigung in die nächste Reaktion eingesetzt.

### (3-Benzyloxy-phenyl)-methansulfonsäure Natriumsalz

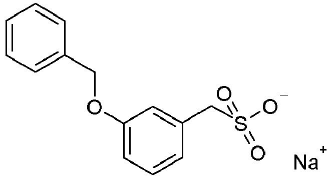

5.80 g Methansulfonsäure-3-benzyloxy-benzylester und 5.21 g Natriumsulfit wurden in 50 ml Wasser gelöst und für 5 Stunden bei 90 °C gerührt. Der Ansatz wurde über Nacht auf Raumtemperatur abkühlen gelassen, anschließend mit 30 ml Ethylacetat versetzt und 1 Stunde im Eisbad gerührt. Der Niederschlag wurde abgesaugt, mit 2 x 10 ml Eiswasser und mit 2 x 10 ml eiskaltem Ethylacetat nachgewaschen und über Nacht im Vakuumtrockenschrank bei 40 °C getrocknet. Man erhielt so das Produkt (2.37 g) mit dem Molekulargewicht 300.3 g / mol (C₁₄H₁₃O₄S.Na); MS (ESI): m / e = 296 (M-Na⁺+H₂O+H⁺).

### (3-Benzyloxy-phenyl)-methansulfonylchlorid

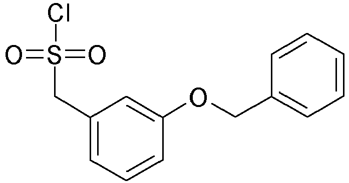

Unter Inertgas wurden 2.36 g (3-Benzyloxy-phenyl)-methansulfonsäure Natriumsalz und 0.61 ml N,N-Dimethyl-formamid in 45 ml THF vorgelegt, anschließend bei einer Temperatur von -20 °C 4.1.73 ml Oxalylchlorid zugetropft und die Reaktionslösung innerhalb von 15 Minuten auf 0 °C kommen gelassen. Die Reaktionslösung wurde mit 100 ml Diethylether verdünnt und mit Wasser, verdünnter wässriger Natriumhydrogencarbonat-Lösung und gesättigter Natriumchlorid-Lösung gewaschen. Die organische Phase wurde über MgSO₄ getrocknet und einrotiert. Der Rückstand (2.30 g) wurde ohne weitere Reinigung in die nächste Reaktion eingesetzt.

### C-(3-Benzyloxy-phenyl)-N-(2,4-dimethoxy-benzyl)-methansulfonamid

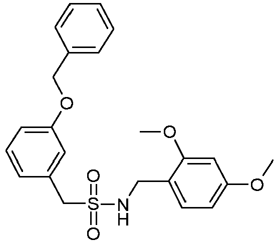

Unter Inertgas wurden 4.89 g (3-Benzyloxy-phenyl)-methansulfonylchlorid 50 ml THF vorgelegt, anschließend bei einer Temperatur von -20 °C 3.00 ml 2,4-Dimethoxybenzylamin zugetropft und die Reaktionslösung innerhalb von 30 Minuten auf 0 °C kommen gelassen. Die Reaktionslösung wurde mit 80 ml Ethylacetat verdünnt und mit Wasser, mit 10%iger wässriger Kaliumhydrogensulfat-Lösung, gesättigter wässriger Natriumhydrogencarbonat-Lösung und gesättigter Natriumchlorid-Lösung gewaschen. Die organische Phase wurde über MgSO₄ getrocknet und einrotiert. Das Rohprodukt wurde mittels Normalphasenchromatographie über den Flashmaster mit einem n-Heptan/Ethylacetat-Gradienten gereinigt. Die produkthaltigen Fraktionen wurden vereinigt, einrotiert und im Hochvakuum getrocknet. Man erhielt so das Produkt (3.12 g) mit dem Molekulargewicht 427.5 g / mol (C₂₃H₂₅NO₅S).

### 1-(3-Benzyloxy-phenyl)-2-hydroxy-2-methyl-propan-1-sulfonsäureamid

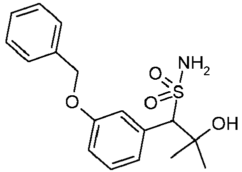

Unter Inertgas wurden 3.10 g C-(3-Benzyloxy-phenyl)-N-(2,4-dimethoxy-benzyl)-methansulfonamid in 60 ml THF vorgelegt und anschließend bei einer Temperatur von -75 °C 12.00 ml einer 1.8 N Phenyllithium-Lösung tropfenweise zugegeben. Die Reaktionsmischung wurde auf 0 °C kommen gelassen und kurz nachgerührt, dann erneut auf -75 °C abgekühlt und 3.80 ml Aceton tropfenweise zugegeben. Nach 10 Minuten Rühren wurden 3 ml Trifluoressigsäure zugegeben und der Ansatz wurde auf Raumtemperatur kommen gelassen. Nach dem Entfernen des Lösungsmittels unter vermindertem Druck wurde der Rückstand in 20 ml Dichlormethan gelöst und mit 5.00 ml Trifluoressigsäure versetzt. Nach 2.5 Stunden Rühren bei Raumtemperatur wurde die Reaktionslösung vorsichtig mit 2 N wässriger Natronlauge neutralisiert und anschließend über Nacht gerührt. Der entstandene Niederschlag wurde abgesaugt, mit Dichlormethan und kaltem Wasser nachgewaschen und 2 x mit Toluol koevaporiert. Das Rohprodukt wurde mittels Normalphasenchromatographie über den Flashmaster mit einem Dichlormethan/Methanol-Gradienten gereinigt. Die produkthaltigen Fraktionen wurden vereinigt und einrotiert. Man erhielt so das Produkt (2.06 g) mit dem Molekulargewicht 335.4 g / mol (C₁₇H₂₁NO₄S).

### [5-(3-Benzyloxy-phenyl)-6,6-dimethyl-4,4-dioxo-5,6-dihydro-4H-4lambda6-[1,4,3]oxathiazin-2-yl]-cyclohexyl-amin

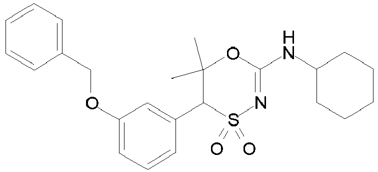

Bei Raumtemperatur wurde eine Lösung aus 1.00 g 1-(3-Benzyloxy-phenyl)-2-hydroxy-2-methyl-propan-1-sulfonsäureamid und 0.45 ml Cylohexylisothiocyanat in 6.5 ml NMP mit 1.49 ml einer 2 N Lösung von Natrium-bis(trimethylsilyl)-amid in THF versetzt. Nach 45 Minuten Rühren wurde die Umsetzung mittels LCMS überprüft. Da die Reaktion noch unvollständig war, wurden zusätzlich 0.22 ml Cylohexylisothiocyanat und 0.74 ml einer 2 N Lösung von Natrium-bis(trimethylsilyl)-amid in THF zugegeben. Nach 20 Minuten Rühren wurden 0.53 g N-Bromsuccinimid zugegeben und eine weitere Stunde bei Raumtemperatur gerührt. Die Reaktionslösung wurde mit 150 ml Wasser versetzt und 2 x mit 100 ml Ethylacetat extrahiert. Die vereinten organischen Phasen wurden mit gesättigter wässriger Natriumchlorid-Lösung gewaschen, über MgSO₄ getrocknet und einrotiert. Der Rückstand wurde im Reinigungslabor über präparative HPLC gereinigt und die produkthaltigen Fraktionen unter vermindertem Druck vom organischen Lösungsmittel befreit. Der wässrige Rückstand wurde lyophilisiert. Man erhielt so das Produkt (587 mg) mit dem Molekulargewicht 442.6 g / mol (C₂₄H₃₀N₂O₄S); MS (ESI): m / e = 443 (M+H⁺).

### 3-(2-Cyclohexylamino-6,6-dimethyl-4,4-dioxo-5,6-dihydro-4H-4lambda6-[1,4,3]oxathiazin-5-yl)-phenol

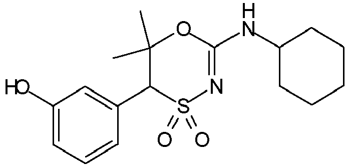

Unter Inertgas wurden 548 mg [5-(3-Benzyloxy-phenyl)-6,6-dimethyl-4,4-dioxo-5,6-dihydro-4H-4lambda6-[1,4,3]oxathiazin-2-yl]-cyclohexyl-amin in 20 ml Methanol vorgelegt und unter Zusatz von 263 mg Pd/C 10 % für 3.5 Stunden unter Wasserstoff-Atmosphäre gerührt. Die Vollständigkeit der Umsetzung wurde mit LCMS geprüft, die Reaktion nochmals mit 263 mg Pd/C 10 % versetzt und für 2 Tage unter Wasserstoff-Atmosphäre gerührt. Nach Filtration von den festen Rückständen wurde das Filtrat unter vermindertem Druck vom Lösungsmittel befreit und im Hochvakuum getrocknet. Ein Teil (105 mg) des so erhaltenenen Rückstandes (468 mg) wurde im Reinigungslabor über präparative HPLC gereinigt und die produkthaltigen Fraktionen unter vermindertem Druck vom organischen Lösungsmittel befreit. Der wässrige Rückstand wurde lyophilisiert. Man erhielt so das Produkt (66 mg) mit dem Molekulargewicht 352.4 g / mol (C₁₇H₂₄N₂O₄S); MS (ESI): m / e = 353 (M+H⁺).

### Cyclohexyl-[5-(3-methoxy-phenyl)-6,6-dimethyl-4,4-dioxo-5,6-dihydro-4H-4lambda6-[1,4,3]oxathiazin-2-yl]-amin

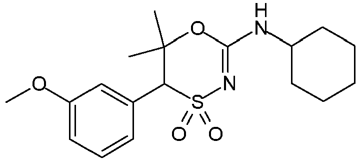

Zu einer Lösung von 120 mg 3-(2-Cyclohexylamino-6,6-dimethyl-4,4-dioxo-5,6-dihydro-4H-4lambda6-[1,4,3]oxathiazin-5-yl)-phenol in 2 ml NMP wurden bei Raumtemperatur 38 mg Kalium-tert-butylat und 97 mg Methyliodid zugegeben und die Reaktionsmischung für 2.5 Stunden bei gleichbleibender Temperatur gerührt. Die Reaktionslösung wurde mit 60 ml Wasser verdünnt, 5 ml 5%iger wässriger Natriumthiosulfat-Lösung zugegeben und das Gemisch 2 x mit 30 ml Ethylacetat extrahiert. Die vereinten organischen Phasen wurden über MgSO₄ getrocknet, einrotiert und der Rückstand im Reinigungslabor über präparative HPLC gereinigt. Die produkthaltige Fraktion wurde lyophilisiert. Man erhielt so das Produkt (54 mg) mit dem Molekulargewicht 366.5 g / mol (C₁₈H₂₆N₂O₄S); MS (ESI): m / e = 367 (M+H⁺).

### [5-(3-Benzyloxy-phenyl)-6,6-dimethyl-4,4-dioxo-5,6-dihydro-4H-4lambda6-1,4,3-oxathiazin-2-yl]-[(S)-3-(tert-butyl-dimethyl-silanyloxy)-1-phenyl-propyl]-amin

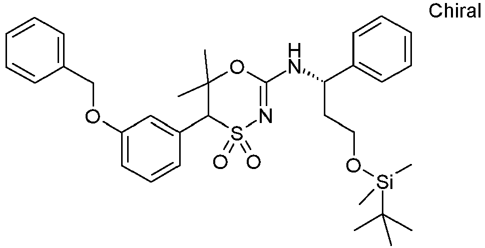

921 mg (S)-3-(tert-Butyl-dimethyl-silanyloxy)-1-phenyl-propylamin wurden in 10 ml Dichlormethan gelöst und mit 638 mg 1,1'- Thiocarbonyldiimidazol versetzt. Nach 30 Minuten Rühren bei Raumtemperatur wurde die Reaktionslösung mit einem Gemisch aus 50 ml Diethylether und 50 ml n-Pentan versetzt und 3 x mit 50 ml Wasser gewaschen, über MgSO₄ getrocknet und einrotiert. Unter Inertgas wurden der Rückstand und 1000 mg 1-(3-Benzyloxy-phenyl)-2-hydroxy-2-methyl-propan-1-sulfonsäureamid in 8 ml NMP gelöst und mit 1.49 ml einer 2 N Lösung von Natrium-bis(trimethylsilyl)-amid in THF tropfenweise versetzt. Nach 15 Minuten Rühren wurden 531 mg N-Bromsuccinimid zugesetzt und die resultierende Reaktionslösung 10 Minuten bei gleichbleibender Temperatur gerührt. Die Reaktionslösung wurde mit 100 ml Wasser versetzt und 2 x mit 100 ml Ethylacetat extrahiert. Die vereinten organischen Phasen wurden über MgSO₄ getrocknet, einrotiert und der Rückstand mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (1.22 g) mit dem Molekulargewicht 608.9 g / mol (C₃₃H₄₄N₂O₅SSi); MS (ESI): m / e = 609 (M+H⁺).

### (S)-3-[5-(3-Benzyloxy-phenyl)-6,6-dimethyl-4,4-dioxo-5,6-dihydro-4H-4lambda6-[1,4,3] oxathiazin-2-ylamino]-3-phenyl-propan-1-ol

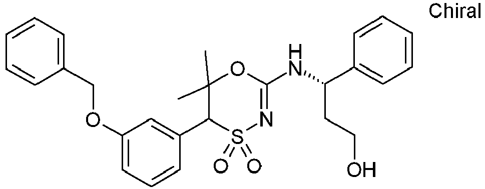

Zur Abspaltung der Schutzgruppe wurden 220 mg [5-(3-Benzyloxy-phenyl)-6,6-dimethyl-4,4-dioxo-5,6-dihydro-4H-4lambda6-1,4,3-oxathiazin-2-yl]-[(S)-3-(tert-butyl-dimethyl-silanyloxy)-1-phenyl-propyl]-amin in 4 ml Methanol gelöst und nach Zugabe von 0.4 ml konzentrierter Salzsäure für 1.75 Stunden bei Raumtemperatur gerührt. Die Reaktionslösung wurde einrotiert, noch 2 x mit 10 ml Toluol koevaporiert und der Rückstand im Reinigungslabor über präparative HPLC gereinigt. Die produkthaltigen Fraktionen wurden vereinigt, das Acetonitril unter vermindertem Druck entfernt und der wässrige Rückstand mit gesättigter wässriger Natriumhydrogencarbonat-Lösung basisch gestellt. Anschließend wurde dieser mit Dichlormethan extrahiert, die vereinten organischen Phasen über eine Phasenseparator-Kartusche (Chromabond^{®} PTS) getrocknet und einrotiert. Der Rückstand wurde erneut in einer Mischung aus Acetonitril und Wasser aufgenommen und lyophilisiert. Man erhielt so das Produkt (129 mg) mit dem Molekulargewicht 494.6 g / mol (C₂₇H₃₀N₂O₅S); MS (ESI): m / e = 495 (M+H⁺).

### 3-[2-((S)-3-Hydroxy-1-phenyl-propylamino)-6,6-dimethyl-4,4-dioxo-5,6-dihydro-4H-4lambda6-[1,4,3]oxathiazin-5-yl]-phenol

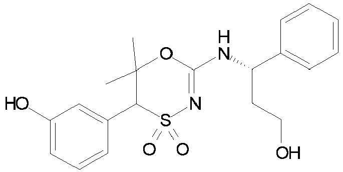

### 3-{2-[(S)-3-(tert-Butyl-dimethyl-silanyloxy)-1-phenyl-propylamino]-6,6-dimethyl-4,4-dioxo-5,6-dihydro-4H-4lambda6-1,4,3-oxathiazin-5-yl}-phenol

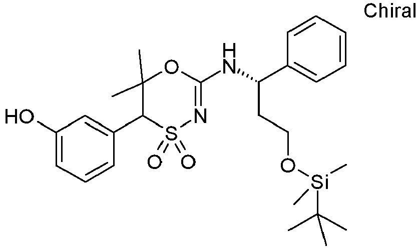

Unter Inertgas wurden 1.03 g [5-(3-Benzyloxy-phenyl)-6,6-dimethyl-4,4-dioxo-5,6-dihydro-4H-41ambda6-1,4,3-oxathiazin-2-yl]-[(S)-3-(tert-butyl-dimethyl-silanyloxy)-1-phenyl-propyl]-amin in 20 ml Methanol vorgelegt und unter Zusatz von 0.24 ml Triethylamin und 359 mg Pd/C 10 % für 3.75 Stundem unter Wasserstoff-Atmosphäre gerührt. Die Vollständigkeit der Umsetzung wurde mit LCMS geprüft, die Reaktion nochmals mit 359 mg Pd/C 10 % versetzt und weitere 16 Stunden unter Wasserstoff-Atmosphäre gerührt. Laut LCMS blieb die Abspaltung der Benzyl-Schutzgruppe unvollständig. Nach Filtration von den festen Rückständen wurde das Filtrat erneut mit 359 mg Pd/C 10 % versetzt. Nach 5 h Rühren unter Wasserstoff-Atmosphäre wurden nochmals 359 mg Pd/C 10 % zugegeben und nach weiteren 2 Stunden wurde die Reaktion nach Überprüfung mit LCMS für vollständig befunden. Nach Filtration von den Katalysatorrückständen wurde das Filtrat unter vermindertem Druck vom Lösungsmittel befreit. Man erhielt so das Produkt (764 mg) mit dem Molekulargewicht 518.8 g / mol (C₂₆H₃₈N₂O₅SSi); MS (ESI): m / e = 519 (M+H⁺).

### 3-[2-((S)-3-Hydroxy-1-phenyl-propylamino)-6,6-dimethyl-4,4-dioxo-5,6-dihydro-4H-4lambda6-[1,4,3]oxathiazin-5-yl]-phenol

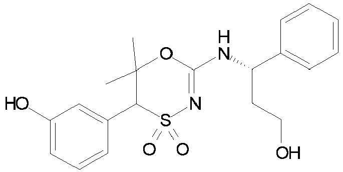

Zur Abspaltung der Schutzgruppe wurden 150 mg 3-{2-[(S)-3-(tert-Butyl-dimethyl-silanyloxy)-1-phenyl-propylamino]-6,6-dimethyl-4,4-dioxo-5,6-dihydro-4H-4lambda6-1,4,3-oxathiazin-5-yl}-phenol in 3 ml Methanol gelöst und nach Zugabe von 0.3 ml konzentrierter Salzsäure für 16 Stunden bei Raumtemperatur gerührt. Die Reaktionslösung wurde einrotiert, noch 2 x mit 10 ml Toluol koevaporiert und der Rückstand im Reinigungslabor über präparative HPLC gereinigt. Die produkthaltigen Fraktionen wurden vereinigt, das Acetonitril unter vermindertem Druck entfernt und der wässrige Rückstand mit gesättigter wässriger Natriumhydrogencarbonat-Lösung basisch gestellt. Anschließend wurde dieser mit Dichlormethan extrahiert, die vereinten organischen Phasen über eine Phasenseparator-Kartusche (Chromabond^{®} PTS) getrocknet und einrotiert. Der Rückstand wurde erneut in einer Mischung aus Acetonitril und Wasser aufgenommen und lyophilisiert. Man erhielt so das Produkt (64.2 mg) mit dem Molekulargewicht 404.5 g / mol (C₂₀H₂₄N₂O₅S); MS (ESI): m / e = 405 (M+H⁺).

### (S)-3-[5-(3-Methoxy-phenyl)-6,6-dimethyl-4,4-dioxo-5,6-dihydro-4H-4lambda6-[1,4,3]oxathiazin-2-ylamino]-3-phenyl-propan-1-ol

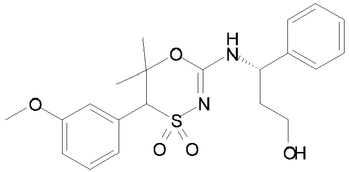

Zu einer Lösung von 452 mg 3-{2-[(S)-3-(tert-Butyl-dimethyl-silanyloxy)-1-phenyl-propylamino]-6,6-dimethyl-4,4-dioxo-5,6-dihydro-4H-4lambda6-1,4,3-oxathiazin-5-yl}-phenol und 148 mg Methyliodid in THF bei 0°C wurden tropfenweise 0.44 ml einer 2 N Lösung von Natrium-bis(trimethylsilyl)-amid in THF zugegeben. Nach 45 Minuten Rühren bei gleichbleibender Temperatur und anschließendem Aufwärmen auf Raumtemperatur wurde die Reaktionslösung weitere 4 Stunden gerührt und dann die Umsetzung mit LCMS geprüft. Es wurden weitere 0.20 ml der 2 N Lösung von Natrium-bis(trimethylsilyl)-amid in THF und 114 mg Methyliodid zugesetzt. Nach 16 Stunden Rühren bei Raumtemperatur wurde die Reaktionslösung mit 60 ml Wasser verdünnt, 5 ml 5%iger wässriger Natriumthiosulfat-Lösung zugegeben und das Gemisch 2 x mit 30 ml Ethylacetat extrahiert. Die vereinten organischen Phasen wurden über MgSO₄ getrocknet und einrotiert. Zur Abspaltung der Schutzgruppe wurde der Rückstand in 5 ml Methanol gelöst und nach Zugabe von 0.5 ml konzentrierter Salzsäure für 1 Stunde bei Raumtemperatur gerührt. Die Reaktionslösung wurde mit 50 ml Wasser verdünnt und 2 x mit Ethylacetat extrahiert. Die vereinten organischen Phasen wurden über MgSO₄ getrocknet, einrotiert und der Rückstand im Reinigungslabor über präparative HPLC gereinigt. Die produkthaltigen Fraktionen wurden vereinigt und lyophilisiert. Man erhielt so das Produkt (41.1 mg) mit dem Molekulargewicht 418.5 g / mol (C₂₁H₂₆N₂O₅S); MS (ESI): m / e = 419 (M+H⁺).

### (S)-3-[5-(3-Chlor-phenyl)-6,6-dimethyl-4,4-dioxo-5,6-dihydro-4H-4lambda6-[1,4,3]oxathiazin-2-ylamino]-3-phenyl-propan-1-ol

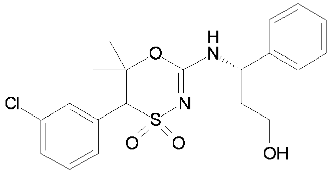

### C-(3-Chlor-phenyl)-N-(2,4-dimethoxy-benzyl)-methansulfonamid

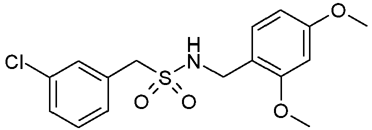

Bei Raumtemperatur wurde 1.06 g 3-Chlorphenylmethansulfonylchlorid in 30 ml Dichlormethan vorgelegt und 1.42 ml 2,4-Dimethoxybenzylamin zugetropft. Die Reaktionsmischung wurde für 30 Minuten gerührt. Dann wurde der Ansatz mit 50 ml Wasser und 50 ml Wasser versetzt und anschließend die wässrige Phase nochmals mit 30 ml Ethylacetat gewaschen. Die vereinten organischen Phasen wurden mit 1 N wässriger Salzsäure gewaschen, über MgSO₄ getrocknet und einrotiert. Der Rückstand (1.65 g) wurde ohne weitere Reinigung in die nächste Reaktion eingesetzt.

### 1-(3 -Chlor-phenyl)-2-hydroxy-2-methyl-propan-1 -sulfonsäureamid

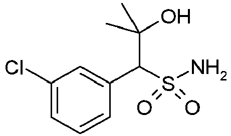

Unter Inertgas wurden 1.65 g C-(3-Chlor-phenyl)-N-(2,4-dimethoxy-benzyl)-methansulfonamid in 30 ml THF vorgelegt und anschließend bei einer Temperatur von -75 °C 6.67 ml einer 1.6 N Methyllithium-Lösung in Diethylether tropfenweise zugegeben und 10 Minuten bei gleichbleibender Temperatur gerührt. Anschließend wurden 1.86 ml Aceton zugegeben. Nach 5 Minuten Rühren wurde die Reaktionslösung mit 15 ml Trifluoressigsäure versetzt, auf Raumtemperatur erwärmen gelassen und das Lösungsmittel unter vermindertem Druck entfernt. Der Rückstand wurde in 10 ml Dichlormethan gelöst und mit 2.5 ml Trifluoressigsäure versetzt. Nach 2 Stunden Rühren bei Raumtemperatur wurde die Reaktionslösung mit 50 ml Wasser versetzt, der dabei ausgefallene Niederschlag abgesaugt und mit Wasser nachgewaschen. Zur Trocknung wurde das Produkt noch 2 x mit Toluol koevaporiert. Man erhielt so das Produkt (1.16 g) mit dem Molekulargewicht 263.7 g / mol (C₁₀H₁₄ClNO₃S).

### (S)-3-[5-(3-Chlor-phenyl)-6,6-dimethyl-4,4-dioxo-5,6-dihydro-4H-4lambda6-[1,4,3]oxathiazin-2-ylamino]-3-phenyl-propan-1-ol

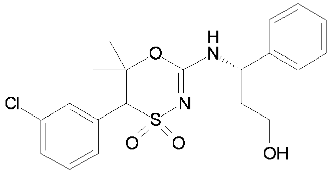

443 mg (S)-3-(tert-Butyl-dimethyl-silanyloxy)-1-phenyl-propylamin wurden in 10 ml Dichlormethan vorgelegt und mit 325 mg 1,1'-Thiocarbonyldiimidazol versetzt. Nach 45 Minuten Rühren bei Raumtemperatur wurde die Reaktionslösung mit einem Gemisch aus 50 ml Diethylether und 50 ml n-Pentan versetzt und 3 x mit 50 ml Wasser gewaschen, über MgSO₄ getrocknet und einrotiert. Unter Inertgas wurden der Rückstand und 400 mg 1-(3-Chlorphenyl)-2-hydroxy-2-methyl-propan-1-sulfonsäureamid in 7 ml NMP gelöst und mit 0.68 ml einer 2 N Lösung von Natrium-bis(trimethylsilyl)-amid in THF tropfenweise versetzt. Nach 10 Minuten Rühren wurden 270 mg N-Bromsuccinimid zugesetzt und die resultierende Reaktionsmischung 10 Minuten bei gleichbleibender Temperatur gerührt. Die Reaktionslösung wurde mit 70 ml Ethylacetat verdünnt und 1 x mit 180 ml und 2 x mit 75 ml Wasser gewaschen. Die organische Phase wurde über MgSO₄ getrocknet, einrotiert, der Rückstand in 10 ml Methanol aufgenommen und nach Zugabe von 1 ml konzentrierter Salzsäure 1.5 Stunden bei Raumtemperatur gerührt. Die Reaktionslösung wurde nach Verdünnung mit 10 ml Wasser mit gesättigter wässriger Natriumhydrogencarbonat-Lösung auf pH 6 eingestellt und mit 3 x 20 ml Ethylacetat extrahiert. Die organische Phase wurde nach dem Trocknen über eine Diatomeenerde-Kartusche (Varian Chem Elut^{®}) einrotiert und der Rückstand im

Reinigungslabor über präparative HPLC gereinigt. Die produkthaltigen Fraktionen wurden vereinigt, das Acetonitril unter vermindertem Druck entfernt und der wässrige Rückstand mit gesättigter wässriger Natriumhydrogencarbonat-Lösung auf pH5 gebracht. Anschließend wurde dieser 3 x mit 20 ml Ethylacetat extrahiert, die vereinten organischen Phasen über eine Diatomeenerde-Kartusche (Varian Chem Elut^{®}) getrocknet und einrotiert. Der Rückstand wurde erneut in einer Mischung aus Acetonitril und Wasser aufgenommen und lyophilisiert. Man erhielt so das Produkt (84.2 mg) mit dem Molekulargewicht 422.9 g / mol (C₂₀H₂₃ClN₂O₄S); MS (ESI): m / e = 423 (M+H⁺).

Die Verbindung 21 wurde mit dieser Herstellungsvorschrift synthetisiert:
(S)-3-[6,6-Dimethyl-4,4-dioxo-5-(3-trifluormethyl-phenyl)-5,6-dihydro-4H-4lambda6-[1,4,3] oxathiazin-2-ylamino]-3-phenyl-propan-1-ol

Lediglich auf der Stufe des 2-Hydroxy-2-methyl-1-(3-trifluormethyl-phenyl)-propan-1-sulfonsäureamids wurde das Rohprodukt zusätzlich mittels Normalphasenchromatographie über den Flashmaster mit einem n-Heptan/Ethylacetat-Gradienten gereinigt.

### Cyclohexyl-(5,6,6-trimethyl-4,4-dioxo-5-phenyl-5,6-dihydro-4H-4lambda6-[1,4,3]oxathiazin-2-yl)-amin

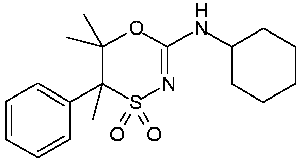

### 1-Phenyl-ethansulfonsäure-2,4-dimethoxy-benzylamid

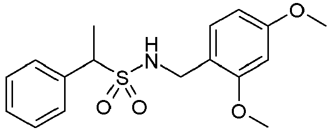

Unter Inertgas wurden 375 mg N-(2,4-Dimethoxy-benzyl)-C-phenyl-methansulfonamid in 40 ml THF vorgelegt und anschließend bei einer Temperatur von -78 °C 1.46 ml einer 1.6 N Butyllithium-Lösung in Hexan tropfenweise zugegeben und 5 Minuten gerührt. Nach der Zugabe von 166 mg Methyliodid wurde die Reaktionsmischung auf Raumtemperatur kommen gelassen. Die Reaktionslösung wurde mit 50 ml Wasser und 50 ml Ethylacetat versetzt, die wässrige Phase mit 50 ml Ethylacetat reextrahiert. Die vereinten organischen Phasen wurden über MgSO₄ getrocknet, einrotiert und der Rückstand mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (334 mg) mit dem Molekulargewicht 335.4 g / mol (C₁₇H₂₁NO₄S).

### 3-Hydroxy-3-methyl-2-phenyl-butan-2-sulfonsäureamid

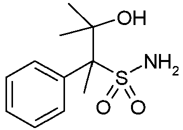

Unter Inertgas wurden 330 mg 1-Phenyl-ethansulfonsäure-2,4-dimethoxy-benzylamid in 5 ml THF vorgelegt und anschließend bei einer Temperatur von -78 °C 1.41 ml einer 1.6 N Butyllithium-Lösung in Hexan tropfenweise zugegeben und 5 Minuten gerührt. Anschließend wurden 0.22 ml Aceton zugegeben, kurz nachgerührt und nach der Zugabe von 1 ml Trifluoressigsäure wurde die Reaktionslösung auf Raumtemperatur kommen gelassen. Die Reaktionsmischung wurde mit 50 ml Wasser und 50 ml Ethylacetat versetzt, die wässrige Phase mit 50 ml Ethylacetat reextrahiert. Die vereinten organischen Phasen wurden über MgSO₄ getrocknet, einrotiert und der Rückstand mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (113 mg) mit dem Molekulargewicht 243.3 g / mol (C₁₁H₁₇NO₃S).

### [5-(3-Brom-phenyl)-5,6,6-trimethyl-4,4-dioxo-5,6-dihydro-4H-4lambda6-[1,4,3]oxathiazin-2-yl]-[(S)-1-(2-fluor-phenyl)-ethyl]-amin

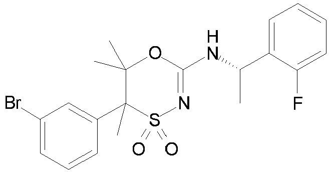

### 1-(3-Brom-phenyl)-ethansulfonsäure-2,4-dimethoxy-benzylamid

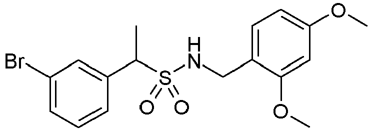

Unter Inertgas wurden 3.50 g C-(3-Brom-phenyl)-N-(2,4-dimethoxy-benzyl)-methansulfonamid in 40 ml THF vorgelegt und anschließend bei einer Temperatur von -76 °C 10.93 ml einer 1.6 N Methyllithium-Lösung in Diethylether tropfenweise zugegeben und 5 Minuten gerührt. Nach der Zugabe von 1.24 g Methyliodid wurde die Reaktionsmischung auf Raumtemperatur kommen gelassen. Die Reaktionslösung wurde mit Wasser und Ethylacetat versetzt, die wässrige Phase mit Ethylacetat reextrahiert. Die vereinten organischen Phasen wurden über MgSO₄ getrocknet, einrotiert und der Rückstand mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (2.83 g) mit dem Molekulargewicht 414.3 g / mol (C₁₇H₂₀BrNO₄S).

### 2-(3-Brom-phenyl)-3-hydroxy-3-methyl-butan-2-sulfonsäureamid

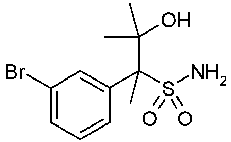

Unter Inertgas wurden 2.82 g 1-(3-Brom-phenyl)-ethansulfonsäure-2,4-dimethoxy-benzylamid in 40 ml THF vorgelegt und anschließend bei einer Temperatur von -76 °C 10.00 ml einer 1.6 N Methyllithium-Lösung in Diethylether tropfenweise zugegeben und 5 Minuten bei gleichbleibender Temperatur gerührt. Anschließend wurden 1.50 ml Aceton zugegeben. Nach 5 Minuten Rühren wurde die Reaktionslösung auf Raumtemperatur erwärmen gelassen und das Lösungsmittel unter vermindertem Druck entfernt. Der Rückstand wurde in 30 ml Dichlormethan gelöst, mit 2 ml Trifluoressigsäure versetzt und 90 Minuten bei Raumtemperatur gerührt. Die Umsetzung wurde mit LCMS geprüft, weitere 2 ml Trifluoressigsäure zugesetzt und die Mischung für 75 Minuten gerührt. Dann wurden unter kräftigem Rühren 40 ml Wasser zugegeben, die Phasen getrennt und die wässrige Phase erneut mit 2 x 30 ml Dichlormethan gewaschen. Die vereinten organischen Phasen wurden einrotiert und 2 x mit Toluol koevaporiert. Das Rohprodukt wurde mittels Normalphasenchromatographie über den Flashmaster mit einem Dichlormethan/Methanol-Gradienten gereinigt. Die produkthaltigen Fraktionen wurden vereinigt und einrotiert. Man erhielt so das Produkt (1.10g) mit dem Molekulargewicht 322.2 g / mol (C₁₁H₁₆BrNO₃S); MS (ESI): m / e = 341 (M+H₂O+H⁺).

### 5-(3-Brom-phenyl)-2-methoxy-5,6,6-trimethyl-5,6-dihydro-1,4,3-oxathiazin-4,4-dioxid

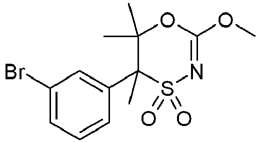

Eine Suspension aus 1.10 g 2-(3-Brom-phenyl)-3-hydroxy-3-methyl-butan-2-sulfonsäureamid in 10 ml Tetramethoxymethan wurde mit 2 ml Essigsäure versetzt und 5 Stunden bei 100°C gerührt. Nach dem Entfernen des Lösungsmittels unter vermindertem Druck wurde das Rohprodukt mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (0.61 g) mit dem Molekulargewicht 362.2 g / mol (C₁₃H₁₆BrNO₄S).

### [5-(3-Brom-phenyl)-5,6,6-trimethyl-4,4-dioxo-5,6-dihydro-4H-4lambda6-1,4,3-oxathiazin-2-yl]-[(S)-1-(2-fluor-phenyl)-ethyl]-amin

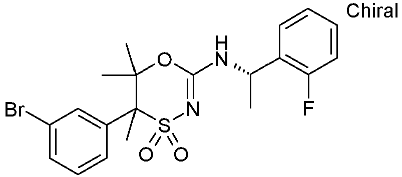

Eine Lösung aus 209 mg 5-(3-Brom-phenyl)-2-methoxy-5,6,6-trimethyl-5,6-dihydro-1,4,3-oxathiazin-4,4-dioxid und 92 mg (S)-1-(2-Fluor-phenyl)-ethylamin in 5 ml Methylenchlorid wurde 1 Stunde bei Raumtemperatur gerührt und die Umsetzung mit LCMS geprüft. Da noch keine Umsetzung stattgefunden hatte, wurde das Lösungsmittel einrotiert und der Rückstand für 19 Stunden bei Raumtemperatur gerührt. Das Rohprodukt wurde mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (170 mg) mit dem Molekulargewicht 469.4 g / mol (C₂₀H₂₂BrFN₂O₃S); MS (ESI): m / e = 469 (M+H⁺).

### (S)-3-[5-(3-Brom-phenyl)-5,6,6-trimethyl-4,4-dioxo-5,6-dihydro-4H-4lambda6-[1,4,3]oxathiazin-2-ylamino]-3-phenyl-propan-1-ol

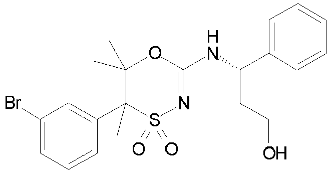

### [5-(3-Brom-phenyl)-5,6,6-trimethyl-4,4-dioxo-5,6-dihydro-4H-4lambda6-1,4,3-oxathiazin-2-yl]-[(S)-3-(tert-butyl-dimethyl-silanyloxy)-1-phenyl-propyl]-amin

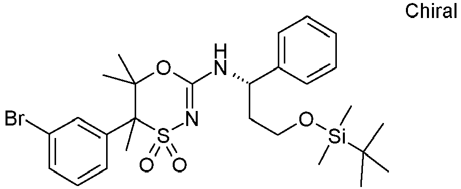

Eine Lösung aus 209 mg 5-(3-Brom-phenyl)-2-methoxy-5,6,6-trimethyl-5,6-dihydro-1,4,3-oxathiazin-4,4-dioxid und 181 mg (S)-3-(tert-Butyl-dimethyl-silanyloxy)-1-phenyl-propylamin in 5 ml Methylenchlorid wurde 1 Stunde bei Raumtemperatur gerührt und die Umsetzung mit LCMS geprüft. Da noch keine Umsetzung stattgefunden hatte, wurde das Lösungsmittel einrotiert und der Rückstand für 19 Stunden bei Raumtemperatur gerührt. Das Rohprodukt wurde mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (237 mg) mit dem Molekulargewicht 595.7 g / mol (C₂₇H₃₉BrN₂O₄SSi); MS (ESI): m / e = 596 (M+H⁺).

### (S)-3-[5-(3-Brom-phenyl)-5,6,6-trimethyl-4,4-dioxo-5,6-dihydro-4H-4lambda6-[1,4,3] oxathiazin-2-ylamino]-3-phenyl-propan-1-ol

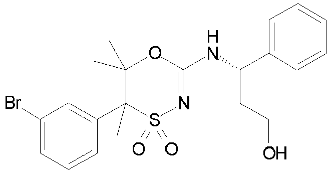

Zur Abspaltung der Schutzgruppe wurden 20 mg [5-(3-Brom-phenyl)-5,6,6-trimethyl-4,4-dioxo-5,6-dihydro-4H-4lambda6-1,4,3-oxathiazin-2-yl]-[(S)-3-(tert-butyl-dimethyl-silanyloxy)-1-phenyl-propyl]-amin in 1 ml Methanol gelöst und nach Zugabe von 0.05 ml konzentrierter Salzsäure für 2 Stunden bei Raumtemperatur gerührt. Die Reaktionslösung wurde einrotiert und der Rückstand im Reinigungslabor über präparative HPLC gereinigt. Die produkthaltigen Fraktionen wurden vereinigt, das Acetonitril unter vermindertem Druck entfernt und der wässrige Rückstand mit gesättigter wässriger Natriumhydrogencarbonat-Lösung basisch gestellt. Anschließend wurde dieser mit Ethylacetat extrahiert, die vereinten organischen Phasen über eine Kartusche mit Diatomeenerde (Varian Chem Elut^{®}) getrocknet und einrotiert. Der Rückstand wurde erneut in einer Mischung aus Acetonitril und Wasser aufgenommen und lyophilisiert. Man erhielt so das Produkt (10.2 mg) mit dem Molekulargewicht 481.4 g / mol (C₂₁H₂₅BrN₂O₄S); MS (ESI): m / e = 482 (M+H⁺).

### (S)-3-[5-(3-Brom-phenyl)-5,6,6-trimethyl-4,4-dioxo-5,6-dihydro-4H-4lambda6-[1,4,3] oxathiazin-2-ylamino]-3-(2-fluor-phenyl)-propan-1-ol

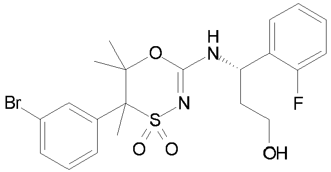

### (S)-3-(tert-Butyl-dimethyl-silanyloxy)-1-(2-fluor-phenyl)-propylamin

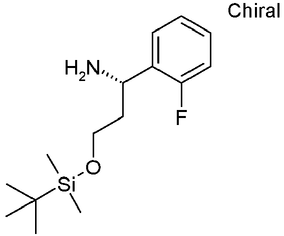

Unter Inertgas wurden zu einer Suspension aus 0.97 g (S)-3-Amino-3-(2-fluor-phenyl)-propionsäure in 80 ml THF 2.80 g Lithiumaluminiumhydrid in Portionen zugegeben und die Reaktionsmischung anschließend bei einer Temperatur von 50 °C 3 Stunden gerührt. 3 ml Wasser, 1 ml 12 N wässrige Natronlauge und erneut 10 ml Wasser wurden zugegeben und die Mischung für 10 Minuten gerührt. Der Niederschlag wurde abfiltriert, das Filtrat einrotiert, der Rückstand nochmals in Methylenchlorid gelöst, die Lösung über MgSO₄ getrocknet und einrotiert. Zur weiteren Reaktion wurde eine Lösung des Rückstandes in Methylenchlorid mit 2.22 ml Triethylamin und 1.20 g tert-Butyl-chlor-dimethyl-silan versetzt. Nach Rühren über Nacht bei Raumtemperatur wurde die Reaktionslösung mit wässriger Natriumhydrogencarbonat-Lösung gewaschen, über MgSO₄ getrocknet und einrotiert. Das Rohprodukt wurde mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (880 g) mit dem Molekulargewicht 283.5 g / mol (C₁₅H₂₆FNOSi), MS (ESI): m / e = 284 (M+H⁺).

### [5-(3-Brom-phenyl)-5,6,6-trimethyl-4,4-dioxo-5,6-dihydro-4H-4lambda6-1,4,3-oxathiazin-2-yl]-[(S)-3-(tert-butyl-dimethyl-silanyloxy)-1-(2-fluor-phenyl)-propyl]-amin

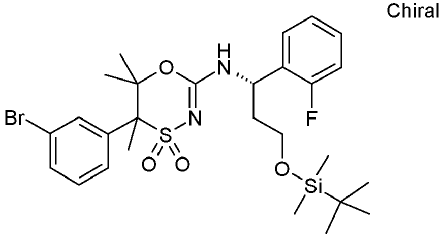

Eine Lösung aus 188 mg 5-(3-Brom-phenyl)-2-methoxy-5,6,6-trimethyl-5,6-dihydro-1,4,3-oxathiazin-4,4-dioxid und 157 mg (S)-3-(tert-Butyl-dimethyl-silanyloxy)-1-(2-fluor-phenyl)-propylamin in 2 ml Methylenchlorid wurde 3 Stunden bei Raumtemperatur gerührt und die Umsetzung mit LCMS geprüft. Da noch keine Reaktion stattgefunden hatte, wurde das Lösungsmittel einrotiert und der Rückstand unter Zusatz von 0.25 ml Methylenchlorid für 17 Stunden bei Raumtemperatur gerührt. Anschließend wurde die Reaktionsmischung 4 Stunden bei 50 °C geührt. Das Rohprodukt wurde mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (206 mg) mit dem Molekulargewicht 613.7 g / mol (C₂₇H₃₈BrFN₂O₄SSi); MS (ESI): m / e = 614 (M+H⁺).

### (S)-3-[5-(3-Brom-phenyl)-5,6,6-trimethyl-4,4-dioxo-5,6-dihydro-4H-4lambda6-[1,4,3] oxathiazin-2-ylamino]-3-(2-fluor-phenyl)-propan-1-ol

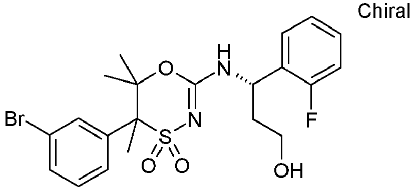

Zur Abspaltung der Schutzgruppe wurden 20 mg [5-(3-Brom-phenyl)-5,6,6-trimethyl-4,4-dioxo-5,6-dihydro-4H-4lambda6-1,4,3-oxathiazin-2-yl]-[(S)-3-(tert-butyl-dimethyl-silanyloxy)-1-(2-fluor-phenyl)-propyl]-amin in 1 ml Methanol gelöst und nach Zugabe von 0.05 ml konzentrierter Salzsäure für 2.5 Stunden bei Raumtemperatur gerührt. Die Reaktionslösung wurde einrotiert und der Rückstand im Reinigungslabor über präparative HPLC gereinigt. Die produkthaltigen Fraktionen wurden vereinigt, das Acetonitril unter vermindertem Druck entfernt und der wässrige Rückstand mit gesättigter wässriger Natriumhydrogencarbonat-Lösung basisch gestellt. Anschließend wurde dieser mit Ethylacetat extrahiert, die vereinten organischen Phasen über MgSO₄ getrocknet und einrotiert. Der Rückstand wurde erneut in einer Mischung aus Acetonitril und Wasser aufgenommen und lyophilisiert. Man erhielt so das Produkt (10.2 mg) mit dem Molekulargewicht 499.4 g / mol (C₂₁H₂₄BrFN₂O₄S); MS (ESI): m / e = 500 (M+H⁺).

### (S)-3-Phenyl-3-(5,6,6-trimethyl-4,4-dioxo-5-phenyl-5,6-dihydro-4H-4lambda6-[1,4,3]oxathiazin-2-ylamino)-propan-1-ol

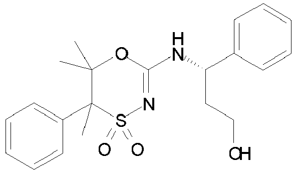

Unter Inertgas wurden 50 mg [5-(3-Brom-phenyl)-5,6,6-trimethyl-4,4-dioxo-5,6-dihydro-4H-4lambda6-1,4,3-oxathiazin-2-yl]-[(S)-3-(tert-butyl-dimethyl-silanyloxy)-1-phenyl-propyl]-amin in 4 ml Methanol vorgelegt und unter Zusatz von 18 mg Pd/C 10 % für 1 Stunde ein leichter Wasserstoff-Strom durchgeleitet. Die Reaktionslösung wurde einrotiert und der Rückstand im Reinigungslabor über präparative HPLC gereinigt. Die produkthaltigen Fraktionen wurden vereinigt, das Acetonitril unter vermindertem Druck entfernt und der wässrige Rückstand mit gesättigter wässriger Natriumhydrogencarbonat-Lösung basisch gestellt. Anschließend wurde dieser mit Ethylacetat extrahiert, die vereinten organischen Phasen über MgSO₄ getrocknet und einrotiert. Der Rückstand wurde erneut in einer Mischung aus Acetonitril und Wasser aufgenommen und lyophilisiert. Man erhielt so das Produkt (22.8 mg) mit dem Molekulargewicht 402.5 g / mol (C₂₁H₂₆N₂O₄S); MS (ESI): m / e = 403 (M+H⁺).

Die Verbindungen 43 und 48 wurden ebenfalls nach dieser Herstellungsvorschrift aus den Bromhaltigen Ausgangsverbindungen synthetisiert:
[(S)-1-(2-Fluor-phenyl)-ethyl]-(5,6,6-trimethyl-4,4-dioxo-5-phenyl-5,6-dihydro-4H-4lambda6-[1,4,3]oxathiazin-2-yl)-amin
(S)-3-(2-Fluor-phenyl)-3-(5,6,6-trimethyl-4,4-dioxo-5-phenyl-5,6-dihydro-4H-4lambda6-[1,4,3] oxathiazin-2-ylamino)-propan-1-ol
(S)-3-(2-Fluor-phenyl)-3-[5,6,6-trimethyl-4,4-dioxo-5-(3-pyrimidin-5-yl-phenyl)-5,6-dihydro-4H-4lambda6-[1,4,3]oxathiazin-2-ylamino]-propan-1-ol

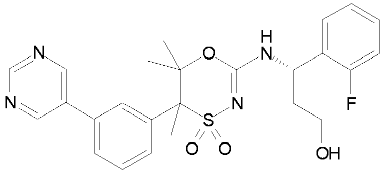

Unter Inertgas wurden 45 mg [5-(3-Brom-phenyl)-5,6,6-trimethyl-4,4-dioxo-5,6-dihydro-4H-4lambda6-1,4,3-oxathiazin-2-yl]-[(S)-3-(tert-butyl-dimethyl-silanyloxy)-1-(2-fluor-phenyl)-propyl]-amin, 11 mg Pyrimidin-5-boronsäure und 96 mg Cäsiumcarbonat in einer Mischung aus 1 ml Dioxan und 0.4 ml Wasser gelöst. Nach 10 Minuten Spülen mit Argon wurden 6 mg Dichlor[1,1'-bis(diphenylphosphino)ferrocen]palladium zugegeben und die Reaktionsmischung für 4 Stunden bei 80 °C gerührt. Die Reaktionsmischung wurde auf Raumtemperatur abkühlen gelassen, mit Ethylacetat verdünnt und mit Wasser gewaschen. Die organische Phase wurde über eine Diatomeenerde-Kartusche (Varian Chem Elut^{®}) getrocknet und einrotiert. Zur Abspaltung der Schutzgruppe wurde der Rückstand in 1.5 ml Methanol gelöst, mit 0.18 ml konzentrierter Salzsäure versetzt und 2.5 Stunden bei Raumtemperatur gerührt. Nach dem Entfernen des Lösungmittels unter vermindertem Druck wurde das Rohprodukt im Reinigungslabor über präparative HPLC gereinigt. Die produkthaltigen Fraktionen wurden vereinigt, das Acetonitril unter vermindertem Druck entfernt, der wässrige Rückstand mit gesättigter wässriger Natriumhydrogencarbonat-Lösung basisch gestellt und mit Ethylacetat extrahiert. Die organische Phase wurde über MgSO₄ getrocknet und einrotiert, der Rückstand erneut in einer Mischung aus Acetonitril und Wasser aufgenommen und lyophilisiert. Man erhielt so das Produkt (12.6 mg) mit dem Molekulargewicht 498.6 g / mol (C₂₅H₂₇N₄O₄S); MS (ESI): m / e = 499 (M+H⁺).

Die Verbindungen 40 und 41 wurden ebenfalls nach dieser Herstellungsvorschrift synthetisiert:
(S)-3-Phenyl-3-[5,6,6-trimethyl-4,4-dioxo-5-(3-pyrimidin-5-yl-phenyl)-5,6-dihydro-4H-4lambda6-[1,4,3]oxathiazin-2-ylamino]-propan-1-ol
[(S)-1-(2-Fluor-phenyl)-ethyl]-[5,6,6-trimethyl-4,4-dioxo-5-(3-pyrimidin-5-yl-phenyl)-5,6-dihydro-4H-4lambda6-[1,4,3]oxathiazin-2-yl]-amin (Abspaltung der Schutzgruppe entfällt)
(S)-3-(2-Fluor-phenyl)-3-{5-[3-(4-methansulfonyl-piperazin-1-yl)-phenyl]-5,6,6-trimethyl-4,4-dioxo-5,6-dihydro-4H-4lambda6-[1,4,3]oxathiazin-2-ylamino}-propan-1-ol

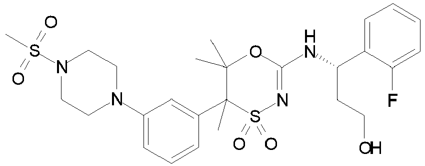

Unter Inertgas wurden 45 mg [5-(3-Brom-phenyl)-5,6,6-trimethyl-4,4-dioxo-5,6-dihydro-4H-4lambda6-1,4,3-oxathiazin-2-yl]-[(S)-3-(tert-butyl-dimethyl-silanyloxy)-1-(2-fluor-phenyl)-propyl]-amin, 4 mg Bis(dibenzylidenaceton)palladium, 10 mg 1,2,3,4,5-Pentaphenyl-1-(di-tert-butylphosphino)ferrocen und 8 mg Kalium-tert-butylat in 1.5 ml Toluol suspendiert. Nach 5 Minuten Spülen mit Argon wurden 15 mg 1-Methansulfonylpiperazin zugegeben und die Reaktionsmischung für 2 Stunden bei 80 °C gerührt. Nach dem Abkühlen auf Raumtemperatur wurde die Mischung mit Ethylacetat verdünnt und mit gesättigter wässriger Natriumchlorid-Lösung gewaschen. Die organische Phase wurde über eine Diatomeenerde-Kartusche (Varian Chem Elut^{®}) getrocknet und einrotiert. Zur Abspaltung der Schutzgruppe wurde der Rückstand in 1.5 ml Methanol gelöst, mit 0.18 ml konzentrierter Salzsäure versetzt und 2.5 Stunden bei Raumtemperatur gerührt. Nach dem Entfernen des Lösungmittels unter vermindertem Druck wurde das Rohprodukt im Reinigungslabor über präparative HPLC gereinigt. Die produkthaltigen Fraktionen wurden vereinigt, das Acetonitril unter vermindertem Druck entfernt, der wässrige Rückstand mit gesättigter wässriger Natriumhydrogencarbonat-Lösung basisch gestellt und mit Ethylacetat extrahiert. Die organische Phase wurde über MgSO₄ getrocknet und einrotiert, der Rückstand erneut in einer Mischung aus Acetonitril und Wasser aufgenommen und lyophilisiert. Man erhielt so das Produkt (21.1 mg) mit dem Molekulargewicht 582.7 g / mol (C₂₆H₃₅FN₄O₆S₂); MS (ESI): m / e = 583 (M+H⁺).

Die Verbindungen 34-36, 38-39, 45 und 49 wurden ebenfalls nach dieser Herstellungsvorschrift synthetisiert:
(S)-3-{5-[3-(4-Methansulfonyl-piperazin-1-yl)-phenyl]-5,6,6-trimethyl-4,4-dioxo-5,6-dihydro-4H-4lambda6-[1,4,3]oxathiazin-2-ylamino}-3-phenyl-propan-1-ol
[(S)-1-(2-Fluor-phenyl)-ethyl]-{5-[3-(4-methansulfonyl-piperazin-1-yl)-phenyl]-5,6,6-trimethyl-4,4-dioxo-5,6-dihydro-4H-4lambda6-[1,4,3]oxathiazin-2-yl}-amin (Abspaltung der Schutzgruppe entfällt)
[(S)-1-(2-Fluor-phenyl)-ethyl]-[5,6,6-trimethyl-4,4-dioxo-5-(3-pyrrolidin-1-yl-phenyl)-5,6-dihydro-4H-4lambda6-[1,4,3]oxathiazin-2-yl]-amin (Abspaltung der Schutzgruppe entfällt)

Bei den nachfolgenden Verbindungen erfolgte die Trennung der Diastereomere mittels RP-Chromatographie im Reinigungslabor.

### (S)-3-Phenyl-3-[5,6,6-trimethyl-4,4-dioxo-5-(3-pyrrolidin-1-yl-phenyl)-5,6-dihydro-4H-4lambda6-[1,4,3]oxathiazin-2-ylamino]-propan-1-ol, Stereomer 1 und 2

HPLC-Anlage: Waters Pump 2525, PAD 996, 2767 Sample Manager

MS-Anlage: Waters Micromass ZQ

Säule: Waters SunFire Prep C18 OBD, 5 µm, 50 x 100 mm

Laufmittel: 0 min 90 % H₂O (0.1 %TFA) - 2.0 min 90 % H₂O - 2.5 min 75 % H₂O - 10.5 min 75 % Acetonitril - 11.5 min 95 % Acetonitril - 13.0 min 95 % Acetonitril (25 °C, Fluss 120 ml / min)

Retentionszeiten:
7.36 Minuten (Stereomer 1, 61.2 mg)
7.66 Minuten (Stereomer 2, 57.7 mg)

### (S)-3-(2-Fluor-phenyl)-3-[5,6,6-trimethyl-4,4-dioxo-5-(3-pyrrolidin-1-yl-phenyl)-5,6-dihydro-4H-4lambda6-[1,4,3]oxathiazin-2-ylamino]-propan-1-ol, Stereomer 1 und 2

HPLC-Anlage: Waters Pump 2525, PAD 996, 2767 Sample Manager

MS-Anlage: Waters Micromass ZQ

Säule: Waters Atlantis dC18 OBD, 5 µm, 50 x 100 mm

Laufmittel: 0 min 90 % H₂O (0.1 %TFA) - 2.0 min 90 % H₂O - 2.5 min 75 % H₂O - 10.5 min 75 % Acetonitril - 11.5 min 95 % Acetonitril - 13.0 min 95 % Acetonitril (25 °C, Fluss 120 ml / min)

Retentionszeiten:
7.31 Minuten (Stereomer 1, 8.7 mg)
7.51 Minuten (Stereomer 2, 9.1 mg)

### (S)-3-[5-(3-Brom-phenyl)-5-fluor-6,6-dimethyl-4,4-dioxo-5,6-dihydro-4H-4lambda6-[1,4,3]oxathiazin-2-ylamino]-3-phenyl-propan-1-ol

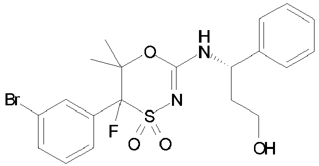

### C-(3-Brom-phenyl)-N,N-bis-(2,4-dimethoxy-benzyl)-methansulfonamid

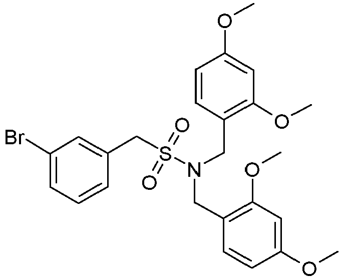

Unter Eiskühlung wurden 4.94 g 3-Brombenzylsulfonylchlorid in 60 ml Dichlormethan vorgelegt und innerhalb von 15 Minuten eine Lösung aus 5.64 g Bis-(2,4-dimethoxybenzyl)-amin in 30 ml Dichlormethan sowie anschließend eine Lösung aus 3.82 ml N,N-Diisopropylethylamin in 5 ml Dichlormethan zugetropft. Die Reaktionsmischung wurde auf Raumtemperatur kommen gelassen und 1 Stunde gerührt. Anschließend wurde der Ansatz mit 50 ml Wasser, mit 50 ml 1 N wässriger Salzsäure, mit 50 ml gesättigter wässriger Natriumhydrogencarbonat-Lösung und abschließend mit 50 ml gesättigter wässriger Natriumchlorid-Lösung gewaschen. Die organische Phase wurde über MgSO₄ getrocknet und einrotiert. Das Rohprodukt wurde mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (9.23 g) mit dem Molekulargewicht 550.5 g / mol (C₂₅H₂₈BrNO₆S),

### C-(3-Brom-phenyl)-N,N-bis-(2,4-dimethoxy-benzyl)-C-fluor-methansulfonamid

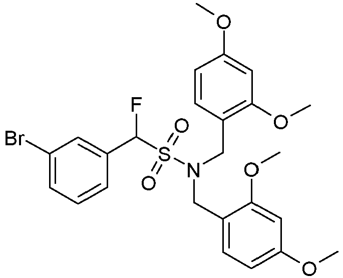

Unter Inertgas wurden 1.97 g C-(3-Brom-phenyl)-N,N-bis-(2,4-dimethoxy-benzyl)-methansulfonamid in 30 ml THF vorgelegt und anschließend bei einer Temperatur von -78 °C 3.56 ml einer 1.6 N Methyllithium-Lösung in Diethylether tropfenweise zugegeben. Die Reaktionsmischung wurde kurz nachgerührt und dann eine Lösung aus 1.49 g N-Fluorphenylsulfonimid in 5 ml THF zugegeben. Nach 10 Minuten Rühren bei gleichbleibender Temperatur wurden 2 ml Essigsäure zugegeben und die Reaktionsmischung auf Raumtemperatur kommen gelassen. Nach dem Einrotieren wurde das Rohprodukt mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (0.64 g) mit dem Molekulargewicht 568.5 g / mol (C₂₅F₂₇BrFNO₆S).

### 1-(3-Brom-phenyl)-1-fluor-2-hydroxy-2-methyl-propan-1-sulfonsäureamid

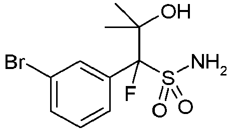

Unter Inertgas wurden 310 mg C-(3-Brom-phenyl)-N,N-bis-(2,4-dimethoxy-benzyl)-C-fluor-methansulfonamid in 10 ml THF vorgelegt und anschließend bei einer Temperatur von -78 °C 0.68 ml einer 1.6 N Methyllithium-Lösung in Diethylether tropfenweise zugegeben und 10 Minuten bei gleichbleibender Temperatur gerührt. Anschließend wurden 0.25 ml Aceton zugegeben. Nach 10 Minuten Rühren wurde die Reaktionslösung mit 0.10 ml Trifluoressigsäure versetzt, die Reaktionsmischung auf Raumtemperatur kommen gelassen und einrotiert. Der Rückstand wurde in 10 ml Dichlormethan gelöst, mit 1.5 ml Trifluoressigsäure versetzt und 20 Minuten bei Raumtemperatur gerührt. Die Reaktionslösung wurde vom Lösungsmittel befreit und das Rohprodukt mittels Normalphasenchromatographie über den Flashmaster mit einem Dichlormethan/Methanol-Gradienten gereinigt. Die produkthaltigen Fraktionen wurden vereinigt und einrotiert. Man erhielt so das Produkt (77.9 mg) mit dem Molekulargewicht 326.2 g / mol (C₁₀H₁₃BrFNO₃S), MS (ESI): m / e = 345 (M+H₂O+H⁺).

### 5-(3-Brom-phenyl)-2-ethoxy-5-fluor-6,6-dimethyl-5,6-dihydro-1,4,3-oxathiazin-4,4-dioxid

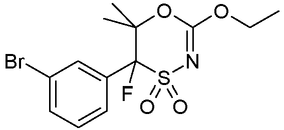

Eine Mischung aus 75 mg 1-(3-Brom-phenyl)-1-fluor-2-hydroxy-2-methyl-propan-1-sulfonsäureamid in 5 ml Tetraethoxymethan und 1 ml Essigsäure wurde für 1.5 Stunden bei 100 °C gerührt. Die Umsetzung wurde mit LCMS geprüft. Da sie unvollständig war, wurde die Reaktionsmischung für weitere 3.5 Stunden bei 100 °C gerührt. Nach dem Entfernen der Lösungsmittel unter vermindertem Druck wurde der Rückstand mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (45.6 mg) mit dem Molekulargewicht 380.2 g / mol (C₁₃H₁₅BrFNO₄S).

### (S)-3-[5-(3-Brom-phenyl)-5-fluor-6,6-dimethyl-4,4-dioxo-5,6-dihydro-4H-4lambda6-[1,4,3]oxathiazin-2-ylamino]-3-phenyl-propan-1-ol

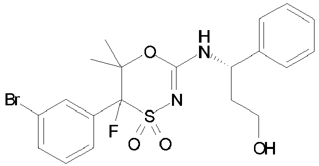

In einem Rundkolben wurden 42 mg 5-(3-Brom-phenyl)-2-ethoxy-5-fluor-6,6-dimethyl-5,6-dihydro-1,4,3-oxathiazin-4,4-dioxid unter Inertgas in 0.2 ml Dichlormethan gelöst und mit 20 mg (S)-3-Amino-3-phenyl-propan-1-ol versetzt. Nach 18 Stunden Rühren bei Raumtemperatur wurde die Umsetzung mit LCMS geprüft. Da nur Spuren des Produktes entstanden waren, wurde die Reaktionsmischung für weitere 4.5 Stunden bei 100 °C gerührt. Nach dem Entfernen des Lösungmittels unter vermindertem Druck wurde das Rohprodukt im Reinigungslabor über präparative HPLC gereinigt. Die produkthaltigen Fraktionen wurden vereinigt, das Acetonitril unter vermindertem Druck entfernt, der wässrige Rückstand mit gesättigter wässriger Natriumhydrogencarbonat-Lösung basisch gestellt und mit Ethylacetat extrahiert. Die organische Phase wurde über MgSO₄ getrocknet und einrotiert, der Rückstand erneut in einer Mischung aus Acetonitril und Wasser aufgenommen und lyophilisiert. Man erhielt so das Produkt (13.1 mg) mit dem Molekulargewicht 485.4 g / mol (C₂₀H₂₂BrFN₂O₄S); MS (ESI): m / e = 485 (M+H⁺).

### (S)-3-(5-Biphenyl-4-yl-6,6-dimethyl-4,4-dioxo-5,6-dihydro-4H-4lambda6-[1,4,3]oxathiazin-2-ylamino)-3-(2-fluor-phenyl)-propan-1-ol

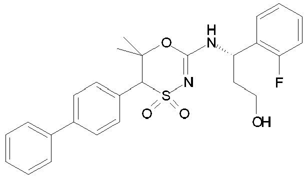

### Biphenyl-4-yl-methansulfonsäure Natriumsalz

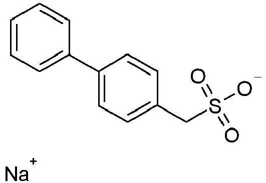

Eine Mischung aus 28.69 g 4-Brommethylbiphenyl und 29.27 g Natriumsulfit in 200 ml einer Mischung aus Wasser und Methanol (1:1) wurde für 12 Stunden bei 60 °C gerührt. Nach dem Abkühlen auf Raumtemperatur wurde das Lösungsmittel unter vermindertem Druck entfernt und der wässrige Rückstand 2 x mit 100 ml Ethylacetat extrahiert. Die wässrige Phase wurde einrotiert und der feste Rückstand mit 200 ml Isopropanol für 1 Stunde gerührt. Nach Filtration von den festen Bestandteilen wurde das Filtrat einrotiert. Man erhielt so das Produkt (15.80 g) mit dem Molekulargewicht 270.3 g / mol (C₁₃H₁₁O₃S.Na).

### C-Biphenyl-4-yl-N-(2,4-dimethoxy-benzyl)-methansulfonamid

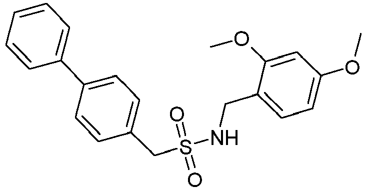

Unter Inertgas wurden 8.00 g Biphenyl-4-yl-methansulfonsäure Natriumsalz und 2.50 ml N,N-Dimethyl-formamid in 165 ml THF vorgelegt, anschließend bei einer Temperatur von -20 °C 6.50 ml Oxalylchlorid zugetropft und die Reaktionslösung innerhalb von 1 Stunde auf 0 °C kommen gelassen. Die Reaktionslösung wurde mit 200 ml Methyl-tert-butylether verdünnt und mit 200 ml Wasser, mit 200 ml verdünnter wässriger Natriumhydrogencarbonat-Lösung und und mit 100 ml gesättigter Natriumchlorid-Lösung gewaschen. Die organische Phase wurde über MgSO₄ getrocknet und einrotiert. Der Rückstand (7.41 g) wurde in 200 ml THF gelöst und anschließend bei einer Temperatur von -20 °C 11.00 ml 2,4-Dimethoxybenzylamin zugetropft und die Reaktionslösung innerhalb von 30 Minuten auf Raumtemperatur kommen gelassen. Nach 48 Stunden Rühren wurde die Reaktionslösung mit 200 ml Ethylacetat verdünnt und mit je 100 ml Wasser, 1 N wässriger Salzsäure, gesättigter wässriger Natriumhydrogencarbonat-Lösung und gesättigter Natriumchlorid-Lösung gewaschen. Die organische Phase wurde über MgSO₄ getrocknet und einrotiert. Das Rohprodukt wurde durch Filtration über eine Kieselgel-Säule gereinigt. Die Säule wurde solange mit Ethylacetat nachgewaschen bis kein Produkt mehr eluierte. Das Eluat wurde einrotiert, der Rückstand in etwas Dichlormethan gelöst, die Lösung erneut einrotiert und der Feststoff anschließend im Hochvakuum getrocknet. Man erhielt so das Produkt (10.89 g) mit dem Molekulargewicht 397.5 g / mol (C₂₂H₂₃NO₄S).

### 1-Biphenyl-4-yl-2-hydroxy-2-methyl-propan-1-sulfonsäureamid

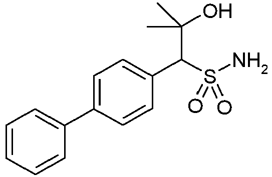

Unter Inertgas wurden 4.01 g C-Biphenyl-4-yl-N-(2,4-dimethoxy-benzyl)-methansulfonamid in 100 ml THF vorgelegt und anschließend bei einer Temperatur von -71 °C 12.60 ml einer 1.6 N Butyllithium-Lösung in Hexan tropfenweise zugegeben. Die Reaktionsmischung wurde für 10 Minuten nachgerührt und dann mit 4.53 ml Aceton tropfenweise versetzt. Die Mischung wurde 10 Minuten bei gleichbleibender Temperatur gerührt, mit 2 ml Trifluoressigsäure versetzt und auf Raumtemperatur kommen gelassen. Der Rückstand wurde in 100 ml Dichlormethan gelöst und mit 20 ml Trifluoressigsäure versetzt. Nach 20 Minuten Rühren bei Raumtemperatur wurde die Reaktionslösung mit gesättigter wässriger Natriumhydrogencarbonat-Lösung gewaschen, die organische Phase gemeinsam mit dem Niederschlag einrotiert und anschließend mit Toluol koevaporiert. Das Rohprodukt wurde mittels Normalphasenchromatographie über den Flashmaster mit einem Dichlormethan/Methanol-Gradienten gereinigt. Die produkthaltigen Fraktionen wurden vereinigt und einrotiert. Man erhielt so das Produkt (3.15 g) mit dem Molekulargewicht 305.4 g / mol (C₁₆H₁₉NO₃S).

### 5-Biphenyl-4-yl-2-ethoxy-6,6-dimethyl-5,6-dihydro-1,4,3-oxathiazin 4,4-dioxid

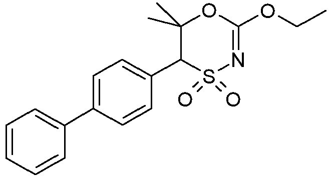

Eine Mischung aus 3.14 g 1-Biphenyl-4-yl-2-hydroxy-2-methyl-propan-1-sulfonsäureamid in 75 ml Tetraethoxymethan und 15 ml Essigsäure wurde für 24 Stunden bei 100 °C gerührt. Die Umsetzung wurde mit LCMS geprüft. Da sie unvollständig war, wurde die Reaktionsmischung mit weiteren 15 ml Essigsäure versetzt und für 4 Stunden bei 120 °C gerührt. Anschließend wurden weitere 20 ml Tetraethoxymethan zugegeben und die Reaktionsmischung für 5 Stunden bei 120 °C gerührt. Da laut Überprüfung mit LCMS keine weitere Umsetzung erreicht werden konnte, wurde das Lösungsmittel unter vermindertem Druck entfernt und der Rückstand mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (1.04 g) mit dem Molekulargewicht 359.5 g / mol (C₁₉H₂₁NO₄S).

### (S)-3-(5-Biphenyl-4-yl-6,6-dimethyl-4,4-dioxo-5,6-dihydro-4H-4lambda6-[1,4,3]oxathiazin-2-ylamino)-3-(2-fluor-phenyl)-propan-1-ol

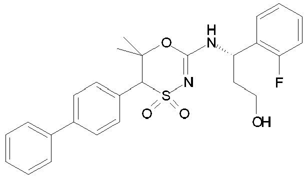

In einem Rundkolben wurden 346 mg 5-Biphenyl-4-yl-2-ethoxy-6,6-dimethyl-5,6-dihydro-1,4,3-oxathiazin 4,4-dioxid unter Inertgas in 2 ml Dichlormethan gelöst und mit 328 mg (S)-3-(tert-Butyl-dimethyl-silanyloxy)-1-(2-fluor-phenyl)-propylamin versetzt. Nach dem Einrotieren wurde der Rückstand in 0.5 ml Dichlormethan suspendiert und für 18 Stunden bei Raumtemperatur gerührt. Die Umsetzung wurde mit LCMS geprüft. Da noch Edukt vorhanden war, wurde die Reaktionsmischung nach der Zugabe von 1 ml Toluol 1 Stunde bei 50 °C gerührt. Die Umsetzung blieb auch nach weiteren 4 Stunden Rühren bei 75 °C unvollständig. Das Lösungsmittel wurde unter vermindertem Druck entfernt. Zur Abspaltung der Schutzgruppe wurde der Rückstand in 1 ml Methanol aufgenommen und nach Zugabe von 0.05 ml konzentrierter Salzsäure 1 Stunde bei Raumtemperatur gerührt. Nach dem Einrotieren wurde der Rückstand im Reinigungslabor über präparative HPLC gereinigt. Die produkthaltigen Fraktionen wurden vereinigt und unter vermindertem Druck vom Lösungsmittel befreit. Der wässrige Rückstand wurde mit Ethylacetat extrahiert, die vereinten organischen Phasen über eine Diatomeenerde-Kartusche (Varian Chem Elut^{®}) getrocknet und einrotiert. Der Rückstand wurde in einer Mischung aus Acetonitril und Wasser gelöst und lyophilisiert. Man erhielt so das Produkt (221 mg) mit dem Molekulargewicht 482.6 g / mol (C₂₆H₂₇FN₂O₄S); MS (ESI): m / e = 483 (M+H⁺).

Es wurde eine chirale Trennung des Diastereomerengemisches durchgeführt.
HPLC-Anlage: Waters 2690UP, PAD 2996
Säule: Chiralpak AD-H/83, 5 µm, 250 x 4.6 mm
Laufmittel: n-Heptan : Ethanol : Methanol 5 : 1 : 1 (30 °C, Fluss 1 ml / min)
Retentionszeiten:
5.722 Minuten (Stereomer 1, 42 mg)
11.110 Minuten (Stereomer 2, 37 mg).

Die Verbindung 52 wurde ebenfalls nach dieser Herstellungsvorschrift synthetisiert:
(5-Biphenyl-4-yl-6,6-dimethyl-4,4-dioxo-5,6-dihydro-4H-4lambda6-[1,4,3]oxathiazin-2-yl)-[(S)-1-(2-fluor-phenyl)-ethyl]-amin (Abspaltung der Schutzgruppe entfällt).

Die Verbindungen 58-60 wurden analog dieser Herstellungsvorschrift synthetisiert. Durch die Umsetzung von 1-(4-tert-Butyl-phenyl)-2-hydroxy-2-methyl-propan-1-sulfonsäureamid mit Tetramethoxymethan wurde 5-(4-tert-Butyl-phenyl)-2-methoxy-6,6-dimethyl-5,6-dihydro-1,4,3-oxathiazin-4,4-dioxid hergestellt, das dann in weiterer Umsetzung mit den jeweiligen Aminen bereits bei Raumtemperatur reagierte.

### (S)-3-[5-(4-tert-Butyl-phenyl)-6,6-dimethyl-4,4-dioxo-5,6-dihydro-4H-4lambda6-[1,4,3]oxathiazin-2-ylamino]-3-(2-fluor-phenyl)-propan-1-ol

### [5-(4-tert-Butyl-phenyl)-6,6-dimethyl-4,4-dioxo-5,6-dihydro-4H-4lambda6-[1,4,3]oxathiazin-2-yl]-[(S)-1-(2-fluor-phenyl)-ethyl]-amin

### (S)-3-[5-(4-tert-Butyl-phenyl)-6,6-dimethyl-4,4-dioxo-5,6-dihydro-4H-4lambda6-[1,4,3]oxathiazin-2-ylamino]-3-phenyl-propan-1-ol

Es wurde eine chirale Trennung des Diastereomerengemisches von Verbindung 58 durchgeführt.
HPLC-Anlage: Waters 2690UP, PAD 2996
Säule: Chiralpak AD-H/44 250x4,6mm
Laufmittel: n-Heptan : Ethanol : Methanol 5: 1:1 (1 mL / min)
Retentionszeiten:
3.836 Minuten (Stereomer 1, 38 mg)
4.969 Minuten (Stereomer 2, 34 mg).

### (S)-3-(5-Biphenyl-4-yl-5,6,6-trimethyl-4,4-dioxo-5,6-dihydro-4H-4lambda6-[1,4,3]oxathiazin-2-ylamino)-3-(2-fluor-phenyl)-propan-1-ol

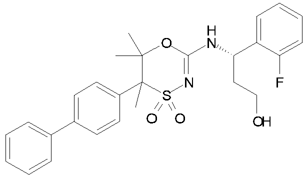

Die Synthese von 1-(4-Brom-phenyl)-ethansulfonsäure-2,4-dimethoxy-benzylamid erfolgte analog zu 1-(3-Brom-phenyl)-ethansulfonsäure-2,4-dimethoxy-benzylamid.

### 2-(4-Brom-phenyl)-3-hydroxy-3-methyl-butan-2-sulfonsäureamid

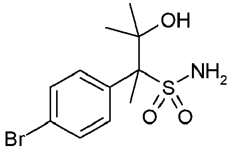

Unter Inertgas wurden 2.58 g 1-(4-Brom-phenyl)-ethansulfonsäure-2,4-dimethoxy-benzylamid in 40 ml THF vorgelegt und anschließend bei einer Temperatur von -78 °C 7.90 ml einer 1.6 N Methyllithium-Lösung in Diethylether tropfenweise zugegeben und 10 Minuten bei gleichbleibender Temperatur gerührt. Anschließend wurden 2.47 ml Aceton zugegeben. Nach 15 Minuten Rühren wurde die Reaktionslösung mit 0.96 ml Trifluoressigsäure versetzt und auf Raumtemperatur erwärmen gelassen. Nach dem Entfernen des Lösungsmittels unter vermindertem Druck wurde der Rückstand in 50 ml Dichlormethan gelöst, mit 10 ml Trifluoressigsäure versetzt und 1 Stunde bei Raumtemperatur gerührt. Es wurden 100 ml Methanol zur Reaktionslösung gegeben und das Lösungsmittel einrotiert. Das Rohprodukt wurde durch Filtration über eine Kieselgel-Säule gereinigt. Die Säule wurde solange mit Ethylacetat nachgewaschen bis kein Produkt mehr eluierte. Das Eluat wurde einrotiert und der Rückstand (2.01 g) ohne weitere Reinigung in die nächste Reaktion eingesetzt.

### 5-(4-Brom-phenyl)-2-methoxy-5,6,6-trimethyl-5,6-dihydro-1,4,3-oxathiazin-4,4-dioxid

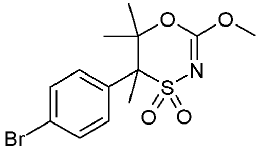

Eine Suspension aus 2.01 g 2-(4-Brom-phenyl)-3-hydroxy-3-methyl-butan-2-sulfonsäureamid in 15 ml Tetramethoxymethan wurde mit 3 ml Essigsäure versetzt und 1.5 Stunden bei 100°C gerührt. Nach dem Entfernen des Lösungsmittels unter vermindertem Druck wurde das Rohprodukt mittels Normalphasenchromatographie über den Flashmaster mit einem n-Heptan/Ethylacetat-Gradienten gereinigt. Die produkthaltigen Fraktionen wurden vereinigt, einrotiert und im Hochvakuum getrocknet. Man erhielt so das Produkt (0.21g) mit dem Molekulargewicht 362.2 g / mol (C₁₃H₁₆BrNO₄S).

### [5-(4-Brom-phenyl)-5,6,6-trimethyl-4,4-dioxo-5,6-dihydro-4H-4lambda6-1,4,3-oxathiazin-2-yl]-[(S)-3-(tert-butyl-dimethyl-silanyloxy)-1-(2-fluor-phenyl)-propyl]-amin

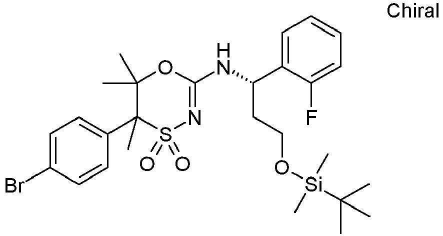

Eine Lösung aus 70 mg 5-(4-Brom-phenyl)-2-methoxy-5,6,6-trimethyl-5,6-dihydro-1,4,3-oxathiazin-4,4-dioxid und 66 mg (S)-3-(tert-Butyl-dimethyl-silanyloxy)-1-(2-fluor-phenyl)-propylamin in 1 ml Methylenchlorid wurde unter leichtem Argonstrom gerührt, sodass das Lösungsmittel langsam verdampfte. Nach Rühren über Nacht bei Raumtemperatur wurde der Rückstand in 20 ml Dichlormethan gelöst, mit 10 ml 0.5 N wässriger Salzsäure und 10 ml Wasser extrahiert. Die organische Phase wurde über eine Phasenseparator-Kartusche (Chromabond^{®} PTS) getrocknet, einrotiert und im Hochvakuum getrocknet. Man erhielt so das Produkt (113 mg) mit dem Molekulargewicht 613.7 g / mol (C₂₇H₃₈BrFN₂O₄SSi); MS (ESI): m / e = 614 (M+H⁺).

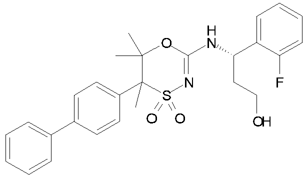

Unter Inertgas wurden 5.50 ml einer 0.5 N Zinkchlorid-Lösung in THF auf -75 °C gekühlt und tropfenweise mit 1.08 ml einer 1.8 N Phenyllithium-Lösung in Dibutylether versetzt. Die Reaktionslösung wurde auf Raumtemperatur kommen gelassen und 30 Minuten gerührt. Dann wurde diese Lösung zu einer Lösung aus 113 mg [5-(4-Brom-phenyl)-5,6,6-trimethyl-4,4-dioxo-5,6-dihydro-4H-4lambda6-1,4,3-oxathiazin-2-yl]-[(S)-3-(tert-butyl-dimethyl-silanyloxy)-1-(2-fluor-phenyl)-propyl]-amin und 8 mg Dichlor[1,1'-bis(diphenylphosphino)ferrocen]palladium in 3 ml THF gegeben. Nach 75 Minuten Rühren bei 60 °C wurde die Umsetzung mit LCMS überprüft und weitere 5 mg des Katalysators zugegeben. Nach 1 Stunde bei gleichbleibender Temperatur wurde noch einmal die Hälfte der oben beschriebenen Menge der selbst hergestellten Lösung aus Phenyllithium und Zinkchlorid zugegeben und weitere 60 Minuten bei 60 °C gerührt. Da laut LCMS keine weitere Umsetzung erfolgte wurde die Reaktionslösung über Kieselgel filtriert und einrotiert. Der Rückstand wurde in 3 ml THF gelöst, mit 8 mg Dichlor[1,1'-bis(diphenylphosphino)ferrocen]palladium und der gleichen Menge der oben beschriebenen selbst hergestellten Lösung aus Phenyllithium und Zinkchlorid versetzt und erneut 60 Minuten bei 60°C gerührt. Da weiterhin keine Umsatzsteigerung zu beobachten war, wurde die Ansatzlösung mit einer Mischung aus 1.5 ml Dioxan und 0.5 ml Wasser, 179 mg Cäsiumcarbonat, 27 mg Phenylboronsäure und 8 mg Dichlor[1,1'-bis(diphenylphosphino)ferrocen]palladium versetzt und weiter bei 60°C gerührt. Nach 2 Stunden wurde die Umsetzung mit LCMS geprüft und für vollständig befunden. Die Reaktionslösung wurde mit Ethylacetat und Wasser versetzt und die organische Phase über MgSO₄ getrocknet. Nach Entfernen des Lösungsmittels unter vermindertem Druck wurde der Rückstand in 1.5 ml Methanol gelöst, mit 0.15 ml konzentrierter Salzsäure versetzt und 16 Stunden bei Raumtemperatur gerührt. Die Reaktionslösung wurde einrotiert, der Rückstand mit 20 ml Ethylacetat und 10 ml Wasser extrahiert und die wässrige Phase nochmals mit Ethylacetat gewaschen. Die vereinten organischen Phasen wurden über eine Phasenseparator-Kartusche (Chromabond^{®} PTS) getrocknet und einrotiert. Der Rückstand wurde mittels Normalphasenchromatographie über den Flashmaster mit einem n-Heptan/Ethylacetat-Gradienten gereinigt. Die produkthaltigen Fraktionen wurden vereinigt, einrotiert und das Rohprodukt im Reinigungslabor über präparative HPLC weiter gereinigt. Die produkthaltigen Fraktionen wurden vereinigt, das Acetonitril unter vermindertem Druck entfernt, der wässrige Rückstand mit gesättigter wässriger Natriumhydrogencarbonat-Lösung basisch gestellt und mit Dichlormethan extrahiert. Die organische Phase wurde über MgSO₄ getrocknet und einrotiert, der Rückstand erneut in einer Mischung aus Acetonitril und Wasser aufgenommen und lyophilisiert. Man erhielt so das Produkt (34.2 mg) mit dem Molekulargewicht 496.6 g / mol (C₂₇H₂₉FN₂O₄S); MS (ESI): m / e = 497 (M+H⁺).

### (S)-3-(5-Biphenyl-4-yl-5,6,6-trimethyl-4,4-dioxo-5,6-dihydro-4H-4lambda6-[1,4,3]oxathiazin-2-ylamino)-3-phenyl-propan-1-ol

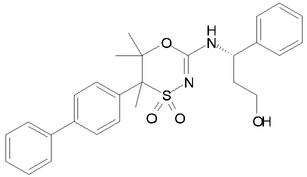

Die Synthese von [5-(4-Brom-phenyl)-5,6,6-trimethyl-4,4-dioxo-5,6-dihydro-4H-4lambda6-1,4,3-oxathiazin-2-yl]-[(S)-3-(tert-butyl-dimethyl-silanyloxy)-1-phenyl-propyl]-amin erfolgte analog zu [5-(4-Brom-phenyl)-5,6,6-trimethyl-4,4-dioxo-5,6-dihydro-4H-4lambda6-1,4,3-oxathiazin-2-yl]-[(S)-3-(tert-butyl-dimethyl-silanyloxy)-1-(2-fluor-phenyl)-propyl]-amin.

Unter Inertgas wurden 5.52 ml einer 0.5 N Zinkchlorid-Lösung in THF auf -75 °C gekühlt und tropfenweise mit 1.02 ml einer 1.8 N Phenyllithium-Lösung in Dibutylether versetzt. Die Reaktionslösung wurde auf Raumtemperatur kommen gelassen und 30 Minuten gerührt. Dann wurde diese Lösung zu einer Lösung aus 110 mg [5-(4-Brom-phenyl)-5,6,6-trimethyl-4,4-dioxo-5,6-dihydro-4H-4lambda6-1,4,3-oxathiazin-2-yl]-[(S)-3-(tert-butyl-dimethyl-silanyloxy)-1-phenyl-propyl]-amin und 8 mg Dichlor[1,1'-bis(diphenylphosphino)ferrocen]palladium in 3 ml THF gegeben. Nach 75 Minuten Rühren bei 60 °C wurde die Reaktionslösung einrotiert. Zur Abspaltung der Schutzgruppe wurde der Rückstand in 2 ml Methanol gelöst, mit 0.2 ml konzentrierter Salzsäure versetzt und 1.5 Stunden bei Raumtemperatur gerührt. Die Reaktionslösung wurde einrotiert und der Rückstand mit 20 ml Dichlormethan und 10 ml Wasser extrahiert und die wässrige Phase nochmals mit 10 ml Dichlormethan gewaschen. Die vereinten organischen Phasen wurden über eine Phasenseparator-Kartusche (Chromabond^{®}) PTS) getrocknet und einrotiert. Der Rückstand wurde mittels Normalphasenchromatographie über den Flashmaster mit einem n-Heptan/Ethylacetat-Gradienten gereinigt. Die produkthaltigen Fraktionen wurden vereinigt, einrotiert und das Rohprodukt im Reinigungslabor über präparative HPLC weiter gereinigt. Die produkthaltigen Fraktionen wurden vereinigt, das Acetonitril unter vermindertem Druck entfernt, der wässrige Rückstand mit gesättigter wässriger Natriumhydrogencarbonat-Lösung basisch gestellt und mit Dichlormethan extrahiert. Die organische Phase wurde über MgSO₄ getrocknet und einrotiert, der Rückstand erneut in einer Mischung aus Acetonitril und Wasser aufgenommen und lyophilisiert. Man erhielt so das Produkt (39.7 mg) mit dem Molekulargewicht 478.6 g / mol (C₂₇H₃₀N₂O₄S); MS (ESI): m / e = 479 (M+H⁺).

### (5-Biphenyl-4-yl-5,6,6-trimethyl-4,4-dioxo-5,6-dihydro-4H-4lambda6-[1,4,3]oxathiazin-2-yl)-[(S)-1-(2-fluor-phenyl)-ethyl]-amin

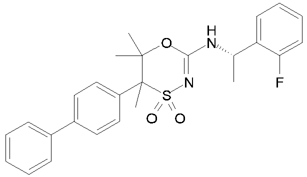

Die Synthese von [5-(4-Brom-phenyl)-5,6,6-trimethyl-4,4-dioxo-5,6-dihydro-4H-4lambda6-1,4,3-oxathiazin-2-yl]-[(S)-1-(2-fluor-phenyl)-ethyl]-amin erfolgte analog zu [5-(4-Brom-phenyl)-5,6,6-trimethyl-4,4-dioxo-5,6-dihydro-4H-4lambda6-1,4,3-oxathiazin-2-yl]-[(S)-3-(tert-butyl-dimethyl-silanyloxy)-1-(2-fluor-phenyl)-propyl]-amin.

Unter Inertgas wurden 5.56 ml einer 0.5 N Zinkchlorid-Lösung in THF auf -75 °C gekühlt und tropfenweise mit 1.03 ml einer 1.8 N Phenyllithium-Lösung in Dibutylether versetzt. Die Reaktionslösung wurde auf Raumtemperatur kommen gelassen und 30 Minuten gerührt. Dann wurde diese Lösung zu einer Lösung aus 87 mg [5-(4-Brom-phenyl)-5,6,6-trimethyl-4,4-dioxo-5,6-dihydro-4H-41ambda6-1,4,3-oxathiazin-2-yl]-[(S)-1-(2-fluor-phenyl)-ethyl]-amin und 8 mg Dichlor[1,1'-bis(diphenylphosphino)ferrocen]palladium in 3 ml THF gegeben. Nach 75 Minuten Rühren bei 60 °C wurde die Umsetzung mit LCMS überprüft. Da sie unvollständig war, wurde die Reaktionsmischung für weitere 2.5 Stunden bei 60 °C gerührt. Da laut LCMS keine weitere Umsetzung stattgefunden hatte, wurde noch einmal 30 % der oben beschriebenen Menge der selbst hergestellten Lösung aus Phenyllithium und Zinkchlorid zugegeben und weitere 60 Minuten bei 60 °C gerührt. Da laut LCMS keine weitere Umsetzung erfolgte wurde die Reaktionslösung einrotiert. Der Rückstand wurde mit Dichlormethan und Wasser versetzt, die organische Phase über MgSO₄ getrocknet und einrotiert. Der Rückstand wurde mittels Normalphasenchromatographie über den Flashmaster mit einem n-Heptan/Ethylacetat-Gradienten gereinigt. Die produkthaltigen Fraktionen wurden vereinigt, einrotiert und das Rohprodukt im Reinigungslabor über präparative HPLC weiter gereinigt. Die produkthaltigen Fraktionen wurden vereinigt und lyophilisiert. Man erhielt so das Produkt (29.1 mg) mit dem Molekulargewicht 466.6 g / mol (C₂₆H₂₇FN₂O₃S); MS (ESI): m / e = 467 (M+H⁺).

### [(S)-1-(2-Fluor-phenyl)-ethyl]-(5,6,6-trimethyl-4,4-dioxo-5-phenyl-5,6-dihydro-4H-4lambda6-[1,4,3]oxathiazin-2-yl)-amin, Stereomer 1

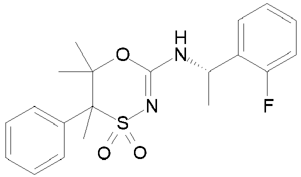

### 5-(3-Brom-phenyl)-2-ethoxy-5,6,6-trimethyl-5,6-dihydro-1,4,3-oxathiazin-4,4-dioxid

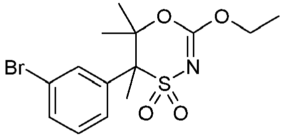

Eine Suspension aus 14.40 g 2-(3-Brom-phenyl)-3-hydroxy-3-methyl-butan-2-sulfonsäureamid (50 %, verunreinigt mit 1-(3-Brom-phenyl)-ethansulfonsäureamid)) in 100 ml Tetraethoxymethan wurde mit 12 ml Essigsäure versetzt und unter starkem Argon-Strom 6 Stunden bei 100°C gerührt. Nach dem Entfernen des Lösungsmittels unter vermindertem Druck (Badtemperatur 70 °C) wurde der zähflüssige Rückstand für 16 Stunden bei 50 °C im Vakuumtrockenschrank getrocknet. Die Umsetzung wurde mit LCMS geprüft und für unvollständig befunden. Daher wurde der ölige Rückstand für 4 Stunden unter vermindertem Druck (Badtemperatur 80 °C) am Rotationsverdampfer reagieren gelassen. Nach der Zugabe von 10 ml Essigsäure wurde die Badtemperatur auf 85 °C erhöht und der Druck auf 150 mbar eingestellt. Anschließend wurden die flüchtigen Bestandteile unter erneut vermindertem Druck entfernt und das Rohprodukt mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (5.97 g) mit dem Molekulargewicht 376.3 g / mol (C₁₄H₁₈BrNO₄S).

Es wurde eine chirale Trennung des Racemats durchgeführt. Anschließend wurden beide Stereomere einzeln weiter umgesetzt.

HPLC-Anlage: Waters 2690UP, PAD 2996
Säule: Chiralpak AS-H/52, 5 µm, 250 x 4.6 mm
Laufmittel: n-Heptan : Ethanol 15 : 1 + 0.1 % Trifluoressigsäure (30 °C, Fluss 1 ml / min)
Retentionszeiten:
8.348 Minuten (Stereomer 1, 1.22 g)
9.862 Minuten (Stereomer 2, 1.54 g)

### [(S)-1-(2-Fluor-phenyl)-ethyl]-(5,6,6-trimethyl-4,4-dioxo-5-phenyl-5,6-dihydro-4H-4lambda6-[1,4,3]oxathiazin-2-yl)-amin, Stereomer 1

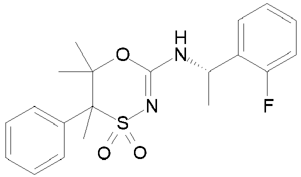

In einem Rundkolben wurden 92 mg 5-(3-Brom-phenyl)-2-ethoxy-5,6,6-trimethyl-5,6-dihydro-1,4,3-oxathiazin-4,4-dioxid Stereomer 1 in 1.3 ml Dichlormethan gelöst und mit 45 mg (S)-1-(2-Fluor-phenyl)-ethylamin versetzt. Nach 18 Stunden Rühren bei Raumtemperatur und unter einem leichten Argon-Strom wurde die Umsetzung mit LCMS geprüft. Da sie unvollständig war, wurde die Reaktionsmischung in 1.5 ml THF aufgenommen und für 5 Stunden bei 50 °C gerührt. Nach der Entfernung des Lösungsmittels unter vermindertem Druck wurde der ölige Rückstand für 2 Stunden bei 95 °C und anschließend für 48 Stunden bei Raumtemperatur gerührt. Laut LCMS blieb die Reaktion unvollständig. Der Rückstand wurde unter Inertgas in 2 ml Ethanol gelöst und unter Zusatz von 50 mg Pd/C 10 % für 16 Stunden unter Wasserstoff-Atmosphäre bei Raumtemperatur gerührt. Nach der Zugabe von weiteren 50 mg Pd/C 10 % wurde die Reaktionsmischung unter gleichbleibenden Bedingungen für weitere 24 Stunden gerührt. Nach Filtration von den festen Bestandteilen wurde das Filtrat einrotiert und der Rückstand im Reinigungslabor über präparative HPLC gereinigt. Die produkthaltigen Fraktionen wurden vereinigt, das Acetonitril unter vermindertem Druck entfernt und der wässrige Rückstand lyophilisiert. Man erhielt so das Produkt (45.7 mg) mit dem Molekulargewicht 390.5 g / mol (C₂₀H₂₃FN₂O₃S); MS (ESI): m / e = 391 (M+H⁺).

Die Verbindung 57 wurde ebenfalls nach dieser Herstellungsvorschrift synthetisiert:
[(S)-1-(2-Fluor-phenyl)-ethyl]-(5,6,6-trimethyl-4,4-dioxo-5-phenyl-5,6-dihydro-4H-4lambda6-[1,4,3]oxathiazin-2-yl)-amin, Stereomer 2
(S)-3-[5-(4-tert-Butyl-phenyl)-5,6,6-trimethyl-4,4-dioxo-5,6-dihydro-4H-4lambda6-[1,4,3]oxathiazin-2-ylamino]-3-(2-fluor-phenyl)-propan-1-ol 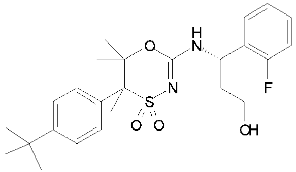
2-(4-tert-Butyl-phenyl)-3-hydroxy-3-methyl-butan-2-sulfonsäure-2,4-dimethoxy-benzylamid 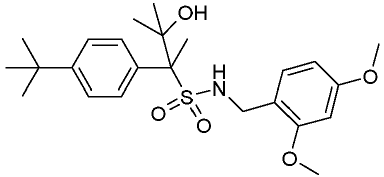
1-(4-tert-Butyl-phenyl)-ethansulfonsäure-2,4-dimethoxy-benzylamid wurde analog zu 1-Phenyl-ethansulfonsäure-2,4-dimethoxy-benzylamid hergestellt.

Unter Inertgas wurden 1.01 g 1-(4-tert-Butyl-phenyl)-ethansulfonsäure-2,4-dimethoxy-benzylamid in 20 ml THF vorgelegt und anschließend bei einer Temperatur von -75 °C 3.23 ml einer 1.6 N Butyllithium-Lösung in Hexan tropfenweise zugegeben und 10 Minuten gerührt. Nach der Zugabe von 1.16 ml Aceton wurde die Reaktionsmischung für 10 Minuten bei gleichbleibender Temperatur nachgerührt. Anschließend wurden 0.45 ml Trifluoressigsäure zugegeben und die Ansatzlösung auf Raumtemperatur kommen gelassen. Nach dem Einrotieren wurde das Rohprodukt über den Flashmaster mit einem n-Heptan/Ethylacetat-Gradienten gereinigt. Die produkthaltigen Fraktionen wurden vereinigt und einrotiert. Man erhielt so das Produkt (484 mg) mit dem Molekulargewicht 449.6 g / mol (C₂₄H₃₅NO₅S).

### 2-(4-tert-Butyl-phenyl)-3-hydroxy-3-methyl-butan-2-sulfonsäureamid

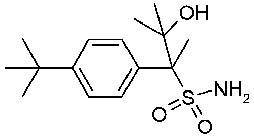

Eine Lösung von 480 mg 2-(4-tert-Butyl-phenyl)-3-hydroxy-3-methyl-butan-2-sulfonsäure-2,4-dimethoxy-benzylamid in einer Mischung aus 21 ml Acetonitril und 7 ml Wasser wurde bei 0°C portionsweise mit 2342 mg Cer(IV)-ammoniumnitrat versetzt. Nach 1 Stunde Rühren bei Raumtemperatur wurde die Reaktionslösung mit 100 ml Dichlormethan und 15 ml Wasser verdünnt und nach der Phasentrennung die organische Phase über eine Phasenseparator-Kartusche (Chromabond^{®} PTS) getrocknet und einrotiert. Das Rohprodukt wurde mittels Normalphasenchromatographie über den Flashmaster mit einem Dichlormethan/Methanol-Gradienten gereinigt. Die produkthaltigen Fraktionen wurden vereinigt und einrotiert. Man erhielt so das Produkt (202 mg) mit dem Molekulargewicht 299.4 g / mol (C₁₅H₂₅NO₃S).

Die weitere Umsetzung zu (S)-3-[5-(4-tert-Butyl-phenyl)-5,6,6-trimethyl-4,4-dioxo-5,6-dihydro-4H-4lambda6-[1,4,3]oxathiazin-2-ylamino]-3-(2-fluor-phenyl)-propan-1-ol erfolgte über die Zwischenstufe des 5-(4-tert-Butyl-phenyl)-2-methoxy-5,6,6-trimethyl-5,6-dihydro-1,4,3-oxathiazin-4,4-dioxids in Analogie zur Herstellung von (S)-3-[5-(3-Brom-phenyl)-5,6,6-trimethyl-4,4-dioxo-5,6-dihydro-4H-4lambda6-[1,4,3]oxathiazin-2-ylamino]-3-phenyl-propan-1-ol.

Die Verbindungen 62 und 63 wurden ebenfalls nach dieser Herstellungsvorschrift synthetisiert:
(S)-3-[5-(4-tert-Butyl-phenyl)-5,6,6-trimethyl-4,4-dioxo-5,6-dihydro-4H-4lambda6-[1,4,3]oxathiazin-2-ylamino]-3-phenyl-propan-1-ol
[5-(4-tert-Butyl-phenyl)-5,6,6-trimethyl-4,4-dioxo-5,6-dihydro-4H-4lambda6-[1,4,3]oxathiazin-2-yl]-[(S)-1-(2-fluor-phenyl)-ethyl]-amin (Schutzgruppenabspaltung entfällt)
(S)-3-[5,6,6-Triimethyl-5-(2'-methyl-biphenyl-4-yl)-4,4-dioxo-5,6-dihydro-4H-4lambda6-[1,4,3]oxathiazin-2-ylamino]-3-(2-fluor-phenyl)-propan-1-ol

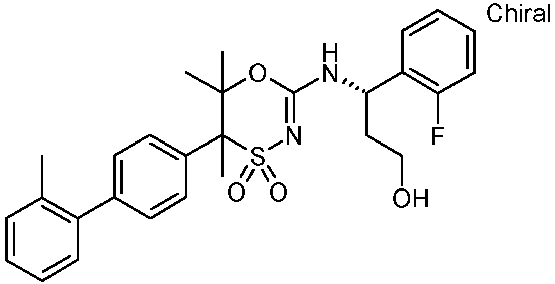

Eine Lösung aus 2-Methylphenyl-boronsäure (57 mg), Cäsiumcarbonat (335 mg) und [5-(4-Brom-phenyl)-5,6,6-trimethyl-4,4-dioxo-5,6-dihydro-4H-4lambda6-[1,4,3]oxathiazin-2-yl]-[(S)-3-(tert-butyl-dimethyl-silanyloxy)-1-(2-fluor-phenyl)-propyl]-amin (210 mg) in Dioxan (3 ml) / Wasser (1 ml) wurde mit Argon zwanzig Minuten gespült. Dann wurde 1,1'-Bis(diphenylphosphino)ferrocen-palladium(II)chlorid (24 mg) zugegeben und fünfundvierzig Minuten bei 80 °C gerührt. Anschließend wurde mit Ethylacetat (20 ml) versetzt und mit Wasser (10 ml) gewaschen. Die organische Phase wurde über eine Kieselgurkartusche getrocknet, filtriert und im Vakuum konzentriert. Anschließend wurde säulenchromatographisch (Ethylacetat / Heptan) gereinigt. Der Rückstand der Wertfraktionen (197 mg) wurde in Methanol (4 ml) aufgenommen und mit konzentrierter Salzsäure versetzt und über Nacht gerührt. Das Lösungsmittel wurde entfernt, der Rückstand in Dichlormethan (20 ml) aufgenommen und mit Wasser (20 ml) gewaschen. Die organische Phase wurde mit eine Phasenseparatorkartusche getrocknet und konzentriert. Der Rückstand wurde säulenchromatographisch (Ethylacetat / Heptan) gereinigt. Man erhielt so das Produkt (124 mg) mit dem Molekulargewicht 496.6 g / mol (C₂₇H₂₉FN₂O₄S), MS (ESI): (M+H+) 421 g / mol.

Auf gleiche Weise wurden folgende Verbindungen erhalten:
(S)-3-[5-(4'-Fluor-biphenyl-4-yl)-5,6,6-trimethyl-4,4-dioxo-5,6-dihydro-4H-4lambda6-[1,4,3]oxathiazin-2-ylamino]-3-(2-fluor-phenyl)-propan-1-ol 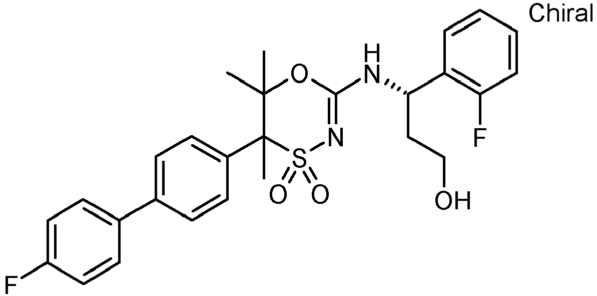
(S)-3-(2-Fluor-phenyl)-3-[5-(4'-methoxy-biphenyl-4-yl)-5,6,6-trimethyl-4,4-dioxo-5,6-dihydro-4H-4lambda6-[1,4,3]oxathiazin-2-ylamino]-propan-1-ol 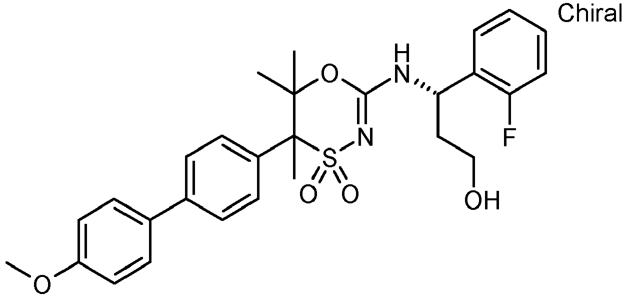
(S)-3-(5-Biphenyl-4-yl-6,6-dimethyl-4,4-dioxo-5,6-dihydro-4H-4lambda6-[1,4,3]oxathiazin-2-ylamino)-3-(2,4-difluor-phenyl)-propan-1-ol

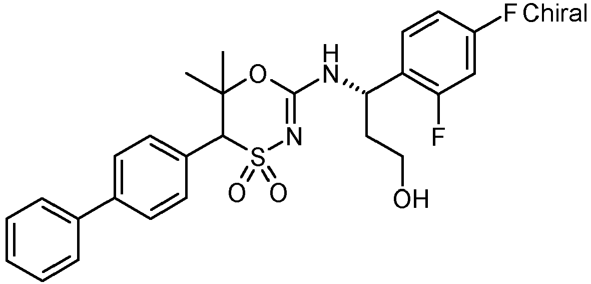

Eine Lösung aus Phenylboronsäure (88 mg), Cäsiumcarbonat (423 mg) und [5-(4-Brom-phenyl)-6,6-dimethyl-4,4-dioxo-5,6-dihydro-4H-4lambda6-[1,4,3]oxathiazin-2-yl]-[(S)-3-(tert-butyl-dimethyl-silanyloxy)-1-(2,4-difluor-phenyl)-propyl]-amin (360 mg) in Dioxan (3.8 ml) / Wasser (1.2 ml) wurde mit Argon zwanzig Minuten gespült. Dann wurde 1,1'-Bis(diphenylphosphino)ferrocen-palladium(II)chlorid (50 mg) zugegeben und dreißig Minuten bei 65 °C gerührt. Anschließend wurde mit Ethylacetat (20 ml) versetzt und mit Wasser (10 ml) gewaschen. Die organische Phase wurde über eine Kieselgurkartusche getrocknet, filtriert und im Vakuum konzentriert. Anschließend wurde säulenchromatographisch (Ethylacetat / Heptan) gereinigt. Der Rückstand der Wertfraktionen (197 mg) wurde in Methanol (4 ml) aufgenommen und mit konzentrierter Salzsäure versetzt und über Nacht gerührt. Das Lösungsmittel wurde entfernt, der Rückstand in Dichlormethan (20 ml) aufgenommen und mit Wasser (20 ml) gewaschen. Die organische Phase wurde mit eine Phasenseparatorkartusche getrocknet und konzentriert. Der Rückstand wurde säulenchromatographisch (Ethylacetat / Heptan) gereinigt. Man erhielt so das Produkt (150 mg) mit dem Molekulargewicht 500.5 g / mol (C₂₆H₂₆F₂N₂O₄S), MS (ESI): (M+H+) 501 g / mol.

Auf gleiche Weise wurden folgende Verbindungen erhalten:
3-(5-Biphenyl-4-yl-6,6-dimethyl-4,4-dioxo-5,6-dihydro-4H-4lambda6-[1,4,3]oxathiazin-2-ylamino)-3-(2,3-difluor-phenyl)-propan-1-ol 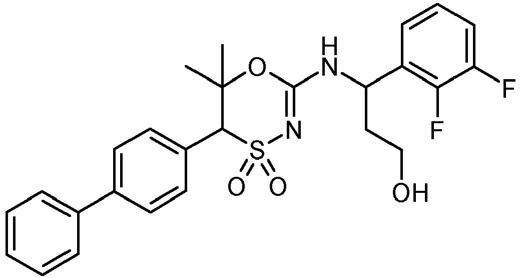
(S)-3-(5-Biphenyl-4-yl-6,6-dimethyl-4,4-dioxo-5,6-dihydro-4H-4lambda6-[1,4,3]oxathiazin-2-ylamino)-3-(2-chlor-phenyl)-propan-1-ol 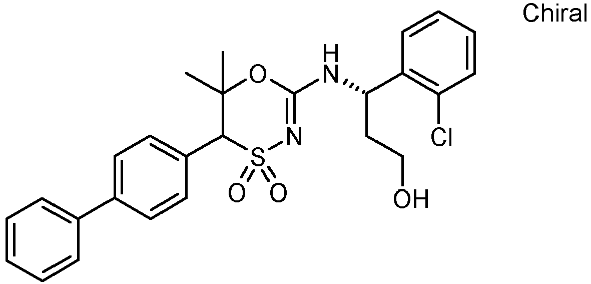
(S)-3-(2-Fluor-phenyl)-3-[5-(3'-methoxy-biphenyl-4-yl)-6,6-dimethyl-4,4-dioxo-5,6-dihydro-4H-4lambda6-[1,4,3]oxathiazin-2-ylamino]-propan-1-ol 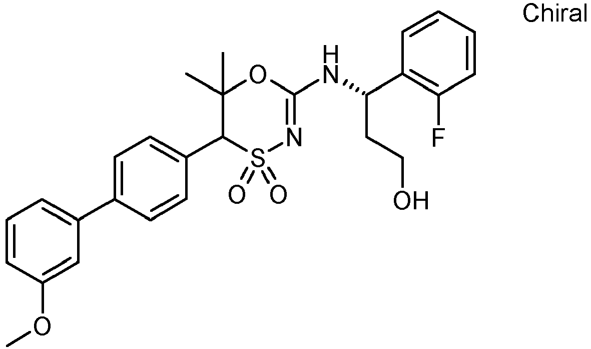
4'-{2-[(S)-1-(2-Fluor-phenyl)-3-hydroxy-propylamino]-6,6-dimethyl-4,4-dioxo-5,6-dihydro-4H-4lambda6-[1,4,3]oxathiazin-5-yl}-biphenyl-2-carbonitril 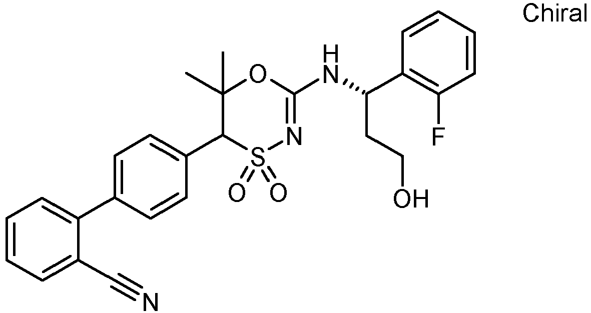
(S)-3-(5-Biphenyl-4-yl-6,6-dimethyl-4,4-dioxo-5,6-dihydro-4H-4lambda6-[1,4,3]oxathiazin-2-ylamino)-3-(4-chlor-phenyl)-propan-1-ol 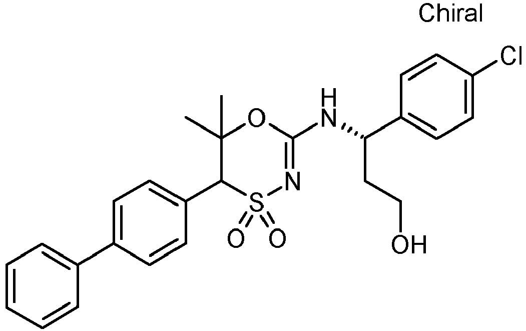
(S)-3-(5-Biphenyl-4-yl-6,6-dimethyl-4,4-dioxo-5,6-dihydro-4H-4lambda6-[1,4,3]oxathiazin-2-ylamino)-3-(3-chlor-phenyl)-propan-1-ol 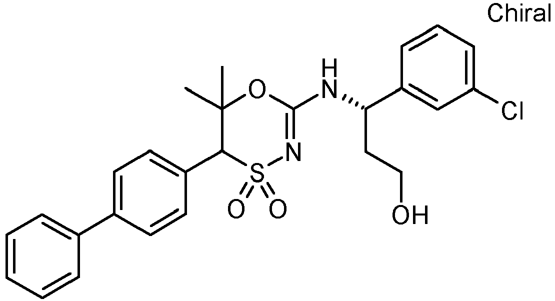
(S)-3-[6,6-Dimethyl-4,4-dioxo-5-(4-pyrimidin-5-yl-phenyl)-5,6-dihydro-4H-4lambda6-[1,4,3]oxathiazin-2-ylamino]-3-(2-fluor-phenyl)-propan-1-ol 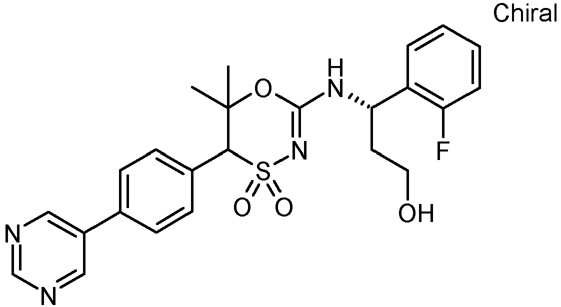
(S)-3-{5-[4-(5-Chlor-thiophen-2-yl)-phenyl]-6,6-dimethyl-4,4-dioxo-5,6-dihydro-4H-4lambda6-[1,4,3]oxathiazin-2-ylamino}-3-(2-fluor-phenyl)-propan-1-ol 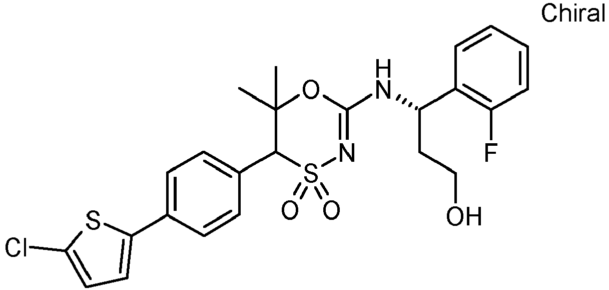
(S)-3-{5-[4-(2,5-Dichlor-thiophen-3-yl)-phenyl]-6,6-dimethyl-4,4-dioxo-5,6-dihydro-4H-4lambda6-[1,4,3]oxathiazin-2-ylamino}-3-(2-fluor-phenyl)-propan-1-ol 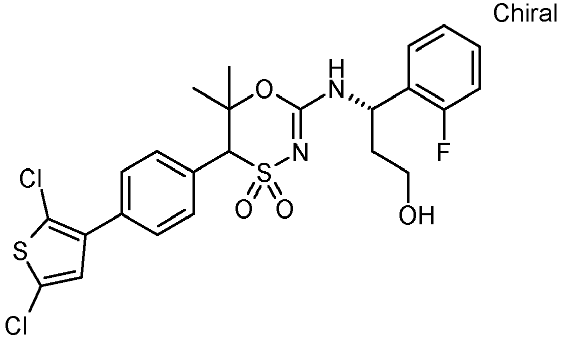
4'-{2-[(S)-1-(2-Fluor-phenyl)-3-hydroxy-propylamino]-6,6-dimethyl-4,4-dioxo-5,6-dihydro-4H-4lambda6-[1,4,3]oxathiazin-5-yl}-biphenyl-3-carbonitril 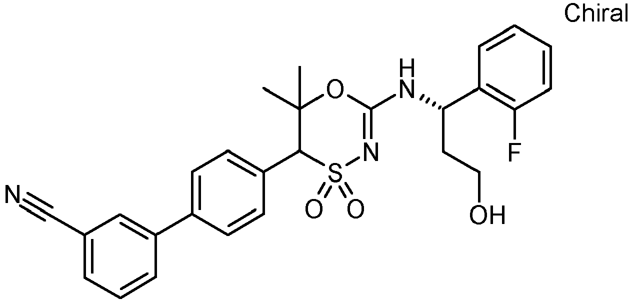
(S)-3-(2-Fluor-phenyl)-3-[5-(2'-methoxy-biphenyl-4-yl)-6,6-dimethyl-4,4-dioxo-5,6-dihydro-4H-4lambda6-[1,4,3]oxathiazin-2-ylamino]-propan-1-ol 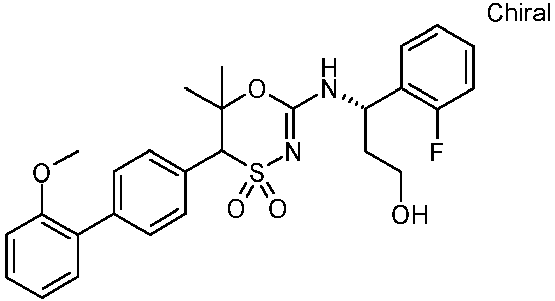
4'-{2-[(S)-1-(2-Fluor-phenyl)-3-hydroxy-propylamino]-6,6-dimethyl-4,4-dioxo-5,6-dihydro-4H-4lambda6-[1,4,3]oxathiazin-5-yl}-biphenyl-4-carbonitril 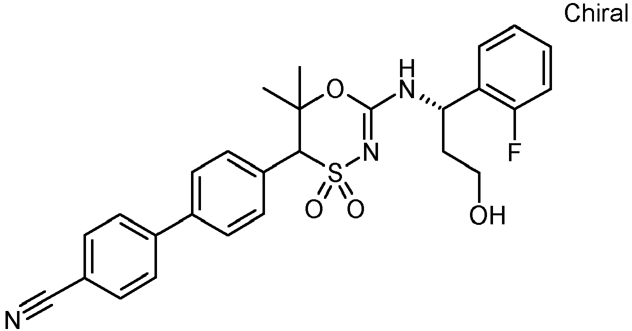
(S)-3-(5-Biphenyl-4-yl-6,6-dimethyl-4,4-dioxo-5,6-dihydro-4H-4lambda6-[1,4,3]oxathiazin-2-ylamino)-3-(2-chlor-3-fluor-phenyl)-propan-1-ol 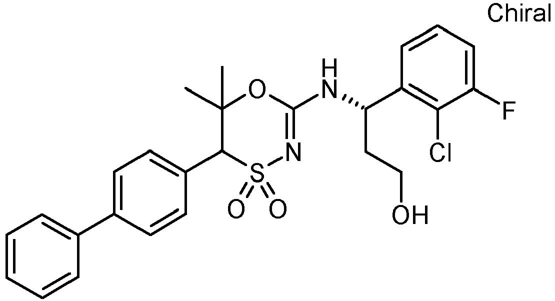
(S)-3-(5-Biphenyl-4-yl-6,6-dimethyl-4,4-dioxo-5,6-dihydro-4H-4lambda6-[1,4,3]oxathiazin-2-ylamino)-3-(2,5-difluor-phenyl)-propan-1-ol 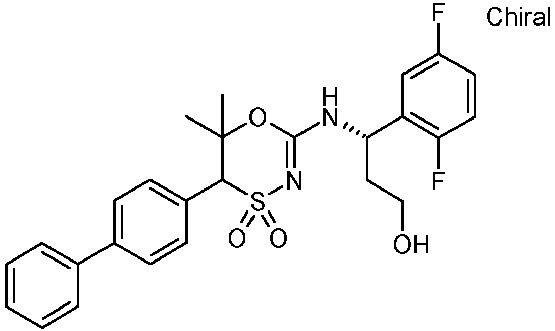
(S)-3-(5-Biphenyl-4-yl-6,6-dimethyl-4,4-dioxo-5,6-dihydro-4H-4lambda6-[1,4,3]oxathiazin-2-ylamino)-3-(5-chlor-2-fluor-phenyl)-propan-1-ol 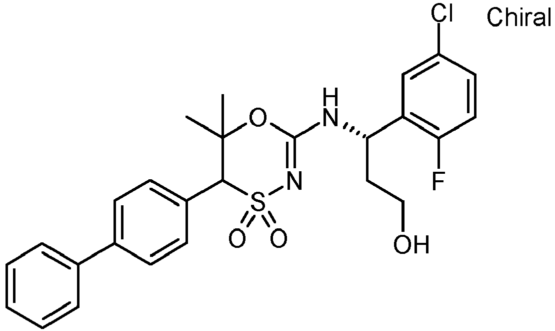
(S)-3-(5-Biphenyl-4-yl-6,6-dimethyl-4,4-dioxo-5,6-dihydro-4H-4lambda6-[1,4,3]oxathiazin-2-ylamino)-3-(5-fluor-2-methyl-phenyl)-propan-1-ol 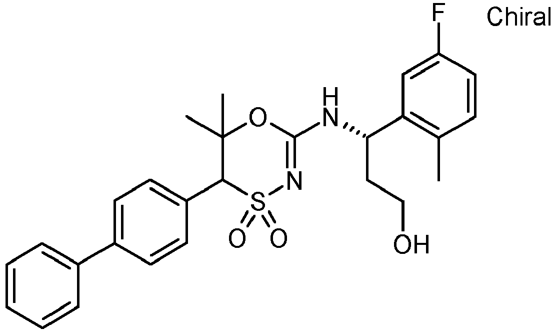
(S)-3-[6,6-Dimethyl-4,4-dioxo-5-(4-thiazol-4-yl-phenyl)-5,6-dihydro-4H-4lambda6-[1,4,3]oxathiazin-2-ylamino]-3-(2-fluor-phenyl)-propan-1-ol 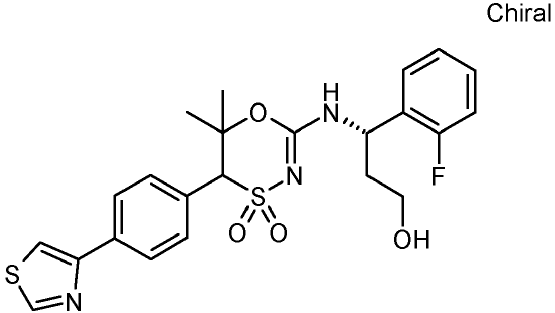 (S)-3-{6,6-Dimethyl-5-[4-(2-methyl-2H-pyrazol-3-yl)-phenyl]-4,4-dioxo-5,6-dihydro-4H-4lambda*6*-[1,4,3]oxathiazin-2-ylamino}-3-(2-fluoro-phenyl)-propan-1-ol 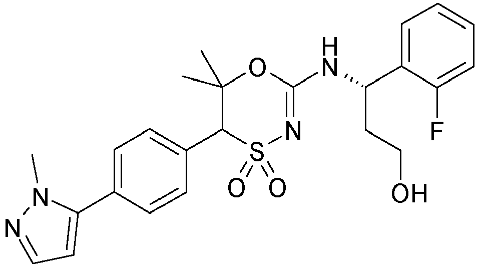
4'-{2-[(S)-1-(2-Fluor-phenyl)-3-hydroxy-propylamino]-6,6-dimethyl-4,4-dioxo-5,6-dihydro-4H-4lambda*6*-[1,4,3]oxathiazin-5-yl}-biphenyl-4-carbonsäureamid 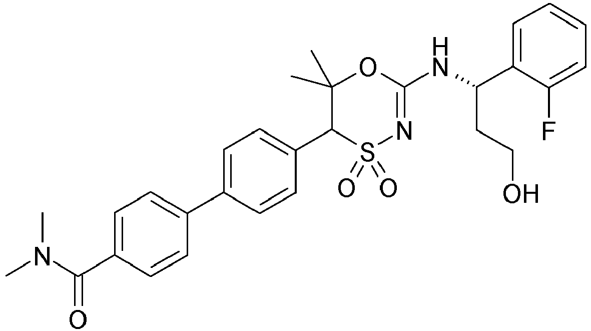
4'-{2-[(S)-1-(2-Fluor-phenyl)-3-hydroxy-propylamino]-6,6-dimethyl-4,4-dioxo-5,6-dihydro-4H-4lambda*6*-[1,4,3]oxathiazin-5-yl}-biphenyl-4-carbonsäureamid 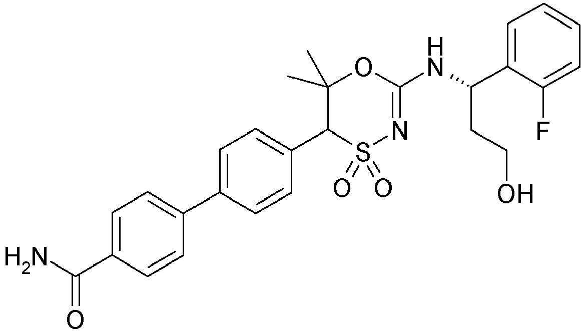
(S)-3-[6,6-Dimethyl-4,4-dioxo-5-(4-pyridin-3-yl-phenyl)-5,6-dihydro-4H-4lambda*6*-[1,4,3]oxathiazin-2-ylamino]-3-(2-fluor-phenyl)-propan-1-ol 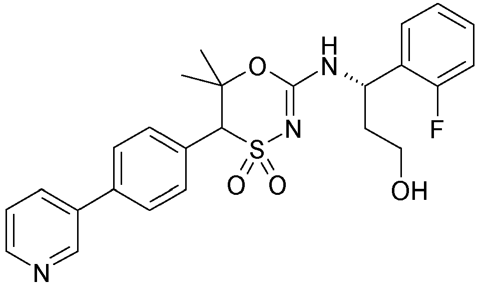
(S)-3-[6,6-Dimethyl-4,4-dioxo-5-(4-pyridin-4-yl-phenyl)-5,6-dihydro-4H-4lambda*6*-[1,4,3]oxathiazin-2-ylamino]-3-(2-fluor-phenyl)-propan-1-ol 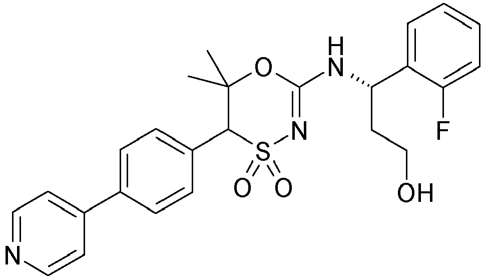
(S)-3-{6,6-Dimethyl-5-[4-(3-methyl-1H-pyrazol-4-yl)-phenyl]-4,4-dioxo-5,6-dihydro-4H-4lambda*6*-[1,4,3]oxathiazin-2-ylamino}-3-(2-fluor-phenyl)-propan-1-ol 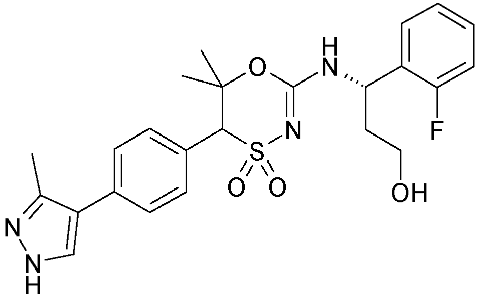
(S)-3-{6,6-Dimethyl-5-[4-(2-morpholin-4-yl-thiazol-4-yl)-phenyl]-4,4-dioxo-5,6-dihydro-4H-4lambda*6*-[1,4,3]oxathiazin-2-ylamino}-3-(2-fluor-phenyl)-propan-1-ol 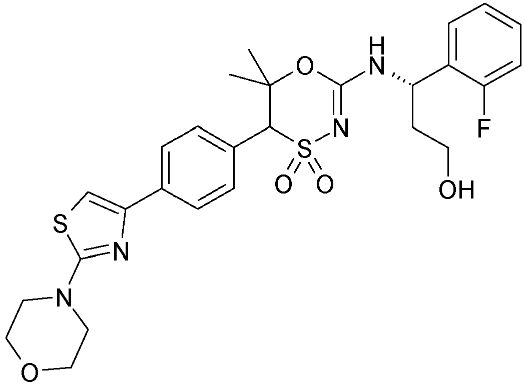
(S)-3-{6,6-Dimethyl-4,4-dioxo-5-[4-(1H-pyrazol-4-yl)-phenyl]-5,6-dihydro-4H-4lambda*6*-[1,4,3]oxathiazin-2-ylamino}-3-(2-fluor-phenyl)-propan-1-ol 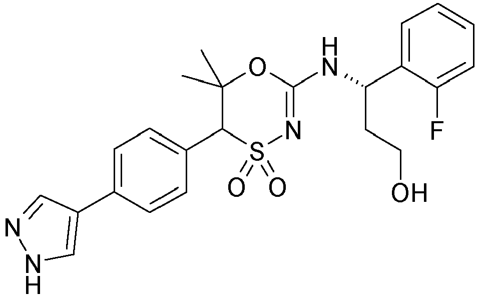
(S)-3-[5-(4'-Dimethylaminomethyl-biphenyl-4-yl)-6,6-dimethyl-4,4-dioxo-5,6-dihydro-4H-4lambda*6*-[1,4,3]oxathiazin-2-ylamino]-3-(2-fluor-phenyl)-propan-1-ol 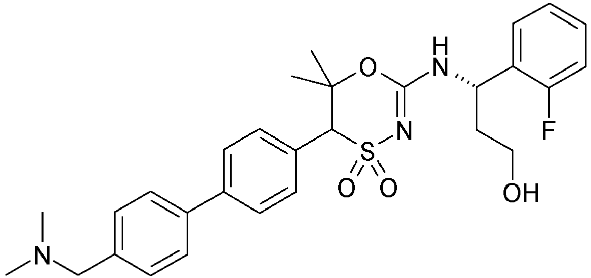
(S)-3-{6,6-Dimethyl-5-[4-(1-methyl-1H-pyrazol-4-yl)-phenyl]-4,4-dioxo-5,6-dihydro-4H-4lambda*6*-[1,4,3]oxathiazin-2-ylamino}-3-(2-fluor-phenyl)-propan-1-ol 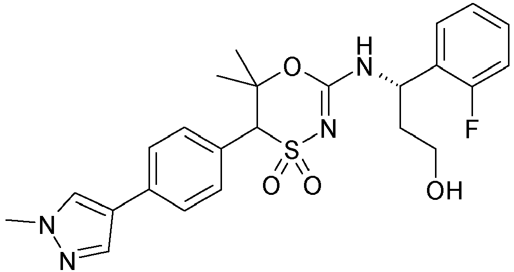
(S)-3-(6,6-Dimethyl-5-{4-[2-(4-methyl-piperazin-1-yl)-pyrimidin-5-yl]-phenyl}-4,4-dioxo-5,6-dihydro-4H-4lambda*6*-[1,4,3]oxathiazin-2-ylamino)-3-(2-fluor-phenyl)-propan-1-ol 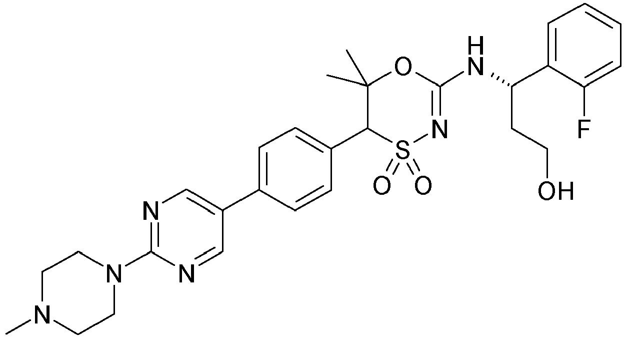
(S)-3-{5-[4-(6-Dimethylamino-pyridin-2-yl)-phenyl]-6,6-dimethyl-4,4-dioxo-5,6-dihydro-4H-4lambda*6*-[1,4,3]oxathiazin-2-ylamino}-3-(2-fluor-phenyl)-propan-1-ol 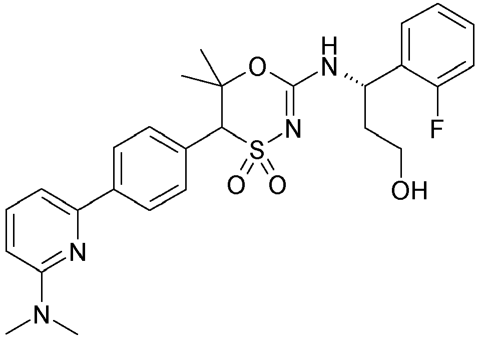
4-(4-{2-[(S)-1-(2-Fluor-phenyl)-3-hydroxy-propylamino]-6,6-dimethyl-4,4-dioxo-5,6-dihydro-4H-4lambda*6*-[1,4,3]oxathiazin-5-yl}-phenyl)-pyridine-2-carbonitril 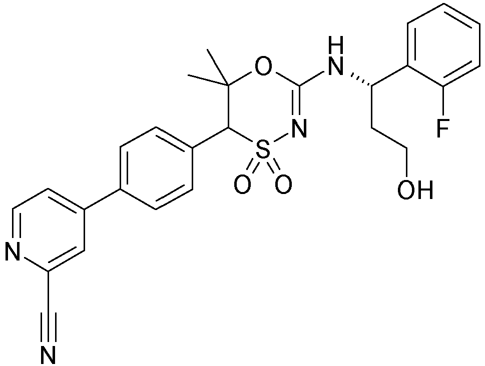
(S)-3-{6,6-Dimethyl-5-[4-(4-methyl-3,4,5,6-tetrahydro-2H-[1,2']bipyrazinyl-5'-yl)-phenyl]-4,4-dioxo-5,6-dihydro-4H-4lambda*6*-[1,4,3]oxathiazin-2-ylamino}-3-(2-fluor-phenyl)-propan-1-ol 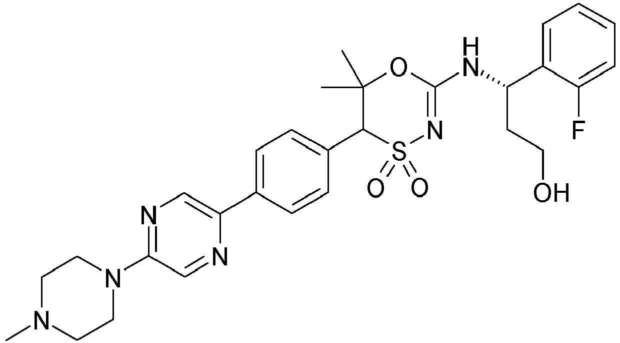
(S)-3-(2-Fluor-phenyl)-3-{5-[4-(2-methoxy-pyrimidin-5-yl)-phenyl]-6,6-dimethyl-4,4-dioxo-5,6-dihydro-4H-4lambda*6*-[1,4,3]oxathiazin-2-ylamino}-propan-1-ol 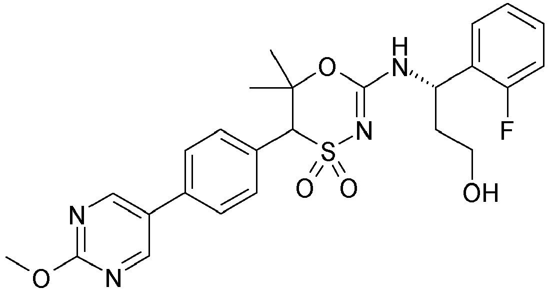
(S)-3-(6,6-Dimethyl-5-{4-[6-(4-methyl-piperazin-1-yl)-pyridin-3-yl]-phenyl}-4,4-dioxo-5,6-dihydro-4H-4lambda*6*-[1,4,3]oxathiazin-2-ylamino)-3-(2-fluor-phenyl)-propan-1-ol 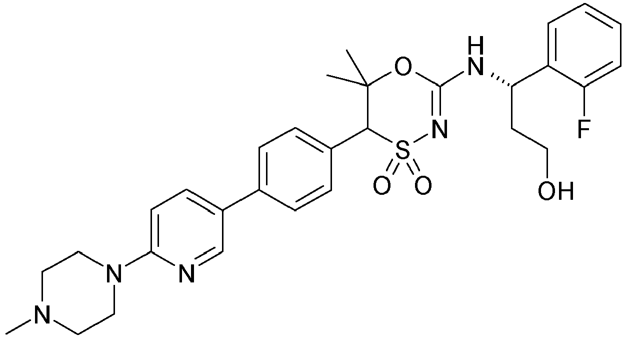
4'-{2-[(S)-1-(2-Fluor-phenyl)-3-hydroxy-propylamino]-6,6-dimethyl-4,4-dioxo-5,6-dihydro-4H-4lambda*6*-[1,4,3]oxathiazin-5-yl}-biphenyl-3-carbonsäureamid 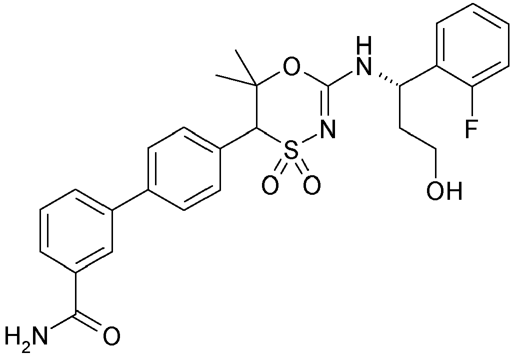
4'-{2-[(S)-1-(2-Fluor-phenyl)-ethylamino]-6,6-dimethyl-4,4-dioxo-5,6-dihydro-4H-4lambda*6*-[1,4,3]oxathiazin-5-yl}-biphenyl-3 -carbonsäureamid 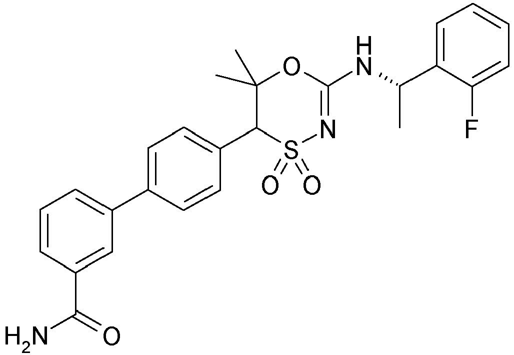
4'-{2-[(S)-1-(2-Fluor-phenyl)-ethylamino]-6,6-dimethyl-4,4-dioxo-5,6-dhydro-4H-4lambda*6*-[1,4,3]oxathiazin-5-yl}-biphenyl-4-carbonsäureamid 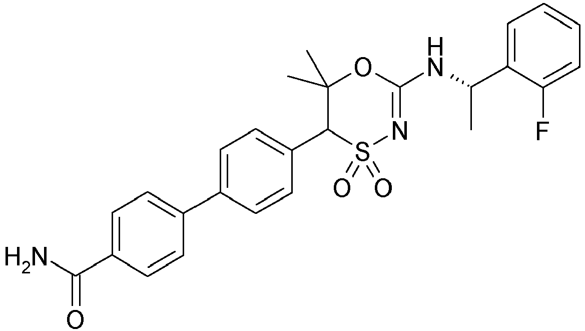
[6,6-Dimethyl-4,4-dioxo-5-(6-phenyl-pyridin-3-yl)-5,6-dihydro-4H-4lambda*6*-[1,4,3]oxathiazin-2-yl]-[(S)-1-(2-fluor-phenyl)-ethyl]-amin 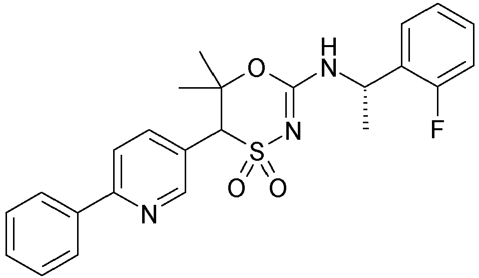
(S)-3-[6,6-Dimethyl-4,4-dioxo-5-(6-phenyl-pyridin-3-yl)-5,6-dihydro-4H-4lambda*6*[1,4,3]oxathiazin-2-ylamino]-3-(2-fluor-phenyl)-propan-1-ol 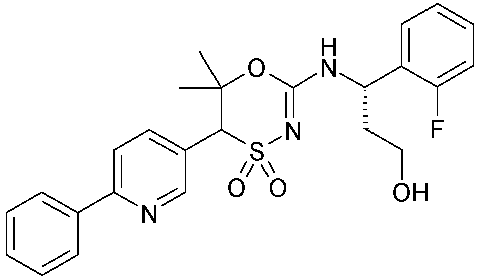
{6,6-Dimethyl-4,4-dioxo-5-[4-(2-piperazin-1-yl-pyrimidin-5-yl)-phenyl]-5,6-dihydro-4H-4lambda*6*-[1,4,3]oxathiazin-2-yl}-[(S)-1-(2-fluor-phenyl)-ethyl]-amine 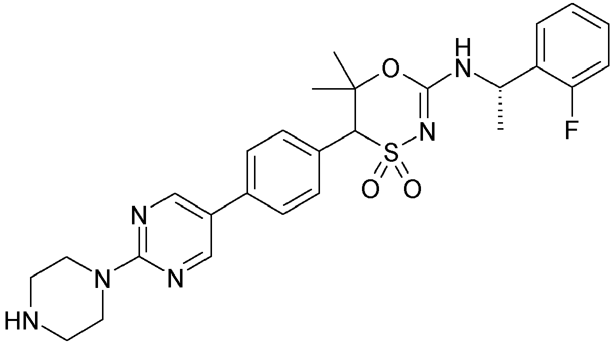
(S)-3-{6,6-Dimethyl-4,4-dioxo-5-[4-(2-piperazin-1-yl-pyrimidin-5-yl)-phenyl]-5,6-dihydro-4H-4lambda*6*-[1,4,3]oxathiazin-2-ylamino}-3-(2-fluor-phenyl)-propan-1-ol 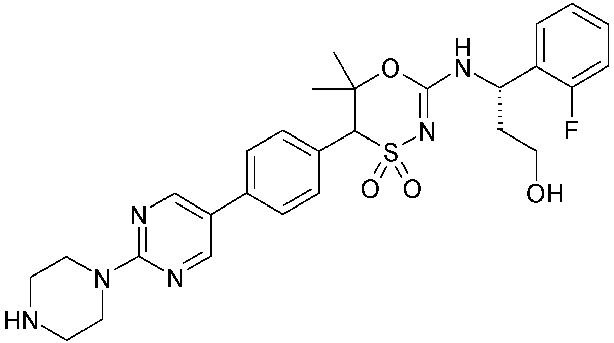
[5-(2'-Dimethylaminomethyl-biphenyl-4-yl)-6,6-dimethyl-4,4-dioxo-5,6-dihydro-4H-4lambda*6*-[1,4,3]oxathiazin-2-yl]-[(S)-1-(2-fluor-phenyl)-ethyl]-amin 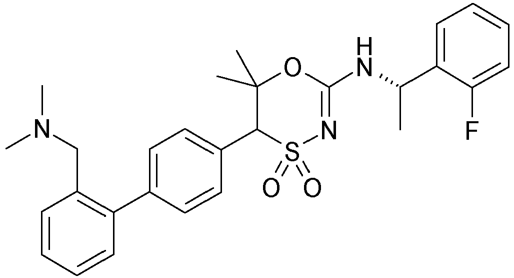
3-(5-{2-[(S)-1-(2-Fluor-phenyl)-ethylamino]-6,6-dimethyl-4,4-dioxo-5,6-dihydro-4H-4lambda*6*-[1,4,3]oxathiazin-5-yl}-pyridin-2-yl)-benzamid 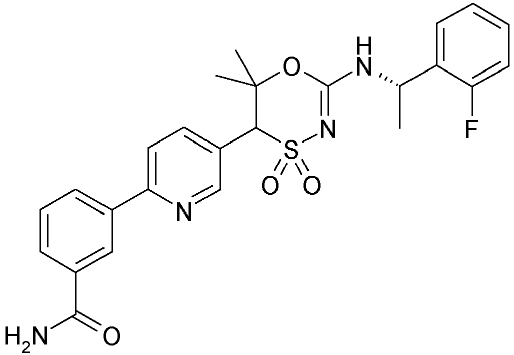
5-(4-{2-[(S)-1-(2-Fluor-phenyl)-ethylamino]-6,6-dimethyl-4,4-dioxo-5,6-dihydro-4H-4lambda*6*-[1,4,3]oxathiazin-5-yl}-phenyl)-nicotinamid 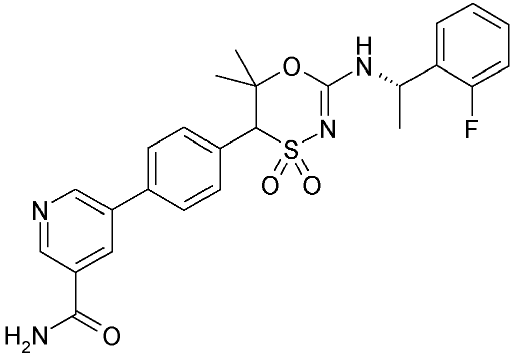
4-(4-{2-[(S)-1-(2-Fluor-phenyl)-ethylamino]-6,6-dimethyl-4,4-dioxo-5,6-dihydro-4H-4lambda*6*-[1,4,3]oxathiazin-5-yl}-phenyl)-pyridin-2-carbonitril 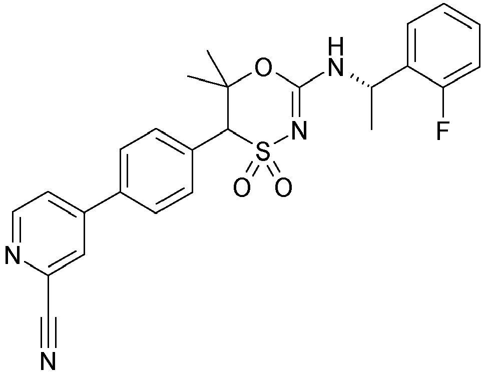
4-(4-{2-[(S)-1-(2-Fluor-phenyl)-3-hydroxy-propylamino]-6,6-dimethyl-4,4-dioxo-5,6-dihydro-4H-4lambda*6*-[1,4,3]oxathiazin-5-yl}-phenyl)-pyridin-2-carbonitril 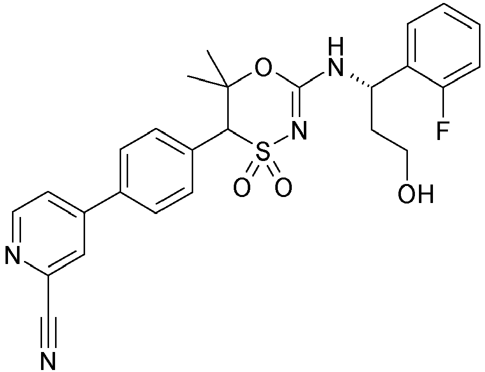
4-(4-{2-[(S)-1-(2-Fluor-phenyl)-3-hydroxy-propylamino]-6,6-dimethyl-4,4-dioxo-5,6-dihydro-4H-4lambda*6*-[1,4,3]oxathiazin-5-yl}-phenyl)-pyridin-2-carbonsäureamid 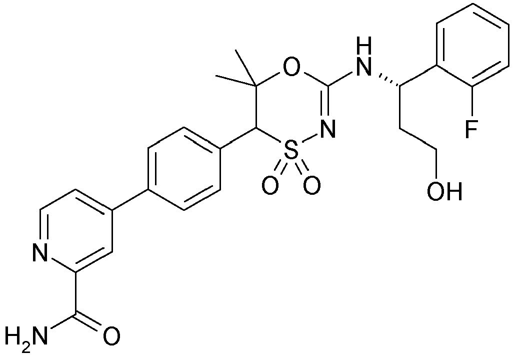
4-(4-{2-[(S)-1-(2-Fluor-phenyl)-3-hydroxy-propylamino]-6,6-dimethyl-4,4-dioxo-5,6-dihydro-4H-4lambda*6*-[1,4,3]oxathiazin-5-yl}-phenyl)-pyridin-2-carbonitril 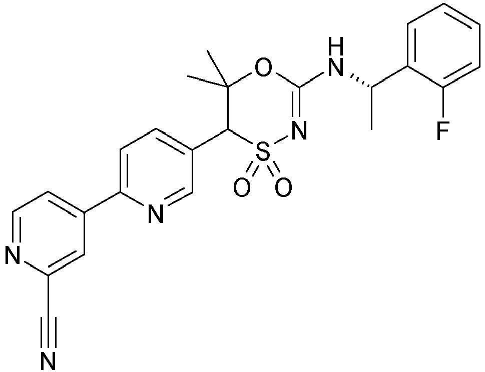
6-(4-{2-[(S)-1-(2-Fluor-phenyl)-ethylamino]-6,6-dimethyl-4,4-dioxo-5,6-dihydro-4H-4lambda*6*-[1,4,3]oxathiazin-5-yl}-phenyl)-benzothiazol-2-ylamin 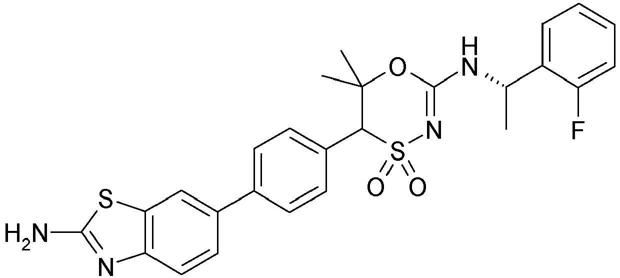
{5-[4'-(2-Amino-pyrimidin-5-yl)-biphenyl-4-yl]-6,6-dimethyl-4,4-dioxo-5,6-dihydro-4H-4lambda*6*-[1,4,3]oxathiazin-2-yl}-[(S)-1-(2-fluor-phenyl)-ethyl]-amin 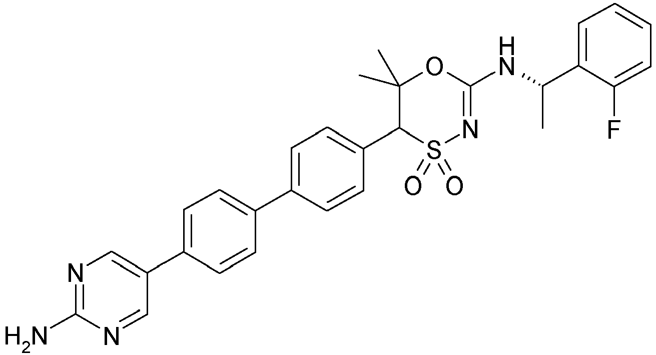
(S)-3-(9-Biphenyl-4-yl-8,8-dioxo-2,5-dioxa-8lambda6-thia-7-aza-spiro[3.5]non-6-en-6-ylamino)-3-(2-fluor-phenyl)-propan-1-ol 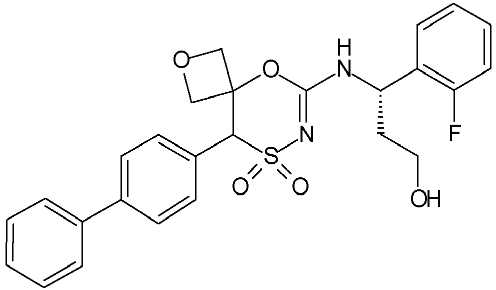
Biphenyl-4-yl-(3-hydroxy-oxetan-3-yl)-methanesulfonsäureamid

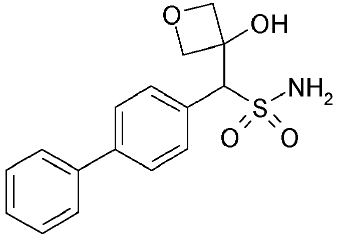

Unter Inertgas wurden 0.500 g C-Biphenyl-4-yl-N-(2,4-dimethoxy-benzyl) methanesulfonsäureamid in 5 ml THF vorgelegt und anschließend wurden bei -78 °C 1.8 ml einer 1.6 N Butyllithium-Lösung in Hexan tropfenweise zugegeben. Die Reaktionsmischung wurde für 5 Minuten gerührt und dann mit 0.24 ml 3-Oxetanon tropfenweise versetzt. Die Mischung wurde 5 Minuten bei gleichbleibender Temperatur gerührt, mit 0.28 ml Trifluoressigsäure versetzt und auf Raumtemperatur kommen gelassen. Das Lösungsmittel wurde unter vermindertem Druck entfernt. Der Rückstand wurde in 2 ml Dichlormethan gelöst und mit 2 ml Trifluoressigsäure versetzt. Nach 60 Minuten Rühren bei Raumtemperatur wurden 50 mL gesättige Kaliumcarbonatlösung hinzugegeben und die Mischung wurde mit Dichlormethan extrahiert, über Magnesiumsulfat getrocknet und eingeengt. Das Rohprodukt wurde mittels Normalphasenchromatographie über den Flashmaster mit einem n-Heptan/Ethylacetat-Gradienten gereinigt. Man erhielt so das Produkt (170 mg) mit dem Molekulargewicht 319.4 g / mol (C₁₆H₁₇NO₄S).

### 9-Biphenyl-4-yl-6-methoxy-2,5-dioxa-8-thia-7-aza-spiro[3.5]non-6-ene 8,8-dioxid

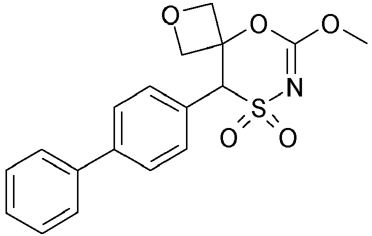

Eine Mischung aus 0.17 g Biphenyl-4-yl-(3-hydroxy-oxetan-3-yl)-methanesulfonsäureamid in 4 ml Tetraethoxymethan und 1 ml Essigsäure wurde für 3 Stunden bei 90 °C gerührt. Das Lösungsmittel wurde unter vermindertem Druck entfernt. Man erhielt so das Rohprodukt, das ohne weitere Reinigung eingesetzt wurde.

### (S)-3-(9-Biphenyl-4-yl-8,8-dioxo-2,5-dioxa-8lambda*6*-thia-7-aza-spiro[3.5]non-6-en-6-ylamino)-3-(2-fluor-phenyl)-propan-1-ol

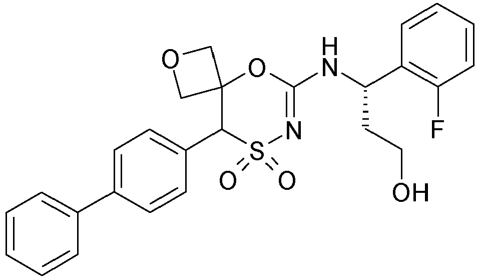

In einem Rundkolben wurde 9-Biphenyl-4-yl-6-methoxy-2,5-dioxa-8-thia-7-aza-spiro[3.5]non-6-ene 8,8-dioxid unter Inertgas in 2 ml Dichlormethan gelöst und mit 151 mg (S)-3-(tert-Butyl-dimethyl-silanyloxy)-1-(2-fluor-phenyl)-propylamin versetzt. Nach dem Einrotieren wurde der Rückstand für 18 Stunden bei Raumtemperatur stehengelassen. Zur Abspaltung der Schutzgruppe wurde der Rückstand in 1 ml Methanol aufgenommen und nach Zugabe von 0.05 ml konzentrierter Salzsäure 1 Stunde bei Raumtemperatur gerührt. Nach dem Einrotieren wurde der Rückstand im Reinigungslabor über präparative HPLC gereinigt. Die produkthaltigen Fraktionen wurden vereinigt und unter vermindertem Druck vom Lösungsmittel befreit..Man erhielt so das Produkt (8 mg) mit dem Molekulargewicht 496.6 g / mol (C₂₆H₂₅FN₂O₅S); MS (ESI): m / e = 497 (M+H⁺).

Auf gleiche Weise wurden folgende Produkte erhalten:
(S)-3-(9-Biphenyl-4-yl-8,8-dioxo-5-oxa-8lambda*6*-thia-7-aza-spiro[3.5]non-6-en-6-ylamino)-3-(2-fluor-phenyl)-propan-1-ol 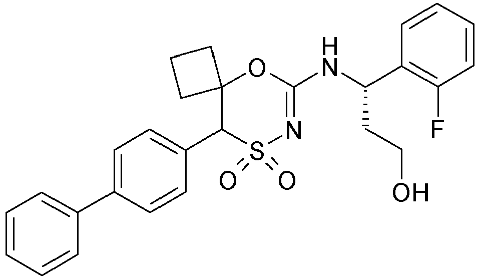
(S)-3-(10-Biphenyl-4-yl-9,9-dioxo-6-oxa-9lambda*6*-thia-8-aza-spiro[4.5]dec-7-en-7-ylamino)-3-(2-fluor-phenyl)-propan-1-ol 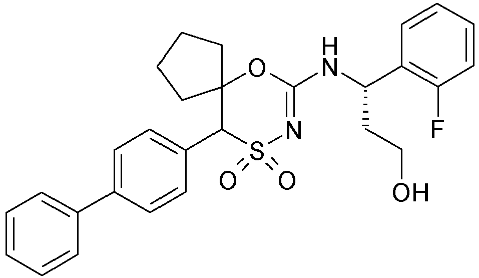
(S)-3-{6,6-Dimethyl-5-[4-(1-methyl-piperidin-4-yl)-phenyl]-4,4-dioxo-5,6-dihydro-4H-4lambda*6*-[1,4,3]oxathiazin-2-ylamino}-3-(2-fluor-phenyl)-propan-1-ol 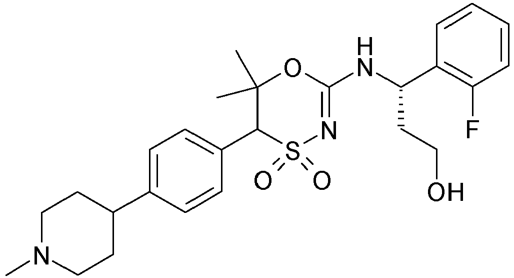
1-Boc-4-(4-{2-[(S)-3-(tert-Butyl-dimethyl-silanyloxy)-1-(2-fluor-phenyl)-propylamino]-6,6-dimethyl-4,4-dioxo-5,6-dihydro-4H-4lambda*6*-[1,4,3]oxathiazin-5-yl}-phenyl)-3,6-dihydro-2H-pyridin

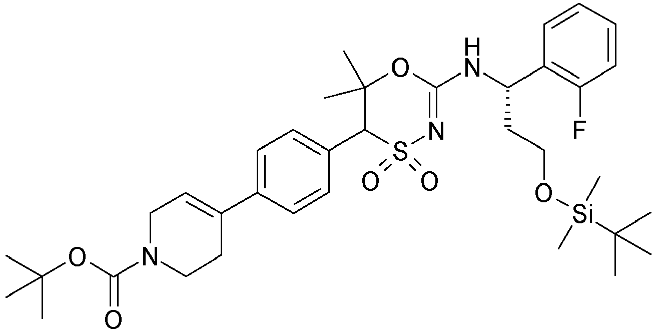

Eine Lösung aus N-boc-4-(4,4,5,5-Tetramethyl-[1,3,2]dioxaborolan-2-yl)-3,6-dihydro-2H-pyridin (408 mg), Cäsiumcarbonat (696 mg) und [5-(4-Brom-phenyl)-6,6-dimethyl-4, 4-dioxo-5,6-dihydro-4H-4lambda*6*-[1,4,3]oxathiazin-2-yl]-[(S)-3-(tert-butyl-dimethyl-silanyloxy)-1-(2-fluor-phenyl)-propyl]-amin (600 mg) in Dioxan (7.0 ml) / Wasser (2.8 ml) wurde mit Argon zwanzig Minuten gespült. Dann wurden 1,2,3,4,5-Pentaphenyl-1'-(di-t-butylphosphino)ferrocen (142 mg) und Bis(dibenzylideneaceton)palladium(58 mg) zugegeben und dreißig Minuten bei 65 °C gerührt. Anschließend wurde mit Ethylacetat (20 ml) versetzt und mit Wasser (10 ml) gewaschen. Die organische Phase wurde über eine Kieselgurkartusche getrocknet, filtriert und im Vakuum konzentriert. Anschließend wurde säulenchromatographisch (Ethylacetat / Heptan) gereinigt. Man erhielt so das Produkt (584 mg) mit dem Molekulargewicht 701.0 g / mol (C₃₆H₅₂FN₃O₆SSi).

### (S)-3-[6,6-Dimethyl-4,4-dioxo-5-(4-piperidin-4-yl-phenyl)-5,6-dihydro-4H-4lambda*6*-[1,4,3]oxathiazin-2-ylamino]-3-(2-fluor-phenyl)-propan-1-ol

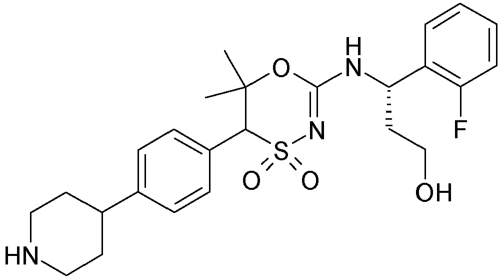

Eine Lösung von 1-Boc-4-(4-{2-[(S)-3-(tert-Butyl-dimethyl-silanyloxy)-1-(2-fluor-phenyl)-propylamino]-6,6-dimethyl-4,4-dioxo-5,6-dihydro-4H-4lambda*6*-[1,4,3]oxathiazin-5-yl}-phenyl)-3,6-dihydro-2H-pyridin (575 mg) in Methanol (25 mL) wurde mit Palladium auf Kohle (10%, 260 mg) versetzt und 1,5 h in einer Wasserstoffatmosphäre gerührt. Die Mischung wurde filtriert und eingeengt. Der Rückstand wurde in 5 mL Dichlormethan gelöst und mit 1 mL Trifluoressigsäure versetzt. Nach 1 h Rühren bei Raumtemperatur wurde das Lösungsmittel im Vakuum entfernt und entstandener TFA Ester durch Behandeln mit 1 N HCl gespalten. Extraktion mit Ethylacetat lieferte das Rohprodukt (91%), das ohne weitere Reinigung eingesetzt wurde

### (S)-3-{6,6-Dimethyl-5-[4-(1-methyl-piperidin-4-yl)-phenyl]-4,4-dioxo-5,6-dihydro-4H-4lambda*6*-[1,4,3]oxathiazin-2-ylamino}-3-(2-fluor-phenyl)-propan-1-ol

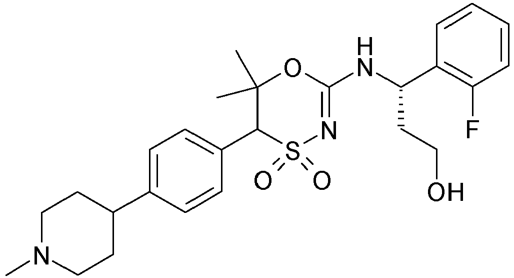

Zu einer Lösung aus (S)-3-[6,6-Dimethyl-4,4-dioxo-5-(4-piperidin-4-yl-phenyl)-5,6-dihydro-4H-4lambda*6*-[1,4,3]oxathiazin-2-ylamino]-3-(2-fluor-phenyl)-propan-1-ol (100 mg) in 5 mL THF/Essigsäure 4:1 wurde Paraformaldehyd (74 mg) und polymergebundenes Natriumcyanoborhydrid (238 mg) gegeben und die Mischung wurde über Nacht bei Raumtemperatur gerührt. Nach dem Filtrieren und Einrotieren wurde der Rückstand im Reinigungslabor über präparative HPLC gereinigt. Man erhielt so das Produkt (75 mg als HCl Salz nach Behandeln mit Salzsäure) mit dem Molekulargewicht 503.2 g / mol (C₂₆H₃₄FN₃O₄S); MS (ESI): m / e = 504 (M+H⁺).

### (S)-3-(2-Fluor-phenyl)-3-(5-{4-[1-(2-hydroxy-ethyl)-piperidin-4-yl]-phenyl}-6,6-dimethyl-4,4-dioxo-5,6-dihydro-4H-4lambda*6*-[1,4,3]oxathiazin-2-ylamino)-propan-1-ol

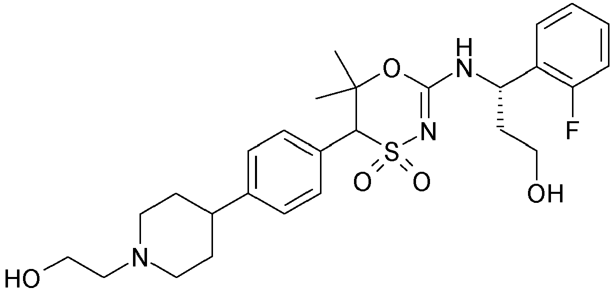

Zu einer Lösung aus (S)-3-[6,6-Dimethyl-4,4-dioxo-5-(4-piperidin-4-yl-phenyl)-5,6-dihydro-4H-4lambda*6*-[1,4,3]oxathiazin-2-ylamino]-3-(2-fluor-phenyl)-propan-1-ol (100 mg)in DMF (1,5 mL) wurde DIPEA (40 mg) und 2-Bromethanol (31 mg) gegeben und die Mischung wurde über Nacht gerührt. Die entstandene Mischung wurde im Reinigungslabor über präparative HPLC gereinigt. Man erhielt so das Produkt (66 mg als HCl Salz nach Behandeln mit Salzsäure) mit dem Molekulargewicht 533.2 g / mol (C₂₇H₃₆FN₃O₅S); MS (ESI): m / e = 534 (M+H⁺).

Analog wurde folgende Verbindungen erhalten:
(S)-3-(5-{4-[1-(2,2-Difluor-ethyl)-piperidin-4-yl]-phenyl}-6,6-dimethyl-4,4-dioxo-5,6-dihydro-4H-4lambda*6*-[1,4,3]oxathiazin-2-ylamino)-3-(2-fluor-phenyl)-propan-1-ol 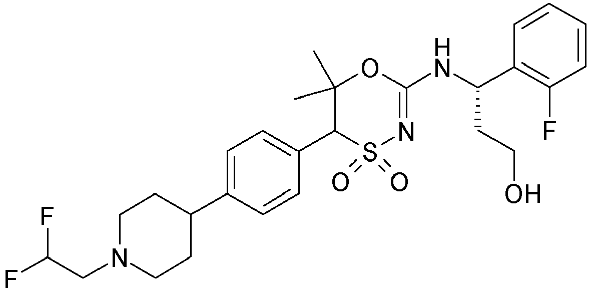
(S)-3-(6,6-Dimethyl-4,4-dioxo-5-{4-[1-(3,3,3-trifluor-propyl)-piperidin-4-yl]-phenyl}-5,6-dihydro-4H-4lambda*6*-[1,4,3]oxathiazin-2-ylamino)-3-(2-fluor-phenyl)-propan-1-ol

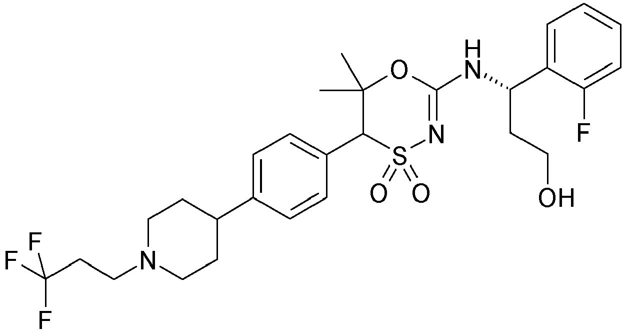

Analog wurden folgende Verbindungen erhalten:
[6,6-Dimethyl-4,4-dioxo-5-(4-piperidin-4-yl-phenyl)-5,6-dihydro-4H-4lambda*6*-[1,4,3]oxathiazin-2-yl]-[(S)-1-(2-fluor-phenyl)-ethyl]-amin 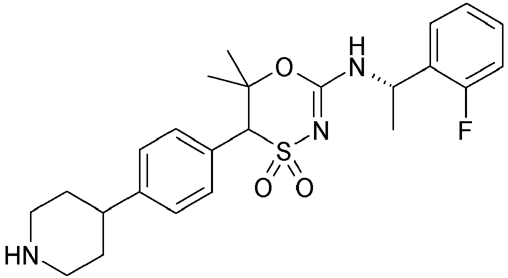
{6,6-Dimethyl-5-[4-(1-methyl-piperidin-4-yl)-phenyl]-4,4-dioxo-5,6-dihydro-4H-4lambda*6*-[1,4,3]oxathiazin-2-yl}-[(S)-1-(2-fluor-phenyl)-ethyl]-amin 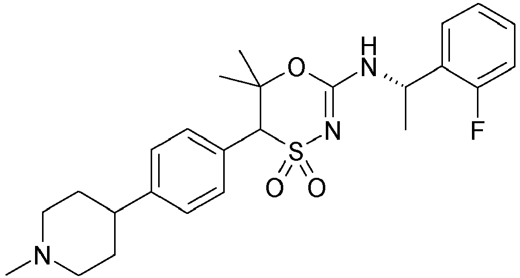
{6,6-Dimethyl-5-[3-(1-methyl-piperidin-4-yl)-phenyl]-4,4-dioxo-5,6-dihydro-4H-4lambda*6*-[1,4,3]oxathiazin-2-yl}-[(S)-1-(2-fluor-phenyl)-ethyl]-amin 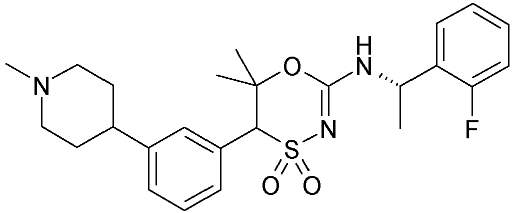
2-[4-(4-{2-[(S)-1-(2-Fluor-phenyl)-ethylamino]-6,6-dimethyl-4,4-dioxo-5,6-dihydro-4H-4lambda*6*-[1,4,3]oxathiazin-5-yl}-phenyl)-piperidin-1-yl]-ethanol 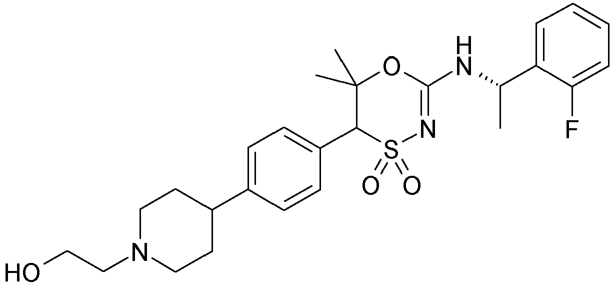
(S)-3-{5-[4-(3-Amino-propyl)-phenyl]-6,6-dimethyl-4,4-dioxo-5,6-dihydro-4H-4lambda*6*-[1,4,3]oxathiazin-2-ylamino}-3-(2-fluor-phenyl)-propan-1-ol 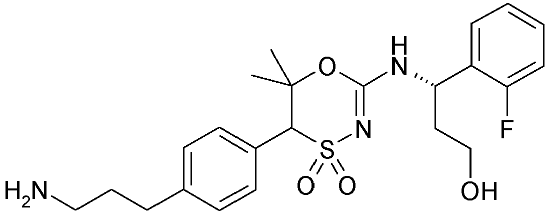
[3-(4-{2-[(S)-3-(tert-Butyl-dimethyl-silanyloxy)-1-(2-fluor-phenyl)-propylamino]-6,6-dimethyl-4,4-dioxo-5,6-dihydro-4H-4lambda*6*-[1,4,3]oxathiazin-5-yl}-phenyl)-prop-2-inyl]-carbaminsäure tert-butyl ester

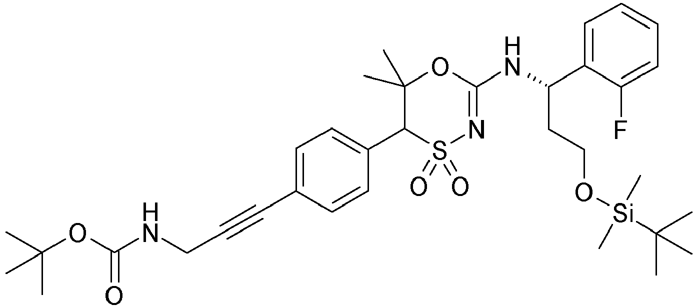

Zu einer Suspension von Bis(triphenylphoshin)palladium(II) dichlorid (10 mg) und Kupfer(I)-iodid (5 mg) in trockenem Dioxan (0.6 mL) wurden nacheinander Triethylamin (120 µL) und eine Lösung von [5-(4-Brom-phenyl)-6,6-dimethyl-4,4-dioxo-5,6-dihydro-4H-4lambda*6*-[1,4,3]oxathiazin-2-yl]-[(S)-3-(tert-butyl-dimethyl-silanyloxy)-1-(2-fluor-phenyl)-propyl]-amin (100 mg) in trockenem Dioxan (0.7 mL) gegeben und die Mischung wurde für 3 h bei 80 °C und 1 h bei 100 °C gerührt. Anschließend wurde mit Ethylacetat versetzt und mit Wasser gewaschen. Die organische Phase wurde über eine Kieselgurkartusche getrocknet, filtriert und im Vakuum konzentriert. Anschließend wurde säulenchromatographisch (Ethylacetat / Heptan) gereinigt. Man erhielt so das Produkt (89 mg) mit dem Molekulargewicht 673.2 g / mol (C₃₄H₄₈FN₃O₆SSi).

### [3-(4-{2-[(S)-3-(tert-Butyl-dimethyl-silanyloxy)-1-(2-fluor-phenyl)-propylamino]-6,6-dimethyl-4,4-dioxo-5,6-dihydro-4H-4lambda*6*-[1,4,3]oxathiazin-5-yl}-phenyl)-propyl]-carbaminsäure tert-butyl ester

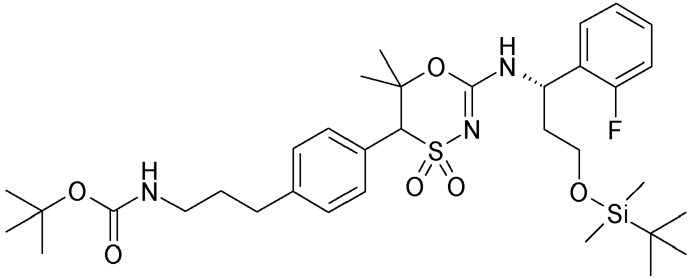

Zu einer Lösung von [3-(4-{2-[(S)-3-(tert-Butyl-dimethyl-silanyloxy)-1-(2-fluor-phenyl)-propylamino]-6,6-dimethyl-4,4-dioxo-5,6-dihydro-4H-4lambda*6*-[1,4,3]oxathiazin-5-yl}-phenyl)-prop-2-inyl]-carbaminsäure tert-butyl ester (89 mg) in Methanol (25 mL) wurde Palaldium auf Kohle gegeben (5%, 25 mg) und die Lösung wurde in einer Wasserstoffatmosphäre für 1 h gerührt. Das Gemisch wurde filtriert und das Filtrat wurde eingeengt. Anschließend wurde säulenchromatographisch (Ethylacetat / Heptan) gereinigt. Man erhielt so das Produkt (49 mg) mit dem Molekulargewicht 678.0 g / mol (C₃₄H₅₂FN₃O₆SSi).

### (S)-3-{5-[4-(3-Amino-propyl)-phenyl]-6,6-dimethyl-4,4-dioxo-5,6-dihydro-4H-4lambda*6*-[1,4,3]oxathiazin-2-ylamino}-3-(2-fluor-phenyl)-propan-1-ol

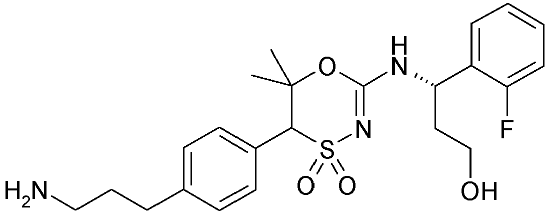

Zu einer Lösung von [3-(4-{2-[(S)-3-(tert-Butyl-dimethyl-silanyloxy)-1-(2-fluor-phenyl)-propylamino]-6,6-dimethyl-4,4-dioxo-5,6-dihydro-4H-4lambda*6*-[1,4,3]oxathiazin-5-yl}-phenyl)-propyl]-carbaminsäure tert-butyl ester (49 mg) in Methanol (2 mL) wurde konzentrierte Salzsäure (0.2 mL) gegeben und die Mischung wurde für 2 Tage gerührt. Die entstandene Mischung wurde im Reinigungslabor über präparative HPLC gereinigt. Man erhielt so das Produkt (6 mg als HCl Salz nach Behandeln mit Salzsäure) mit dem Molekulargewicht 463.2 g / mol (C₂₂H₃₀FN₃O₄S); MS (ESI): m / e = 464 (M+H⁺).

Analog wurden folgende Verbindungen erhalten:
(S)-3-{5-[4-(3-Dimethylamino-propyl)-phenyl]-6,6-dimethyl-4,4-dioxo-5,6-dihydro-4H-4lambda*6*-[1,4,3]oxathiazin-2-ylamino}-3-(2-fluor-phenyl)-propan-1-ol 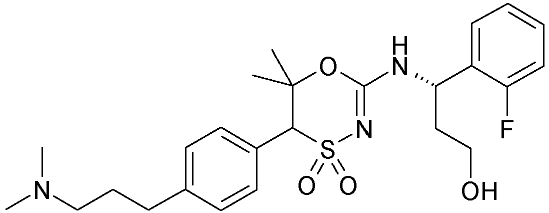
(S)-3-{6,6-Dimethyl-5-[4-(3-morpholin-4-yl-propyl)-phenyl]-4,4-dioxo-5,6-dihydro-4H-4lambda*6*-[1,4,3]oxathiazin-2-ylamino}-3-(2-fluor-phenyl)-propan-1-ol 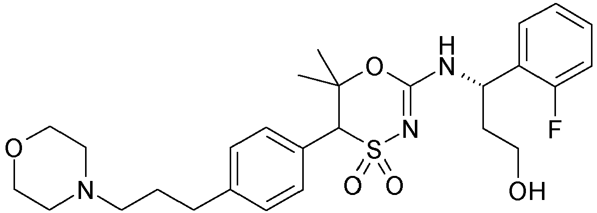
(S)-3-{6,6-Dimethyl-5-[4-(3-methylamino-propyl)-phenyl]-4,4-dioxo-5,6-dihydro-4H-4lambda*6*-[1,4,3]oxathiazin-2-ylamino}-3-(2-fluor-phenyl)-propan-1-ol 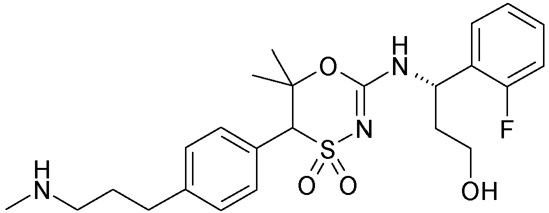
(S)-3-(2-Fluor-phenyl)-3-[5-(4-{3-[(2-hydroxy-ethyl)-methyl-amino]-propyl}-phenyl)-6,6-dimethyl-4,4-dioxo-5,6-dihydro-4H-41ambda*6*-[1,4,3]oxathiazin-2-ylamino]-propan-1-ol

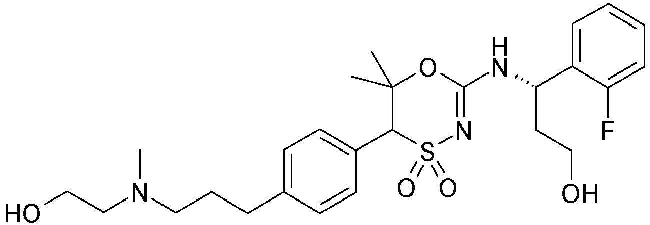

Eine Lösung von (S)-3-{6,6-Dimethyl-5-[4-(3-methylamino-propyl)-phenyl]-4,4-dioxo-5,6-dihydro-4H-4lambda*6*-[1,4,3]oxathiazin-2-ylamino}-3-(2-fluor-phenyl)-propan-1-ol (175 mg) und 2-Bromethanol (51 mg) in DMF (2.5 mL) wurde bei 50 °C für 8 h gerührt. Die entstandene Mischung wurde im Reinigungslabor über präparative HPLC gereinigt. Man erhielt so das Produkt (75 mg als HCl Salz nach Behandeln mit Salzsäure) mit dem Molekulargewicht 521.2 g / mol (C₂₆H₃₆FN₃O₅S); MS (ESI): m / e = 522 (M+H⁺).

Analog wurde folgende Verbindung erhalten:
(S)-3-[5-(4-{3-[(2,2-Difluor-ethyl)-methyl-amino]-propyl}-phenyl)-6,6-dimethyl-4,4-dioxo-5,6-dihydro-4H-4lambda*6*-[1,4,3]oxathiazin-2-ylamino]-3-(2-fluor-phenyl)-propan-1-ol 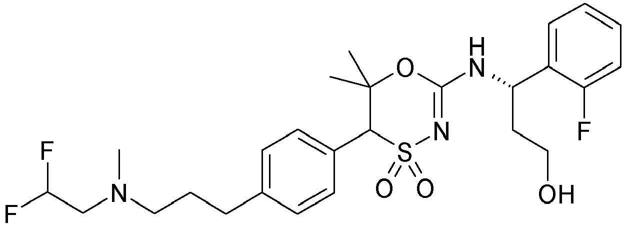
(S)-3-[6,6-Dimethyl-5-(4-morpholin-4-yl-phenyl)-4,4-dioxo-5,6-dihydro-4H-4lambda*6*-[1,4,3]oxathiazin-2-ylamino]-3-(2-fluor-phenyl)-propan-1-ol 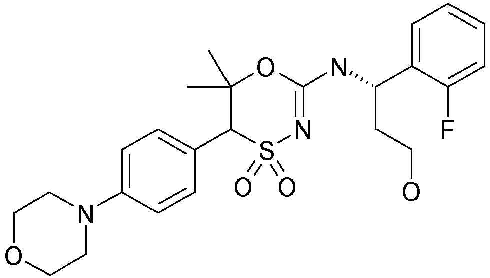
N-(2,4-Dimethoxy-benzyl)-C-(4-morpholin-4-yl-phenyl)-methanesulfonamid

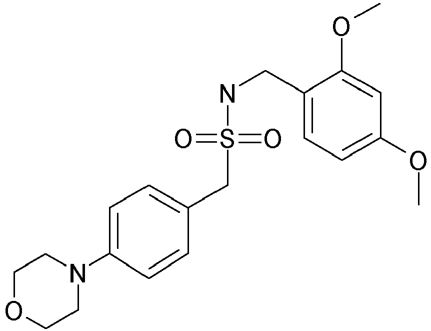

Eine Mischung aus C-(4-Brom-phenyl)-N-(2,4-dimethoxy-benzyl)-methanesulfonamid (1 g), Morpholin (0.3 mL), Pd₂(dba)₃ (51 mg) und Cäsiumcarbonat (1.3 g) in Dioxan wurde bei 80 °C für 16 h gerührt. Die Mischung wurde mit Ethylacetat verdünnt, mit Wasser gewaschen, über Magnesiumsulfat getrocknet und eingeengt. Anschließend wurde säulenchromatographisch (Ethylacetat / Heptan) gereinigt. Man erhielt so das Produkt (267 mg) mit dem Molekulargewicht 406.5 g / mol (C₂₀H₂₆N₂O₅S).

### 2-Hydroxy-2-methyl-1-(4-morpholin-4-yl-phenyl)-propan-1-sulfonamid

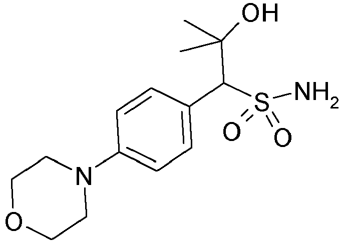

Unter Inertgas wurden 0.264 g N-(2,4-Dimethoxy-benzyl)-C-(4-morpholin-4-yl-phenyl)-methansulfonamid in 3 ml THF vorgelegt und anschließend wurden bei -78 °C 0.85 ml einer 1.6 N Butyllithium-Lösung in Hexan tropfenweise zugegeben. Die Reaktionsmischung wurde für 10 Minuten gerührt und dann mit 0.22 mL Aceton tropfenweise versetzt. Die Mischung wurde 30 Minuten bei gleichbleibender Temperatur gerührt, mit 0.102 ml Trifluoressigsäure versetzt und auf Raumtemperatur kommen gelassen. Das Lösungsmittel wurde unter vermindertem Druck entfernt. Der Rückstand wurde in 4 ml Dichlormethan gelöst und mit 1.2 mL Trifluoressigsäure versetzt. Nach 16 h Rühren bei Raumtemperatur wurde das Rohprodukt im Reinigungslabor über präparative HPLC gereinigt. Man erhielt so das Produkt mit dem Molekulargewicht 428.43 g/mol (C₁₄H₂₂N₂O₄S),

### 2-Methoxy-6,6-dimethyl-5-(4-morpholin-4-yl-phenyl)-5,6-dihydro-[1,4,3]oxathiazin 4,4-dioxid

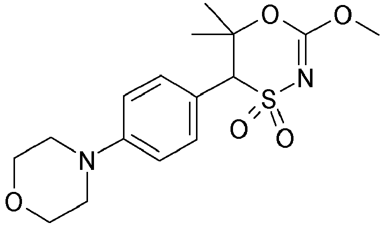

2-Hydroxy-2-methyl-1-(4-morpholin-4-yl-phenyl)-propan-1-sulfonamid (41 mg) wurde mit Tetramethylorthocarbonat (0.2 mL), Eisessig (0.01 mL) und Dioxan (0.1 mL) bei 90 °C für 4 Stunden gerührt. Die Lösungsmittel wurden im Vakuum entfernt und der Rückstand wurde ohne weitere Reinigung weiter eingesetzt.

### (S)-3-[6,6-Dimethyl-5-(4-morpholin-4-yl-phenyl)-4,4-dioxo-5,6-dihydro-4H-4lambda*6*-[1,4,3] oxathiazin-2-ylamino]-3-(2-fluor-phenyl)-propan-1-ol

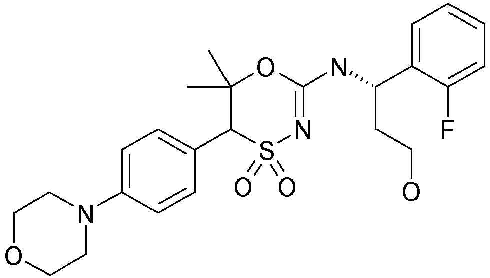

Zu einer Lösung von 2-Methoxy-6,6-dimethyl-5-(4-morpholin-4-yl-phenyl)-5,6-dihydro-[1,4,3]oxathiazin 4,4-dioxid (50 mg) in Dichlormethan (1 mL) wurde (S)-3-(tert-Butyl-dimethyl-silanyloxy)-1-(2-fluor-phenyl)-propylamin (50 mg) gegeben und das Lösungsmittel wurde verdampft. Der Rückstand wurde 16 h stehen gelassen und anschließend in Methanol (0.5 mL) gelöst und mit 0.05 mL conc. HCl versetzt. Die entstandene Mischung wurde im Reinigungslabor über präparative HPLC gereinigt. Man erhielt so das Produkt (28 mg als HCl Salz nach Behandeln mit Salzsäure) mit dem Molekulargewicht 491.2 g / mol (C₂₄H₃₀FN₃O₅S); MS (ESI): m / e = 492 (M+H⁺).

### (S)-3-{6,6-Dimethyl-5-[4-(4-methyl-piperazin-l-yl)-phenyl]-4,4-dioxo-5,6-dihydro-4H-4lambda*6*-[1,4,3]oxathiazin-2-ylamino}-3-(2-fluor-phenyl)-propan-1-ol

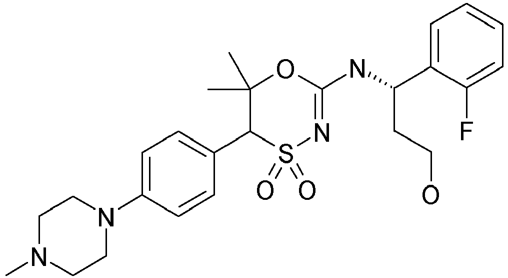

### [(S)-3-(tert-Butyl-dimethyl-silanyloxy)-1-(2-fluor-phenyl)-propyl]-{6,6-dimethyl-4,4-dioxo-5-[4-(4,4,5,5-tetramethyl-[1,3,2]dioxaborolan-2-yl)-phenyl]-5,6-dihydro-4H-4lambda*6*-[1,4,3]oxathiazin-2-yl}-amin

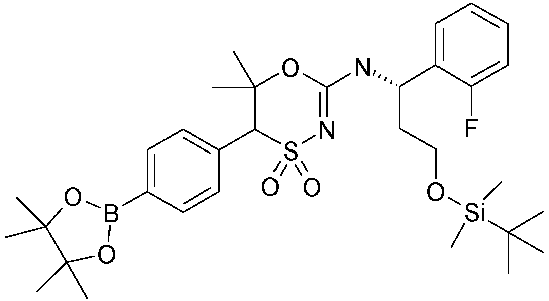

Zu einer Lösung von [5-(4-Brom-phenyl)-6,6-dimethyl-4,4-dioxo-5,6-dihydro-4H-4lambda*6*-[1,4,3]oxathiazin-2-yl]-[(S)-3-(tert-butyl-dimethyl-silanyloxy)-1-(2-fluor-phenyl)-propyl]-amin (490 mg) in Dioxan wurde Pd(dppf)Cl₂ (67 mg), Bis(pinacolato)diboron (311 mg) und Kaliumacetat (241 mg) gegeben und die Mischung wurde 1 h bei 80 °C gerührt. Nach dem Verdünnen mit Ethylacetat wurde mit Wasser und Natriumchloridlösung gewaschen, über Kieselgur getrocknet und eingeengt. Anschließend wurde säulenchromatographisch (Ethylacetat / Heptan) gereinigt. Man erhielt so das Produkt (447 mg) mit dem Molekulargewicht 646.7 g / mol (C₃₂H₄₈BFN₂O₆SSi).

### [5-(4-Boroxyphenyl)-6,6-dimethyl-4,4-dioxo-5,6-dihydro-4H-4lambda*6*-[1,4,3]oxathiazin-2-yl]-[(S)-3-(tert-butyl-dimethyl-silanyloxy)-1-(2-fluor-phenyl)-propyl]-amin

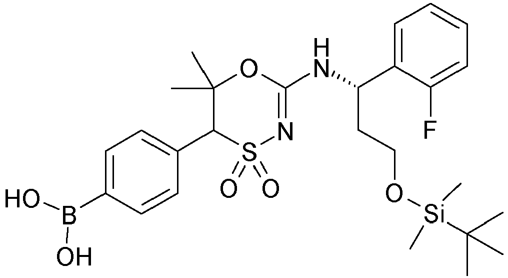

Zu einer Lösung von [(S)-3-(tert-Butyl-dimethyl-silanyloxy)-l-(2-fluor-phenyl)-propyl]-{6,6-dimethyl-4,4-dioxo-5-[4-(4,4,5,5-tetramethyl-[1,3,2]dioxaborolan-2-yl)-phenyl]-5,6-dihydro-4H-4lambda*6*-[1,4,3]oxathiazin-2-yl}-amin (1,15 g) in Aceton/Wasser 1:1 (24 mL) wurde bei Raumtemperatur Ammoniumacetat (308 mg) und Natriumperiodat (859 mg) gegeben und die Mischung wurde für 16 h gerührt. Nach dem Verdünnen mit Ethylacetat wurde mit Wasser und Natriumchloridlösung gewaschen, über Magnesiumsulfat getrocknet und eingeengt. Anschließend wurde säulenchromatographisch (Ethylacetat / Heptan) gereinigt. Man erhielt so das Produkt (567 mg) mit dem Molekulargewicht 564.6 g / mol (C₂₆H₃₈BFN₂O₆SSi),

Analog wurde folgendes Intermediat hergestellt:
[5-(3-Boroxyphenyl)-6,6-dimethyl-4,4-dioxo-5,6-dihydro-4H-4lambda*6*-[1,4,3]oxathiazin-2-yl]-[(S)-1-(2-fluor-phenyl)-ethyl]-amin 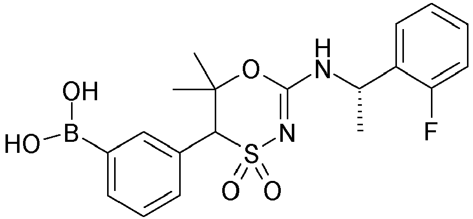
(S)-3-{6,6-Dimethyl-5-[4-(4-methyl-piperazin-1-yl)-phenyl]-4,4-dioxo-5,6-dihydro-4H-4lambda*6*-[1,4,3]oxathiazin-2-ylamin}-3-(2-fluor-phenyl)-propan-l-ol

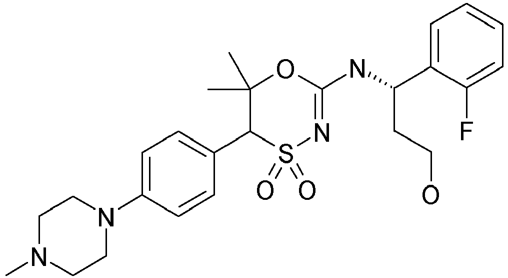

Zu einer Lösung von [(S)-3-(tert-Butyl-dimethyl-silanyloxy)-1-(2-fluor-phenyl)-propyl]-{6,6-dimethyl-4,4-dioxo-5-[4-(4,4,5,5-tetramethyl-[1,3,2]dioxaborolan-2-yl)-phenyl]-5,6-dihydro-4H-4lambda*6*-[1,4,3]oxathiazin-2-yl}-amin (270 mg) in Pyridin wurden Kupfer(II)-acetat (76 mg) und Molekularsieb 0,4 nm gegeben und die Mischung wurde für 10 min gerührt. Anschließend wurde N-Methylpiperazin (63 mg) gegeben und die Mischung wurde bei 80 °C für 16 h gerührt. Nach dem Verdünnen mit Ethylacetat wurde mit Natriumbicarbonatlösung und Natriumchloridlösung gewaschen, über Magnsiumsulfat getrocknet und eingeengt. Der Rückstand wurde in Methanol (5 mL) gelöst und mit conc. HCl (0,5 mL) behandelt. Nach 16 h wurde die Lösung im Reinigungslabor über präparative HPLC gereinigt. Man erhielt so das Produkt (61 mg als HCl Salz nach Behandeln mit Salzsäure) mit dem Molekulargewicht 504.2 g / mol (C₂₅H₃₃FN₄O₄S); MS (ESI): m / e = 505 (M+H⁺).

Analog wurden folgende Verbindungen erhalten:
(S)-3-{6,6-Dimethyl-4,4-dioxo-5-[4-(pyrimidin-2-ylamino)-phenyl]-5,6-dihydro-4H-4lambda*6*-[1,4,3]oxathiazin-2-ylamino}-3-(2-fluor-phenyl)-propan-1-ol 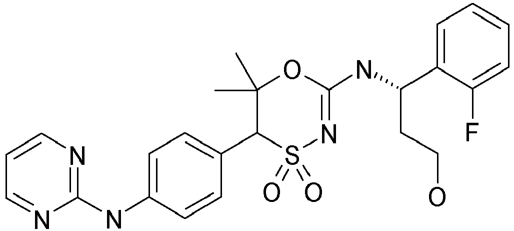
(S)-3-[6,6-Dimethyl-4,4-dioxo-5-(4-piperazin-1-yl-phenyl)-5,6-dihydro-4H-4lambda*6*-[1,4,3]oxathiazin-2-ylamino]-3-(2-fluor-phenyl)-propan-1-ol 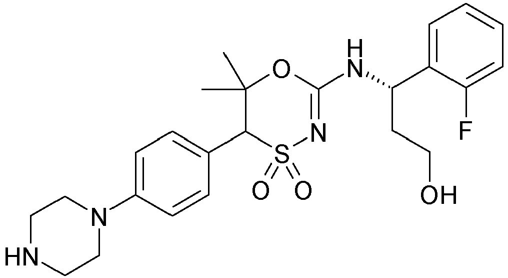
[6,6-Dimethyl-4,4-dioxo-5-(4-piperazin-1-yl-phenyl)-5,6-dihydro-4H-4lambda*6*-[1,4,3]oxathiazin-2-yl]-[(S)-1-(2-fluor-phenyl)-ethyl]-amin 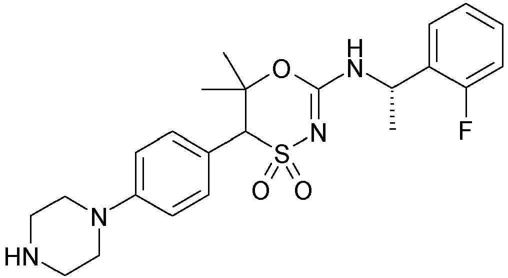
{6,6-Dimethyl-5-[4-(4-methyl-piperazin-1-yl)-phenyl]-4,4-dioxo-5,6-dihydro-4H-4lambda*6*-[1,4,3]oxathiazin-2-yl}-[(S)-1-(2-fluor-phenyl)-ethyl]-amin 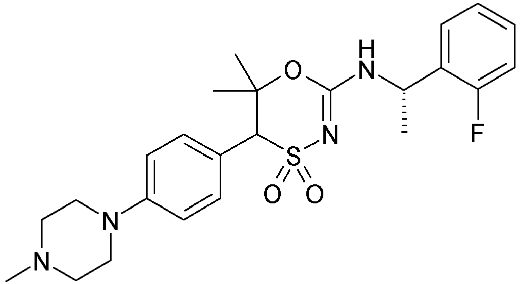
1-(4-{2-[(S)-1-(2-Fluor-phenyl)-3-hydroxy-propylamino]-6,6-dimethyl-4,4-dioxo-5,6-dihydro-4H-4lambda*6*-[1,4,3]oxathiazin-5-yl}-phenyl)-pyrrolidin-2-on 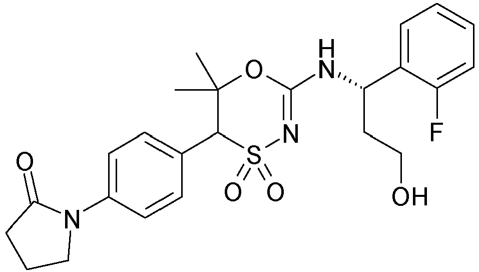
(S)-3-(2-Fluor-phenyl)-3-(5-{4-[4-(2-hydroxy-ethyl)-piperazin-1-yl]-phenyl}-6,6-dimethyl-4,4-dioxo-5,6-dihydro-4H-4lambda*6*-[1,4,3]oxathiazin-2-ylamino)-propan-1-ol 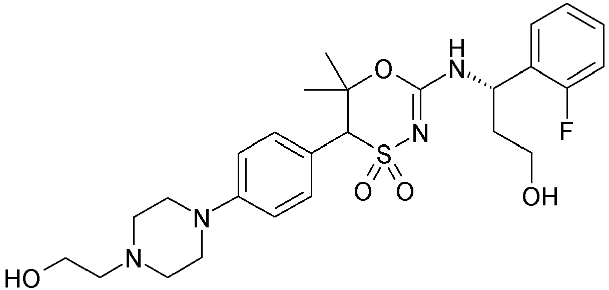
(S)-3-{6,6-Dimethyl-4,4-dioxo-5-[4-(2-piperazin-1-yl-ethoxy)-phenyl]-5,6-dihydro-4H-4lambda*6*-[1,4,3]oxathiazin-2-ylamino}-3-(2-fluor-phenyl)-propan-l-ol 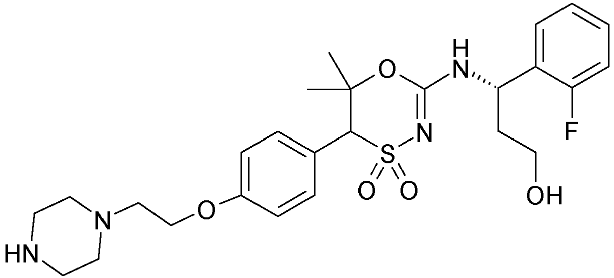
(S)-3-{5-[4-(4-tert-Butyl-piperazin-1-yl)-phenyl]-6,6-dimethyl-4,4-dioxo-5,6-dihydro-4H-4lambda*6*-[1,4,3]oxathiazin-2-ylamino}-3-(2-fluor-phenyl)-propan-1-ol 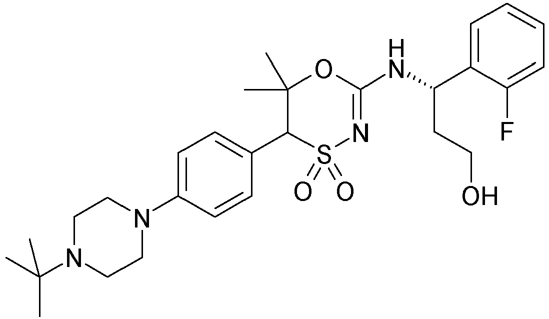
2-(4-{2-[(S)-1-(2-Fluor-phenyl)-3-hydroxy-propylamino]-6,6-dimethyl-4,4-dioxo-5,6-dihydro-4H-4lambda*6*-[1,4,3]oxathiazin-5-yl}-phenyl)-isoindole-1,3-dion 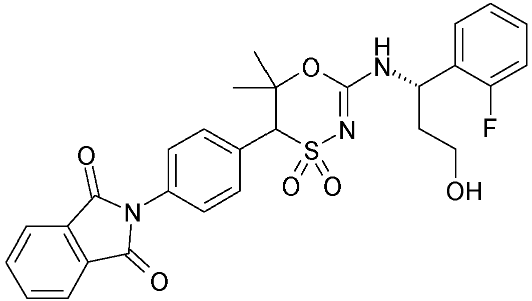
(S)-3-{5-[4-(1-Cyclopropyl-piperidin-4-ylamino)-phenyl]-6,6-dimethyl-4,4-dioxo-5,6-dihydro-4H-4lambda*6*-[1,4,3]oxathiazin-2-ylamino}-3-(2-fluor-phenyl)-propan-1-ol 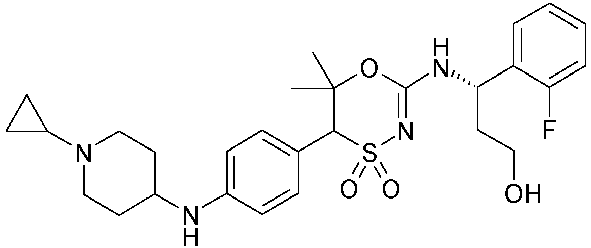
(6,6-Dimethyl-4,4-dioxo-5-{4-[4-(3,3,3-trifluor-propyl)-piperazin-1-yl]-phenyl}-5,6-dihydro-4H-4lambda*6*-[1,4,3]oxathiazin-2-yl)-[(S)-1-(2-fluor-phenyl)-ethyl]-amin 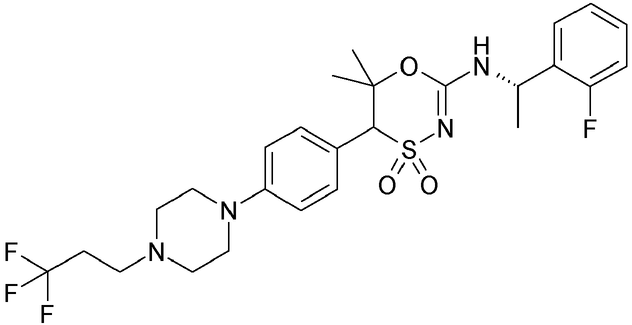 (S)-3-[6,6-Dimethyl-4,4-dioxo-5-(4-pyridin-2-yl-phenyl)-5,6-dihydro-4H-4lambda*6*-[1,4,3]oxathiazin-2-ylamino]-3-(2-fluor-phenyl)-propan-1-ol

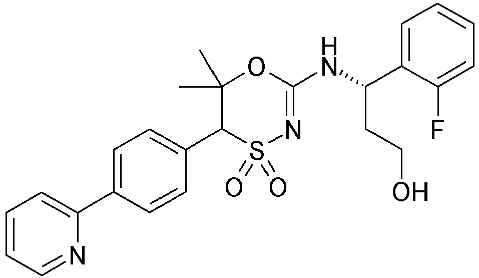

Zu einer Lösung von [(S)-3-(tert-Butyl-dimethyl-silanyloxy)-1-(2-fluor-phenyl)-propyl]-{6,6-dimethyl-4,4-dioxo-5-[4-(4,4,5,5-tetramethyl-[1,3,2]dioxaborolan-2-yl)-phenyl]-5,6-dihydro-4H-4lambda*6*-[1,4,3]oxathiazin-2-yl}-amin (300 mg) in Dioxan/Wasser 3:1 (4 mL) wurde 2-Brompyridin (92 mg), Pd(dba)₂ (27 mg) und CTC-Q-Phos (66 mg) gegeben und die Mischung wurde für 40 Minuten bei 62 °C gerührt. Nach dem Verdünnen mit Ethylacetat wurde mit Wasser gewaschen, über einer Kieselgurkartusche getrocknet und eingeengt. Anschließend wurde säulenchromatographisch (Ethylacetat / Heptan) gereinigt. Das noch silylierte Zwischenprodukt wurde in Methanol (2 mL) gelöst und mit conc. HCl (0,25 mL) behandelt. Nach 2 h wurde die Lösung im Reinigungslabor über präparative HPLC gereinigt. Man erhielt so das Produkt (129 mg als HCl Salz nach Behandeln mit Salzsäure) mit dem Molekulargewicht 483.6 g / mol (C₂₅H₂₆FN₃O₄S); MS (ESI): m / e = 484 (M+H⁺).

Analog wurde folgende Verbindung erhalten:
(S)-3-(6,6-Dimethyl-5-{4-[5-(4-methyl-piperazin-1-yl)-pyridin-2-yl]-phenyl}-4,4-dioxo-5,6-dihydro-4H-4lambda*6*-[1,4,3]oxathiazin-2-ylamino)-3-(2-fluor-phenyl)-propan-1-ol 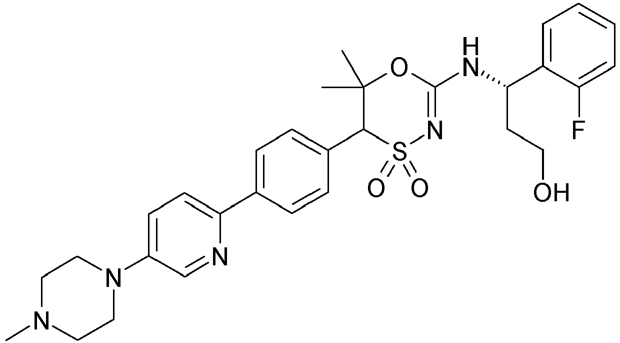
(S)-3-{5-[4-(1-tert-Butyl-piperidin-4-yl)-phenyl]-6,6-dimethyl-4,4-dioxo-5,6-dihydro-4H-4lambda*6*-[1,4,3]oxathiazin-2-ylamino}-3-(2-fluor-phenyl)-propan-1-ol 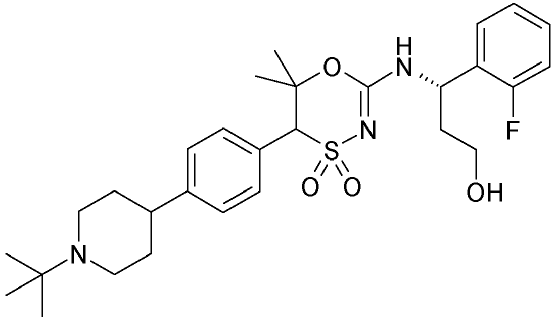
[(S)-3-(tert-Butyl-dimethyl-silanyloxy)-1-(2-fluor-phenyl)-propyl]-{5-[4-(1-tert-butyl-1,2,3,6-tetrahydro-pyridin-4-yl)-phenyl]-6,6-dimethyl-4,4-dioxo-5,6-dihydro-4H-4lambda* 6*-[1,4,3]oxathiazin-2-yl}-amin

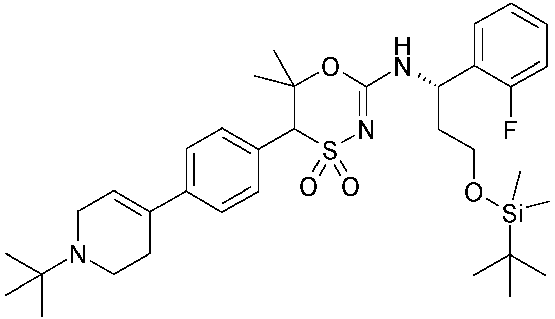

Zu einer Lösung von [(S)-3-(tert-Butyl-dimethyl-silanyloxy)-1-(2-fluor-phenyl)-propyl]-{6,6-dimethyl-4,4-dioxo-5-[4-(4,4,5,5-tetramethyl-[1,3,2]dioxaborolan-2-yl)-phenyl]-5,6-dihydro-4H-4lambda*6*-[1,4,3]oxathiazin-2-yl}-amin (300 mg), Trifluormethanesulfonsäure 1-tert-butyl-1,2,3,6-tetrahydro-pyridin-4-yl ester (208 mg) und Cäsiumcarbonat (519 mg) in Dioxan/Wasser 3:1 (4 mL) wurde Pd(dba)₂ (31 mg) und CTC-Q-Phos (75 mg) gegeben und die Mischung wurde für 40 min bei 62 °C gerührt. Nach dem Verdünnen mit Ethylacetat wurde mit Wasser gewaschen, über einer Kieselgurkartusche getrocknet und eingeengt. Anschließend wurde säulenchromatographisch (Ethylacetat / Heptan) gereinigt. Man erhielt so das Produkt (207 mg) mit dem Molekulargewicht 658.0 g / mol (C₃₅H₅₂FN₃O₄SSi),

### (S)-3-{5-[4-(1-tert-Butyl-piperidin-4-yl)-phenyl]-6,6-dimethyl-4,4-dioxo-5,6-dihydro-4H-4lambda*6*-[1,4,3]oxathiazin-2-ylamino}-3-(2-fluor-phenyl)-propan-1-ol

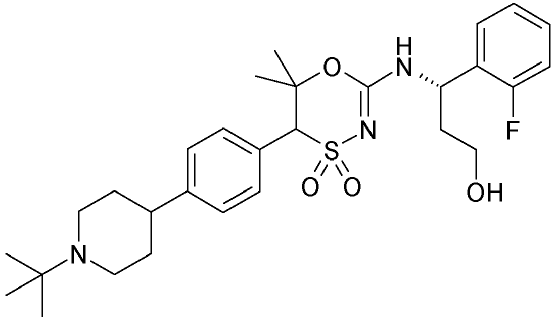

Zu einer Lösung von [(S)-3-(tert-Butyl-dimethyl-silanyloxy)-1-(2-fluor-phenyl)-propyl]-{5-[4-(1-tert-butyl-1,2,3,6-tetrahydro-pyridin-4-yl)-phenyl]-6,6-dimethyl-4,4-dioxo-5,6-dihydro-4H-4lambda*6*-[1,4,3]oxathiazin-2-yl}-amin (203 mg) in Methanol (10 mL) wurde Palladium auf Kohle (5%, 66 mg) gegeben und die Mischung wurde in einer Wasserstoffatmosphäre für 11 h gerührt. Die Mischung wurde filtriert und das Filtrat wurde eingeengt. Der Rückstand wurde in Methanol (10 mL) gelöst und mit conc. HCl (1 mL) behandelt. Nach 2 h wurde die Lösung im Reinigungslabor über präparative HPLC gereinigt. Man erhielt so das Produkt (69 mg als HCl Salz nach Behandeln mit Salzsäure) mit dem Molekulargewicht 545.7 g / mol (C₂₉H₄₀FN₃O₄S); MS (ESI): m / e = 546 (M+H⁺).

Analog wurdenfolgende Verbindungenrhalten:
(S)-3-{5-[4-(1-Cyclopropyl-piperidin-4-yl)-phenyl]-6,6-dimethyl-4,4-dioxo-5,6-dihydro-4H-4lambda*6*-[1,4,3]oxathiazin-2-ylamino}-3-(2-fluor-phenyl)-propan-1-ol 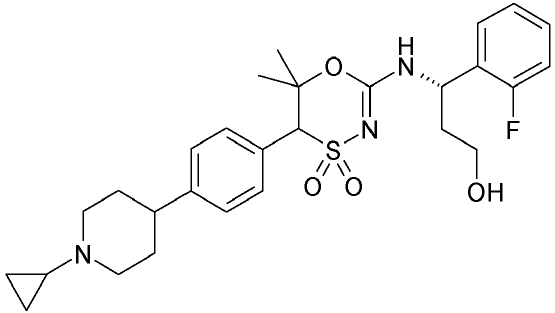
{5-[4-(1-Cyclopropyl-piperidin-4-yl)-phenyl]-6,6-dimethyl-4,4-dioxo-5,6-dihydro-4H-4lambda*6*-[1,4,3]oxathiazin-2-yl}-[(S)-1-(2-fluor-phenyl)-ethyl]-amin 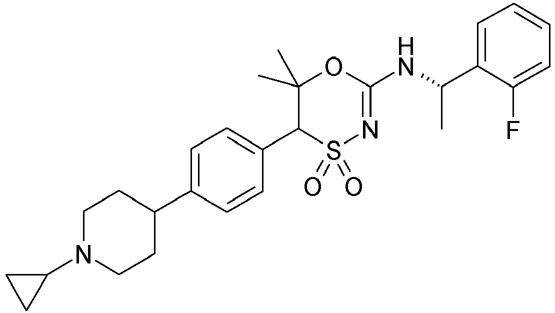
{5-[4-(1-tert-Butyl-piperidin-4-yl)-phenyl]-6,6-dimethyl-4,4-dioxo-5,6-dihydro-4H-4lambda*6*-[1,4,3]oxathiazin-2-yl}-[(S)-1-(2-fluor-phenyl)-ethyl]-amin 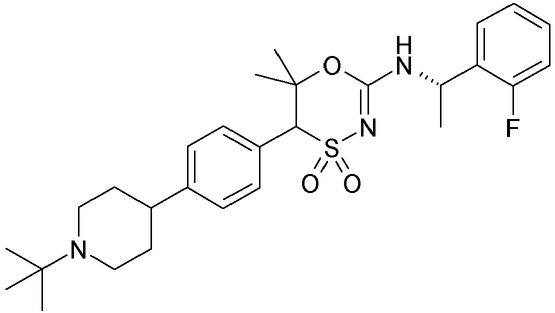
(S)-3-[6,6-Dimethyl-5-(4-morpholin-4-ylmethyl-phenyl)-4,4-dioxo-5,6-dihydro-4H-4lambda*6*-[1,4,3]oxathiazin-2-ylamino]-3-(2-fluor-phenyl)-propan-1-ol 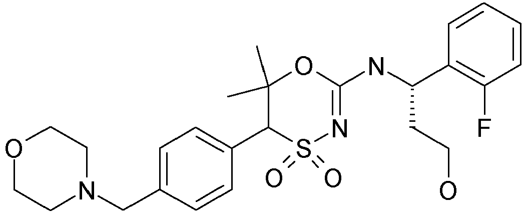
4-[(2,4-Dimethoxy-benzylsulfamoyl)-methyl]-benzoesäure methyl ester

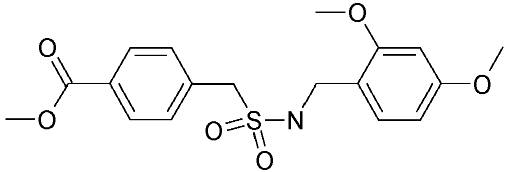

Unter Inertgas wurde 10 g 4-[(chlorsulfonyl)methyl]benzoesäuremethylester in 100 mL DCM vorgelegt, anschließend bei einer Temperatur von 0 °C 12.1 mL 2,4-Dimethoxybenzylamin zugetropft. Die Reaktionsmischung wurde filtriert und das Filtrat wurde mit 1 N HCl gewaschen. Die organische Phase wurde über MgSO₄ getrocknet und einrotiert. Man erhielt so das Produkt (14.2 g) mit dem Molekulargewicht 379.4 g / mol (C₁₈H₂₁NO₆S).

Analog wurde folgendes Intermediat synthetisiert:
3-[(2,4-Dimethoxy-benzylsulfamoyl)-methyl]-benzoesäuremethylester 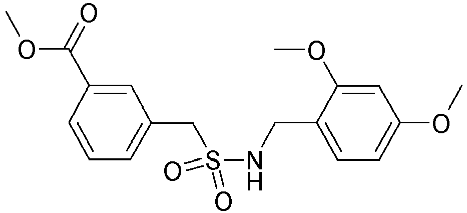
N-(2,4-Dimethoxy-benzyl)-C-[4-(morpholin-4-carbonyl)-phenyl]-methanesulfonamid

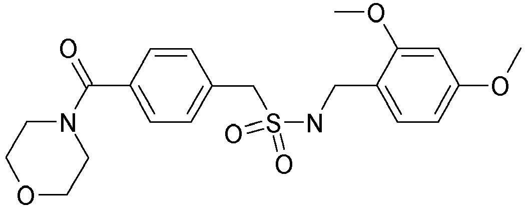

Zu einer Lösung von Morpholin (0.69 mL) in THF (10 mL) wurde bei -78 °C N-butyllithium (1.6 M, 3.3 mL) gegeben und die Lösung wurde für 10 min gerührt. Eine Lösung von 4-[(2,4-Dimethoxy-benzylsulfamoyl)-methyl]-benzoesäure methyl ester (1 g) wurde hinzugegeben und die Lösung wurde bis auf Raumtemperatur aufgewärmt und weitere 30 Minuten gerührt. Nach der Zugabe von Wasser (100 mL) wurde die Mischung mit Ethylacetat extrahiert (2 x 50 mL). Die organischen Phasen wurden über Magnesiumsulfat getrocknet und eingeengt. Anschließend wurde säulenchromatographisch (Ethylacetat / Heptan) gereinigt. Man erhielt so das Produkt (450 mg) mit dem Molekulargewicht 434.5 g / mol (C₂₁H₂₆N₂O₆S).

### N-(2,4-Dimethoxy-benzyl)-C-(4-morpholin-4-ylmethyl-phenyl)-methanesulfonamid

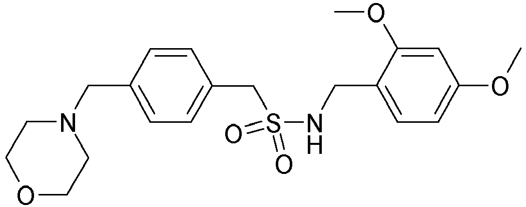

Zu einer Lösung von N-(2,4-Dimethoxy-benzyl)-C-[4-(morpholin-4-carbonyl)-phenyl]-methanesulfonamid (440 mg) in THF (5 mL) wurde bei 0 °C LAH (96 mg) gegeben und die Mischung wurde bis auf Raumtemperatur aufgewärmt und weitere 60 Minuten gerührt. Nach der Zugabe von Wasser (100 µL), Natronlauge (300 µL) und nochmals Wasser (300 µL) wurde die Mischung filtriert und das Filtrat eingeengt. Man erhielt so das Produkt (415 mg) mit dem Molekulargewicht 420.5 g / mol (C₂₁H₂₈N₂O₅S).

### 2-Hydroxy-2-methyl-1-(4-morpholin-4-ylmethyl-phenyl)-propan-1-sulfonamid

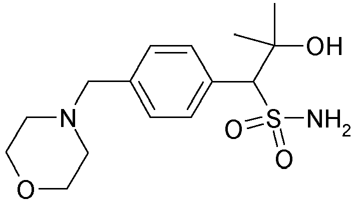

Unter Inertgas wurden 0.410 g N-(2,4-Dimethoxy-benzyl)-C-(4-morpholin-4-ylmethyl-phenyl)-methanesulfonamid in 5 ml THF vorgelegt und anschließend wurden bei -78 °C 1.34 ml einer 1.6 N Butyllithium-Lösung in Hexan tropfenweise zugegeben. Die Reaktionsmischung wurde für 5 Minuten gerührt und dann mit 0.29 mL Aceton tropfenweise versetzt. Die Mischung wurde 5 Minuten bei gleichbleibender Temperatur gerührt, mit 0.22 ml Trifluoressigsäure versetzt und auf Raumtemperatur kommen gelassen. Das Lösungsmittel wurde unter vermindertem Druck entfernt. Der Rückstand wurde in 10 ml Dichlormethan gelöst und mit 2 mL Trifluoressigsäure versetzt. Nach 16 h Rühren bei Raumtemperatur wurden 20 mL 1M HCl zugegeben und die wässrige Phase wurde abgetrennt und eingedampft. Man erhielt so das Rohprodukt mit dem Molekulargewicht 364.9 g / mol (C₁₅H₂₅N₂O₄S), das ohne weitere Reinigung eingesetzt wurde.

### 2-Methoxy-6,6-dimethyl-5-(4-morpholin-4-ylmethyl-phenyl)-5,6-dihydro-[1,4,3]oxathiazin 4,4-dioxid

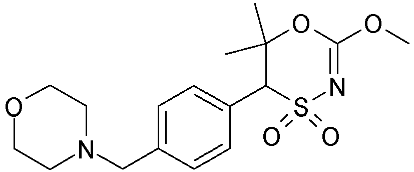

2-Hydroxy-2-methyl-1-(4-morpholin-4-ylmethyl-phenyl)-propan-1-sulfonamid (350 mg) wurde mit tetramethylorthocarbonat (5 mL) und Eisessig (1 mL) bei 80 °C für 16 Stunden gerührt. Die Lösungsmittel wurden im Vakuum entfernt und der Rückstand wurde ohne weitere Reinigung weiter eingesetzt.

### (S)-3-[6,6-Dimethyl-5-(4-morpholin-4-ylmethyl-phenyl)-4,4-dioxo-5,6-dihydro-4H-4lambda*6*-[1,4,3]oxathiazin-2-ylamino]-3-(2-fluor-phenyl)-propan-1-ol

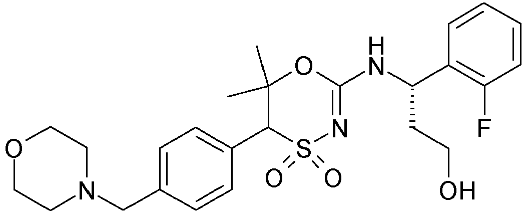

Zu einer Lösung von 2-Methoxy-6,6-dimethyl-5-(4-morpholin-4-ylmethyl-phenyl)-5,6-dihydro-[1,4,3]oxathiazin 4,4-dioxid in Dichlormethan (1 mL) wurde (S)-3-(tert-Butyl-dimethyl-silanyloxy)-1-(2-fluor-phenyl)-propylamin (283 mg) gegeben und das Lösungsmittel wurde verdampft. Der Rückstand wurde 16 h stehen gelassen und anschließend in Methanol (0.5 mL) gelöst und mit 0.05 mL conc. HCl versetzt. Die entstandene Mischung wurde im Reinigungslabor über präparative HPLC gereinigt. Man erhielt so das Produkt (230 mg als HCl Salz nach Behandeln mit Salzsäure) mit dem Molekulargewicht 505.2 g / mol (C₂₅H₃₂FN₃O₅S); MS (ESI): m / e = 506 (M+H⁺).

Analog wurde folgende Verbindung erhalten:
(S)-3-{6,6-Dimethyl-5-[4-(4-methyl-piperazin-1-ylmethyl)-phenyl]-4,4-dioxo-5,6-dihydro-4H-4lambda*6*-[1,4,3]oxathiazin-2-ylamino}-3-(2-fluor-phenyl)-propan-1-ol 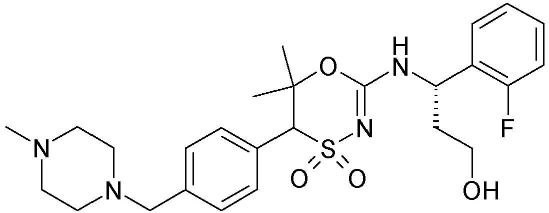
(S)-3-(6,6-Dimethyl-4,4-dioxo-5-{4-[4-(3,3,3-trifluoro-propyl)-piperazin-1-yl]-phenyl}-5,6-dihydro-4H-4lambda*6*-[1,4,3]oxathiazin-2-ylamino)-3-(2-fluor-phenyl)-propan-1-ol

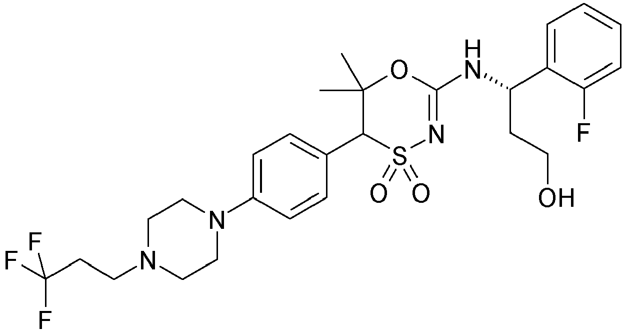

Zu einer Lösung von (S)-3-[6,6-Dimethyl-4,4-dioxo-5-(4-piperazin-1-yl-phenyl)-5,6-dihydro-4H-4lambda*6*-[1,4,3]oxathiazin-2-ylamino]-3-(2-fluor-phenyl)-propan-1-ol (73 mg) in DMF (1 mL) wurde 3-Brom-1,1,1-trifluorpropan (105 mg) gegeben und die Mischung wurde für 16 h bei 50 °C gerührt. Die entstandene Mischung wurde im Reinigungslabor über präparative HPLC gereinigt. Man erhielt so das Produkt (61 mg als HCl Salz nach Behandeln mit Salzsäure) mit dem Molekulargewicht 586.6 g / mol (C₂₇H₃₄F₄N₄O₄S); MS (ESI): m / e = 587 (M+H⁺).

### (S)-3-(6,6-Dimethyl-5-{4-[2-(4-methyl-piperazin-1-yl)-ethoxy]-phenyl}-4,4-dioxo-5,6-dihydro-4H-4lambda*6*-[1,4,3]oxathiazin-2-ylamino)-3-(2-fluor-phenyl)-propan-1-ol

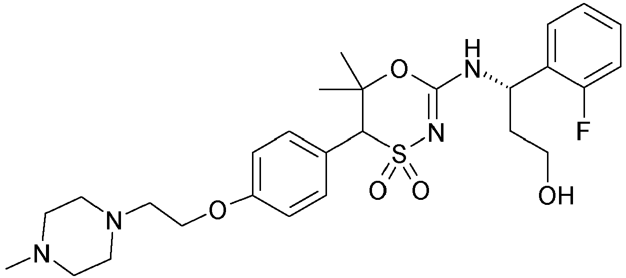

Zu einer Lösung aus (S)-3-{6,6-Dimethyl-4,4-dioxo-5-[4-(2-piperazin-1-yl-ethoxy)-phenyl]-5,6-dihydro-4H-4lambda*6*-[1,4,3]oxathiazin-2-ylamino}-3-(2-fluor-phenyl)-propan-1-ol (26 mg) in 1.3 mL THF/Essigsäure 4:1 wurde Paraformaldehyd (17 mg) und polymergebundenes Natriumcyanoborhydrid (56 mg) gegeben und die Mischung wurde über Nacht bei Raumtemperatur gerührt. Nach dem Filtrieren und Einrotieren wurde der Rückstand im Reinigungslabor über präparative HPLC gereinigt. Man erhielt so das Produkt (16 mg als HCl Salz nach Behandeln mit Salzsäure) mit dem Molekulargewicht 548.7 g / mol (C₂₇H₃₇FN₄O₅S); MS (ESI): m / e = 549 (M+H⁺).

### (S)-3-{6,6-Dimethyl-5-[4-(2-morpholin-4-yl-ethyl)-phenyl]-4,4-dioxo-5,6-dihydro-4H-4lambda*6*-[1,4,3]oxathiazin-2-ylamino}-3-(2-fluor-phenyl)-propan-1-ol

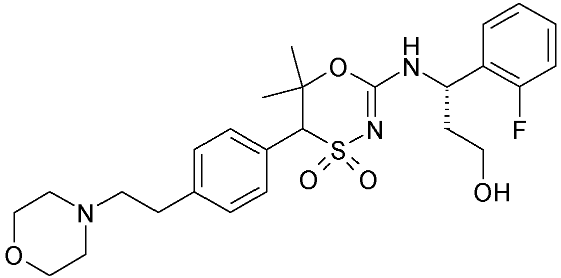

### N-(2,4-Dimethoxy-benzyl)-C-(4-hydroxymethyl-phenyl)-methanesulfonamid

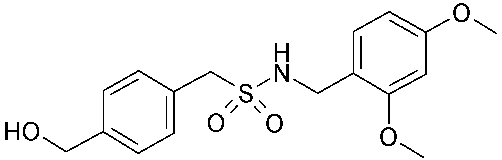

Zu einer Lösung von 4-[(2,4-Dimethoxy-benzylsulfamoyl)-methyl]-benzoesäuremethylester (6 g) in THF (300 mL) wurde bei 0 °C portionsweise Lithiumaluminiumhydrid (1,5 g) gegeben und die Mischung wurde für 1 h bei Raumtemperatur gerührt. Nacheinander wurde dann Wasser ( 9 mL), 6 M NaOH (7 mL) und nochmals Wasser (27 mL) zugegeben. Vom Feststoff wurde abfiltriert und zum Filtrat wurde Dichlormethan gegeben. Die organische Phase wurde abgetrennt, über Magnesiumsulfat getrocknet und eingeengt. Man erhielt so das Produkt (6 g) mit dem Molekulargewicht 351.4 g / mol (C₁₇H₂₁NO₅S),

### Methansulfonsäure 4-[(2,4-dimethoxy-benzylsulfamoyl)-methyl]-benzyl ester

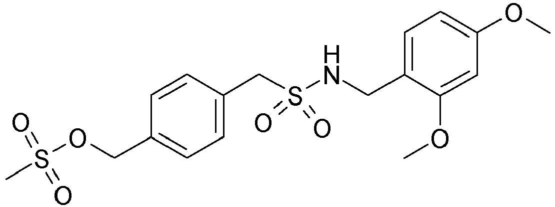

Zu einer Lösung von N-(2,4-Dimethoxy-benzyl)-C-(4-hydroxymethyl-phenyl)-methansulfonamid (6 g) wurde bei 0 °C Triethylamin (4,5 mL) und langsam Methansulfonsäurechlorid in Dichlormethan (2,2 mL in 50 mL) gegeben. Die Mischung wurde für 1,5 h gerührt und dann mit Ethylacetat verdünnt. Nach dem Waschen mit Wasser, 1 M Salzsäure und Natriumchloridlösung wurde die organische Phase über Magnesiumsulfat getrocknet und eingeengt. Man erhielt so das Produkt (7 g) mit dem Molekulargewicht 429.5 g / mol (C₁₈H₂₃NO₇S₂).

### C-(4-Cyanomethyl-phenyl)-N-(2,4-dimethoxy-benzyl)-methansulfonamid

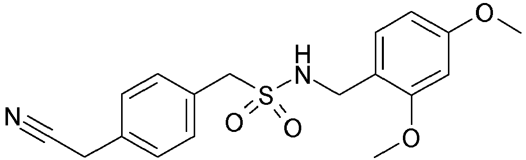

Zu einer Lösung von C-(4-Cyanomethyl-phenyl)-N-(2,4-dimethoxy-benzyl)-methansulfonamid (4,8 g) in DMF (30 mL) wurde Natriumcyanid (1,4 g) gegeben und die Mischung wurde bei 85 °C für 1,5 h gerührt. Die Mischung wurde in Wasser (400) gegeben. Nach der Extraktion mit Ethylacetat wurde die organische Phase mit Wasser und Natriumchloridlösung gewaschen, über Magnesiumsulfat getrocknet und eingeengt. Anschließend wurde säulenchromatographisch (Ethylacetat / Heptan) gereinigt. Man erhielt so das Produkt (1,8 g) mit dem Molekulargewicht 360.4 g / mol (C₁₈H₂₀N₂O₄S).

### {4-[(2,4-Dimethoxy-benzylsulfamoyl)-methyl]-phenyl}-essigsäure

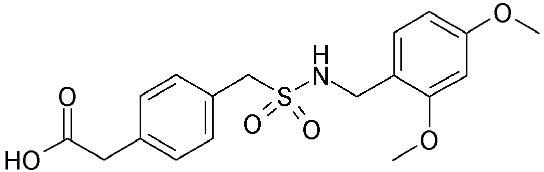

Zu einer Suspension von C-(4-Cyanomethyl-phenyl)-N-(2,4-dimethoxy-benzyl)-methansulfonamid (1,7 g) in Ethanol (50 mL) wurde 25% NaOH gegeben und die Mischung wurde bei 60 °C für 5 h gerührt. Nach dem Abkühlen wurde das organische Lösungsmittel abrotiert und die verbleibende Lösung wurde mit Diethylether gewaschen. Die wässrige Phase wurde mit conc. HCl leicht angesäuert und anschließend mit Ethylacetat extrahiert. Die organische Phase wurde über Magnsiumsulfat getrocknet und eingeengt. Man erhielt so das Produkt (1 g) mit dem Molekulargewicht 379.4 g / mol (C₁₈H₂₁NO₆S),

### N-(2,4-Dimethoxy-benzyl)-C-[4-(2-morpholin-4-yl-2-oxo-ethyl)-phenyl]-methansulfonamid

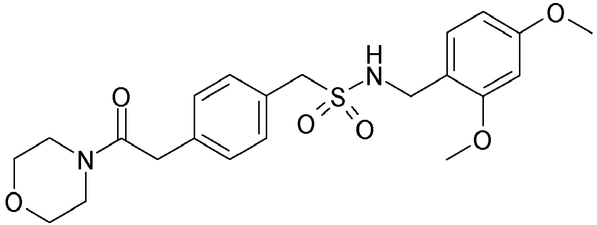

Zu einer Lösung von {4-[(2,4-Dimethoxy-benzylsulfamoyl)-methyl]-phenyl}-essigsäure (356 mg) in DMF (15 mL) wurde Triethylamin (237 mg), Morpholin (97 mg) und HATU (392 mg) gegeben. Die Mischung wurde bei Raumtemperatur für 17 h gerührt, anschließend in Wasser (200 mL) gegeben und mit Ethylacetat extrahiert. Nach dem Waschen mit Wasser, 1 M Salzsäure, Natriumhydrogencarbonatlösung und Natriumchloridlösung wurde die organische Phase über Magnesiumsulfat getrocknet und eingeengt. Man erhielt so das Produkt (313 mg) mit dem Molekulargewicht 448.5 g / mol (C₂₂H₂₈N₂O₆S),

### N-(2,4-Dimethoxy-benzyl)-C-[4-(2-morpholin-4-yl-ethyl)-phenyl]-methansulfonamid

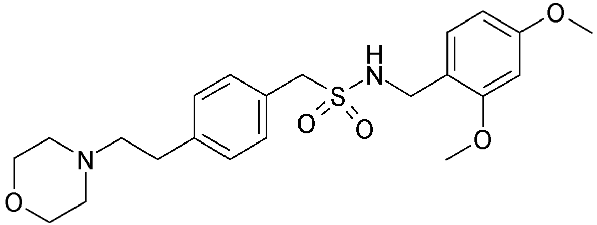

Zu einer Lösung von N-(2,4-Dimethoxy-benzyl)-C-[4-(2-morpholin-4-yl-2-oxo-ethyl)-phenyl]-methansulfonamid (313 mg) in THF (10 mL) wurde bei 0 °C portionsweise Lithiumaluminiumhydrid (66 mg) gegeben und die Mischung wurde für 1 h bei Raumtemperatur gerührt. Nacheinander wurde dann Wasser (0,4 mL), 6 M NaOH (0,3 mL) und nochmals Wasser (1,2 mL) zugegeben. Vom Feststoff wurde abfiltriert und zum Filtrat wurde Dichlormethan gegeben. Die organische Phase wurde abgetrennt, über Magnesiumsulfat getrocknet und eingeengt. Das Rohprodukt wurde über präparative HPLC gereinigt Man erhielt so das Produkt (57 mg) mit dem Molekulargewicht 434.6 g / mol (C₂₂H₃₀N₂O₅S).

### 2-Hydroxy-2-methyl-1-[4-(2-morpholin-4-yl-ethyl)-phenyl]-propan-1-sulfonamid

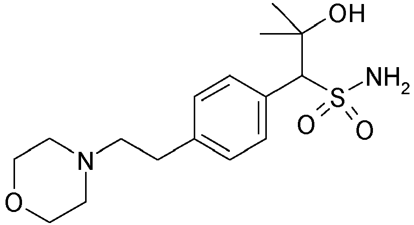

Eine Lösung von N-(2,4-Dimethoxy-benzyl)-C-[4-(2-morpholin-4-yl-ethyl)-phenyl]-methansulfonamid (50 mg) in THF (1 mL) wurde vorgelegt und anschließend wurde bei -78 °C 1.6 N Butyllithium-Lösung in Hexan (0,3 mL) tropfenweise zugegeben. Die Reaktionsmischung wurde für 10 Minuten gerührt und dann mit Aceton (50 µL) tropfenweise versetzt. Die Mischung wurde 30 Minuten bei gleichbleibender Temperatur gerührt, mit Trifluoressigsäure (40 µL) versetzt und auf Raumtemperatur kommen gelassen. Das Lösungsmittel wurde unter vermindertem Druck entfernt. Der Rückstand wurde in 2 ml Dichlormethan gelöst und mit 250 µL Trifluoressigsäure versetzt. Nach 2 h Rühren bei Raumtemperatur wurde mit Dichlormethan verdünnt und die Mischung wurde mit 1 M Salzsäure extrahiert. Die wässrige Phase wurde eingedampft und der Rückstand wurde mit Toluol coevaporiert. Man erhielt so das Rohprodukt mit dem Molekulargewicht 378.9 g / mol (C₁₆H₂₆N₂O₄S), das ohne weitere Reinigung eingesetzt wurde.

### 2-Methoxy-6,6-dimethyl-5-[4-(2-morpholin-4-yl-ethyl)-phenyl]-5,6-dihydro-[1,4,3]oxathiazin 4,4-dioxid

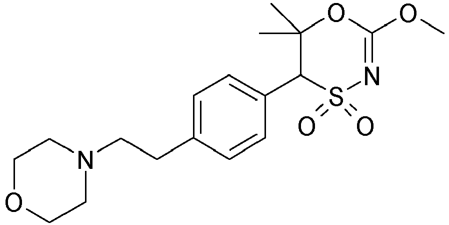

2-Hydroxy-2-methyl-1-[4-(2-morpholin-4-yl-ethyl)-phenyl]-propan-1-sulfonamid (44 mg) wurde mit Tetramethylorthocarbonat (1 mL) und Eisessig (0,2 mL) bei 80 °C für 17 Stunden gerührt. Die Lösungsmittel wurden im Vakuum entfernt. Man erhielt so das Rohprodukt mit dem Molekulargewicht 382.5 g / mol (C₁₈H₂₆N₂O₅S), das ohne weitere Reinigung eingesetzt wurde.

### (S)-3-{6,6-Dimethyl-5-[4-(2-morpholin-4-yl-ethyl)-phenyl]-4,4-dioxo-5,6-dihydro-4H-4lambda*6*-[1,4,3]oxathiazin-2-ylamino}-3-(2-fluor-phenyl)-propan-1-ol

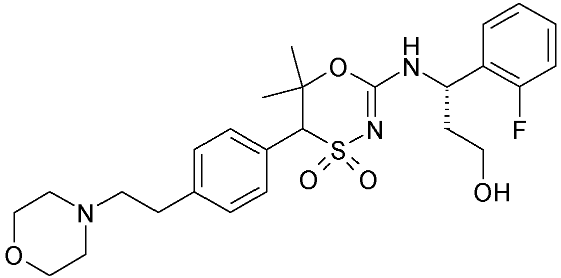

Zu einer Lösung von 2-Methoxy-6,6-dimethyl-5-[4-(2-morpholin-4-yl-ethyl)-phenyl]-5,6-dihydro-[1,4,3]oxathiazin 4,4-dioxid (19 mg) in Dichlormethan (1 mL) wurde (S)-3-(tert-Butyl-dimethyl-silanyloxy)-1-(2-fluor-phenyl)-propylamin (15 mg) gegeben und das Lösungsmittel wurde verdampft. Der Rückstand wurde 2,5 Tage stehen gelassen und anschließend in Methanol (1 mL) gelöst und mit 0,1 mL conc. HCl versetzt. Die entstandene Mischung wurde im Reinigungslabor über präparative HPLC gereinigt. Man erhielt so das Produkt (2,7 mg als HCl Salz nach Behandeln mit Salzsäure) mit dem Molekulargewicht 519.6 g / mol (C₂₆H₃₄FN₃O₅S); MS (ESI): m / e = 520 (M+H⁺).

Analog wurde folgende Verbindung erhalten:
(S)-3-(6,6-Dimethyl-5-{4-[2-(4-methyl-piperazin-1-yl)-ethyl]-phenyl}-4,4-dioxo-5,6-dihydro-4H-4lambda*6*-[1,4,3]oxathiazin-2-ylamino)-3-(2-fluor-phenyl)-propan-1-ol 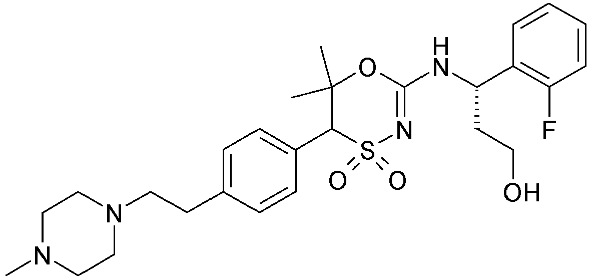
(S)-3-(2-Fluor-phenyl)-3-{5-[4-(1-hydroxy-1-methyl-ethyl)-phenyl]-6,6-dimethyl-4,4-dioxo-5,6-dihydro-4H-4lambda*6*-[1,4,3]oxathiazin-2-ylamino}-propan-1-ol 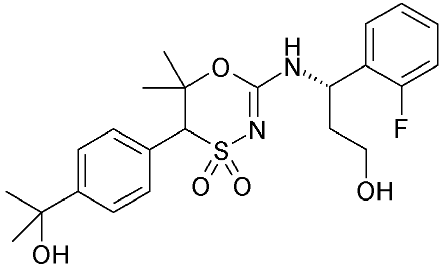
[5-(4-Boroxyphenyl)-6,6-dimethyl-4,4-dioxo-5,6-dihydro-4H-4lambda*6*-[1,4,3]oxathiazin-2-yl]-[(S)-3-(tert-butyl-dimethyl-silanyloxy)-1-(2-fluor-phenyl)-propyl]-amin

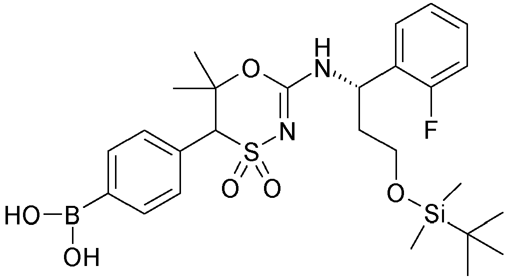

Zu einer Lösung von [(S)-3-(tert-Butyl-dimethyl-silanyloxy)-1-(2-fluoro-phenyl)-propyl]-{6,6-dimethyl-4,4-dioxo-5-[4-(4,4,5,5-tetramethyl-[1,3,2]dioxaborolan-2-yl)-phenyl]-5,6-dihydro-4H-4lambda*6*-[1,4,3]oxathiazin-2-yl}-amin (1,7 g) in Aceton/Wasser 1:1 (36 mL) wurde Ammoniumacetat (463 mg) und Natriumperjodat (1,3 g) gegeben und die Mischung wurde bei Raumtemperatur für 16 h und anschließend bei 50 °C für 1 h gerührt. Die Mischung wurde mit Ethylacetat verdünnt, mit Wasser gewaschen, überr Magnesiumsulfat getrocknet und eingeengt. Anschließend wurde säulenchromatographisch (Ethylacetat / Heptan / Methanol) gereinigt. Man erhielt so das Produkt (1,1 g) mit dem Molekulargewicht 564.6 g / mol (C₂₆H₃₈BN₂O₆SSi).

### 1-(4-{2-[(S)-3-(tert-Butyl-dimethyl-silanyloxy)-1-(2-fluor-phenyl)-propylamino]-6,6-dimethyl-4,4-dioxo-5,6-dihydro-4H-4lambda*6*-[1,4,3]oxathiazin-5-yl}-phenyl)-ethanon

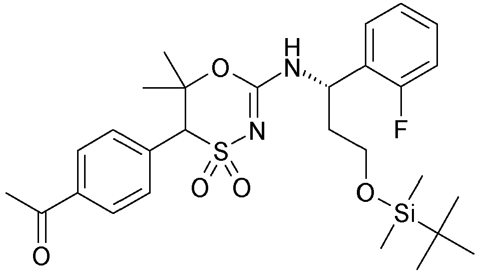

Zu einer Suspension von [5-(4-Boroxyphenyl)-6,6-dimethyl-4,4-dioxo-5,6-dihydro-4H-4lambda*6*-[1,4,3]oxathiazin-2-yl]-[(S)-3-(tert-butyl-dimethyl-silanyloxy)-1-(2-fluor-phenyl)-propyl]-amin (400 mg) und Kaliumacetat (140 mg) in Dioxan (6 mL) wurde Bis(triphenylphosphin)palladium(II) dichlorid (60 mg) und Acetylchlorid (120 µL) gegeben und die Mischung wurde über Nacht bei 50 °C gerührt. Die Mischung wurde mit Ethylacetat verdünnt, mit Wasser gewaschen, überr Magnesiumsulfat getrocknet und eingeengt. Anschließend wurde säulenchromatographisch (Ethylacetat / Heptan) gereinigt. Man erhielt so das Produkt (172 mg) mit dem Molekulargewicht 562.8 g / mol (C₂₈H₃₉FN₂O₅SSi).

Als weiteres Produkt wurde in dieser Reaktion erhalten:

### Essigsäure 4-{2-[(S)-3-(tert-butyl-dimethyl-silanyloxy)-1-(2-fluor-phenyl)-propylamino]-6,6-dimethyl-4,4-dioxo-5,6-dihydro-4H-4lambda*6*-[1,4,3]oxathiazin-5-yl}-phenyl ester

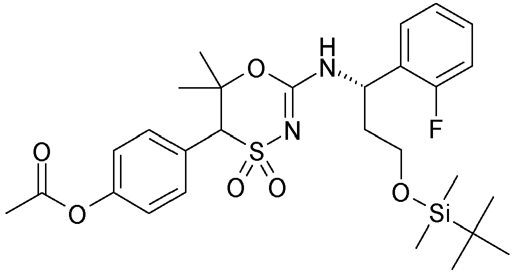

### (S)-3-(2-Fluor-phenyl)-3-{5-[4-(1-hydroxy-1-methyl-ethyl)-phenyl]-6,6-dimethyl-4,4-dioxo-5,6-dihydro-4H-4lambda*6*-[1,4,3]oxathiazin-2-ylamino}-propan-1-ol

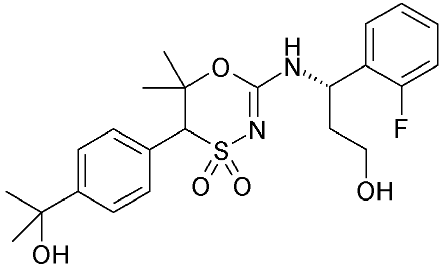

Zu einer Lösung von 1-(4-{2-[(S)-3-(tert-Butyl-dimethyl-silanyloxy)-1-(2-fluor-phenyl)-propylamino]-6,6-dimethyl-4,4-dioxo-5,6-dihydro-4H-4lambda*6*-[1,4,3]oxathiazin-5-yl}-phenyl)-ethanon (174 mg) in THF (2,5 mL) wurde bei -78 °C eine Lösung von Methylmagnesiumbromid (1.4 M, 0.44 mL) zugetropft. Die Mischung wurde nach 30 min bei - 78 °C mit Ammoniumchloridlösung versetzt. Nach der Zugabe von Wasser wurde mit Ethylacetat extrahiert, über Magnesiumsulfat getrocknet und eingeengt. Der Rückstand wurde in Methanol (2 mL) gelöst und mit 0.2 mL conc. HCl versetzt. Die entstandene Mischung wurde im Reinigungslabor über präparative HPLC gereinigt. Man erhielt so das Produkt (28 mg) mit dem Molekulargewicht 464.6 g / mol (C₂₃H₂₉FN₂O₅S); MS (ESI): m / e = 465 (M+H⁺).

Bei dieser Reaktion wurde ebenfalls isoliert:

### 1-(4-{2-[(S)-1-(2-Fluor-phenyl)-3-hydroxy-propylamino]-6,6-dimethyl-4,4-dioxo-5,6-dihydro-4H-4lambda*6*-[1,4,3]oxathiazin-5-yl}-phenyl)-ethanon

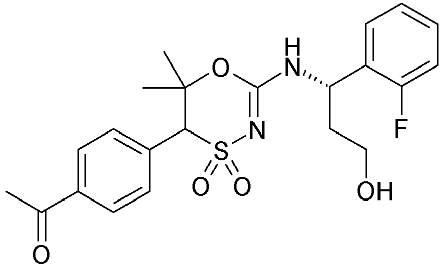

Analog wurden folgende Verbindungen erhalten:
2-(4-{2-[(S)-1-(2-Fluor-phenyl)-ethylamino]-6,6-dimethyl-4,4-dioxo-5,6-dihydro-4H-4lambda*6*-[1,4,3]oxathiazin-5-yl}-phenyl)-propan-2-ol 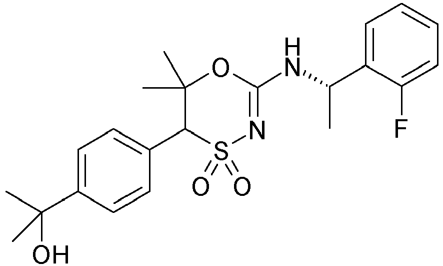
1-(4-{2-[(S)-1-(2-Fluor-phenyl)-ethylamino]-6,6-dimethyl-4,4-dioxo-5,6-dihydro-4H-4lambda*6*-[1,4,3]oxathiazin-5-yl}-phenyl)-ethanon

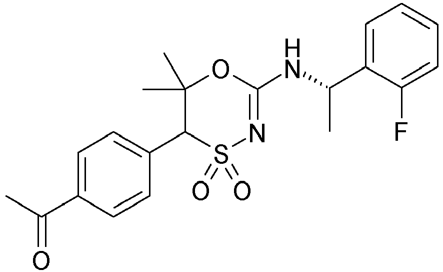

### Essigsäure 4-{2-[(S)-1-(2-fluor-phenyl)-3-hydroxy-propylamino]-6,6-dimethyl-4,4-dioxo-5,6-dihydro-4H-4lambda*6*-[1,4,3]oxathiazin-5-yl}-phenyl ester

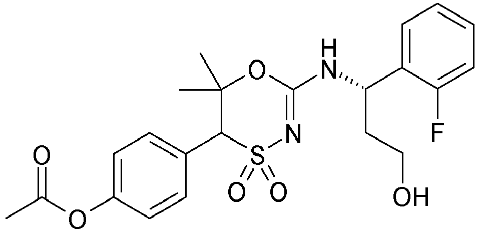

Diese Verbindung wurde durch saures Entschützen von Essigsäure 4-{2-[(S)-3-(tert-butyl-dimethyl-silanyloxy)-1-(2-fluor-phenyl)-propylamino]-6,6-dimethyl-4,4-dioxo-5,6-dihydro-4H-4lambda*6*-[1,4,3]oxathiazin-5-yl}-phenyl ester erhalten.

### (S)-3-(6,6-Dimethyl-4,4-dioxo-5-pyridin-3-yl-5,6-dihydro-4H-4lambda*6*-[1,4,3]oxathiazin-2-ylamino)-3-(2-fluor-phenyl)-propan-1-ol

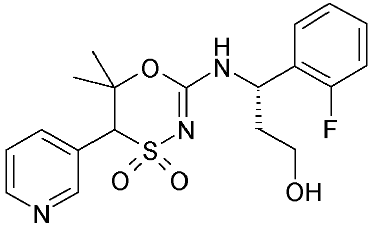

Zu einer Lösung von [(S)-3-(tert-Butyl-dimethyl-silanyloxy)-1-(2-fluoro-phenyl)-propyl]-[5-(6-chloro-pyridin-3-yl)-6,6-dimethyl-4,4-dioxo-5,6-dihydro-4H-4lambda*6*-[1,4,3]oxathiazin-2-yl]-amin (310 mg) in Methanol wurde palladium auf Kohle (5%, 119 mg) gegeben und die Mischung wurde für 19 h in einer Wasserstoffatmosphäre gerührt. Die Mischung wurde filtriert und das Filtrat wurde eingeengt. Der Rückstand wurde in Methanol (10 mL) gelöst und mit conc. HCl (1 mL) behandelt. Nach 2 h wurde die Lösung im Reinigungslabor über präparative HPLC gereinigt. Man erhielt so das Produkt (108 mg) mit dem Molekulargewicht 407.5 g / mol (C₁₉H₂₂FN₃O₄S); MS (ESI): m / e = 408 (M+H⁺).

Analog wurde folgende Verbindung erhalten:
(6,6-Dimethyl-4,4-dioxo-5-pyridin-3-yl-5,6-dihydro-4H-4lambda*6*-[1,4,3]oxathiazin-2-yl)-[(S)-1-(2-fluor-phenyl)-ethyl]-amin 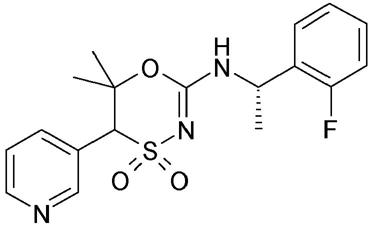
3-[(2,4-Dimethoxy-benzylsulfamoyl)-methyl]-benzoesäure

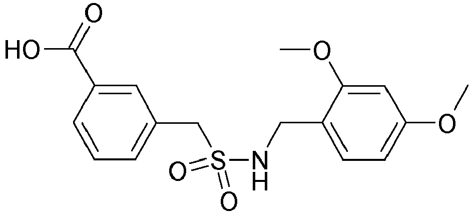

Zu einer Lösung von 3-[(2,4-Dimethoxy-benzylsulfamoyl)-methyl]-benzoesäure methyl ester (5 g) in Methanol (20 mL) wurde 2M NaOH (20 mL) gegeben und die Lösung wurde für 16 h bei Raumtemperatur gerührt. Das Methanol wurde unter vermindertem Druck entfernt und die wässrige Lösung wurde mit Ethylacetat gewaschen, mit HCl angesäuert und mit Ethylacetat extrahiert. Die organische Phase wurde über Magnesiumsulfat getrocknet und eingeengt. Man erhielt so das Produkt (4,1 g) mit dem Molekulargewicht 365.4 g / mol (C₁₇H₁₉NO₆S).

### 3-(2-Hydroxy-2-methyl-1-sulfamoyl-propyl)-benzoesäure

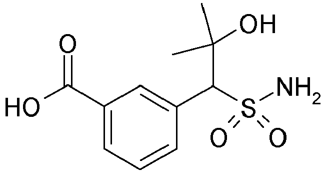

Zu einer Lösung von 3-[(2,4-Dimethoxy-benzylsulfamoyl)-methyl]-benzoesäure (4,1 g) in THF (50 mL) wurde bei - 78 °C Butyllithium (1,6 M, 25 mL) getropft. Die Mischung wurde für 10 min gerührt. Anschließend wurde Aceton (4,5 mL) zugegeben und die Mischung wurde für weitere 30 min bei - 78 °C gerührt. Die Reaktionsmischung wurde dann mit Trifluoressigsäure (4 mL) versetzt und eingeengt. Der Rückstand wurde in Dichlormethan suspendiert und und Trifluoressigsäure (10 mL) versetzt. Nach 2 h wurde Wasser zugegeben und filtriert. Die wässrige Phase wurde abgetrennt und gefriergetrocknet. Der Rückstand wurde über präparative HPLC gereinigt. Man erhielt so das Produkt (1,1 g) mit dem Molekulargewicht 273.3 g / mol (C₁₁H₁₅NO₅S).

### 3-(2-Methoxy-6,6-dimethyl-4,4-dioxo-5,6-dihydro-4H-4lambda*6*-[1,4,3]oxathiazin-5-yl) benzoesäure methyl ester

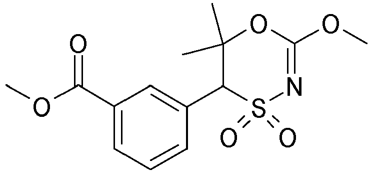

Eine Suspension von 3-(2-Hydroxy-2-methyl-1-sulfamoyl-propyl)-benzoesäure (1,1 g) in Tetramethylorthocarbonat (12 mL) und Essigsäure (60 µL) wurde für 16 h bei 85 °C gerührt. Die Mischung wurde eingeengt, mit Ethylacetat versetzt und mit Natriumbicarbonatlösung und mit Natriumchloridlösung gewaschen. Die organische Phase wurde über Magnesiumsulfat getrocknet und eingeengt. Man erhielt so das Produkt (1,3 g) mit dem Molekulargewicht 327.4 g/mol (C₁₄H₁₇NO₆S).

### 3-{2-[(S)-1-(2-Fluor-phenyl)-ethylamino]-6,6-dimethyl-4,4-dioxo-5,6-dihydro-4H-4lambda*6*-[1,4,3]oxathiazin-5-yl}-benzoesäure methyl ester

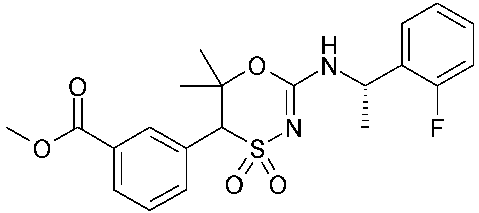

Eine Lösung von 3-(2-Methoxy-6,6-dimethyl-4,4-dioxo-5,6-dihydro-4H-4lambda*6*-[1,4,3]oxathiazin-5-yl) benzoesäure methyl ester (1,3 g) und (S)-1-(2-fluorophenyl)ethylamine (713 mg) in Dichlormethan (15 mL) wurde unter einem Argonstrom gerührt und so aufkonzentriert. Der Rückstand wurde für 4 h stehen gelassen, in Ethylacetat gelöst, mit 1 M HCl und Natriumbicarbonatlösung gewaschen, über Magnesiumsulfat getrocknet und eingeengt. Anschließend wurde säulenchromatographisch (Ethylacetat / Heptan) gereinigt. Man erhielt so das Produkt (1,2 g) mit dem Molekulargewicht 434.5 g / mol (C₂₁H₂₃FN₂O₅S).

### 3-{2-[(S)-1-(2-Fluor-phenyl)-ethylamino]-6,6-dimethyl-4,4-dioxo-5,6-dihydro-4H-4lambda*6*-[1,4,3]oxathiazin-5-yl}-benzoesäure

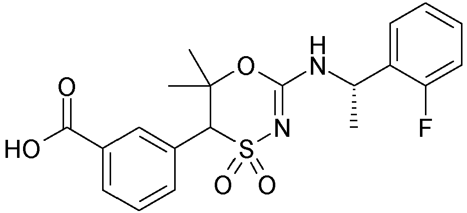

Zu einer Lösung von 3-{2-[(S)-1-(2-Fluor-phenyl)-ethylamino]-6,6-dimethyl-4,4-dioxo-5,6-dihydro-4H-4lambda*6*-[1,4,3]oxathiazin-5-yl}-benzoesäure methyl ester (1,7 mg) in Dioxan (15 mL) wurde Wasser (5 mL) und Schwefelsäure (0,3 mL) gegeben und die Mischung wurde für 16 h bei 100 °C gerührt. Die Mischung wurde mit Natriumcarbonatlösung alkalisch gestellt, mit Wasser verdünnt und mit Ethylacetat gewaschen. Die wässrige Phase wurde angesäuert, mit Ethylacetat extrahiert, über Magnesiumsulfat getrocknet und eingeengt. Man erhielt so das Produkt (551 mg) mit dem Molekulargewicht 420.5 g / mol (C₂₀H₂₁FN₂O₅S),

### (3-{2-[(S)-1-(2-Fluor-phenyl)-ethylamino]-6,6-dimethyl-4,4-dioxo-5,6-dihydro-4H-4lambda*6*-[1,4,3]oxathiazin-5-yl}-phenyl)-(4-methyl-piperazin-1-yl)-methanon

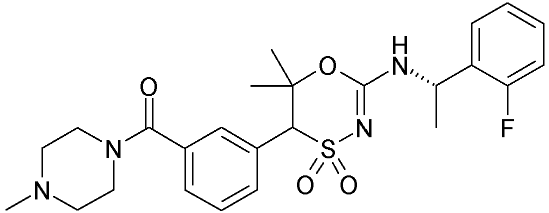

Zu einer Lösung von 3-{2-[(S)-1-(2-Fluor-phenyl)-ethylamino]-6,6-dimethyl-4,4-dioxo-5,6-dihydro-4H-4lambda*6*-[1,4,3]oxathiazin-5-yl}-benzoesäure (100 mg) in DMF (2,5 mL) wurde DIPEA (0,1 mL), N-Methylpiperazin (29 mg) und HATU (100 mg) gegeben. Die Mischung wurde für 1,5 h bei Raumtemperatur gerührt. Die Mischung wurde mit Ethylacetat verdünnt, mit Natriumhydrogencarbonatlösung und Natriumchloridlösung gewaschen, über Magnesiumsulfat getrocknet und eingeengt. Die entstandene Mischung wurde im Reinigungslabor über präparative HPLC gereinigt. Man erhielt so das Produkt (87 mg als HCl Salz nach Behandeln mit Salzsäure) mit dem Molekulargewicht 502.2 g / mol (C₂₅H₃₁FN₄O₄S); MS (ESI): m / e = 503 (M+H⁺).

Analog wurden folgende Verbindungen hergestellt:
(3-{2-[(S)-1-(2-Fluor-phenyl)-ethylamino]-6,6-dimethyl-4,4-dioxo-5,6-dihydro-4H-4lambda*6*-[1,4,3]oxathiazin-5-yl}-phenyl)-(4-methyl-[1,4]diazepan-1-yl)-methanon 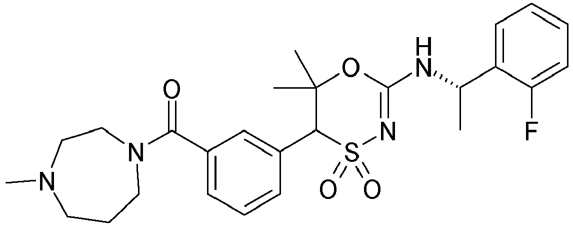
(3-{2-[(S)-1-(2-Fluor-phenyl)-ethylamino]-6,6-dimethyl-4,4-dioxo-5,6-dihydro-4H-4lambda*6*-[1,4,3]oxathiazin-5-yl}-phenyl)-morpholin-4-yl-methanon 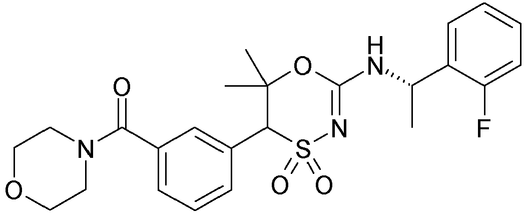
3-{2-[(S)-1-(2-Fluor-phenyl)-ethylamino]-6,6-dimethyl-4,4-dioxo-5,6-dihydro-4H-4lambda*6*-[1,4,3]oxathiazin-5-yl}-N-pyridin-2-ylmethyl-benzamid

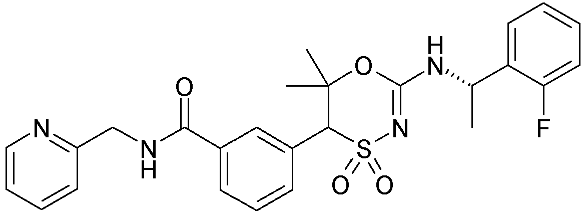

## Patentansprüche

1. Verbindungen der Formel I 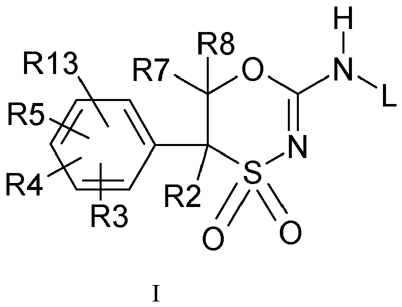 worin bedeuten
L R1, -CH(R10)(R11);
R10, R11 unabhängig voneinander H, F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅, (C₁-C₆)-Alkylen-(R6), (C₃-C₈)-Cycloalkylen-(R6), (C₁-C₆)-Alkylen-(C₃-C₈)-Cycloalkylen-(R6), (C₆-C₁₀)-Aryl, (C₁-C₆)-Alkylen-((C₆-C₁₀)-Aryl, wobei der Arylrest ein bis 3-fach substituiert sein kann mit F, Cl, Br, I, OH, CF₃, CHF, CH₂F, NO₂, CN, OCF₃, OCHF₂, O-(C₁-C₆)-Alkyl, (C₁-C₆)-Alkyl, NH, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)- Alkyl)₂, SF₅;
R1 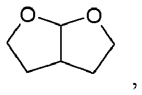 (C₆-C₁₀)-Aryl, (C₃-C₈)-Cycloalkyl, wobei der 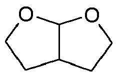 rest, Arylrest oder Cycloalkylrest ein bis 3-fach substituiert sein kann mit F, Cl, Br, I, OH, CF₃, CHF, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
R2 H, F, (C₁-C₃)-Alkyl wobei der Alkylrest ein bis 3-fach mit Fluor substituiert sein kann;
R3, R4, R5, R13 unabhängig voneinander H, F, Cl, Br, I, OH, CF₃, CHF, CH₂F, NO₂, CN, OCF₃, OCHF₂, -(C₁-C₆)-Alkylen-(R9), O-(C₁-C₆)-Alkylen-(R9), tertiär-Butyl, iso-Propylen-(R9), (C=O)-(C₁-C₆)-Alkylen-(R9), (C₁-C₆)-Alkylen-(R9), NH₂, NH(C₁-C₆)-Alkylen-(R9), N((C₁-C₆)-Alkylen-R9)₂, (C₁-C₆)-Alkylen-NH₂, (C₁-C₆)-Alkylen-NH(C₁-C₆)-Alkylen-(R9), (C₁-C₆)-Alkylen-N((C₁-C₆)-Alkylen-R9)₂, -O-(C₁-C₆)-Alkylen-NH₂, -O-(C₁-C₆)-Alkylen-NH(C₁-C₆)-Alkylen-(R9), -O-(C₁-C₆)-Alkylen-N((C₁-C₆)-Alkylen-R9)₂, -NH-(C₁-C₆)-Alkylen-NH₂, -NH-(C₁-C₆)-Alkylen-NH(C₁-C₆)-Alkylen-(R9), -NH-(C₁-C₆)-Alkylen-N((C₁-C₆)-Alkylen-R9)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;(C₆-C₁₀)-Aryl, -(C₁-C₆)-Alkylen-(C₆-C₁₀)-Aryl, , wobei der Arylrest ein bis 3-fach substituiert sein kann mit F, Cl, Br, I, OH, CF₃, CHF, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅, (C₆-C₁₀)-Aryl, (C₃-C₈)-Cycloalkyl, 4 bis12-gliedriger Heterocyclus; wobei der (C₆-C₁₀)-Arylrest, (C₃-C₈)-Cycloalkylrest, 4 bis12-gliedriger Heterocyclusrest ein bis 3-fach substituiert sein kann mit F, Cl, Br, I, OH, CF₃, CHF, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅; 4 bis 12-gliedriger Heterocyclus, -(C₁-C₆)-Alkylen-4 bis12-gliedriger Heterocyclus, wobei der Heterocyclylrest ein bis 3-fach substituiert sein kann mit F, Cl, Br, I, OH, CF₃, CHF, CH₂F, NO₂, CN, OCF₃, OCHF₂, O-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-(C₁-C₆)-Alkylen-(R9), SO₂-C₂H₂F₃, SO₂-CH₃, SO₂-NH₂, SO₂-NH(C₁-C₆)-Alkylen-(R9), SO₂-N((C₁-C₆)-Alkyl)₂, COOH, COO-(C₁-C₆)-Alkylen-(R9), CONH₂, CONH(C₁-C₆)-Alkylen-(R9), CON((C₁-C₆)-Alkyl)₂, SF₅, (C₆-C₁₀)-Aryl, (C₃-C₈)-Cycloalkyl, 4 bis12-gliedriger Heterocyclus; wobei der (C₆-C₁₀)-Arylrest, (C₃-C₈)-Cycloalkylrest, 4 bis12-gliedriger Heterocyclusrest ein bis 3-fach substituiert sein kann mit F, Cl, Br, I, OH, CF₃, CHF, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
R6 H, OH, CF₃, CHF, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, O-(CO)-NH₂, SF₅;
R7, R8 unabhängig voneinander H, (C₁-C₃)-Alkyl, wobei der Alkylrest ein bis 3-fach mit Fluor substituiert sein kann, oder R7 und R8 bilden gemeinsam mit dem Kohlenstoffatom an das sie gebunden sind einen 3-8 gliedrigen Carbocyclus oder Heterocyclus;
R9 H, OH, OCH₃, OCF₃, CHF, CF₃;
sowie deren pharmazeutisch verträgliche Salze.

2. Verbindungen der Formel I, gemäß Anspruch 1, **dadurch gekennzeichnet, dass** darin bedeuten
L R1, -CH(R10)(R11);
R10 F, Cl, Br, I, OH, CF₃, CHF, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅; (C₁-C₆)-Alkylen-(R6), (C₃-C₈)-Cycloalkylen-(R6), (C₁-C₆)-Alkylen-(C₃-C₈)-Cycloalkylen-(R6), (C₆-C₁₀)-Aryl, (C₁-C₆)-Alkylen-((C₆-C₁₀)-Aryl, wobei der Arylrest ein bis 3-fach substituiert sein kann mit F, Cl, Br, I, OH, CF₃, CHF, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)- Alkyl)₂, SF₅;
R11 H, F, Cl, Br, I, OH, CF₃, CHF₂, CH₂F, NO₂, CN, OCF₃, OCHF₂, O-(C₁-C₆)-Alkyl, (C₁-C₆)-Alkyl, NH, NH(C₁-C₆)-Alkyl, N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅; (C₁-C₆)-Alkylen-(R6), (C₃-C₈)-Cycloalkylen-(R6), (C₁-C₆)-Alkylen-(C₃-C₅)-Cycloalkylen-(R6), (C₆-C₁₀)-Aryl, (C₁-C₆)-Alkylen-((C₆-C₁₀)-Aryl; wobei der Arylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)- Alkyl)₂, SF₅;
R1 (C₃-C₈)-Cycloalkyl, wobei der Cycloalkylrest ein bis 3-fach substituiert sein kann mit F, Cl, Br, I, OH, CF₃, CHF, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
R2 H, F, (C₁-C₃)-Alkyl, wobei der Alkylrest ein bis 3-fach mit Fluor substituiert sein kann;
R3, R4, R5, R13 unabhängig voneinander H, F, Cl, Br, I, OH, CF₃, CHF₂, CH₂F, NO₂, CN, OCF₃, OCHF₂, O-(C₁-C₆)-Alkylen-(R9), -(C₁-C₆)-Alkylen-(R9), (C=O)-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅; (C₆-C₁₀)-Aryl, -(C₁-C₆)-Alkylen-(C₆-C₁₀)-Aryl, wobei der Arylrest ein bis 3-fach substituiert sein kann mit F, Cl, Br, I, OH, CF₃, CHF, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅; 4 bis 12-gliedriger Heterocyclus, -(C₁-C₆)-Alkylen-4 bis12-gliedriger Heterocyclus, wobei der Heterocyclylrest ein bis 3-fach substituiert sein kann mit F, Cl, Br, I, OH, CF₃, CHF, CH₂F, NO₂, CN, OCF₃, OCHF₂, O-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-(C₁-C₆)-Alkylen-(R9), SO₂-C₂H₂F₃, SO₂-CH₃, SO₂-NH₂, SO₂-NH(C₁-C₆)-Alkylen-(R9), SO₂-N((C₁-C₆)-Alkyl)₂, COOH, COO-(C₁-C₆)-Alkylen-(R9), CONH₂, CONH(C₁-C₆)-Alkylen-(R9), CON((C₁-C₆)-Alkyl)₂, SF₅, (C₆-C₁₀)-Aryl, (C₃-C₈)-Cycloalkyl, 4-12-gliedriger Heterocyclus;
R6 H, OH, O-(CO)-NH₂, SO₂NH₂;
R7, R8 unabhängig voneinander H, (C₁-C₃)-Alkyl, wobei der Alkylrest ein bis 3-fach mit Fluor substituiert sein kann;
R9 H, OH, OCH₃, OCF₃, CHF₂, CF₃;
sowie deren pharmazeutisch verträgliche Salze.

3. Verbindungen der Formel I, gemäß Anspruch 1 oder 2, **dadurch gekennzeichnet, dass** darin bedeuten
L R1, -CH(R10)(R11);
R10 (C₆-C₁₀)-Aryl, (C₁-C₆)-Alkylen-((C₆-C₁₀)-Aryl, wobei der Arylrest ein bis 3-fach substituiert sein kann mit F, Cl, Br, I, OH, CF₃, CHF, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)- Alkyl)₂, SF₅;
R11 F, Cl, Br, I, OH, CF₃, CHF, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅; (C₁-C₆)-Alkylen-(R6);
R1 (C₃-C₈)-Cycloalkyl, wobei der Cycloalkylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
R2 H, (C₁-C₃)-Alkyl;
R3, R4, R5, R13 unabhängig voneinander H, F, Cl, Br, I, OH, CF₃, CHF, CH₂F, NO₂, CN, OCF₃, OCHF₂, O-(C₁-C₆)-Alkylen-(R9), -(C₁-C₆)-Alkylen-(R9), (C=O)-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅; (C₆-C₁₀)-Aryl, -(C₁-C₆)-Alkylen-(C₆-Cₗₒ)-Aryl, , wobei der Arylrest ein bis 3-fach substituiert sein kann mit F, Cl, Br, I, OH, CF₃, CHF, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅; 4 bis 12-gliedriger Heterocyclus, -(C₁-C₆)-Alkylen-4 bis12-gliedriger Heterocyclus, wobei der Heterocyclylrest ein bis 3-fach substituiert sein kann mit F, Cl, Br, I, OH, CF₃, CHF, CH₂F, NO₂, CN, OCF₃, OCHF₂, O-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-(C₁-C₆)-Alkylen-(R9), SO₂-C₂H₂F₃, SO₂-CH₃, SO₂-NH₂, SO₂-NH(C₁-C₆)-Alkylen-(R9), SO₂-N((C₁-C₆)-Alkyl)₂, COOH, COO-(C₁-C₆)-Alkylen-(R9), CONH₂, CONH(C₁-C₆)-Alkylen-(R9), CON((C₁-C₆)-Alkyl)₂, SF₅, (C₆-C₁₀)-Aryl, (C₃-C₈)-Cycloalkyl, 4-12-gliedriger Heterocyclus;
R6 H, OH, O-(CO)-NH₂, SO₂NH₂;
R7, R8 unabhängig voneinander (C₁-C₃)-Alkyl;
R9 H, OH, OCH₃, OCF₃, CHF₂, CF₃;
sowie deren pharmazeutisch verträgliche Salze.

4. Verbindungen der Formel I, gemäß einem oder mehreren der Ansprüche 1 bis 3, **dadurch gekennzeichnet, dass** darin bedeuten
L R1, -CH(R10)(R11);
R10 Phenyl, wobei der Phenylrest ein bis 3-fach substituiert sein kann mit F, Cl, Br, - (C₁-C₆)-Alkyl;
R11 (C₁-C₆)-Alkyl, (C₁-C₆)-Alkylen-(R6);
R1 (C₃-C₈)-Cycloalkyl, wobei der Cycloalkylrest ein bis 3-fach substituiert sein kann mit F, Cl, Br, OH;
R2 H, (C₁-C₃)-Alkyl;
R3, R4, R5, R13 unabhängig voneinander H, F, Cl, Br, I, OH, CF₃, CHF, CH₂F, NO₂, CN, OCF₃, CHF₂, O-(C₁-C₆)-Alkylen-(R9), -(C₁-C₆)-Alkylen-(R9), (C=O)-(C1-C6)-Alkylen-(R9), (C₁-C₆)-Alkylen-(R9), NH₂, NH(C₁-C₆)-Alkylen-(R9), N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅; (C₆-C₁₀)-Aryl, -(C₁-C₆)-Alkylen-(C₆-C₁₀)-Aryl,, wobei der Arylrest ein bis 3-fach substituiert sein kann mit F, Cl, Br, I, OH, CF₃, CHF, CH₂F, NO₂, CN, OCF₃, CHF₂, O-(C₁-C₆)-Alkyl, (C₁-C₆)-Alkyl, NH, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅; 4 bis 12-gliedriger Heterocyclus, -(C₁-C₆)-Alkylen-4 bis12-gliedriger Heterocyclus, wobei der Heterocyclylrest ein bis 3-fach substituiert sein kann mit F, Cl, Br, I, OH, CF₃, CHF₂, CH₂F, NO₂, CN, OCF₃, OCHF₂, O-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-(C₁-C₆)-Alkylen-(R9), SO₂-C₂H₂F₃, SO₂-CH₃, SO₂-NH₂, SO₂-NH(C₁-C₆)-Alkylen-(R9), SO₂-N((C₁-C₆)-Alkyl)₂, COOH, COO-(C₁-C₆)-Alkylen-(R9), CONH₂, CONH(C₁-C₆)-Alkylen-(R9), CON((C₁-C₆)-Alkyl)₂, SF₅, (C₆-C₁₀)-Aryl, (C₃-C₈)-Cycloalkyl, 4-12-gliedriger Heterocyclus;
R6 OH;
R7, R8 unabhängig voneinander (C₁-C₃)-Alkyl;
R9 H, OH, OCH₃, OCF₃, CHF₂, CF₃;
sowie deren pharmazeutisch verträgliche Salze.

5. Verbindungen der Formel I, gemäß einem oder mehreren der Ansprüche 1 bis 4, zur Anwendung als Arzneimittel.

6. Arzneimittel enthaltend Verbindungen der Formel I, gemäß einem oder mehreren der Ansprüche 1 bis 4.

7. Arzneimittel enthaltend Verbindungen der Formel I, gemäß einem oder mehreren der Ansprüche 1 bis 4 und mindestens einen weiteren Wirkstoff.

8. Arzneimittel, gemäß Anspruch 7, **dadurch gekennzeichnet, dass** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, PPAR delta Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen Synthetase Inhibitoren, Lipoprotein(a) Antagonisten, HM74A Rezeptor Agonisten, Lipase Inhibitoren, Insuline, Sulfonylharnstoffe, Biguanide, Meglitinide, Thiazolidindione, α-Glukosidase-Inhibitoren, auf den ATP-abhängigen Kaliumkanal der Betazellen wirkende Wirkstoffe, Glykogen Phosphorylase Inhibitoren, Glukagon-Rezeptor-Antagonisten, Aktivatoren der Glukokinase, Inhibitoren der Glukoneogenese, Inhibitoren der Fructose-1,6-biphosphatase, Modulatoren des Glukosetransporters-4, Inhibitoren der Glutamin-Fructose-6-Phosphat-Amidotransferase, Inhibitoren der Dipeptidylpeptidase-IV, Hemmstoffe der 11-beta-Hydroxysteroid-Dehydrogenase-1, Inhibitoren der Protein-Tyrosin-Phosphatase-1B, Modulatoren des natrium-abhängigen Glukosetransporters 1 oder 2, Modulatoren des GPR40, Inhibitoren der hormon-sensitiven Lipase, Hemmstoffe der Acetyl-CoA Carboxylase, Inhibitoren der Phosphoenolpyruvatcarboxykinase, Inhibitoren der Glykogen Synthase Kinase-3 beta, Inhibitoren der Protein Kinase C beta, Endothelin-A-Rezeptor Antagonisten, Inhibitoren der I kappaB Kinase, Modulatoren des Glukocorticoidrezeptors, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, , CB1-Rezeptor Antagonisten ,MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten, Lipase/Amylase-Inhibitoren, PPAR-Modulatoren, RXR-Modulatoren oder TR-β-Agonisten oder Amphetamine enthält.

9. Verfahren zur Herstellung eines Arzneimittels enthaltend die Verbindungen der Formel I, gemäß einem oder mehreren der Ansprüche 1 bis 4, **dadurch gekennzeichnet, daß** der Wirkstoff mit einem pharmazeutisch geeigneten Träger vermischt wird und diese Mischung in eine für die Verabreichung geeignete Form gebracht wird.

10. Verwendung der Verbindungen der Formel I, gemäß einem oder mehreren der Ansprüche 1 bis 4 zur Herstellung eines Medikaments zur Behandlung von Hyperglykämie.

11. Verwendung der Verbindungen der Formel I, gemäß einem oder mehreren der Ansprüche 1 bis 4 zur Herstellung eines Medikaments zur Behandlung des Diabetes.

12. Verwendung der Verbindungen der Formel I, gemäß einem oder mehreren der Ansprüche 1 bis 4 zur Herstellung eines Medikaments zur Behandlung von Insulin Resistenz.

13. Set (Kit) bestehend aus getrennten Packungen von
a) einer wirksamen Menge einer Verbindung der Formel I, gemäß einem oder mehreren der Ansprüche 1 bis 4 und
b) einer wirksamen Menge eines weiteren Arzneimittelswirkstoffs.

## Claims

1. Compounds of the formula I 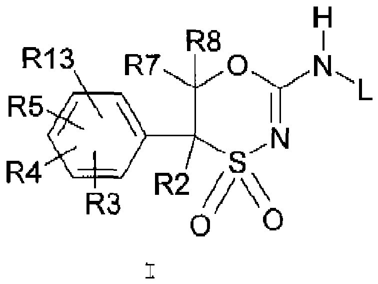 in which
L is R1, -CH(R10)(R11);
R10, R11 are each independently H, F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH (C₁-C₆) -alkyl, N((C₁-C₆)-alkyl)₂, COOH, COO-(C₁-C₆)-alkyl, CONH₂, CONH(C₁-C₆)-alkyl, CON((C₁-C₆)-alkyl)₂, SF₅, (C₁-C₆)-alkylene-(R6), (C₃-C₈)-cycloalkylene-(R6), (C₁-C₆)-alkylene-(C₃-C₈)-cycloalkylene-(R6), (C₆-C₁₀) -aryl, (C₁-C₆)-alkylene- ((C₆-C₁₀)-aryl; where the aryl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH_{z}, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
R1 is 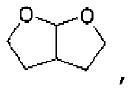 (C₆-C₁₀) -aryl, (C₃-C₈) -cycloalkyl, where the 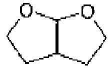 radical, aryl radical or cycloalkyl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
R2 is H, F, (C₁-C₃) -alkyl where the alkyl radical may be mono- to trisubstituted by fluorine;
R3, R4, R5, R13 are each independently H, F, Cl, Br, I, OH, CF₃, CHF₂, CH₂F, NO₂, CN, OCF₃, OCHF₂, -(C₁-C₆)-alkylene-(R9), O-(C₁-C₆)-alkylene-(R9), tert-butyl, isopropylene-(R9), (C=O)-(C₁-C₆)-alkylene-(R9), (C₁-C₆)-alkylene-(R9), NH₂, NH(C₁-C₆)-alkylene-(R9), N((C₁-C₆)-alkylene-R9) ₂, (C₁-C₆) -alkylene-NH₂, (C₁-C₆)-alkylene-NH(C₁-C₆)-alkylene-(R9), (C₁-C₆)-alkylene-N((C₁-C₆)-alkylene-R9)₂, -O-(C₁-C₆)-alkylene-NH₂, -O-(C₂-C₆)-alkylene-NH(C₁-C₆)-alkylene-(R9), -O-(C₁-C₆)-alkylene-N((C₁-C₆)-alkylene-R9) ₂, -NH-(C₁-C₆)-alkylene-NH₂, -NH-(C₁-C₆)-alkylene-NH(C₁-C₆)-alkylene-(R9), -NH-(C₁-C₅)-alkylene-N((C₁-C₆)-alkylene-R9)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅; (C₆-C₁₀)-aryl, - (C₁-C₆)-alkylene-(C₆-C₁₀)-aryl, where the aryl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅, (C₆-C₁₀)-aryl, (C₃-C₈)-cycloalkyl, 4- to 12-membered heterocycle; where the (C₆-C₁₀)-aryl radical, (C₃-C₈)-cycloalkyl radical, 4- to 12-membered heterocycle radical may be mono- to trisubstituted by F, Cl, Br, I, OH, CF₃, CHF₂, CH₂F, NO₂, CN, OCF₃, OCHF₂, O- (C₁-C₆) -alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂. SF₅; 4- to 12-membered heterocycle, -(C₁-C₆)-alkylene-4- to -12-membered heterocycle, where the heterocyclyl radical may be mono- to trisubstituted by F, Cl, Br, I, OH, CF₃, CHF₂, CH₂F, NO₂, CN, OCF₃, OCHF₂, O-(C₁-C₆)-alkylene-(R9), (C₁-C₆)-alkylene-(R9), NH₂, NH(C₁-C₆)-alkylene-(R9), N((C₁-C₆)-alkyl)₂, SO₂-(C₁-C₆)-alkylene-(R9), SO₂-C₂H₂F₃, SO₂-CH₃, SO₂-NH₂, SO₂-NH(C₁-C₆)-alkylene-(R9), SO₂-N((C₁-C₅)-alkyl)₂, COOH, COO-(C₁-C₆)-alkylene-(R9), CONH₂, CONH(C₁-C₆)-alkylene-(R9), CON ((C₁-C₆)-alkyl)₂, SF₅, (C₆-C₁₀)-aryl, (C₃-C₈)-cycloalkyl, 4- to 12-membered heterocycle; where the (C₆-C₁₀) -aryl radical, (C₃-C₈)-cycloalkyl radical, 4- to 12-membered heterocycle radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH (C₁-C₆) -alkyl, SO₂-N ((C₁-C₆)-alkyl)₂, COOH, COO-(C₁-C₆)-alkyl, CONH₂, CONH(C₁-C₆)-alkyl, CON ((C₁-C₅)-alkyl)SF₅;
R6 is H, OH, CF₃, CHF₂, CH₂F, NO₂, CN, OCF₃, OCHF₂, O-(C₁-C₆)-alkyle, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, O-(CO)-NH₂, SF₅;
R7, R8 are each independently H, (C₁-C₃)-alkyl, where the alkyl radical may be mono- to trisubstituted by fluorine, or R7 and R8 together with the carbon atom to which they are bonded form a 3-8-membered carbocycle or heterocycle;
R9 is H, OH, OCH₃, OCF₃, CHF₂, CF₃:
and pharmaceutically acceptable salts thereof.

2. Compounds of the formula I according to Claim 1, **characterized in that**
L is R1, -CH(R10)(R11);
R10 is F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, COOH, COO-(C₁-C₆)-alkyl, CONH₂, CONH(C₁-C₆)-alkyl, CON((C₁-C₅)-alkyl)₂, SF₅, (C₁-C₆)-alkylene- (R6), (C₃-C₈)-cycloalkylene-(R6), (C₁-C₆)-alkylene-(C₃-C₈)-cycloalkylene-(R6), (C₆-C₁₀)-aryl, (C₂-C₆)-alkylene-(C₆-C₁₀)-aryl; where the aryl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
R11 is H, F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, COOH, COO-(C₁-C₆)-alkyl, CONH₂, CONH(C₁-C₆)-alkyl, CON ((C₁-C₆)-alkyl)₂, SF₅, (C₁-C₆)-alkylene-(R6), (C₃-C₈)-cycloalkylene-(R6), (C₁-C₆)-alkylene-(C₃-C₈) -cycloalkylene- (R6), (C₆-C₁₀)-aryl, (C₁-C₆)-alkylene-(C₆-C₁₀)-aryl; where the aryl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH (C₁-C₆) -alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂ SF₅;
R1 is (C₃-C₈)-cycloalkyl, where the cycloalkyl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
R2 is H, F, (C₁-C₃)-alkyl where the alkyl radical may be mono- to trisubstituted by fluorine;
R3, R4, R5, R13 are each independently H, F, Cl, Br, I, OH, CF₃, CHF₂, CH₂F, NO₂, CN, OCF₃, OCHF₂, 0- (C₁-C₆) -alkylene-(R9), -(C₁-C₆)-alkylene-(R9), (C=O)-(C1-C6)-alkylene-(R9), (C₁-C₆)-alkylene-(R9), NH₂, NH(C₁-C₆)alkylene-(R9), N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅; (C₅-C₁₀)-aryl, -(C₁-C₆)-alkylene- (C₆-C₁₀)-aryl, where the aryl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO- (C₁-C₆) -alkyl, CONH₂, CONH(C₁-C₆)-alkyl, CON((C₁-C₆)-alkyl)₂, SF₅; 4- to 12-membered heterocycle, -(C₁-C₆)-alkylene-4- to -12-membered heterocycle, where the heterocyclyl radical may be mono- to trisubstituted by F, Cl, Br, I, OH, CF₃, CHF₂, CH₂F, NO₂, CN, OCF₃, OCHF₂, O- (C₁-C₆) -alkylene- (R9), (C₁-C₆)-alkylene-(R9), NH₂, NH (C₁-C₆)-alkylene-(R9), N((C₁-C₆)-alkyl)₂, SO₂-(C₁-C₆)-alkylene-(R9), SO₂-C₂H₂F₃, SO₂-CH₃, SO₂-NH₂, SO₂-NH(C₁-C₆)-alkylene-(R9), SO₂-N((C₁-C₆)-alkyl)₂, COOH, COO-(C₁-C₆)-alkylene-(R9), CONH₂, CONH(C₁-C₆)-alkylene-(R9), CON ((C₁-C₆) -alkyl)₂, SF₅, (C₆-C₁₀)-aryl, (C₃-C₈)-cycloalkyl, 4- to 12-membered heterocycle;
R6 is H, OH, O-(CO)-NH₂, SO₂NH₂;
R7, R8 are each independently H, (C₁-C₃)-alkyl where the alkyl radical may be mono- to trisubstituted by fluorine;
R9 is H, OH, OCH₃, OCF₃, CHF₂, CF₃;
and pharmaceutically acceptable salts thereof.

3. Compound of the formula I according to Claim 1 or 2, **characterized in that**
L is R1, -CH(R10)(R11);
R10 is (C₆-C₁₀) -aryl, (C₁-C₆) -alkylene- (C₆-C₁₀) -aryl; where the aryl radical may be mono- to trisubstituted by F, Cl, Br, I, OH, CF₃, CHF₂, CH₂F, NO₂, CN, OCF₃, OCHF₂, O- (C₁-C₆) -alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH (C₁-C₆)-alkyl, CON ((C₁-C₆)-alkyl₂, SF₅;
R11 is F, Cl, B, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, COOH, COO-(C₁-C₆)-alkyl, CONH₂, CONH(C₁-C₆)-alkyl, CON((C₁-C₆)-alkyl)₂, SF₅, (C₁-C₆)-alkylene-(R6);
R1 is (C₃-C₈)-cycloalkyl, where the cycloalkyl radical may be mono- to trisubstituted by F, Cl, B, I, OH, CF₃, CHF₂, CH₃F, NO₂, CN, OCF₃, OCHF₂, 0-(C₁-C₆)-alkyl, (C₁-C₆) -alkyl, NH₂, NH(C₁-C₆)-alkyl, N((C₁-C₆)-alkyl)₂, SO₂-CH₃, SO₂-NH₂, SO₂-NH(C₁-C₅) -alkyl, SO₂-N((C₁-C₆)-alkyl)₂, COOH, COO-(C₁-C₆)-alkyl, CONH₂, CONH(C₁-C₆)-alkyl, CON((C₁-C₆)alkyl)₂, SF₅;
R2 is H, (C₁-C₃)-alkyl;
R3, R4, R5, R13 are each independently H, F, Cl, Br, I, OH, CF₃, CHF₂, CH₂F, NO₂, CN, OCF₃, OCHF₂, O-(C₁-C₆)-alkylene-(R9),-(C₂-C₆) -alkylene-(R9) , (C=O)-(C1-C6)-alkylene-(R9), (C₁-C₆)-alkylene-(R9), NH₂, NH(C₁-C₆)-alkylene-(R9), N((C₁-C₆)-alkyl)₂, SO₂-CH₃, SO₂-NH₂, SO₂-NH(C₁-C₆)alkyl, SO₂-N((C₁-C₆)-alkyl)₂, COOH, COO-(C₁-C₆)-alkyl, CONH₂, CONH (C₁-C₆) -alkyl, CON((C₁-C₆)-alkyl)₂, SF₅; (C₆-C₁₀) -aryl, - (C₁-C₆) -alkylene- (C₆-C₁₀) -aryl, where the aryl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅; 4- to 12-membered heterocycle, -(C₁-C₆)-alkylene-4- to -12-menbered heterocycle, where the heterocyclyl radical may be mono- to trisubstituted by F, Cl, Br, I, OH, CF₃, CHF₂, CH₂F, NO₂, CN, OCF₃, OCHF₂, 0- (C₁-C₆) -alkylene- (R9), (C₁-C₆)-alkylene-(R9), NH₂, NH(C₁-C₆)-alkylene-(R9), N((C₁-C₆)-alkyl)₂, SO₂-(C₁-C₆)-alkylene-(R9), SO₂-C₂H₂F₃, SO₂-CH₃, SO₂-NH₂, SO₂-NH(C₁-C₆)- alkylene-(R9), SO₂-N((C₁-C₆)-alkyl)₂, COOH, COO-(C₁-C₆)-alkylene-(R9), CONH₂, CONH(C₁-C₆)-alkylene-(R9), CON ((C₁-C₆)-alkyl)₂, SF₅, (C₆-C₁₀)-aryl, (C₃-C₈)-cycloalkyl, 4- to 12-membered heterocycle;
R6 is H, OH, O-(CO)-NH₂, SO₂NH_{2;}
R7, R8 are each independently (C₁-C₃)-alkyl;
R9 is H, OH, OCH₃, OCF₃, CHF₂, CF₃;
and pharmaceutically acceptable salts thereof.

4. Compound of the formula I according to one or more of Claims 1 to 3, **characterized in that**
L is R1, -CH(R10)(R11);
R10 is phenyl, where the phenyl radical may be mono- to trisubstituted by F, Cl, Br, -(C₁-C₆)-alkyl;
R11 is (C₁-C₆)-alkyl, (C₁-C₈)-alkylene-(R6);
R1 is (C₃-C₈)-cycloalkyl, where the cycloalkyl radical may be mono- to trisubstituted by F, Cl, Br, OH;
R2 is H, (C₁-C₃)-alkyl;
R3, R4, R5, R13 are each independently H, F, Cl, Br, I, OH, CF₃, CHF₂, CH₂F, NO₂, CN, OCF₃, OCHF₂, O-(C₁-C₆-alkylene-(R9), -(C₁-C₆)-alkylene-(R9), (C=O)-(C1-C6)-alkylene-(R9), (C₁-C₆)-alkylene-(R9), NH₂, NH(C₁-C₆)-alkylene-(R9), N((C₁-C₆)-alkyl-)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅; (C₆-C₁₀))-aryl, -(C₁-C₆)-alkylene-(C₆-C₁₀)-aryl, where the aryl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅; 4- to 12-membered heterocycle, -(C₁-C₆)-alkylene-4- to -12-membered heterocycle, where the heterocyclyl radical may be mono- to trisubstituted by F, Cl, Br, I, OH, CF₃, CHF₂, CH₂F, NO₃, CN, OCF₃, OCHF₂, O-(C₁-C₆)-alkylene-(R9), (C₁-C₆)-alkylene-(R9), N-H₂, NH(C₁-C₆)-alkylene-(R9), N((C₁-C₆)-alkyl)₂, SO₂-(C₁-C₆)-alkylene-(R9), SO₂-C₂H₂F₃, SO₂-CH₃, SO₂-NH₂, SO₂-NH(C₁-C₆)-alkylene-(R9), SO₂-N((C₁-C₆)-alkyl)₂, COOH, COO-(C₁-C₆)-alkylene-(R9), CONH₂, CONH(C₁-C₆)-alkylene-(R9), CON((C₁-C₆)-alkyl)₂, SF₅, (C₆-C₁₀)-aryl, (C₃-C₈)-cycloalkyl, 4- to 12-membered heterocycle;
R6 is OH;
R7, R8 are each independently (C₁-C₃)-alkyl;
R9 is H, OH, OCH₃, OCF₃, CHF₂, CF₃;
and pharmaceutically acceptable salts thereof.

5. Compound of the formula I according to one or more of Claims 1 to 4 for use as medicaments.

6. Medicament comprising compounds of the formula I according to one or more of Claims 1 to 4.

7. Medicament comprising compounds of the formula I according to one or more of Claims 1 to 4 and at least one further active ingredient.

8. Medicament according to Claim 7, **characterized in that** it comprises, as a further active ingredient, one or more antidiabetics, active hypoglycemic ingredients, HMG-CoA reductase inhibitors, cholesterol absorption inhibitors, PPAR gamma agonists, PPAR alpha agonists, PPAR alpha/gamma agonists, PPAR delta agonists, fibrates, MTP inhibitors, bile acid absorption inhibitors, CETP inhibitors, polymeric bile acid adsorbers, LDL receptor inducers, ACAT inhibitors, antioxidants, lipoprotein lipase inhibitors, ATP citrate lyase inhibitors, squalene synthetase inhibitors, lipoprotein(a) antagonists, HM74A receptor agonists, lipase inhibitors, insulins, sulfonylureas, biguanides, meglitinides, thiazolidinediones, α-glucosidase inhibitors, active ingredients which act on the ATP-dependent potassium channel of the beta cells, glycogen phosphorylase inhibitors, glucagon receptor antagonists, activators of glucokinase, inhibitors of gluconeogenesis, inhibitors of fructose 1,6-biphosphatase, modulators of glucose transporter 4, inhibitors of glutamine:fructose-6-phosphate amidotransferase, inhibitors of dipeptidylpeptidase IV, inhibitors of 11-beta-hydroxysteroid dehydrogenase 1, inhibitors of protein tyrosine phosphatase 1B, modulators of the sodium-dependent glucose transporter 1 or 2, GPR40 modulators, inhibitors of hormone-sensitive lipase, inhibitors of acetyl-CoA carboxylase, inhibitors of phosphoenolpyruvate carboxykinase, inhibitors of glycogen synthase kinase-3 beta, inhibitors of protein kinase C beta, endothelin-A receptor antagonists, inhibitors of I kappaB kinase, modulators of the glucocorticoid receptor, CART agonists, NPY agonists, MC4 agonists, orexin agonists, H3 agonists, TNF agonists, CRF agonists, CRF BP antagonists, urocortin agonists, β3 agonists, CB1 receptor antagonists, MSH (melanocyte-stimulating hormone) agonists, CCK agonists, serotonin reuptake inhibitors, mixed serotoninergic and noradrenergic compounds, 5HT agonists, bombesin agonists, galanin antagonists, growth hormones, growth hormone-releasing compounds, TRH agonists, decoupling protein 2 or 3 modulators, leptin agonists, DA agonists, lipase/amylase inhibitors, PPAR modulators, RXR modulators, or TR β agonists or amphetamines.

9. Process for producing a medicament comprising the compounds of the formula I according to one or more of Claims 1 to 4, **characterized in that** the active ingredient is mixed with a pharmaceutically suitable carrier and this mixture is converted to a form suitable for administration.

10. Use of the compounds of the formula I according to one or more of Claims 1 to 4 for producing a medicament for treatment of hyperglycemia.

11. Use of the compounds of the formula I according to one or more of Claims 1 to 4 for producing a medicament for treatment of diabetes.

12. Use of the compounds of the formula I according to one or more of Claims 1 to 4 for producing a medicament for treatment of insulin resistance.

13. Set (kit) consisting of separate packages of
a) an effective amount of a compound of the formula I according to one or more of Claims 1 to 4 and
b) an effective amount of a further active medicament ingredient.

## Revendications

1. Composés de formule I 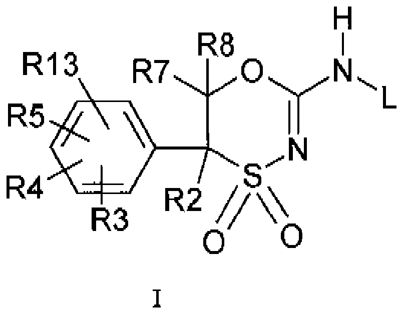 dans laquelle
L signifie R1, -CH(R10)(R11) ;
R10, R11 signifient, indépendamment l'un de l'autre, H, F, Cl, Br, I, OH, CF₃, CHF₂, CH₂F, NO₂, CN, OCF₃, OCHF₂, O-(C₁-C₆)-alkyle, (C₁-C₆)-alkyle, NH₂, NH(C₁-C₆)-alkyle, N((C₁-C₆)-alkyle)₂, COOH, COO-(C₁-C₆)-alkyle, CONH₂, CONH (C₁-C₆)-alkyle, CON((C₁-C₆)-alkyle) SF₅, (C₁-C₆) -alkylène- (R6), (C₃-C₈)-cycloalkylène-(R6), (C₁-C₆)-alkylène-(C₃-C₈)-cycloalkylène-(R6), (C₆-C₁₀)-aryle, (C₁-C₆)-alkylène-(C₆-C₁₀)-aryle ; le radical aryle pouvant être monosubstitué à trisubstitué par F, Cl, Br, I, OH, CF₃, CHF₂, CH₂F, NO₂, CN, OCF₃, OCHF₂, O-(C₁-C₆)-alkyle, (C₁-C₆-alkyle, NH₂, NH (C₁-C₆) -alkyle, N((C₁-C₆) -alkyle)₂, SO₂-CH₃, SO₂-NH₂, SO₂-NH (C₁-C₆-alkyle, SO₂-N((C₁-C₅)-alkyle)₂, COOH, COO-(C₁-C₆)-alkyle, CONH₂, CONH(C₁-C₆)-alkyle, CON((C₁-C₆)-alkyle)₂, SF₅ ;
R1 signifie 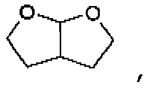 (C₆-C1₀) -aryle, (C₃-C₈) - cycloalkyle, le radical 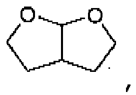 aryle ou cycloalkyle pouvant être monosubstitué à trisubstitué par F, Cl, Br, I, OH, CF₃, CHF₂, CH₂F, NO₂, CN, OCF₃, OCHF₂, O-(C₁-C₆)-alkyle, (C₁-C₆)-alkyle, NH₂, NH(C₁-C₆)-alkyle, N((C₁-C₆)-alkyle)₂, SO₂-CH₃, SO₂-NH₂, SO₂-NH(C₁-C₆)-alkyle, SO₂-N((C₁-C₆)-alkyle)₂, COOH, COO-(C₁-C₆) -alkyle, CONH₂, CONH(C₁-C₆)-alkyle, CON((C₁-C₆)-alkyle)₂, SF₅ ;
R2 signifie H, F, (C₁-C₃)-alkyle, le radical alkyle pouvant être monosubstitué à trisubstitué par fluor ;
R3, R4, R5, R13 signifient, indépendamment les uns des autres, H, F, Cl, Br, I, OH, CF₃, CHF₂, CH₂F, NO₂, CN, OCF₃, OCHF₂, -(C₁-C₆)-alkylène-(R9), O-(C₁-C₆)-alkylène-(R9), tert-butyle, iso-propylène-(R9), (C=O)-(C₁-C₆)-alkylène-(R9), (C₁-C₆)-alkylène-(R9), NH₂, NH (C₁-C₆)-alkylène- (R9), N((C₁-C₆)-alkylène-R9)₂, (C₁-C₆)-alkylène-NH₂, (C₁-C₆)-alkylène-NH(C₁-C₆) -alkylène-(R9), (C₁-C₆)-alkylène-N((C₁-C₆)-alkylène-R9)₂, -O-(C₁-C₆)-alkylène-NH₂, -O-(C₁-C₆)-alkylène-NH(C₁-C₆)-alkylène-(R9), -O-(C₁-C₆)-allkylène-N((C₁-C₆) -alkylène-R9)₂, -NH-(C₁-C₆)-alkylène-NH₂, -NH-(C₁-C₆)-alkylène-NH(C₁-C₆)-alkyléne-(R9), -NH-(C₁-C₆)-alkylène-N((C₁-C₆)-alkylène-R9)₂, SO₂-CH₃, SO₂-NH₂, SO₂-NH(C₁-C₆)-alkyle, SO₂-N((C₁-C₆)-alkyle)₂, COOH, COO- (C₁-C₆)-alkyle, CONH₂, CONH (C₁-C₆)-alkyle, CON((C₁-C₆)-alkyle)₂, SF₅ ; (C₆-C₁₀)-aryle, -(C₁-C₆)-alkylène- (C₆-C₁₀)-aryle, le radical aryle pouvant être monosubstitué à trisubstitués par F, Cl, Br, I, OH, CF₃, CHF₂, CH₂F, NO₂, CN, OCF₃, OCHF₂, O-(C₁-C₆)-alkyle, (C₁-C₆) -alkyle, NH₂, NH (C₁-C₆) -alkyle, N((C₁-C₆)-alkyle)₂, SO₂-CH₃, SO₂-NH₂, SO₂-NH (C₁-C₆)-alkyle, SO₂-N((C₁-C₆)-alkyle)₂, COOH, COO-(C₁-C₆)-alkyle, CONH₂, CONH(C₁-C₆)-alkyle, CON((C₁-C₆)-alkyle)₂, SF₅, (C₆-C₁₀)-aryle, (C₃-C₃)-cycloalkyle, hétérocycle de 4 à 12 chaînons ; le radical (C₆-C₁₀) -aryle, le radical (C₃-C₈)-cycloalkyle, le radical hétérocyclique de 4 à 12 chaînons pouvant être monosubstitués à trisubstitués par F, Cl, Br, I, OH, CF₃, CHF₂, CH₂F, NO₂, CN, OCF₃, OCHF₂, O-(C₁-C₆)-alkyle, (C₁-C₆)-alkyle, NH₂, NH(C₁-C₆)-alkyle, N((C₁-C₆)-alkyle)₂, SO₂-CH₃, SO₂-NH₂, SO₂-NH(C₁-C₆)-alkyle, SO₂-N((C₁-C₆)-alkyle)₂, COOH, COO-(C₁-C₆)-alkyle, CONH₂ CONH(C₁-C₆) -alkyle, CON((C₁-C₆)-alkyle)₂, SF₅ ; hétérocycle de 4 à 12 chaînons, -(C₁-C₆)-alkylène-hétérocycle de 4 à 12 chaînons, le radical hétérocyclyle pouvant être monosubstitué à trisubstitué par F, Cl, Br, I, OH, CF₃, CHF₂, CH₂F, NO₂, CN, OCF₃, OCHF₂, O-(C₁-C₆)-alkylène- (R9), (C₁-C₆)-alkylène-(R9), NH₂, NH(C₁-C₆)-alkylène-(R9), N((C₁-C₆)-alkyle)₂, SO₂-(C₁-C₆)-alkylène-(R9), SO₂-C₂H₂F₃, SO₂-CH₃, SO₂-NH₂, SO₂-NH(C₁-C₆)-alkylène-(R9), SO₂-N((C₁-C₆)-alkyle)₂, COOH, COO-(C₁-C₆)-alkylène-(R9), CONH₂, CONH(C₁-C₆)-alkylène-(R9), CON (C₁-C₆)-alkyle)₂, SF₅, (C₆-C₁₀)-aryle, (C₃-C₈)-cycloalkyle, hétérocycle de 4 à 12 chaînons ; le radical (C₆-C₁₀) -aryle, le radical (C₃-C₈) - cycloalkyle, le radical hétérocyclique de 4 à 12 chaînons pouvant être monosubstitués à trisubstitués par F, Cl, Br, I, OH, CF₃, CHF₂, CH₂F, NO₂, CN, OCF₃, OCHF₂, O-(C₁-C₆)-alkyle, (C₁-C₆)-alkyle, NH₂, NH (C₁-C₆)-alkyle, N((C₁-C₆)-alkyle)₂, SO₂-CH₃, SO)₂-NH₂, SO₂-NH (C₁-C₆)-alkyle, SO₂-N((C₁-C₆)-alkyle)₂, COOH, COO-(C₁-C₆)-alkyle, CONH₂, CONH(C₁-C₆)-alkyle, CON((C₁-C₆)-alkyle)₂, SF₅ ;
R6 signifie H, OH, CF₃, CHF₂, CH₂F, NO₂, CN, OCF₃, OCHF₂, O-(C₁-C₆)-alkyle, (C₁-C₆)-alkyle, NH₂, NH(C₁-C₆)-alkyle, N((C₁-C₆)-alkyle)₂, SO₂-CH₃, SO₂-NH₂, SO₂-NH(C₁-C₆)-alkyle, SO₂-N((C₁-C₆)-alkyle)₂, COOH, COO-(C₁-C₆)-alkyle, CONH₂, CONH(C₁-C₆)-alkyle, CON((C₁-C₆)-alkyle)₂, O-(CO)-NH₂, SF₅ ;
R7, R8 signifient, indépendamment l'un de l'autre, H, (C₁-C₃)-alkyle, le radical alkyle pouvant être monosubstitué à trisubstitué par fluor, ou R7 et R8 forment ensemble avec l'atome de carbone auquel ils sont liés un carbocycle ou un hétérocycle de 3-8 chaînons;
R9 signifie H, OH, OCH₃, OCF₃, CHF₂, CF₃ ;
ainsi que leurs sels pharmaceutiquement acceptables.

2. Composés de formule I, selon la revendication 1, **caractérisés en ce que**
L signifie R1, -CH(R10)(R11) ;
R10 signifie F, Cl, Br, I, OH, CF₃, CHF₂, CH₂F, NO₂, CN, OCF₃, OCHF₂, O-(C₁-C₆)-alkyle, (C₁-C₆)-alkyle, NH₂, NH(C₁-C₆)-alkyle, N((C₁-C₆)-alkyle)₂, COOH, COO-(C₁-C₆)-alkyle, CONH₂, CONH(C₁-C₆)-alkyle, CON((C₁-C₆)-alkyle)₂, SF₅, (C₁-C₆)-alkylène-(R6), (C₃-C₈)-cycloalkylène-(R6), (C₁-C₆)-alkylène-(C₃-C₈)-cycloalkylène-(R6), (C₆-C₁₀)-aryle, (C₁-C₆)-alkylène-(C₆-C₁₀)-aryle ; le radical aryle pouvant être monosubstitués à trisubstitué par F, Cl, Br, I, OH, CF₃, CHF₂, CH₂F, NO₂, CN, OCF₃, OCHF₂, O-(C₁-C₆)-alkyle, (C₁-C₆)-alkyle, NH₂, NH(C₁-C₆)-alkyle, N((C₁-C₆)-alkyle)₂, SO₂-CH₃, SO₂-NH₂, SO₂-NH(C₁-C₆)-alkyle, SO₂-N((C₁-C₆)-alkyle)₂, COOH, COO-(C₁-C₆)-alkyle, CONH₂, CONH(C₁-C₆)-alkyle, CON((C₁-C₆)-alkyle)₂, SF₅ ;
R11 signifie H, F, Cl, Br, I, OH, CF₃, CHF₂, CH₂F, NO₂, CN, OCF₃, OCHF₂, O-(C₁-C₆)-alkyle, (C₁-C₆)-alkyle, NH₂, NH(C₁-C₆)-alkyle, N((C₁-C₆)-alkyle)₂, COOH, COO-(C₁-C₆)-alkyle, CONH₂, CONH(C₁-C₆)-alkyle, CON((C₁-C₆)-alkyle)₂, SF₅, (C₁-C₆)-alkylène-(R6), (C₃-C₈)-cycloalkylène-(R6), (C₁-C₆)-alkylène-(C₃-C₈)-cycloalkylène-(R6), (C₆-C₁₀)-aryle, (C₁-C₆)-alkylène-(C₆-C₁₀)-aryle ; le radical aryle pouvant être monosubstitués à trisubstitué par F, Cl, Br, I, CH, CF₂, CHF₂, CH₂F, NO₂, CN, OCF₃, OCHF₂, O-(C₁-C₆)-alkyle, (C₁-C₆)-alkyle, NH₂, NH (C₁-C₆)-alkyle, N((C₁-C₆)-alkyle)₂, SO₂-CH₃, SO₂-NH₂, SO₂-NH(C₁-C₆)-alkyle, SO₂-N((C₁-C₆)-alkyle)₂, COOH, COO-(C₁-C₆)-alkyle, CONH₂, CONH(C₁-C₆)-alkyle, CON((C₁-C₆)-alkyle)₂, SF₅ ;
R1 signifie (C₃-C₈)-cycloalkyle, le radical cycloalkyle pouvant être monosubstitué à trisubstitué par F, Cl, Br, I, OH, CF₃, CHF₂, CH₂F, NO₂, CN, OCF₃, OCHF₂, O-(C₁-C₆)-alkyle, (C₁-C₆)alkyle, NH₂, NH(C₁-C₆)-alkyle, N((C₁-C₆)-alkyle)₂, SO₂-CH₃, SO₂-NF₂, SO₂-NH(C₁-C₆)-alkyle, SO₂-N((C₁-C₆)-alkyle)₂, COOH, COO-(C₁-C₆)-alkyle, CONH₂, CONH(C₁-C₆)-alkyle, CON((C₁-C₆)-alkyle)₂, SF₅ ;
R2 signifie H, F, (C₁-C₃)-alkyle, le radical alkyle pouvant être monosubstitué à trisubstitué par fluor ;
R3, R4, R5, R13 signifient, indépendamment les uns des autre, H, F, Cl, Br, I, OH, CF₃, CHF₂, CH₂F, NO₂, CN, OCF₃, OCHF₂, O-(C₁-C₆)-alkylène-(R9), - (C₁-C₆-alkylène-(R9), (C=O)-(C₁-C₆)-alkylène-(R9), (C₁-C₆)-alkylène-(R9), NH₂, NH(C₁-C₆)-alkylène-(R9), N((C₁-C₆)-alkyle)2, SO₂-CH₃, SO₂-NH₂, SO₂-NH(C₁-C₆)-alkyle, SO₂-N(C₁-C₆)-alkyle)₂, COOH, COO-(C₁-C₆)-alkyle, CONH₂, CONH (C₁-C₆)-alkyle, CON((C₁-C₆)-alkyle)₂, SF₅ ; (C₆-C₁₀) -aryle, - (C₁-C₆) -alkylène- (C₆-C₁₀) -aryle, le radical aryle pouvant être monosubstitué à trisubstitué par F, Cl, Br, I, OH, CF₃, CHF₂, CH₂F, NO₂, CN, OCF₃, OCHF₂, O-(C₁-C₆)-alkyle, (C₁-C₆)-alkyle, NH₂, NH(C₁-C₆)-alkyle, N((C₁-C₆)-alkyle)₂, SO₂-CH₃, SO₂-NH₂, SO₂-NH (C₁-C₆)-alkyle, SO₂-N((C₁-C₆)-alkyle)₂, COOH, COO-(C₁-C₆)-alkyle, CONH₂, CONH (C₁-C₆)-alkyle, CON(C₁-C₆)-alkyle)₂, SF₅ ; hétérocycle de 4 à 12 chaînons, -(C₁-C₆)-alkylène-hétérocycle de 4 à 12 chaînons, le radical hétérocyclyle pouvant être monosubstitué à trisubstitué par F, Cl, Br, I, OH, CF₃, CHF₂, CH₂F, NO₂, CN, OCF₃, OCHF₂, O-(C₁-C₆)-alkylène-(R9), (C₁-C₆)-alkylène-(R9), NH₂, NH(C₁-C₆)-alkylène-(R9), N((C₁-C₆)-alkyle)₂, SO₂-(C₁-C₆)-alkylène-(R9), SO₂-C₂H₂F₃, SO₂-CH₃, SO₂-NH₂, SO₂-NH(C₁-C₆)-alkylène-(R9), SO₂-N((C₁-C₆)-alkyle)₂, COOH, COO-(C₁-C₆)-alkyléne-(R9), CONH₂, CONH(C₁-C₆)-alkylène-(R9), CON((C₁-C₆)-alkyle)₂, SF₅, (C₅-C₁₀)-aryle, (C₃-C₈)-cycloalkyle, hétérocycle de 4 à 12 chaînons ;
R6 signifie H, OH, O-(CO)-NH₂, SO₂NH₂ ;
R7, R8 signifient, indépendamment l'un de l'autre, H, (C₁-C₃)-alkyle, le radical alkyle pouvant être monosubstitué à trisubstitué par fluor ;
R9 signifie H, OH, OCH₃, OCF₃, CHF₂, CF₃ ;
ainsi que leurs sels pharmaceutiquement acceptables.

3. Composés de formule I, selon la revendication 1
ou 2, **caractérisés en ce que**
L signifie R1, -CH(R10)(R11) ;
R10 signifie (C₆-C₁₀)-aryle, (C₁-C₆)-alkylène-((C₆-C₁₀)-aryle ; le radical aryle pouvant être monosubstitué à trisubstitué par F, Cl, Br, I, OH, CF₃, CHF₂, CH₂F, NO₂, CN, OCF₃, OCHF₂, O-(C₁-C₆)-alkyle, (C₁-C₆)-alkyle, NH₂, NH (C₁-C₆) -alkyle, N((C₁-C₆)-alkyle)₂, SO₂-CH₃, SO₂-NH₂, SO₂-NH (C₁-C₆)-alkyle, SO₂-N((C₁-C₆)-alkyle)₂, COOH, COO-(C₁-C₆)-alkyle, CONH₂, CONH(C₁-C₆)-alkyle, CON((C₁-C₆)-alkyle)₂, SF₅ ;
R11 signifie F, Cl, Br, I, OH, CF₃, CHF₂, CH₂F, NO₂, CN, OCF₃, OCHF₂, O- (C₁-C₆)-alkyle, (C₁-C₆)-alkyle, NH₂, NH(C₁-C₆-alkyle, N((C₁-C₆)-alkyle)₂, COOH, COO-(C₁-C₆)-alkyle, CONH₂, CONH(C₁-C₆) -alkyle, CON((C₁-C₆)-alkyle)₂, SF₅, (C₁-C₆) -alkylène-(R6) ;
R1 signifie (C₃-C₈)-cycloalkyle, le radical cycloalkyle pouvant être monosubstitué à trisubstitué par F, Cl, Br, I, OH, CF₃, CHF₂, CH₂F, NO₂, CN, OCF₃, OCHF₂, O-(C₁-C₆)-alkyle, (C₁-C₆)-alkyle, NH₂, NH(C₁-C₆)-alkyle, N ((C₁-C₆)-alkyle)₂, SO₂-CH₃, SO₂-NH₂, SO₂-NH(C₁-C₆)-alkyle, SO₂-N ((C₁-C₆)-alkyle)₂, COOH, COO-(C₁-C₆)-alkyle, CONH₂, CONH(C₁-C₆) -alkyle, CON((C₁-C₆)-alkyle)₂, SF₅ ;
R2 signifie H, (C₁-C₃)-alkyle ;
R3, R4, R5, R13 signifient, indépendamment les uns des autres, H, F, Cl, Br, I, OH, CF₃, CHF₂, CH₂F, NO₂, CN, OCF₃, OCHF₂, O-(C₁-C₆)-alkylène-(R9), - (C₁-C₆) -alkylène-(R9), (C=O)-(C₁-C₆)₋alkylène-(R9), (C₁-C₆)-alkylène-(R9), NH₂, NH (C₁-C₆)-alkylène-(R9), N((C₁-C₆)-alkyle)₂, SO₂-CH₃, SO₂-NH₂, SO₂-NH (C₁-C₆)-alkyle, SO₂-N( (C₁-C₆)-alkyle)₂, COOH, COO- (C₁-C₆) -alkyle, CONH₂, CONH (C₁-C₆)-alkyle, CON ((C₁-C₆) -alkyle)₂, SF₅ ; (C₆-C₁₀) -aryle, - (C₁-C₆) -alkylène- (C₆-C₁₀) -aryle, le radical aryle pouvant être monosubstitué à trisubstitué par F, Cl, Br, I, OH, CF₃, CHF₂, CH₂F, NO₂, CN, OCF₃, OCHF₂, O-C₁-C₆)-alkyle, (C₁-C₆)-alkyle, NH₂, NH (C₁-C₆) -alkyle, N ((C₁-C₆) -alkyle) ₂, SO₂-CH₃, SO₂-NH₂, SO₂-NH (C₁-C₆)-alkyle, SO₂-N ((C₁-C₆) -alkyle) ₂, COOH, COO-(C₁-C₆)-alkyle, CONH₂, CONH(C₁-C₆) -alkyle, CON((C₁-C₆)-alkyle)₂, SF₅ ; hétérocycle de 4 à 12 chaînons, -(C₁-C₆)-alkylène-hétérocycle de 4 à 12 chaînons, le radical hétérocyclyle pouvant être monosubstitué à trisubstitué par F, Cl, Br, I, OH, CF₃, CHF₂, CH₂F, NO₂, CN, OCF₃, OCHF₂, O-(C₁-C₆)-alkylène-(R9), (C₁-C₆)-alkylène-(R9), NH₂, NH (C₁-C₆) -alkylène- (R9), N ((C₁-C₆)-alkyle)₂, SO₂-(C₁-C₆)-alkylène-(R9), SO₂-C₂H₂F₃, SO₂-CH₃, SO₂-NH₂, SO₂-NH (C₁-C₆) -alkylène- (R9), SO₂-N ((C₁-C₆)-alkyle)₂, COOH, COO-(C₁-C₆)-alkylène-(R9), CONH₂, CONH (C₁-C₆)-alkyléne-(R9), CON ((C₁-C₆) -alkyle)₂, SF₅, (C₆-C₁₀)-aryle, (C₃-C₈)-cycloalkyle, hétérocycle de 4 à 12 chaînons ;
R6 signifie H, OH, O-(CO)-NH₂, SO₂NH₂ ;
R7, R8 signifient, indépendamment l'un de l'autre, (C₁-C₃)-alkyle ;
R9 signifie H, OH, OCH₃, OCF₃, CHF₂, CF₃ ;
ainsi que leurs sels pharmaceutiquement acceptables.

4. Composés de formule I selon l'une ou plusieurs des revendications 1 à 3, **caractérisés en ce que**
L signifie R1, -CH(R10)(R11) ;
R10 signifie phényle, le radical phényle pouvant être monosubstitué à trisubstitué par F, Cl, Br, - (C₁-C₆)-alkyle ;
R11 signifie (C₁-C₆)-alkyle, (C₁-C₆)-alkyléne-(R6) ;
R1 signifie (C₃-C₈)-cycloalkyle, le radical cycloalkyle pouvant être monosubstitué à trisubstitué par F, Cl, Br, OH ;
R2 signifie H, (C₁-C₃)-alkyle ;
R3, R4, R5, R13 signifient, indépendamment les uns des autres, H, F, Cl, Br, I, OH, CF₃, CHF₂, CH₂F, NO₂, CN, OCF₃, OCHF₂, O-(C₁-C₆)-alkylène-(R9), - (C₁-C₆)-alkylène-(R9), (C=O)-(C₁-C₆)-alkylène-(R9), (C₁-C₆)-alkylène-(R9), NH₂, NH(C₁-C₆)-alkylène-(R9), N ((C₁-C₆)-alkyle)₂, SO₂-CH₃, SO₂-NH₂, SO₂-NH(C₁-C₆)-alkyle, SO₂-N((C₁-C₆)-alkyle)₂, COOH, COO-(C₁-C₆)-alkyle, CONH₂, CONH (C₁-C₆) -alkyle, CON((C₁-C₆)-alkyle)₂, SF₅ ; (C₆-C₁₀)-aryle, -(C₁-C₆) -alkylène- (C₆-C₁₀) -aryle, le radical aryle pouvant être monosubstitué à trisubstitué par F, Cl, Br, I, OH, CF₃, CHF₂. CH₂F, NO₂, CN, OCF₃, CHF₂, O-(C₁-C₅)-alkyle, (C₁-C₆) -alkyle, NH₂, NH (C₁-C₆) -alkyle, N((C₁-C₆)-alkyle) ₂, SO₂-CH₃, SO₂-NH₂, SO₂-NH (C₁-C₆)-alkyle, SO₂-N((C₁-C₆)-alkyle)₂, COOH, COO-(C₁-C₆)-alkyle, CONH₂, CONH (C₁-C₆) -alkyle, CON((C₁-C₆)-alkyle)₂, SF₅ ; hétérocycle de 4 à 12 chaînons, -(C₁-C₆)-alkylène-hétérocycle de 4 à 12 chaînons, le radical hétérocyclyle pouvant être monosubstitué à trisubstitué par F, Cl, Br, I, OH, CF₃, CHF₂, CH₂F, NO₂, CN, OCF₃, OCHF₂, O-C₁-C₆)-alkylène-(R9), (C₁-C₆)-alkylène-(R9), NH₂, NH(C₁-C₆)-alkylène-(R9), N((C₁-C₆)-alkyle)₂, SO₂-(C₁-C₆)-alkylène-(R9), SO₂-C₂H₂F₃, SO₂-CH₃, SO₂-NH₂, SO₂-NH (C₁-C₆) -alkylène-(R9), SO₂-N((C₁-C₆)-alkyle)₂, COOH, COO-(C₁-C₆)-alkyléne-(R9), CONH₂, CONH(C₁-C₆)-alkylène-(R9), CON((C₁-C₆)-alkyle)₂, SF₅, (C₆-C₁₀)-aryle, (C₃-C₈)-cycloalkyle, hétérocycle de 4 à 12 chaînons ;
R6 signifie OH ;
R7, R8 signifient, indépendamment l'un de l'autre, (C₁-C₃)-alkyle ;
R9 signifie H, OH, OCH₃, OCF₃, CHF₂, CF₃ ;
ainsi que leurs sels pharmaceutiquement acceptables.

5. Composés de formule I selon l'une ou plusieurs des revendications 1 à 4 pour une utilisation comme médicament.

6. Médicament contenant des composés de formule I selon l'une ou plusieurs des revendications 1 à 4.

7. Médicament contenant des composés de formule I selon l'une ou plusieurs des revendications 1 à 4 et au moins une autre substance active.

8. Médicament selon la revendication 7, **caractérisé en ce qu'**il contient comme autre substance active un(e) ou plusieurs antidiabétiques, substances actives hypoglycémiantes, inhibiteurs de la HMGCoA-réductase, inhibiteurs de la résorption du cholestérol, agonistes de PPAR gamma, agonistes de PPAR alpha, agonistes de PPAR alpha/gamma, agonistes de PPAR delta, fibrates, inhibiteurs de MTP, inhibiteurs de la résorption de l'acide biliaire, inhibiteurs de CETP, adsorbants polymères de l'acide biliaire, inducteurs des récepteurs de LDL, inhibiteurs d'ACAT, antioxydants, inhibiteurs de la lipoprotéine-lipase, inhibiteurs de l'ATP-citrate-lyase, inhibiteurs de la squalène synthétase, antagonistes de la lipoprotéine (a), agonistes du récepteur de HM74A, inhibiteurs de lipase, insulines, sulfonylurées, biguanides, méglitinides, thiazolidinediones, inhibiteurs de l'α-glucosidase, substances actives agissant sur le canal calcique dépendant de l'ATP des cellules bêta, inhibiteurs de la glycogène phosphorylase, antagonistes du récepteur du glucagon, activateurs de la glucokinase, inhibiteurs de la gluconéogenèse, inhibiteurs de la fructose-1,6-biphosphatase, modulateurs du transporteur 4 du glucose, inhibiteurs de la glutamine-fructose-6-phosphate-amidotransférase, inhibiteurs de la dipeptidylpeptidase-IV, inhibiteurs de la 11-bêta-hydroxystéroïde-déshydrogénase-1, inhibiteurs de la protéine-tyrosine-phosphatase-1B, modulateurs du transporteur 1 ou 2 du glucose dépendant du sodium, modulateurs de GPR40, inhibiteurs de la lipase sensible aux hormones, inhibiteurs de l'acétyl-CoA carboxylase, inhibiteurs de la phosphoénolpyruvate-carboxykinase, inhibiteurs de la glycogène synthase kinase-3 bêta, inhibiteurs de la protéine kinase C bêta, antagonistes du récepteur de l'endothéline-A, inhibiteurs de la I kappa B kinase, modulateurs du récepteur des glucocorticoïdes, agonistes de CART, agonistes de NPY, agonistes de MC4, agonistes d'orexine, agonistes de H3, agonistes de TNF, agonistes de CRF, antagonistes de CRF BP, agonistes d'urocortine, agonistes ß3, antagonistes du récepteur de CB1, agonistes de MSH (hormone stimulant les mélanocytes), agonistes de CCK, inhibiteurs de la réabsorption de la sérotonine, composés sérotoninergiques et noradrénergiques mixtes, agonistes de 5HT, agonistes de bombésine, agonistes de galanine, hormones de croissance, composés libérant l'hormone de croissance, agonistes de TRH, modulateurs de la protéine découplante 2 ou 3, agonistes de leptine, agonistes de DA, inhibiteurs de lipase/amylase, modulateurs de PPAR, modulateurs de RXR ou agonistes de TR-ß ou amphétamines.

9. Procédé pour la préparation d'un médicament contenant les composés de formule I selon l'une ou plusieurs des revendications 1 à 4, **caractérisé en ce que** la substance active est mélangée avec un support pharmaceutiquement approprié et ce mélange est amené dans une forme appropriée pour l'administration.

10. Utilisation des composés de formule I selon l'une ou plusieurs des revendications 1 à 4 pour la préparation d'un médicament destiné au traitement de l'hyperglycémie.

11. Utilisation des composés de formule I, selon l'une ou plusieurs des revendications 1 à 4 pour la préparation d'un médicament destiné au traitement du diabète.

12. Utilisation des composés de formule I selon l'une ou plusieurs des revendications 1 à 4 pour la préparation d'un médicament destiné au traitement de la résistance à l'insuline.

13. Kit constitué par des emballages séparés de
a) une quantité efficace d'un composé de formule I selon l'une ou plusieurs des revendications 1 à 4
et
b) une quantité efficace d'une autre substance médicamenteuse.
